# EUROPEAN PATENT APPLICATION

(11) **EP 4 682 141 A1**
(43) Date of publication of application: **21.01.2026**
(21) Application number: 24770882.9
(22) Date of filing: 12.03.2024
(51) Int. Cl.: C07D 313/06, A61K 31/34, A61K 31/335, A61P 9/10, A61P 13/12, A61P 27/06, A61P 43/00, C07D 407/06, C07D 493/04

(54) **TRICYCLIC COMPOUND**

(30) Priority: 13.03.2023 JP 2023038845
(71) Applicant: ONO Pharmaceutical Co., Ltd., Osaka-shi, Osaka 541-0045 (JP)
(72) Inventor: OHTA, Nobukazu, Mishima-gun, Osaka 618-8585 (JP); OGAWA, Seiji, Mishima-gun, Osaka 618-8585 (JP); TOKURA, Hiroshi, Mishima-gun, Osaka 618-8585 (JP); YAMAZAKI, Yuji, Tokyo 100-0004 (JP); KITAYAMA, Tetsuya, Tokyo 100-0004 (JP); UBE, Yuko, Tokyo 100-0004 (JP); MAKITANI, Kouki, Tokyo 100-0004 (JP)
(74) Representative: Grünecker Patent- und Rechtsanwälte PartG mbB
(86) International application number: PCT/JP2024/009559
(87) International publication number: WO 2024/190777

(57) **Abstract**

To provide a compound having EP3 agonist activity. A compound represented by general formula (I) (in the formula, all symbols are as defined in the description) or a salt thereof has strong EP3 agonist activity and can therefore be used as an EP3 agonist.

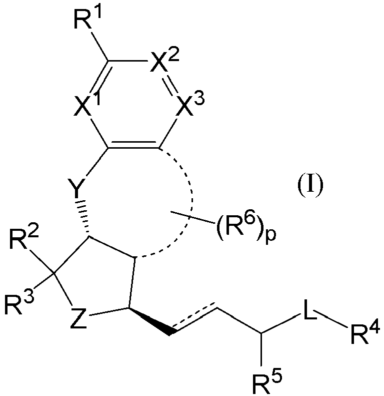

## Description

### TECHNICAL FIELD

The present disclosure relates to a compound represented by the following general formula (I): 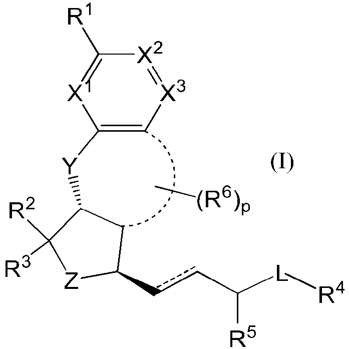 (in which all symbols represent the same meaning as described later) or a salt thereof (hereinafter, the compound of the present invention may be abbreviated as the present compound).

### BACKGROUND ART

Prostaglandin is known as a metabolite in the arachidonic acid cascade, and its effect is known to have a cytoprotective effect, uterine contraction, pain generating effect, promotion of peristaltic movement of the digestive tract, awakening effect, gastric acid secretion suppressing effect, blood pressure lowering effect, diuretic effect, and the like. It is also known that there are PGE (prostaglandin E) receptors having different roles for each subtype. There are roughly four subtypes known at the present time, which are called EP1, EP2, EP3, and EP4 (Non Patent Literature 1).

Non Patent Literature 2 and Non Patent Literature 3 related to a tricyclic compound are known.

### CITATION LIST

### NON-PATENT LITERATURE

Non-Patent Literature 1: Journal of Biological Chemistry, vol. 282, no. 16, p. 11613-11617
Non-Patent Literature 2: Phytochemistry, vol. 16, no. 4, p. 494-495
Non-Patent Literature 3: Journal of Organic Chemistry, vol. 64, no. 17, p. 6380-6386

### SUMMARY OF INVENTION

### TECHNICAL PROBLEM

An object of the present invention is to provide a compound having EP3 agonist activity.

### SOLUTION TO PROBLEM

As a result of intensive studies to solve the above problems, the present inventors have found that a compound shown below has a potent EP3 agonist activity.

The present invention has, for example, the following aspects.
[1] A compound represented by general formula (I) or a salt thereof: 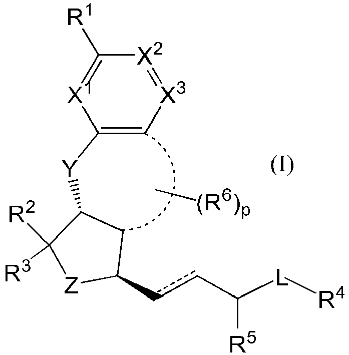 [in which
   R¹ represents COOH, COOR¹¹, CONHSO₂R¹² or tetrazolyl,
   R¹¹ represents C1-6 alkyl,
   R¹² represents C1-6 alkyl or phenyl, which phenyl is optionally substituted with 1 to 5 (1) halogen atoms or (2) C1-4 alkyl groups,
   X¹, X² and X³ each independently represent N, CH or CR²¹,
   a plurality of R²¹s each independently represent (1) halogen, (2) C1-4 alkyl, (3) C1-4 haloalkyl, (4) -(CH₂)ₜ-(C3-4 saturated carbocycle), or (5) vinyl optionally substituted with 1 to 3 halogen atoms,
   t represents an integer of 0 or 1, 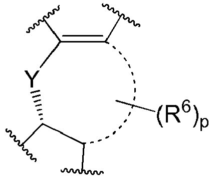
   represents 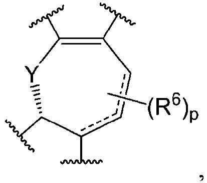 or 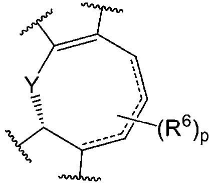
   R⁶ represents (1) halogen, (2) C1-4 alkyl, (3) C1-4 haloalkyl, or (4) -(CH₂)_{q}-(C3-4 saturated carbocycle),
   q represents an integer of 0 or 1,
   p represents an integer of 0 to 3,
   a plurality of R⁶s may form a C3-4 saturated carbocycle optionally substituted with 1 to 6 halogen atoms together with a carbon atom to which each R⁶ is bonded,
   Y represents an oxygen atom or a sulfur atom,
   R² and R³ each independently represent (1) a hydrogen atom, (2) halogen, (3) C1-4 alkyl, or (4) C1-4 haloalkyl,
   R² and R³ may form a C3-4 saturated carbocycle optionally substituted with 1 to 6 halogen atoms together with a carbon atom to which R² and R³ are bonded,
   Z represents CHR¹⁰ or an oxygen atom,
   R¹⁰ represents a hydrogen atom, halogen, a hydroxyl group, or a methoxy group,
   L represents -CH₂-, -CHR^{L1}-, -CR^{L2}R^{L3}- or -C(=CH₂)-,
   R^{L1} represents (1) halogen, (2) C1-4 alkyl, (3) C1-4 haloalkyl, (4) C2-4 alkenyl, (5) C2-4 haloalkenyl, (6) C2-4 alkynyl or (7) C2-4 haloalkynyl,
   R^{L2} and R^{L3} each independently represent (1) halogen, (2) C1-4 alkyl, (3) C1-4 haloalkyl, (4) C2-4 alkenyl, (5) C2-4 haloalkenyl, (6) C2-4 alkynyl, (7) C2-4 haloalkynyl or (8) a hydroxyl group, or
   R^{L2} and R^{L3} may form (1) a C3-6 saturated carbocycle optionally substituted with 1 to 10 halogen atoms or (2) a 3-6 membered saturated heterocycle optionally substituted with 1 to 8 halogen atoms, together with a carbon atom to which R^{L2} and R^{L3} are bonded,
   R⁴ represents (1) C3-7 alkyl optionally substituted with 1 to 5 R⁴¹s, (2) C3-7 alkenyl optionally substituted with 1 to 5 R⁴²s, (3) C3-7 alkynyl optionally substituted with 1 to 5 R⁴³s, (4) C2-6 alkoxy optionally substituted with 1 to 5 R⁴⁴s, (5) Cyc¹ or (6) -O-Cyc¹,
   when R⁴ represents C3-7 alkyl optionally substituted with 1 to 5 R⁴¹s, one carbon atom in the C3-7 alkyl may be substituted with an oxygen atom,
   R⁴¹, R⁴², R⁴³ and R⁴⁴ each independently represent (1) halogen, (2) a C3-6 carbocycle or (3) a 3-6 membered heterocycle, in which the C3-6 carbocycle and the 3-6 membered heterocycle is optionally substituted with 1 to 10 (1) halogen atoms or (2) C1-2 alkyl groups,
   Cyc¹ represents (1) a C5-10 carbocycle optionally substituted with 1 to 5 R⁵¹s or (2) a 5-10 membered heterocycle optionally substituted with 1 to 5 R⁵²s,
   R⁵¹ and R⁵² each independently represent (1) halogen, (2) C1-4 alkyl, (3) C1-4 haloalkyl, (4) C2-4 alkenyl, (5) C2-4 haloalkenyl, (6) C2-4 alkynyl, (7) C2-4 haloalkynyl, (8) C1-4 alkoxy, (9) C1-4 haloalkoxy, (10) a hydroxyl group, (11) a C3-6 saturated carbocycle or (12) a 3-6 membered saturated heterocycle,
   R^{L1} and R⁵¹ may form (1) a C3-6 saturated carbocycle or (2) a 3-6 membered saturated heterocycle, together with a carbon atom to which R^{L1} and R⁵¹ are bonded,
   R⁵ represents a hydrogen atom or a hydroxyl group,
   in which a plurality of R⁶s, R⁴¹s, R⁴²s, R⁴³s, R⁴⁴s, R⁵¹s and R⁵²s may be the same or different,

   -̅ -̅ -̅ -̅ -̅ -̅ represents a single bond or a double bond, provided that adjacent
   -̅ -̅ -̅ -̅ -̅ -̅ are not simultaneously a double bond, 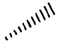
   represents a bond beyond the sheet surface (that is, an α configuration), 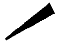
   represents a bond in front of the sheet surface (that is, a β configuration), in which 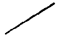
   bonded to an asymmetric atom represents the α configuration, the β configuration, or a mixture of them in any ratio, unless otherwise indicated].
[2] The compound or the salt thereof according to [1], in which X¹, X² and X³ are each independently CH or CR²¹.
[3] The compound or the salt thereof according to [1] or [2], in which Y is an oxygen atom.
[4] The compound or the salt thereof according to any one of [1] to [3], in which R⁴ is C3-7 alkyl optionally substituted with 1 to 3 R⁴¹s, C3-7 alkenyl optionally substituted with 1 to 3 R⁴²s, or Cyc¹.
[5] The compound or the salt thereof according to any one of [1] to [4], in which Z is CHR¹⁰, and R¹⁰ is a hydroxyl group.
[6] The compound or the salt thereof according to any one of [1] to [5], in which R¹ is COOH.
[7] The compound or the salt thereof according to [1], in which general formula (I) is represented by general formula (I-3) 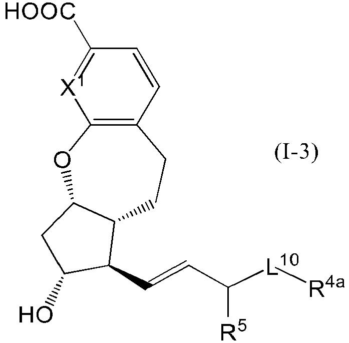 [in which L¹⁰ represents -CHR^{L1}- or -CR^{L2}R^{L3}-, R^{4a} represents C3-7 alkyl optionally substituted with 1 to 3 R⁴¹s, C3-7 alkenyl optionally substituted with 1 to 3 R⁴²s, or Cyc¹, and the other symbols represent the same meaning as described above].
[8] The compound or a salt thereof according to [1], in which the compound represented by general formula (I) or the salt thereof is
   (1) (1R,2R,3aS,10aR)-1-[(1E,3R,4S)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]loxepine-6-carboxylic acid,
   (2) (1R,2R,3 aS,10aR)-5-fluoro-1-[(1E)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid,
   (3) (1R,2R,3aS,10aR)-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
   (4) (1R,2R,3aS,10aR)-1-{(1E,3ξ)-3-[1-(2,5-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
   (5) (1R,2R,3aS,10aR)-5-chloro-1-[(1E,3ξ)-4-(2,6-difluorophenyl)-4,4-difluoro-3-hydroxy-1-buten-1-yl]-2-hydroxy-2,3,3a,9,10,10a-hexahydro-1H-benzo[b]cyclopenta[f]oxepine-6-carboxylic acid,
   (6) (1R,2R,3aS,10aR)-1-{(1E,3ξ)-3-[1-(4-fluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid,
   (7) (1R,2R,3aS,10aR)-1-[(1E,3ξ,4ξ)-4-ethyl-3-hydroxy-7-methyl-1-octen-1-yl]-2-hydroxy-5-methyl-2,3,3a,9,10,10a-hexahydro-1H-benzo[b]cyclopenta[f]oxepine-6-carboxylic acid,
   (8) (1R,2R,3aS,10aR)-2-hydroxy-5-methyl-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid,
   (9) (1R,2R,3aS,10aR)-2-hydroxy-5-methyl-1-[(1E,4S)-8,8,8-trifluoro-4-hydroxy-4-methyl-1-octen-1-yl]-2,3,3a,9,10,10a-hexahydro-1H-benzo[b]cyclopenta[f]oxepine-6-carboxylic acid,
   (10) (1R,2R,3aS,10aR)-2-hydroxy-1-[(1E,4ξ)-4-hydroxy-4,7-dimethyl-1-octen-1-yl]-5-methyl-2,3,3a,9,10,10a-hexahydro-1H-benzo[b]cyclopenta[f]oxepine-6-carboxylic acid,
   (11) (1R,3aS,10aR)-5-fluoro-1-[(1E,3ξ)-3-hydroxy-4-methyl-4-phenyl-1-penten-1-yl]-2,3,3a,9,10,10a-hexahydro-1H-benzo[b]cyclopenta[f]oxepine-6-carboxylic acid,
   (12) (1R,3aS,10aR)-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
   (13) (1R,3aS,10aR)-5-chloro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
   (14) (1R,3aS,10aR)-5-chloro-1-[(1E,3ξ)-3-hydroxy-6-methoxy-4-methylene-1-hexen-1-yl]-2,3,3a,9,10,10a-hexahydro-1H-benzo[b]cyclopenta[f]oxepine-6-carboxylic acid,
   (15) (1R,3aS,10aR)-5-chloro-1-[(1E,3ξ,4S)-4-ethyl-3-hydroxy-1-octen-1-yl]-2,3,3a,9,10,10a-hexahydro-1H-benzo[b]cyclopenta[f]oxepine-6-carboxylic acid,
   (16) (1R,3aS,10aR)-5-chloro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid,
   (17) (1R,2R,3aS,10aR)-2,5-difluoro-1-[(1E,3ξ)-3-hydroxy-4-methyl-4-phenyl-1-penten-yl]-2,3,3a,9,10,10a-hexahydro-1H-benzo[b]cyclopenta[f]oxepine-6-carboxylic acid,
   (18) (1R,2R,3aS,10aR)-2,5-difluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]loxepine-6-carboxylic acid,
   (19) (1R,2R,3aS,10aR)-2-chloro-5-fluoro-1-{(1E,3ξ)-3-[3-(2-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid,
   (20) (1R,2R,3aS,10aR)-2-chloro-5-fluoro-1-{(1E,3ξ)-3-hydroxy-3-[(2ξ)-2-phenyl-2-oxetanyl]-1-propen-1-yl}-2,3,3a,9,10,10a-hexahydro-1H-benzo[b]cyclopenta[f]oxepine-6-carboxylic acid,
   (21) (1R,3aR,10aR)-1-[(1E,3ξ,4S)-3-hydroxy-4-methyl-1-octen-1-yl]-5-methyl-1,3,3a,9,10,10a-hexahydrofuro[3,4-b][1]benzoxepine-6-carboxylic acid,
   (22) (1R,3aR,10aR)-1-[(1E,3ξ)-3-hydroxy-3-(1-phenoxycyclobutyl)-1-propen-1-yl]-5-methyl-1,3,3a,9,10,10a-hexahydrofuro[3,4-b][1]benzoxepine-6-carboxylic acid,
   (23) (1R,2R,3aS,10aR)-5-fluoro-2-hydroxy-1-{(1E,3ξ)-3-hydroxy-3-[1-(2-methylphenyl)cyclopropyl]-1-propen-1-yl}-2,3,3a,9,10,10a-hexahydro-1H-benzo[b]cyclopenta[f]oxepine-6-carboxylic acid, or
   (24) (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]loxepine-6-carboxylic acid,
      or a salt thereof.
[9] A pharmaceutical composition containing the compound or the salt thereof according to any one of [1] to [8].
[10] An EP3 agonist containing the compound or the salt thereof according to any one of [1] to [8].
[11] The pharmaceutical composition according to [9] or the EP3 agonist according to [10], for use in prevention and/or treatment of a disease of myocardial infarction, dysuria, or glaucoma.
[12] An agent for preventing and/or treating a renal disease, which contains the compound or the salt thereof according to any one of [1] to [8].
[13] The agent according to [12], in which the renal disease is a chronic renal disease.
[14] The agent according to [13], in which the chronic renal disease is diabetic renal disease, nephrosclerosis, IgA nephropathy, GBM nephritis, ANCA-associated nephritis, mesangial proliferative nephritis, lupus nephritis, minimal change nephrotic syndrome, focal segmental glomerulosclerosis, membranous nephropathy, Alport syndrome, or polycystic kidney disease.
[15] The agent according to [12], in which the renal disease is an acute renal damage.
[16] The agent according to [15], in which the acute renal damage is acute renal damage associated with organ surgery, acute renal damage associated with sepsis, acute renal damage associated with kidney transplantation, acute renal damage associated with rhabdomyolysis, contrast nephropathy, or drug-induced renal damage.
[17] A method for preventing and/or treating kidney disease, characterized by administering an effective amount of the compound or the salt thereof according to any one of [1] to [8] to a mammal (preferably a patient in need thereof).
[18] The compound or the salt thereof according to any one of [1] to [8], for use in prevention and/or treatment of a renal disease.
[19] Use of the compound or the salt thereof according to any one of [1] to [8] for producing a preventive and/or therapeutic agent for a renal disease.
[20] The pharmaceutical composition according to [9] or the EP3 agonist according to [10], for use in prevention and/or treatment of a renal disease.
[21] The pharmaceutical composition according to [9] or the EP3 agonist according to [10] for producing an agent for preventing and/or treating a renal disease.
[22] A method for producing the compound or the salt thereof according to any one of [1] to [8], including the step of:
   subjecting a compound represented by general formula (I') 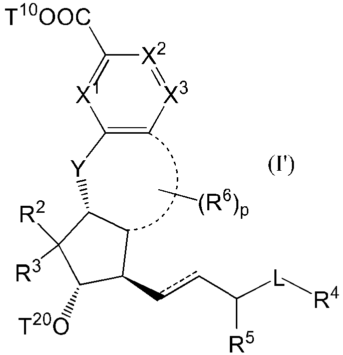 [in which:
   T¹⁰ is a hydrogen atom or R¹¹, T²⁰ is a hydrogen atom or a protecting group for a hydroxyl group, T¹⁰ and T²⁰ are not simultaneously a hydrogen atom, and the other symbols represent the same meaning as defined in general formula (I)] to a hydrolysis reaction and/or a deprotection reaction.
[23] The producing method according to [22], for producing a compound of general formula (I) (R¹ = COOH, Z = CHR¹⁰, R¹⁰ = a hydroxyl group) or a salt thereof, including the step of (A) subjecting the compound represented by general formula (I') (T¹⁰ = R¹¹, T²⁰ = a hydrogen) to a hydrolysis reaction.

### ADVANTAGEOUS EFFECTS OF INVENTION

Since the present compound has a potent EP3 agonist activity, it is useful as a preventive and/or therapeutic agent for renal disease and other diseases that occur in relation to the EP3 receptor.

### DESCRIPTION OF EMBODIMENTS

Hereinafter, a detailed description is made of the present invention.

In the present invention, C1-6 alkyl includes linear or branched C1-6 alkyl, including methyl, ethyl, propyl, isopropyl, butyl, sec-butyl, tert-butyl, pentyl, isopentyl, tert-pentyl, neopentyl, hexyl, isohexyl, 3-methylpentyl, and isomers thereof.

In the present invention, halogen includes fluorine, chlorine, bromine, and iodine.

In the present invention, C1-4 alkyl includes linear or branched C1-4 alkyl, including methyl, ethyl, propyl, isopropyl, butyl, sec-butyl, tert-butyl, and isomers thereof.

In the present invention, C1-2 alkyl means methyl or ethyl.

In the present invention, examples of a C3-4 saturated carbocycle include cyclopropane and cyclobutane.

In the present invention, C1-4 haloalkyl refers to a group in which any hydrogen atom of a linear or branched C1-4 alkyl group is substituted with one or more halogen atoms, including fluoromethyl, chloromethyl, bromomethyl, iodomethyl, difluoromethyl, trifluoromethyl, 1-fluoroethyl, 2-fluoroethyl, 2-chloroethyl, pentafluoroethyl, 1-fluoropropyl, 2-chloropropyl, 3-fluoropropyl, 3-chloropropyl, 4,4,4-trifluorobutyl, 4-bromobutyl, and isomers thereof.

In the present invention, C2-4 alkenyl includes linear or branched C2-4 alkenyl, including ethenyl, propenyl, butenyl, and isomers thereof.

In the present invention, C2-4 haloalkenyl refers to a group in which any hydrogen atom of a linear or branched C2-4 alkenyl group is substituted with one or more halogen atoms, including 1-fluoroethenyl, 2-fluoroethenyl, 2-chloroethenyl, trifluoroethenyl, 1-fluoropropenyl, 2-chloropropenyl, 3-fluoropropenyl, 3-chloropropenyl, 4,4,4-trifluorobutenyl, 4-bromobutenyl, and isomers thereof.

In the present invention, C2-4 alkynyl includes linear or branched C2-4 alkynyl groups, including ethynyl, propynyl, butynyl, and isomers thereof.

In the present invention, C2-4 haloalkynyl refers to a group in which any hydrogen atom of a linear or branched C2-4 alkynyl group is substituted with one or more halogen atoms, including 1-fluoroethynyl, 2-fluoroethynyl, 2-chloroethynyl, 1-fluoropropynyl, 2-chloropropynyl, 3-fluoropropynyl, 3-chloropropynyl, 4,4,4-trifluorobutynyl, 4-bromobutynyl, and isomers thereof.

In the present invention, examples of a C3-6 saturated carbocycle include cyclopropane, cyclobutane, cyclopentane, and cyclohexane.

In the present invention, a 3-6 membered saturated heterocycle refers to 3-6 membered saturated heterocycles containing a heteroatom selected from an oxygen atom, a nitrogen atom and a sulfur atom, including aziridine, azetidine, pyrrolidine, imidazolidine, triazolidine, tetrazolidine, pyrazolidine, piperidine, piperazine, perhydropyrimidine, perhydropyridazine, oxirane, oxetane, tetrahydrofuran, tetrahydropyran, thiirane, thietane, tetrahydrothiophene, tetrahydrothiopyran, tetrahydrooxazole (oxazolidine), tetrahydroisoxazole (isoxazolidine), tetrahydrothiazole (thiazolidine), tetrahydroisothiazole (isothiazolidine), tetrahydrofurazan, tetrahydrooxadiazole (oxadiazolidine), tetrahydrooxazine, tetrahydrooxadiazine, tetrahydrothiadiazole (thiadiazolidine), tetrahydrothiazine, tetrahydrothiadiazine, morpholine, thiomorpholine, oxathiane, dioxolane, dioxane, dithiolane, and dithiane.

In the present invention, C3-7 alkyl includes linear or branched C3-7 alkyl, including propyl, butyl, pentyl, hexyl, heptyl, isopropyl, isobutyl, sec-butyl, tert-butyl, 1-methylbutyl, 1-ethylpropyl, 1,1-dimethylpropyl, 1,2-dimethylpropyl, 2-methylbutyl, 3-methylbutyl, 2,2-dimethylpropyl, 1-methylpentyl, 1-ethylbutyl, 2-ethylbutyl, 1-ethyl-1-methylpropyl, 1-ethyl-2-methylpropyl, 1,1-dimethylbutyl, 1,2-dimethylbutyl, 1,3-dimethylbutyl, 2-methylpentyl, 3-methylpentyl, 4-methylpentyl, 2,3-dimethylbutyl, 1-methylhexyl, 1-ethylpentyl, 2-ethylpentyl, 1-propylbutyl, 2-methyl-3-hexyl, 1,2-dimethylpentyl, 1,3-dimethylpentyl, 1,4-dimethylpentyl, 1-ethyl-1-methylbutyl, 1-methyl-2-ethylbutyl, 1-ethyl-2-methylbutyl, 1-ethyl-3-methylbutyl, 1,1-dimethylpentyl, 1,1,3-trimethylbutyl, 1,1-diethylpropyl, 2-methylhexyl, 3-methylhexyl, 4-methylhexyl, 5-methylhexyl, 3-ethylpentyl, and isomers thereof.

In the present invention, C3-5 alkyl includes linear or branched C3-5 alkyl, including propyl, butyl, pentyl, isopropyl, isobutyl, sec-butyl, tert-butyl, 1-methylbutyl, 1-ethylpropyl, 1,1-dimethylpropyl, 1,2-dimethylpropyl, 2-methylbutyl, 3-methylbutyl, 2,2-dimethylpropyl, and isomers thereof.

In the present invention, C3-7 alkenyl includes linear or branched C3-7 alkenyl, including propenyl, butenyl, pentenyl, hexenyl, heptenyl, isopropenyl, isobutenyl, 1-methylbutenyl, 1-ethylpropenyl, 1,1-dimethylpropenyl, 1,2-dimethylpropenyl, 2-methylbutenyl, 3-methylbutenyl, 1-methylpentenyl, 1-ethylbutenyl, 2-ethylbutenyl, 1-ethyl-1-methylpropenyl, 1-ethyl-2-methylpropenyl, 1,1-dimethylbutenyl, 1,2-dimethylbutenyl, 1,3-dimethylbutenyl, 2-methylpentenyl, 3-methylpentenyl, 4-methylpentenyl, 2,3-dimethylbutenyl, 1-methylhexenyl, 1-ethylpentenyl, 2-ethylpentenyl, 1-propylbutenyl, 2-methyl-3-hexenyl, 1,2-dimethylpentenyl, 1,3-dimethylpentenyl, 1,4-dimethylpentenyl, 1-ethyl-1-methylbutenyl, 1-methyl-2-ethylbutenyl, 1-ethyl-2-methylbutenyl, 1-ethyl-3-methylbutenyl, 1,1-dimethylpentenyl, 1,1,3-trimethylbutenyl, 1,1-diethylpropenyl, 2-methylhexenyl, 3-methylhexenyl, 4-methylhexenyl, 5-methylhexenyl, 3-ethylpentenyl, and isomers thereof.

In the present invention, C3-5 alkenyl includes linear or branched C3-5 alkenyl, including propenyl, butenyl, pentenyl, isopropenyl, isobutenyl, 1-methylbutenyl, 1-ethylpropenyl, 1,1-dimethylpropenyl, 1,2-dimethylpropenyl, 2-methylbutenyl, 3-methylbutenyl, and isomers thereof.

In the present invention, C3-7 alkynyl includes linear or branched C3-7 alkynyl, including propynyl, butynyl, pentynyl, hexynyl, heptynyl, 1-methylbutynyl, 1-ethylpropynyl, 1,1-dimethylpropynyl, 2-methylbutynyl, 3-methylbutynyl, 1-methylpentynyl, 1-ethylbutynyl, 2-ethylbutynyl, 1-ethyl-1-methylpropynyl, 1,1-dimethylbutynyl, 1,2-dimethylbutynyl, 2-methylpentynyl, 3-methylpentynyl, 4-methylpentynyl, 1-methylhexynyl, 1-ethylpentynyl, 2-ethylpentynyl, 1-propylbutynyl, 2-methyl-3-hexynyl, 1,2-dimethylpentynyl, 1,3-dimethylpentynyl, 1,4-dimethylpentynyl, 1-ethyl-1-methylbutynyl, 1-methyl-2-ethylbutynyl, 1-ethyl-2-methylbutynyl, 1,1-dimethylpentynyl, 1,1-diethylpropynyl, 2-methylhexynyl, 3-methylhexynyl, 4-methylhexynyl, 5-methylhexynyl, 3-ethylpentynyl, and isomers thereof.

In the present invention, C2-6 alkoxy includes linear or branched C2-6 alkoxy groups, including ethoxy, propoxy, isopropoxy, butoxy, isobutyloxy, tert-butoxy, n-pentyloxy, isopentyloxy, neopentyloxy, n-hexyloxy, and isohexyloxy.

In the present invention, C1-4 alkoxy includes linear or branched C1-4 alkoxy groups, including methoxy, ethoxy, propoxy, isopropoxy, butoxy, isobutyloxy, tert-butoxy, and isomers thereof.

In the present invention, C1-4 haloalkoxy refers to a group in which any hydrogen atom of a linear or branched C1-4 alkoxy group is substituted with one or more halogen atoms, including trifluoromethoxy, trichloromethoxy, chloromethoxy, bromomethoxy, fluoromethoxy, iodomethoxy, difluoromethoxy, dibromomethoxy, 2-chloroethoxy, 2,2,2-trifluoroethoxy, 2,2,2-trichloroethoxy, 3-bromopropoxy, 3-chloropropoxy, 2,3-dichloropropoxy, 1-fluorobutoxy, 4-fluorobutoxy, and 1-chlorobutoxy.

In the present invention, examples of C3-6 carbocycle include cyclopropane, cyclobutane, cyclopentane, cyclohexane, cyclopentene, cyclohexene, cyclopentadiene, cyclohexadiene, and benzene.

In the present invention, a 3-6 membered heterocycle refers to a 3-6 membered heterocycle containing a hetero atom selected from an oxygen atom, a nitrogen atom and a sulfur atom, including pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, oxirane, oxetane, dihydrofuran, tetrahydrofuran, dihydropyran, tetrahydropyran, thiirane, thietane, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, Oxathiane, dioxolane, dioxane, dithiolane, and dithiane.

In the present invention, examples of a C5-10 carbocycle include cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, spiro[4.4]nonane, and spiro[4.5]decane.

In the present invention, a 5-10 membered heterocycle refers to a 5-10 membered heterocycle containing a heteroatom selected from an oxygen atom, a nitrogen atom and a sulfur atom, including pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxolane, dioxane, dithiolane, dithiane, dioxaindane, benzodioxane, chroman, benzodithiolane, benzodithiane, azaspiro[4.4]nonane, oxazaspiro[4.4]nonane, dioxaspiro[4.4]nonane, azaspiro[4.5]decane, thiaspiro[4.5]decane, dithiaspiro[4.5]decane, dioxaspiro[4.5]decane, and oxazaspiro[4.5]decane.

In the present invention, examples of a C5-6 carbocycle include cyclopentane, cyclohexane, cyclopentene, cyclohexene, cyclopentadiene, cyclohexadiene, and benzene.

In the present invention, a 5-6 membered heterocycle includes isoxazole, isothiazole, imidazole, imidazolidine, imidazoline, oxadiazine, oxadiazole, oxazine, oxazole, oxathiane, dioxane, dioxolane, dithiane, dithiolane, dihydroisoxazole, dihydroisothiazole, dihydrooxadiazine, dihydrooxadiazole, dihydrooxazine, dihydrooxazole, dihydrothiadiazine, dihydrothiadiazole, dihydrothiazine, dihydrothiazole, dihydrothiopyran, dihydrothiophene, dihydropyrazine, dihydropyran, dihydropyridine, dihydropyridazine, dihydropyrimidine, dihydrofurazan, dihydrofuran, thiadiazine, thiadiazole, thiazine, thiazole, thiopyran, thiophene, thiomorpholine, tetrazole, tetrazolidine, tetrazoline, tetrahydroisoxazole (isoxazolidine), tetrahydroisothiazole (isothiazolidine), tetrahydrooxadiazine, tetrahydrooxadiazole (oxadiazolidine), tetrahydrooxazine, tetrahydrooxazole (oxazolidine), tetrahydrothiadiazine, tetrahydrothiadiazole (thiadiazolidine), tetrahydrothiazine, tetrahydrothiazole (thiazolidine), tetrahydrothiopyran, tetrahydrothiophene, tetrahydropyrazine, tetrahydropyran, tetrahydropyridine, tetrahydropyridazine, tetrahydropyrimidine, tetrahydrofurazan, tetrahydrofuran, triazole, triazolidine, triazoline, perhydropyridazine, perhydropyrimidine, piperazine, piperidine, pyrazine, pyrazole, pyrazolidine, pyrazoline, pyran, pyridine, pyridazine, pyrimidine, pyrrole, pyrrolidine, pyrroline, furazan, furan, and morpholine.

In the present invention, the following partial structure 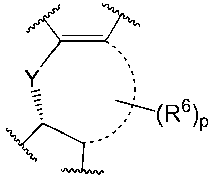 in each general formula represents 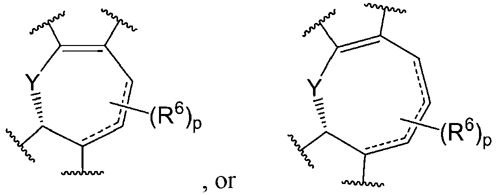 and
the partial structure includes, for example,

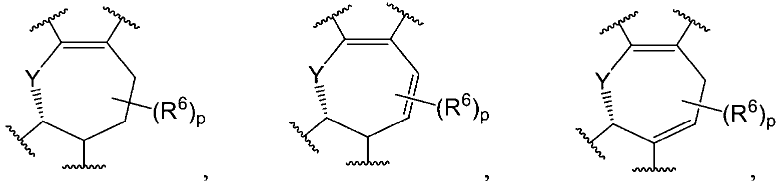

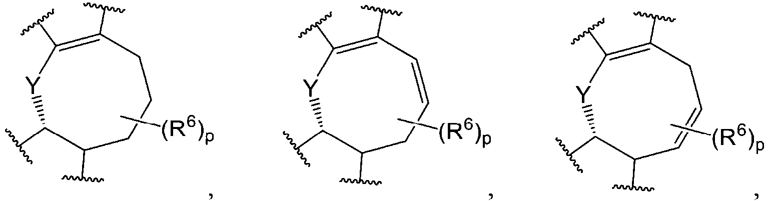

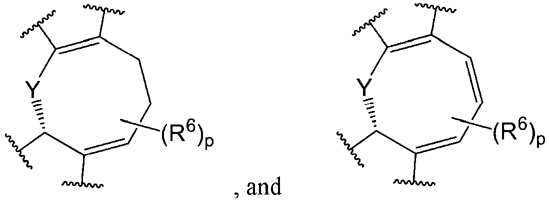

In the present invention, R¹ is preferably COOH.

In the present invention, R¹¹ is preferably C1-2 alkyl.

In the present invention, R¹² is preferably C1-6 alkyl, more preferably C1-2 alkyl, and still more preferably methyl.

In the present invention, X¹ is preferably CH or CR²¹, and more preferably CR²¹.

In the present invention, X² is preferably CH or CR²¹, and more preferably CH.

In the present invention, X³ is preferably CH or CR²¹, and more preferably CH.

In the present invention, R²¹ is preferably (1) halogen, (2) C1-4 alkyl, or (3) vinyl, and more preferably halogen or methyl.

In the present invention, the following partial structure in general formula (I) 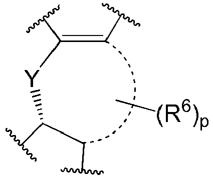 is preferably 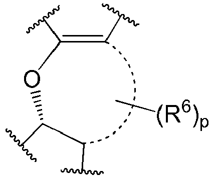 more preferably 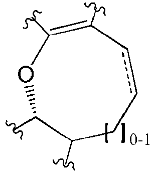 still more preferably 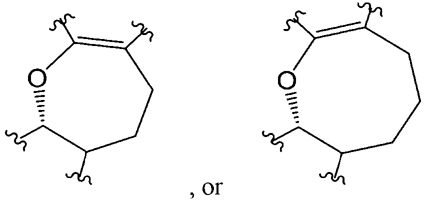 and particularly preferably

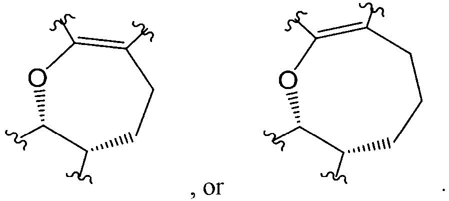

In the present invention, R⁶ is preferably C1-4 alkyl, and more preferably methyl.

In the present invention, p is preferably 0.

In the present invention, Y is preferably an oxygen atom.

In the present invention, R² is preferably a hydrogen atom.

In the present invention, R³ is preferably a hydrogen atom.

In the present invention, Z is preferably CHR¹⁰.

In the present invention, R¹⁰ is preferably a hydroxyl group.

In the present invention, L is preferably -CHR^{L1}- or -CR^{L2}R^{L3}-, and more preferably -CR^{L2}R^{L3}-.

In the present invention, L is preferably 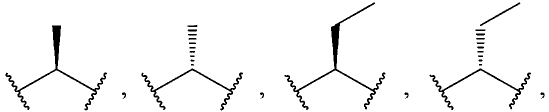 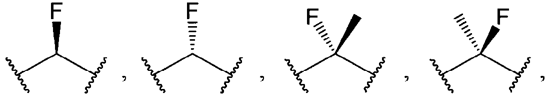 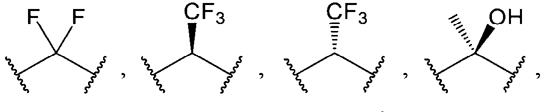 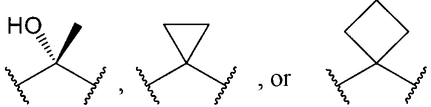 and more preferably

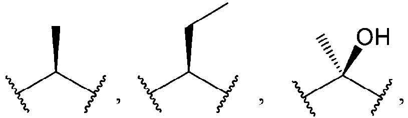

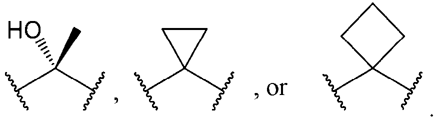

In the present invention, R^{L1} is preferably (1) halogen, (2) C1-4 alkyl, or (3) C1-4 haloalkyl, more preferably C1-4 alkyl, and still more preferably methyl or ethyl.

In the present invention, R^{L2} is preferably (1) halogen, (2) C1-4 alkyl, (3) C1-4 haloalkyl, or (4) a hydroxyl group, and more preferably C1-4 alkyl or a hydroxyl group.

In the present invention, R^{L3} is preferably (1) halogen, (2) C1-4 alkyl, (3) C1-4 haloalkyl, or (4) a hydroxyl group, and more preferably halogen or a hydroxyl group.

In the present invention, when R^{L2} and R^{L3} form a ring together with the carbon atom to which they are bonded, the ring is preferably a C3-6 saturated carbocycle, more preferably a C3-4 saturated carbocycle, and still more preferably cyclobutane.

In the present invention, R⁴ is preferably (1) C3-7 alkyl optionally substituted with 1 to 5 R⁴¹s, (2) C3-7 alkenyl optionally substituted with 1 to 5 R⁴²s, or (3) Cyc¹. R⁴ is preferably (1) C3-7 alkyl optionally substituted with 1 to 3 R⁴¹s, (2) C3-7 alkenyl optionally substituted with 1 to 3 R⁴²s, or (3) Cyc¹. R⁴ is more preferably (1) C3-5 alkyl optionally substituted with 1 to 3 R⁴¹s, (2) C3-5 alkenyl optionally substituted with 1 to 3 R⁴²s, or (3) Cyc¹.

In the present invention, R⁴¹ and R⁴² are each independently preferably halogen.

In the present invention, Cyc¹ is preferably (1) a C5-6 carbocycle optionally substituted with 1 to 5 R⁵¹s or (2) a 5-6 membered heterocycle optionally substituted with 1 to 5 R⁵²s, more preferably (1) phenyl optionally substituted with 1 to 5 R⁵¹s, (2) thiophene optionally substituted with 1 to 3 R⁵²s or (3) pyridine optionally substituted with 1 to 4 R⁵²s, still more preferably phenyl optionally substituted with 1 to 5 R⁵¹s, and particularly preferably phenyl optionally substituted with 1 to 3 halogen atoms.

In the present invention, Cyc¹ is also preferably 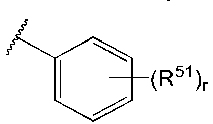 (in which r represents an integer of 0 to 5, and the other symbols represent the same meaning as described above), and more preferably 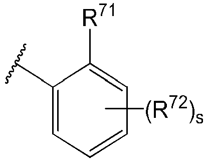 (in which R⁷¹ represents (1) halogen or (2) C1-4 alkyl, R⁷² represents (1) halogen or (2) C1-4 alkyl, and s represents an integer of 0 or 1).

In the present invention, R⁷¹ is preferably halogen.

In the present invention, R⁷² is preferably halogen.

In the present invention, r is preferably an integer of 0 to 2.

In the present invention, R⁵¹ is preferably (1) halogen, (2) C1-4 alkyl, or (3) C1-4 haloalkyl, and more preferably halogen.

In the present invention, R⁵² is preferably (1) halogen, (2) C1-4 alkyl, or (3) C1-4 haloalkyl, and more preferably halogen.

In the present invention, an aspect of a compound represented by general formula (I) is a compound represented by general formula (I-1): 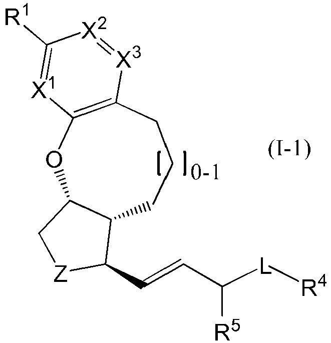 (in which all symbols represent the same meaning as described above), and an aspect of a compound represented by general formula (I-1) is a compound represented by general formula (I-2): 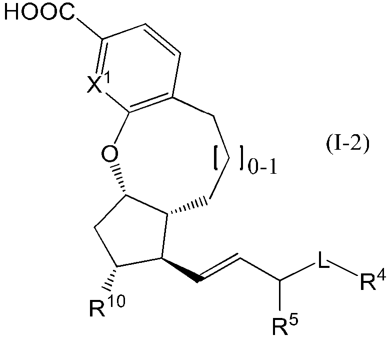 (in which all symbols represent the same meaning as described above), and an aspect of the compound represented by general formula (I-2) is a compound represented by general formula (I-3): 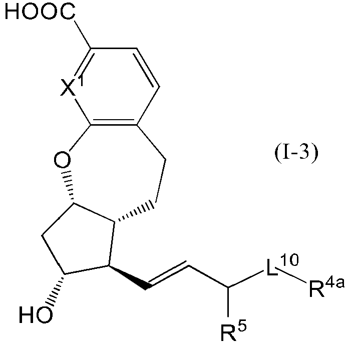 (in which L¹⁰ represents -CHR^{L1}- or -CR^{L2}R^{L3}-, R^{4a} represents C3-7 alkyl optionally substituted with 1 to 3 R⁴¹s, C3-7 alkenyl optionally substituted with 1 to 3 R⁴²s, or Cyc¹, and the other symbols represent the same meaning as described above).

Another aspect of the compound represented by general formula (I) is a compound represented by general formula (I-4): 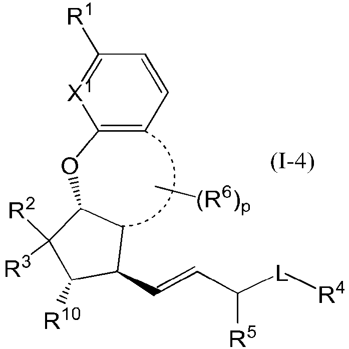 (in which all symbols represent the same meaning as described above), and an aspect of the compound represented by general formula (I-4) is a compound represented by general formula (I-5): 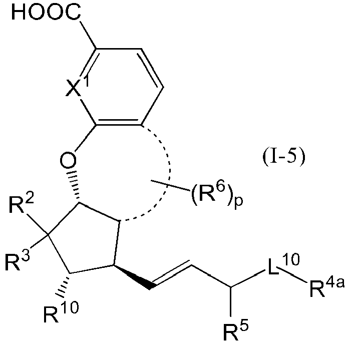 (in which all symbols represent the same meaning as described above).

Another aspect of the compound represented by general formula (I) is a compound represented by general formula (I-6): 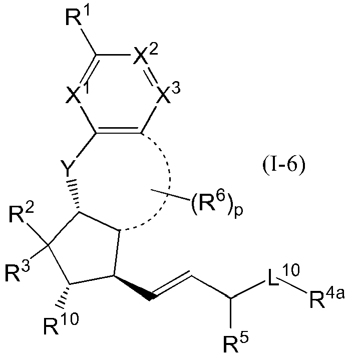 (in which all symbols represent the same meaning as described above), and an aspect of the compound represented by general formula (I-6) is a compound represented by general formula (I-7): 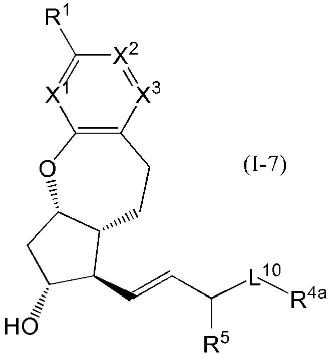 (in which all symbols represent the same meaning as described above).

In the present invention, an embodiment of the compound represented by general formula (I) or a salt thereof is
(1) (1R,2R,3aS,10aR)-1-[(1E,3R,4S)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]loxepine-6-carboxylic acid,
(2) (1R,2R,3 aS,10aR)-5-fluoro-1-[(1E)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid,
(3) (1R,2R,3aS,10aR)-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
(4) (1R,2R,3aS,10aR)-1-{(1E,3ξ)-3-[1-(2,5-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
(5) (1R,2R,3aS,10aR)-5-chloro-1-[(1E,3ξ)-4-(2,6-difluorophenyl)-4,4-difluoro-3-hydroxy-1-buten-1-yl]-2-hydroxy-2,3,3a,9,10,10a-hexahydro-1H-benzo[b]cyclopenta[f]oxepine-6-carboxylic acid,
(6) (1R,2R,3aS,10aR)-1-{(1E,3ξ)-3-[1-(4-fluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid,
(7) (1R,2R,3aS,10aR)-1-[(1E,3ξ,4ξ)-4-ethyl-3-hydroxy-7-methyl-1-octen-1-yl]-2-hydroxy-5-methyl-2,3,3a,9,10,10a-hexahydro-1H-benzo[b]cyclopenta[f]oxepine-6-carboxylic acid,
(8) (1R,2R,3aS,10aR)-2-hydroxy-5-methyl-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid,
(9) (1R,2R,3aS,10aR)-2-hydroxy-5-methyl-1-[(1E,4S)-8,8,8-trifluoro-4-hydroxy-4-methyl-1-octen-1-yl]-2,3,3a,9,10,10a-hexahydro-1H-benzo[b]cyclopenta[f]oxepine-6-carboxylic acid,
(10) (1R,2R,3aS,10aR)-2-hydroxy-1-[(1E,4ξ)-4-hydroxy-4,7-dimethyl-1-octen-1-yl]-5-methyl-2,3,3a,9,10,10a-hexahydro-1H-benzo[b]cyclopenta[f]oxepine-6-carboxylic acid,
(11) (1R,3aS,10aR)-5-fluoro-1-[(1E,3ξ)-3-hydroxy-4-methyl-4-phenyl-1-penten-1-yl]-2,3,3a,9,10,10a-hexahydro-1H-benzo[b]cyclopenta[f]oxepine-6-carboxylic acid,
(12) (1R,3aS,10aR)-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
(13) (1R,3aS,10aR)-5-chloro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
(14) (1R,3aS,10aR)-5-chloro-1-[(1E,3ξ)-3-hydroxy-6-methoxy-4-methylene-1-hexen-1-yl]-2,3,3a,9,10,10a-hexahydro-1H-benzo[b]cyclopenta[f]oxepine-6-carboxylic acid,
(15) (1R,3aS,10aR)-5-chloro-1-[(1E,3ξ,4S)-4-ethyl-3-hydroxy-1-octen-1-yl]-2,3,3a,9,10,10a-hexahydro-1H-benzo[b]cyclopenta[f]oxepine-6-carboxylic acid,
(16) (1R,3aS,10aR)-5-chloro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid,
(17) (1R,2R,3aS,10aR)-2,5-difluoro-1-[(1E,3ξ)-3-hydroxy-4-methyl-4-phenyl-1-penten-yl]-2,3,3a,9,10,10a-hexahydro-1H-benzo[b]cyclopenta[f]oxepine-6-carboxylic acid,
(18) (1R,2R,3aS,10aR)-2,5-difluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid,
(19) (1R,2R,3aS,10aR)-2-chloro-5-fluoro-1-{(1E,3ξ)-3-[3-(2-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid,
(20) (1R,2R,3aS,10aR)-2-chloro-5-fluoro-1-{(1E,3ξ)-3-hydroxy-3-[(2ξ)-2-phenyl-2-oxetanyl]-1-propen-1-yl }-2,3,3a,9,10,10a-hexahydro-1H-benzo[b]cyclopenta[f]oxepine-6-carboxylic acid,
(21) (1R,3aR,10aR)-1-[(1E,3ξ,4S)-3-hydroxy-4-methyl-1-octen-1-yl]-5-methyl-1,3,3a,9,10,10a-hexahydrofuro[3,4-b][1]benzoxepine-6-carboxylic acid,
(22) (1R,3aR,10aR)-1-[(1E,3ξ)-3-hydroxy-3-(1-phenoxycyclobutyl)-1-propen-1-yl]-5-methyl-1,3,3a,9,10,10a-hexahydrofuro[3,4-b][1]benzoxepine-6-carboxylic acid,
(23) (1R,2R,3aS,10aR)-5-fluoro-2-hydroxy-1-{(1E,3ξ)-3-hydroxy-3-[1-(2-methylphenyl)cyclopropyl]-1-propen-1-yl}-2,3,3a,9,10,10a-hexahydro-1H-benzo[b]cyclopenta[f]oxepine-6-carboxylic acid, or
(24) (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]loxepine-6-carboxylic acid,
   or a salt thereof.

### [Isomer]

In the present invention, 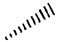
represents a bond beyond the sheet surface (that is, α configuration), and 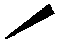
represents a bond in front of the sheet surface (that is, β configuration).

In the present invention, 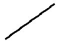 or a mixture of them in any ratio, unless otherwise indicated.

In the present invention, 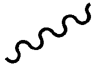 bonded to an asymmetric atom represents either the α configuration or the β configuration.

In addition, the description of ξ in the chemical name indicates that the configuration of a substituent bonded to an asymmetric atom is either the α configuration or the β configuration (IUPAC. Compendium of Chemical Terminology, 2nd ed. (the "Gold Book"). Compiled by A. D. McNaught and A.Wilkinson. Blackwell Scientific Publications, Oxford (1997). Online version (2019-) created by S. J. Chalk. ISBN 0-9678550-9-8. https://doi.org/10.1351/goldbook.).

For example, the chemical name of Example 7-3 and Example 7-4 that is
(1R,2R,3aS,10aR)-2-hydroxy-1-[(1E,3ξ,4ξ)-3-hydroxy-4-(trifluoromethyl)-1-octen-1-yl]-2,3,3a,9,10,10a-hexahydro-1H-benzo[b]cyclopenta[f]oxepine-6-carboxylic acid represents
(1R,2R,3aS,10aR)-2-hydroxy-1-[(1E,3ξ,4R)-3-hydroxy-4-(trifluoromethyl)-1-octen-1-yl]-2,3,3a,9,10,10a-hexahydro-1H-benzo[b]cyclopenta[f]oxepine-6-carboxylic acid or
(1R,2R,3aS,10aR)-2-hydroxy-1-[(1E,3ξ,4S)-3-hydroxy-4-(trifluoromethyl)-1-octen-1-yl]-2,3,3a,9,10,10a-hexahydro-1H-benzo[b]cyclopenta[f]oxepine-6-carboxylic acid, or represents
(1R,2R,3aS,10aR)-2-hydroxy-1-[(1E,3R,4R)-3-hydroxy-4-(trifluoromethyl)-1-octen-1-yl]-2,3,3a,9,10,10a-hexahydro-1H-benzo[b]cyclopenta[f]oxepine-6-carboxylic acid,
(1R,2R,3aS,10aR)-2-hydroxy-1-[(1E,3S,4R)-3-hydroxy-4-(trifluoromethyl)-1-octen-1-yl]-2,3,3a,9,10,10a-hexahydro-1H-benzo[b]cyclopenta[f]oxepine-6-carboxylic acid,
(1R,2R,3aS,10aR)-2-hydroxy-1-[(1E,3R,4S)-3-hydroxy-4-(trifluoromethyl)-1-octen-1-yl]-2,3,3a,9,10,10a-hexahydro-1H-benzo[b]cyclopenta[f]oxepine-6-carboxylic acid, or
(1R,2R,3aS,10aR)-2-hydroxy-1-[(1E,3S,4S)-3-hydroxy-4-(trifluoromethyl)-1-octen-1-yl]-2,3,3a,9,10,10a-hexahydro-1H-benzo[b]cyclopenta[f]oxepine-6-carboxylic acid.

For example, in a certain aspect, when Example 7-3 represents (1R,2R,3aS,10aR)-2-hydroxy-1-[(1E,3ξ,4R)-3-hydroxy-4-(trifluoromethyl)-1-octen-1-yl]-2,3,3a,9,10,10a-hexahydro-1H-benzo[b]cyclopenta[f]oxepine-6-carboxylic acid, Example 7-4 represents (1R,2R,3aS,10aR)-2-hydroxy-1-[(1E,3ξ,4S)-3-hydroxy-4-(trifluoromethyl)-1-octen-1-yl]-2,3,3a,9,10,10a-hexahydro-1H-benzo[b]cyclopenta[f]oxepine-6-carboxylic acid. In another aspect, when Example 7-3 represents (1R,2R,3aS,10aR)-2-hydroxy-1-[(1E,3ξ,4S)-3-hydroxy-4-(trifluoromethyl)-1-octen-1-yl]-2,3,3a,9,10,10a-hexahydro-1H-benzo[b]cyclopenta[f]oxepine-6-carboxylic acid, Example 7-4 represents (1R,2R,3aS,10aR)-2-hydroxy-1-[(1E,3ξ,4R)-3-hydroxy-4-(trifluoromethyl)-1-octen-1-yl]-2,3,3a,9,10,10a-hexahydro-1H-benzo[b]cyclopenta[f]oxepine-6-carboxylic acid.

As will be apparent to those skilled in the art, the other present compound in which the configuration of a substituent bonded to an asymmetric atom is represented by ξ can be interpreted in the same way.

In the present invention, all isomers encompass the above interpretation. For example, alkyl, alkenyl, alkynyl, alkoxy, alkylthio, alkylene, alkenylene, alkynylene, alkylidene and alkenylidene include linear and branched ones. Furthermore, isomers in a double bond, a ring, and a fused ring (E, Z, cis, and trans isomer), isomers due to the presence of asymmetric carbon or the like (R, S form, α, β configuration, enantiomer, diastereomer), optically active materials having optical rotation (D, L, d, l form), polar materials caused by chromatographic separation (high polarity material, low polarity material), equilibrium compounds, rotational isomers, mixtures of them in any arbitrary proportion, racemic mixtures, and tautomers are all included in the present invention.

The optically active compounds in the present invention are not only 100% pure, but may contain less than 50% of other optical isomers.

In the present invention, all references to the present compound include a compound represented by general formula (I) and the like, a salt thereof, a solvate (for example, hydrate) thereof, an N-oxide thereof or a cocrystal thereof, or a solvate (for example, hydrate) of the salt of the compound represented by general formula (I) and the like or a cocrystal thereof.

The compound represented by general formula (I) and the like is converted into a corresponding salt by a known method. The salt is preferably water-soluble. The salt is also preferably a pharmaceutically acceptable salt. Such salts include salts of alkali metals (lithium, potassium, sodium, etc.), salts of alkaline earth metals (calcium, magnesium, etc.), salts of other metals (silver, zinc, etc.), ammonium salts (tetramethylammonium salts), salts of pharmaceutically acceptable organic amines (tetramethylammonium, choline, triethylamine, methylamine, dimethylamine, ethylamine, diethylamine, cyclopentylamine, benzylamine, phenethylamine, tert-butylamine, ethylenediamine, piperidine, piperazine, monoethanolamine, diethanolamine, tris (hydroxymethyl) aminomethane, N-benzyl-2-phenethylamine, deanol, 2-(diethylamino)ethanol, 1-(2-hydroxyethyl)pyrrolidine, lysine, arginine, N-methyl-D-glucamine, etc.), acid adduct salts (inorganic acid salts (hydrochloride, hydrobromide, hydroiodide, sulfate, phosphate, nitrate, etc.), organic acid salts (acetate, trifluoroacetate, lactate, tartrate, oxalate, fumarate, maleate, benzoate, citrate, methanesulfonate, ethanesulfonate, benzenesulfonate, toluene sulfonate, isethionate, napadisylate, glucuronate, gluconate, etc.) etc.).

The compound represented by general formula (I) and the like or a salt thereof may exist in a non-solvated form, or may exist in a solvated form with a pharmaceutically acceptable solvent such as water or ethanol. The solvate is preferably low toxic and water-soluble, and more preferably a hydrate. The compound represented by general formula (I) and the like or a salt thereof can be converted into a solvate by a known method.

The N-oxide of the compound represented by general formula (I) and the like represents a compound obtained by oxidizing a nitrogen atom of the compound represented by general formula (I) and the like. In addition, the N-oxide of the compound represented by general formula (I) and the like may further be the alkali (earth) metal salt, the ammonium salt, the organic amine salt, or the acid adduct salt described above.

The compound represented by general formula (I) and the like or a salt thereof may exist in a form in which a cocrystal is formed with an appropriate cocrystal forming agent. The cocrystal is preferably a pharmaceutically acceptable one formed with a pharmaceutically acceptable cocrystal forming agent. The cocrystal is typically defined as a crystal formed by interaction between two or more different molecules. The cocrystal may also be a complex of a neutral molecule and a salt. The cocrystal can be prepared by known methods, for example melting crystallization, recrystallization from a solvent, or physically grinding of components together. Suitable cocrystal forming agents include organic acids (malic acid, succinic acid, adipic acid, gluconic acid, tartaric acid, benzoic acid, 4-hydroxybenzoic acid, 3-hydroxybenzoic acid, nicotinic acid, isonicotinic acid, etc.), organic amines (imidazole, diethanolamine, triethanolamine, tris(hydroxymethyl)aminomethane, N-benzyl-phenethylamine, deanol, 2-(diethylamino)ethanol, 1-(2-hydroxyethyl)pyrrolidine, 4-(2-hydroxyethyl)morpholine, N-methyl-D-glucamine, glycine, histidine, proline, etc.), and other organic compounds (caffeine, saccharin, etc.).

A prodrug of the compound represented by general formula (I) and the like refers to a compound that is converted into the compound represented by general formula (I) and the like through a reaction with an enzyme, gastric acid, or the like in a living body. The prodrug of the compound represented by general formula (I) and the like includes a compound having, when the compound represented by general formula (I) and the like has an amino group, the amino group acylated, alkylated, or phosphorylated (for example, a compound having the amino group of the compound represented by general formula (I) and the like eicosanoylated, alanylated, pentylaminocarbonylated, (5-methyl-2-oxo-1,3-dioxolene-4-yl)methoxycarbonylated, tetrahydrofuranylated, pyrrolidylmethylated, pivaloyloxymethylated, acetoxymethylated, tert-butylated, or the like); a compound having, when the compound represented by general formula (I) and the like has a hydroxyl group, the hydroxyl group acylated, alkylated, phosphorylated, or borated (for example, a compound having the hydroxyl group of the compound represented by general formula (I) and the like acetylated, palmitoylated, propanoylated, pivaloylated, succinylated, fumarylated, alanylated, dimethylaminomethylcarbonylated, or the like); and a compound having, when the compound represented by general formula (I) and the like has a carboxy group, the carboxy group esterified or amidated (for example, a compound having the carboxy group of the compound represented by general formula (I) and the like ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolene-4-yl)methyl-esterified, cyclohexyloxycarbonylethyl-esterified, methylamidated, or the like). These compounds can be produced by a method known per se. The prodrug of the compound represented by general formula (I) and the like may be a solvate. In addition, the prodrug of the compound represented by general formula (I) and the like may be one that is changed to the compound represented by general formula (I) and the like under physiological conditions as described in "Molecular Design", p. 163 to 198 in "Pharmaceutical research and development", vol. 7, published by Hirokawa Shoten, 1990. Furthermore, the compound represented by general formula (I) and the like may be labeled with an isotope (for example, ²H, ³H, ¹¹C, ¹³C, ¹⁴C, ¹³N, ¹⁵N, ¹⁵O, ¹⁷O, ¹⁸O, ³⁵S, ¹⁸F, ³⁶Cl, ¹²³I, ¹²⁵I, or the like) or the like.

### [Method for producing the present compound]

The present compound can be produced by appropriately improving and using known methods, for example, a method described in Comprehensive Organic Transformations: A Guide to Functional Group Preparations, 3rd Edition (Richard C. Larock, John Wiley & Sons Inc, 2018), a method shown in Examples, and the like in combination.

Among the compounds represented by general formula (I), compounds represented by general formula (I-A) 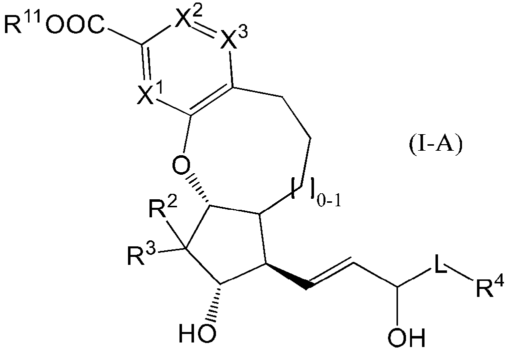 (in which all symbols represent the same meaning as described above) can be produced by the following Reaction Scheme 1. 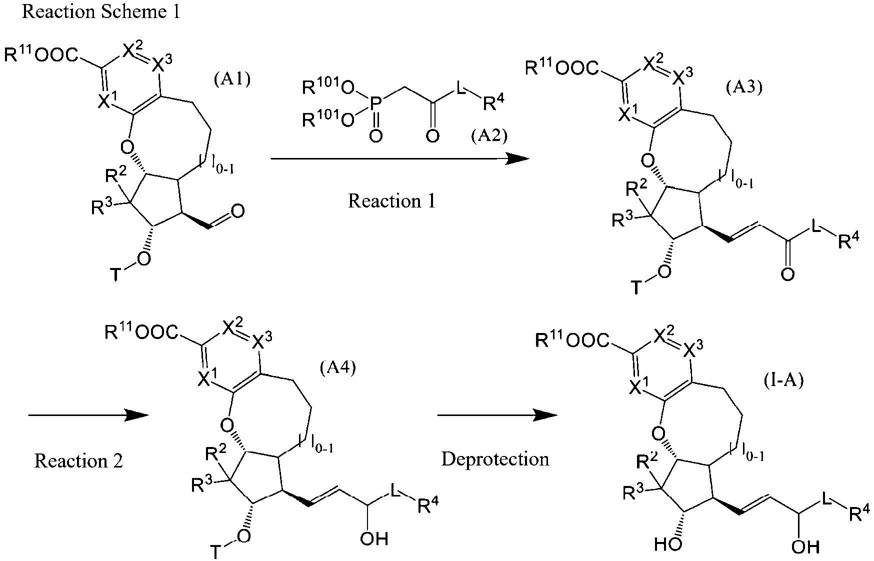 (in which T represents a protecting group for a hydroxyl group, R¹⁰¹s each independently represent C1-6 alkyl, and the other symbols represent the same meaning as described above.)

In Reaction Scheme 1, Reaction 1 is known, and is performed, for example, by reacting a compound represented by general formula (A1) with a compound represented by general formula (A2) in an organic solvent (for example, tetrahydrofuran (THF), dimethylformamide (DMF), dimethoxyethane (DME), dioxane, acetonitrile, ethanol, or dichloromethane), water, or a mixture thereof, in the presence of a base (for example, sodium hydride, sodium hydroxide, potassium hydroxide, potassium phosphate, potassium tert-butoxide, potassium carbonate, or tertiary amine + lithium chloride) at temperatures of -20 to 70°C.

In Reaction Scheme 1, Reaction 2 is known, and is performed by reacting the compound represented by general formula (A3) obtained in Reaction 1 in an organic solvent (for example, THF, DME, toluene, dichloromethane, diethyl ether, and dioxane) in the presence or absence of cerium chloride using a reducing agent (for example, sodium borohydride or zinc borohydride) at -20 to 50°C. When only one stereoisomer is selectively produced, the production is performed using an asymmetric reducing agent (for example, chlorodiisopinocamphenylborane) or a combination of an asymmetric auxiliary and a reducing agent ((R)-2-methyl-CBS-oxazaborolidine and a borohydride/tetrahydrofuran complex or a borane dimethyl sulfide complex, (S)-(-)-binaphthol and lithium aluminum hydride, or the like) at temperatures of -100 to 50°C.

In Reaction Scheme 1, a deprotection reaction of a protecting group is known, and can be performed by the following method. Examples of the method include (1) a deprotection reaction by alkaline hydrolysis, (2) a deprotection reaction under acidic conditions, (3) a deprotection reaction by hydrogenolysis, (4) a deprotection reaction of a silyl group, (5) a deprotection reaction performed using a metal, and (6) a deprotection reaction performed using a metal complex.

These methods are specifically described as follows:
(1) The deprotection reaction by alkaline hydrolysis is performed, for example, in an organic solvent (for example, methanol, tetrahydrofuran, dioxane, or the like) using a hydroxide of an alkali metal (for example, sodium hydroxide, potassium hydroxide, lithium hydroxide, or the like), a hydroxide of an alkaline earth metal (for example, barium hydroxide, calcium hydroxide, or the like), a carbonate (for example, sodium carbonate, potassium carbonate, or the like), an aqueous solution thereof, or a mixture thereof at 0 to 40°C.
(2) The deprotection reaction under acidic conditions is performed, for example, in an organic solvent (for example, dichloromethane, chloroform, dioxane, ethyl acetate, methanol, isopropyl alcohol, tetrahydrofuran, anisole, or the like), an organic acid (for example, acetic acid, trifluoroacetic acid, methanesulfonic acid, p-tosylic acid, or the like), or an inorganic acid (for example, hydrochloric acid, sulfuric acid, or the like) or a mixture thereof (for example, hydrogen bromide/acetic acid, or the like), in the presence or absence of 2,2,2-trifluoroethanol, at 0 to 100°C.
(3) The deprotection reaction by hydrogenolysis is performed, for example, in a solvent (for example, an ether-based solvent (for example, tetrahydrofuran, dioxane, dimethoxyethane, diethyl ether, or the like), an alcohol-based solvent (for example, methanol, ethanol, or the like), a benzene-based solvent (for example, benzene, toluene, or the like), a ketone-based solvent (for example, acetone, methyl ethyl ketone, or the like), a nitrile-based solvent (for example, acetonitrile or the like), an amide-based solvent (for example, N,N-dimethylformamide or the like), water, ethyl acetate, acetic acid, or a mixed solvent of two or more thereof, or the like), in the presence of a catalyst (for example, palladium-carbon, palladium black, palladium hydroxide-carbon, platinum oxide, Raney nickel, or the like), in a hydrogen atmosphere under normal pressure or pressure, or in the presence of ammonium formate at 0 to 200°C.
(4) The deprotection reaction of a silyl group is performed, for example, in an organic solvent (for example, tetrahydrofuran, acetonitrile, or the like) miscible with water using tetrabutylammonium fluoride at 0 to 40°C. In addition, for example, the reaction is performed in an organic acid (for example, acetic acid, trifluoroacetic acid, methanesulfonic acid, p-tosylic acid, or the like), an inorganic acid (for example, hydrochloric acid, sulfuric acid, or the like), or a mixture thereof (for example, hydrogen bromide/acetic acid, or the like) at -10 to 100°C.
(5) The deprotection reaction performed using a metal is performed, for example, in an acidic solvent (for example, acetic acid, a buffer solution having a pH of 4.2 to 7.2, or a mixture of these solutions with an organic solvent such as tetrahydrofuran) in the presence of powdered zinc while applying ultrasonic waves if necessary at 0 to 40°C.
(6) The deprotection reaction performed using a metal complex is performed, for example, in an organic solvent (for example, dichloromethane, N,N-dimethylformamide, tetrahydrofuran, ethyl acetate, acetonitrile, dioxane, ethanol, or the like), water, or a mixture thereof, in the presence of a trap reagent (for example, tributyltin hydride, triethylsilane, dimedone, morpholine, diethylamine, pyrrolidine, or the like), an organic acid (for example, acetic acid, formic acid, 2-ethylhexanoic acid, or the like), and/or an organic acid salt (for example, sodium 2-ethylhexanoate, potassium 2-ethylhexanoate, or the like), in the presence or absence of a phosphine-based reagent (for example, triphenylphosphine or the like), using a metal complex (for example, tetrakistriphenylphosphine palladium(0), bis(triphenylphosphine)palladium(II) dichloride, palladium(II) acetate, tris(triphenylphosphine)rhodium(I) chloride, or the like) at 0 to 40°C.

In addition to the above, the deprotection reaction can be performed by, for example, the method described in T. W. Greene, Protective Groups in Organic Synthesis, Wiley, New York, 2014.

Examples of the protecting group of a hydroxyl group (including T described in Reaction Scheme 1) include a methyl group, a trityl group, a methoxymethyl (MOM) group, a 1-ethoxyethyl (EE) group, a methoxyethoxymethyl (MEM) group, a 2-tetrahydropyranyl (THP) group, a trimethylsilyl (TMS) group, a triethylsilyl (TES) group, a t-butyldimethylsilyl (TBDMS) group, a t-butyldiphenylsilyl (TBDPS) group, an acetyl (Ac) group, a pivaloyl group, a benzoyl group, a p-phenylbenzoyl group, a benzyl (Bn) group, a p-methoxybenzyl group, an allyloxycarbonyl (Alloc) group, a 2,2,2-trichloroethoxycarbonyl (Troc) group, and the like.

Examples of the protecting group of an amino group include a benzyloxycarbonyl group, a t-butoxycarbonyl group, an allyloxycarbonyl (Alloc) group, a 1-methyl-1-(4-biphenyl)ethoxycarbonyl (Bpoc) group, a trifluoroacetyl group, a 9-fluorenylmethoxycarbonyl group, a benzyl (Bn) group, a p-methoxybenzyl group, a benzyloxymethyl (BOM) group, a 2-(trimethylsilyl)ethoxymethyl (SEM) group, and the like.

The protecting group of a hydroxyl group is preferably a 2-tetrahydropyranyl (THP) group or a tert-butyldimethylsilyl (TBDMS) group.

In addition to the groups described above, the protecting group of a hydroxyl group is not particularly limited as long as it is a group that can be easily and selectively detached. For example, those described in T. W. Greene, Protective Groups in Organic Synthesis, Wiley, New York, 2014 are used.

Among the compounds represented by general formula (I), compounds represented by general formula (I-B) 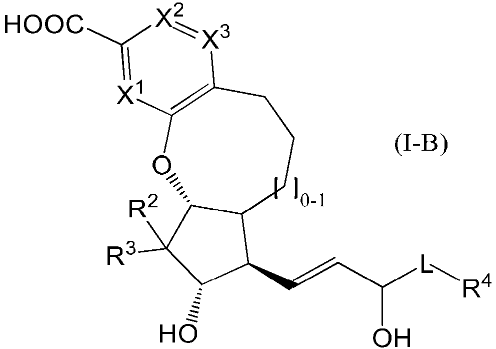 (in which all symbols represent the same meaning as described above) can be produced by subjecting the compounds represented by general formula (I-A) to a hydrolysis reaction.

This hydrolysis reaction (deprotection reaction of a carboxyl group) is known, and examples thereof include the above-described alkaline hydrolysis.

If necessary, the reaction may be followed by a known method for converting the compound into a desired salt or a solvate thereof (for example, hydrate).

The compound represented by general formula (A1) in Reaction Scheme 1 can be produced by the following Reaction Scheme 2. 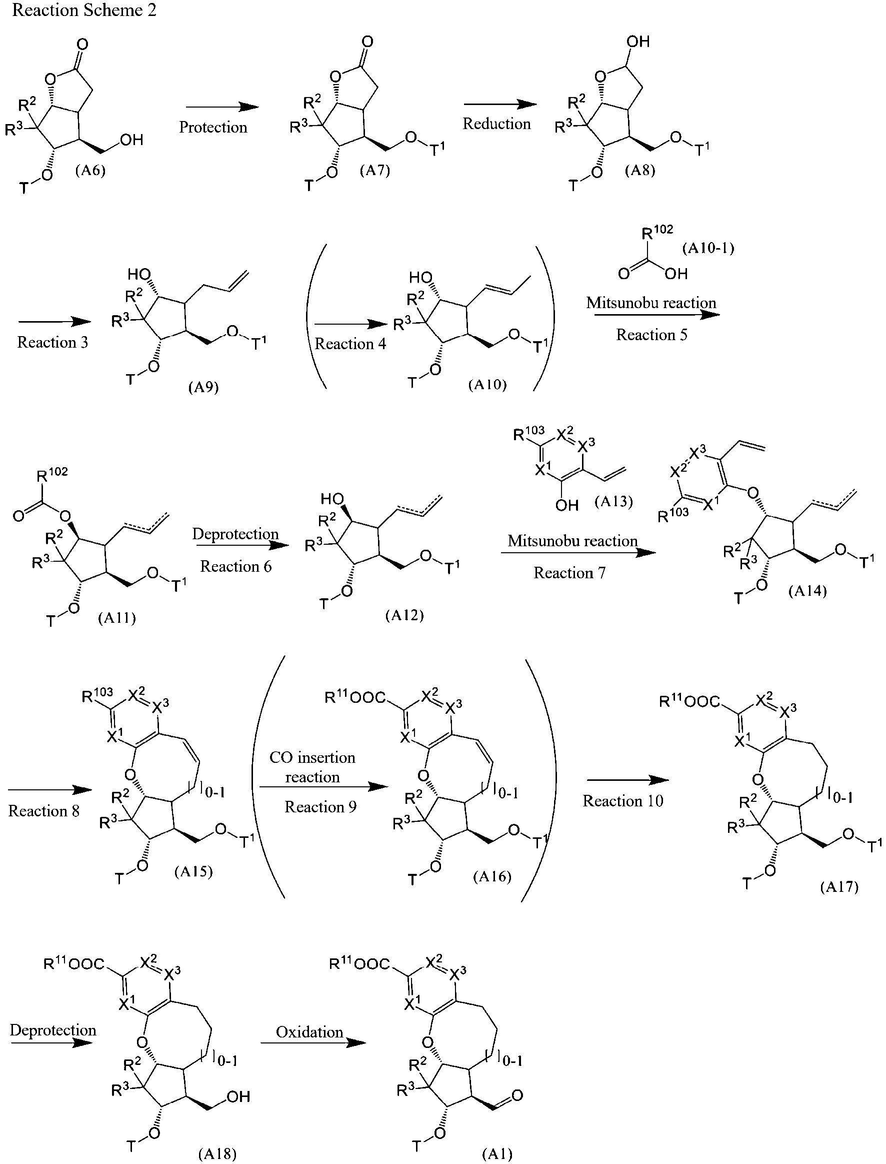 (in which T¹ represents a protecting group for a hydroxyl group, R¹⁰² represents a hydrogen atom, phenyl or substituted phenyl (for example, 4-fluorophenyl or the like), R¹⁰³ represents a halogen atom or COOR¹¹, and the other symbols represent the same meaning as described above. The protecting group for a hydroxyl group represented by T¹ is the same as the protecting group T described above.)

In Reaction Scheme 2, a compound represented by general formula (A7) can be produced by subjecting a compound represented by general formula (A6) to a protection reaction. For example, the reaction is performed using a base (for example, imidazole or the like) and a silane compound (for example, trimethylsilane chloride (TESCl), tert-butyldimethylsilane chloride (TBSCl), tert-butyldiphenylsilane chloride (TBDPSCl), or the like) in an organic solvent (for example, DMF or the like) at temperatures of -100 to 50°C.

In Reaction Scheme 2, a compound represented by general formula (A8) can be produced by subjecting a compound represented by general formula (A7) to a reduction reaction. For example, the reaction is performed using a reducing agent (for example, diisobutylaluminum hydride (DIBAL), lithium aluminum hydride, or the like) in an organic solvent (for example, toluene, ethanol, tetrahydrofuran, hexane, or the like) at -78 to 80°C.

In Reaction Scheme 2, Reaction 3 is known, and is performed, for example, using a Wittig reagent (for example, methyltriphenylphosphonium bromide or the like) in an organic solvent (for example, anhydrous toluene, dimethoxyethane, tetrahydrofuran, or the like) in the presence of a base (for example, lithium hexamethyldisilazane (LHMDS), lithium diisopropylamide (LDA), butyllithium, potassium tert-butoxide, sodium hydride, or the like) at temperatures of -78 to 50°C.

In Reaction Scheme 2, Reaction 4 is known, and is performed, for example, using a ruthenium catalyst (for example, carbonyl chlorohydride tris(triphenylphosphine)ruthenium(II), a metathesis catalyst, or the like) or a cobalt catalyst (for example, a Jacobsen catalyst or the like) in an organic solvent (for example, anhydrous toluene, benzene, 1,4-dioxane, dimethoxyethane, methanol, or the like) at temperatures of 20 to 100°C.

In Reaction Scheme 2, the Mitsunobu reaction in Reaction 5 and Reaction 7 is known, and is performed, for example, in an organic solvent (for example, dichloromethane, diethyl ether, tetrahydrofuran, acetonitrile, benzene, toluene) in the presence of (1) an azo compound (for example, diethyl azodicarboxylate (DEAD), diisopropyl azodicarboxylate, 1,1'-(azodicarbonyl)dipiperidine (ADDP), 1,1'-azobis(N,N-dimethylformamide)) and a phosphine compound (for example, triphenylphosphine, tributylphosphine, trimethylphosphine, polymer-supported triphenylphosphine) or in the presence of (2) an ylide compound (cyanomethylene tributylphosphorane, cyanomethylene trimethylphosphorane) at 0 to 100°C.

In Reaction Scheme 2, Reaction 6 is known, and is performed, for example, using a hydroxide of an alkali metal (sodium hydroxide, potassium hydroxide, lithium hydroxide, or the like), a hydroxide of an alkaline earth metal (barium hydroxide, calcium hydroxide, or the like), a carbonate (sodium carbonate, potassium carbonate, or the like), an aqueous solution thereof, or a mixture thereof in an organic solvent (methanol, tetrahydrofuran, dioxane, or the like) at temperatures of 0 to 100°C.

In Reaction Scheme 2, Reaction 8 is known, and is performed, for example, using a metathesis catalyst (for example, 2,6-diisopropyl phenylimide neophylidenemorbidenium(VI) bis(tert-butoxide), 2,6-diisopropyl phenylimide neophylidenemorbidenium(VI) bis(hexafluoro tert-butoxide), or the like) in an organic solvent (for example, toluene, dichloromethane, dichloroethane, or the like) at temperatures of 20 to 80°C.

In Reaction Scheme 2, Reaction 9 is known, and is performed, for example, in an organic solvent (for example, toluene, THF, DMF, or the like) in the presence of a nucleophilic agent (for example, alcohol or the like) and a palladium catalyst (for example, bis(tri-tert-butylphosphine)palladium (Pd(P(t-Bu)₃)₂), tetrakis(triphenylphosphine)palladium (Pd(PPh₃)₄), bis(triphenylphosphine)palladium dichloride (PdCl₂(PPh₃)₂), or the like) under a carbon monoxide stream at temperatures of 50 to 100°C.

In Reaction Scheme 2, Reaction 10 is known, and is performed through (1) a reaction performed using a metal catalyst (for example, palladium carbon, platinum oxide, rhodium-alumina, Raney nickel, a Wilkinson complex, a ruthenium catalyst, an iridium catalyst, or the like) in an organic solvent (for example, methanol, ethanol, ethyl acetate, dichloromethane, dichloroethane, or the like), for example, using hydrogen gas under atmospheric pressure or high pressure at 0°C to 80°C, or
(2) a reaction performed using a reducing agent (for example, sodium borohydride or the like) in an organic solvent (for example, methanol, ethanol, or the like) in the presence or absence of cerium chloride or the like as an additive at -40 to 80°C.

In Reaction Scheme 2, a compound represented by general formula (A18) can be produced by subjecting a compound represented by general formula (A17) to the deprotection reaction.

In Reaction Scheme 2, the compound represented by general formula (A1) can be produced by subjecting a compound represented by general formula (A18) to an oxidation reaction. Examples of the oxidation reaction include
(1) a method performed using DMSO oxidation (e.g., Swern oxidation),
(2) a method performed using a Dess-Martin Reagent,
(3) a method performed using a TEMPO (2,2,6,6-tetramethylpiperidine 1-oxyl) reagent,
and the like.

As a specific example, the method performed using DMSO oxidation is performed, for example, by reacting an alcohol compound in an organic solvent (for example, chloroform, dichloromethane, ethyl acetate, or the like) in the presence of an activating agent (for example, oxalyl chloride, acetic anhydride, pyridine-sulfur trioxide complex, or the like) and an oxidizing agent (for example, dimethylsulfoxide (DMSO) or the like), and further reacting the alcohol compound with a tertiary amine (for example, triethylamine, N,N-diisopropylethylamine, N-methylmorpholine, N-ethylpiperidine, diazabicyclo[5.4.0]undec-7-ene, or the like) at -78 to 40°C.

In each reaction in the present specification, the compound having a chiral center may be a compound of a desired optical isomer fractionated by performing optical resolution using a conventional method (for example, a method performed using an optical resolution column).

In Reaction Scheme 2, Reaction 4 and/or Reaction 9 can be appropriately omitted depending on the compound to be obtained or the intermediate to be used.

Among the compounds represented by general formula (I), compounds represented by general formula (I-C) 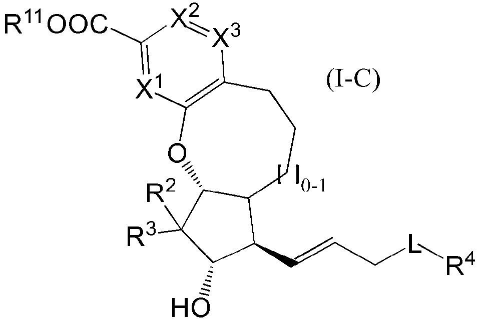 (in which all symbols represent the same meaning as described above) can be produced by the following Reaction Scheme 3. 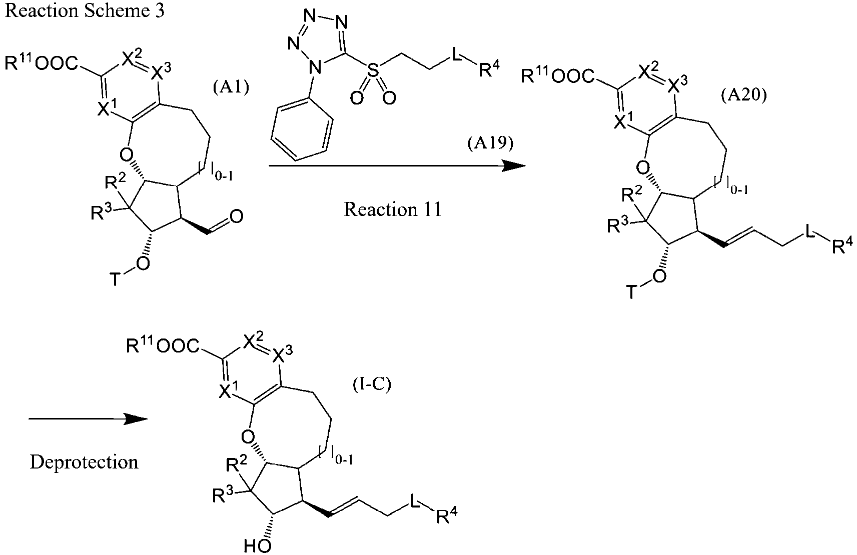 (in which all symbols represent the same meaning as described above.)

In Reaction Scheme 3, Reaction 11 is known, and is performed, for example, by reacting the compound represented by general formula (A1) with a compound represented by general formula (A19) in an organic solvent (for example, tetrahydrofuran (THF), dimethylformamide (DMF), dimethoxyethane (DME), toluene, or the like) in the presence of a base (for example, potassium hexamethyldisilazide, lithium diisopropylamide, butyllithium, or the like) at temperatures of -100 to -20°C.

In Reaction Scheme 3, a deprotection reaction of a protecting group is known, and can be performed by the method of deprotection reaction described above.

Among the compounds represented by general formula (I), compounds represented by general formula (I-D) 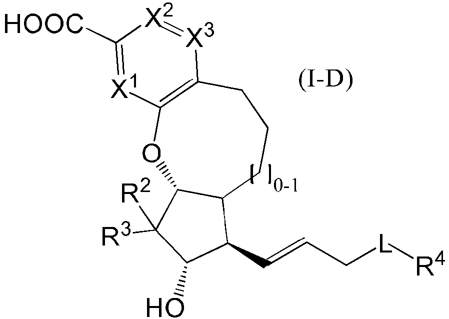 (in which all symbols represent the same meaning as described above) can be produced by subjecting the compounds represented by general formula (I-C) to a hydrolysis reaction.

This hydrolysis reaction (deprotection reaction of a carboxyl group) is known, and examples thereof include the above-described alkaline hydrolysis.

If necessary, the reaction may be followed by a known method for converting the compound into a desired salt.

In each of the reactions herein, compounds used as starting materials, for example, compounds represented by general formula (A2), general formula (A6), general formula (A10-1), general formula (A13) and general formula (A19) are known, or can be easily produced by known methods.

In each of the reactions herein, a reaction involving heating can be performed using a water bath, an oil bath, a sand bath, or a microwave, as will be apparent to those skilled in the art.

In each of the reactions herein, a solid-phase supported reagent supported on a high-molecular polymer (for example, polystyrene, polyacrylamide, polypropylene, polyethylene glycol, or the like) may be appropriately used.

In each of the reactions herein, the reaction product can be purified by ordinary purification means, for example, distillation under normal pressure or reduced pressure, high performance liquid chromatography performed using silica gel or magnesium silicate, thin layer chromatography, ion exchange resin, scavenger resin or column chromatography, washing, recrystallization, or the like. Purification may be performed for each reaction or may be performed after completion of some reactions.

Specific aspects of the method for producing the present compound include the following.

A method for producing a compound of general formula (I) or a salt thereof, including the step of:
subjecting a compound represented by general formula (I') 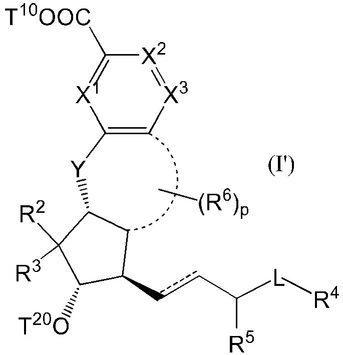 [in which:
T¹⁰ is a hydrogen atom or R¹¹, T²⁰ is a hydrogen atom or a protecting group for a hydroxyl group, T¹⁰ and T²⁰ are not simultaneously a hydrogen atom, and the other symbols represent the same meaning as defined in general formula (I)] to a hydrolysis reaction and/or a deprotection reaction.

The protecting group for a hydroxyl group represented by T²⁰ is the same as the protecting group T described above.

The hydrolysis reaction (deprotection reaction of a carboxyl group) when T¹⁰ is R¹¹ is known, and examples thereof include alkaline hydrolysis.

When T²⁰ is a protecting group for a hydroxyl group, a deprotection reaction for this can be performed by a known method. Examples of the method include (1) a deprotection reaction by alkaline hydrolysis, (2) a deprotection reaction under acidic conditions, (3) a deprotection reaction by hydrogenolysis, (4) a deprotection reaction of a silyl group, (5) a deprotection reaction performed using a metal, and (6) a deprotection reaction performed using a metal complex. Details have been described above.

If necessary, these reactions may be followed by a known method for converting the compound into a desired salt.

In the above producing method, the hydrolysis reaction may be
(A) a step of subjecting a compound represented by general formula (I') (T¹⁰ = R¹¹, T²⁰ = a hydrogen atom) to a hydrolysis reaction, and in that case, the compound of general formula (I) (R¹ = COOH, Z = CHR¹⁰, R¹⁰ = a hydroxyl group) or a salt thereof can be produced.

### [Toxicity]

The toxicity of the present compound is sufficiently so low that the preventive and/or therapeutic agent can be safely used as a pharmaceutical product.

### [Application to pharmaceutical products]

The present compound can be used as an EP3 agonist having EP3 agonist activity.

Since the present compound has EP3 agonist activity, it is considered to be useful for treatment and/or prevention of diseases such as renal disease (for example, chronic renal disease or acute renal damage), myocardial infarction, dysuria, and glaucoma.

The present compound is useful, for example, for treatment and/or prevention of renal disease. Certain aspects of renal disease are chronic renal disease and acute renal damage.

In a preferred embodiment, the chronic renal disease is, for example, chronic renal disease based on lifestyle diseases, chronic glomerulonephritis, nephrotic syndrome, or hereditary renal disease. Among them, the chronic renal disease based on lifestyle diseases include diabetic renal disease and nephrosclerosis. The chronic glomerulonephritis includes IgA nephropathy, GBM nephritis, ANCA-associated nephritis, mesangial proliferative nephritis, and lupus nephritis. The nephrotic syndrome includes minimal change nephrotic syndrome, focal segmental glomerulosclerosis, and membranous nephropathy. The hereditary renal disease includes Alport syndrome and polycystic kidney disease. The polycystic kidney disease includes, for example, autosomal dominant polycystic kidney disease and autosomal recessive polycystic kidney disease.

Another embodiment of the chronic renal disease includes, for example, chronic symptoms of renal failure and/or renal insufficiency, renal hypoperfusion, hypertensive nephrosclerosis, immunological renal damage, and pyelonephritis.

Another embodiment of the chronic renal disease includes, for example, dehydration via forced diuresis after partial resection of the kidney, uncontrolled blood pressure elevation with malignant hypertension, urinary tract obstruction, and infections and amyloidosis and systemic diseases with involvement of glomeruli, e.g. rheumatoid immunologic systemic disorders, chronic renal insufficiency, for example in lupus erythematosus, and renal artery stenosis, renal artery thrombosis, renal vein thrombosis, analgesic nephropathy and renal tubular acidosis, X-ray contrast agent-induced and drug-induced chronic interstitial renal damages, and chronic renal insufficiency due to metabolic syndrome and dyslipidemia.

In a preferred embodiment, the chronic renal disease includes pathological symptoms and diseases caused by chronic renal disease. Examples of the pathological symptoms and diseases caused by chronic renal disease include obstructive uropathy, tubulointerstitial disorders, glomerular and tubular proteinuria, abnormal reduction of renal edema, hematuria, creatinine and/or water excretion, abnormal elevation of blood concentrations of urea, nitrogen, potassium and/or creatinine, a change in the activity of a kidney enzyme, for example glutamyl synthetase, a change in urine osmolar concentration or urine volume, elevation of microalbuminuria, overt albuminuria, lesions of glomeruli and arterioles, tubular dilatation, and hyperphosphatemia.

In a preferred embodiment, the acute renal damage is, for example, acute renal damage accompanied by ischemia reperfusion damage or drug (antimicrobials, anticancer agents, etc.)-induced renal damage. Among them, the acute renal damage accompanied by ischemia reperfusion damage includes acute renal damage associated with organ surgery such as cardiac surgery, liver surgery, or lung surgery, acute renal damage associated with sepsis, acute renal damage associated with kidney transplantation, acute renal damage associated with rhabdomyolysis, and contrast nephropathy.

Another embodiment of the acute renal damage includes, for example, partial resection of the kidney, dehydration via forced diuresis, uncontrolled blood pressure elevation with malignant hypertension, urinary tract obstruction, and infections and amyloidosis and systemic diseases with involvement of glomeruli, e.g. rheumatoid immunologic systemic diseases, for example in lupus erythematosus, renal artery thrombosis, renal vein thrombosis, analgesic nephropathy and renal tubular acidosis, and X-ray contrast agent-induced and drug-induced acute interstitial renal damages.

Preferable examples of the renal disease to be treated and/or prevented using the present compound include diabetic renal disease, IgA nephropathy, Alport syndrome, and autosomal dominant polycystic kidney disease.

The present compound may be administered as a concomitant drug in combination with another agent for
1) complementation and/or enhancement of the preventive and/or therapeutic effect of the compound,
2) improvement of kinetics and absorption, and reduction of dosage of the compound, and/or
3) reduction of side effects of the compound

The concomitant drug of the present compound and another drug may be administered in the form of a compounding agent in which both components are compounded in one formulation, or may be administered in the form of separate formulations. When administration is performed in this separate formulations, simultaneous administration or administration with a time difference is included. In addition, as to the administration with a time difference, the present compound may be administered first, and the other drug may be administered later, or the other drug may be administered first, and the present compound may be administered later. The respective administration methods may be the same or different.

Disease on which a therapeutic and/or preventive effect is exhibited by the concomitant drug is not particularly limited, as long as the concomitant drug complements and/or enhances the therapeutic and/or preventive effect of the present compound.

In addition, the concomitant drug obtained by combination with the present compound includes not only those that have been found so far but also those that will be found in the future.

The pharmaceutical composition in the present invention contains the present compound and any pharmaceutically acceptable carrier. For use for the purpose of preventing and/or treating the above-mentioned diseases, the pharmaceutical composition containing the present compound is usually prepared by formulating the present compound, which is an active ingredient, with a pharmaceutically acceptable carrier such as various additives or solvents, and then administered systemically or topically in an oral or parenteral form. Here, the pharmaceutically acceptable carrier means a substance other than the active ingredient, which substance is generally used for pharmaceutical formulation. Preferably, the pharmaceutically acceptable carrier does not exhibit a pharmacological effect, is harmless, and does not interfere with the therapeutic effect of the active ingredient at the dosage of the formulation. The pharmaceutically acceptable carrier can also be used for the purpose of, for example, enhancing the usefulness of the active ingredient and the formulation, facilitating formulation, stabilizing the quality, or improving the usability. Specifically, substances described in "Iyakuhin Tenkazai Jiten (Japanese Pharmaceutical Excipients Directory)" published in 2000 by Yakuji Nippo Limited (edited by International Pharmaceutical Excipients Council Japan) and the like may be appropriately selected depending on the purpose.

In order to use the present compound or the concomitant drug of the present compound and another drug for the above purpose, the present compound or the concomitant drug is usually administered systemically or locally, in an oral or parenteral form. The dosage varies depending on age, body weight, symptom, therapeutic effect, administration method, treatment time, and the like, but is usually orally administered once to several times a day in the range of 1 ng to 1000 mg per adult for each administration, or parenterally administered once to several times a day in the range of 0.1 ng to 10 mg per adult for each administration, or continuously administered intravenously in the range of 1 hour to 24 hours per day. Of course, as described above, since the dosage varies depending on various conditions, an amount smaller than the above dosage may be sufficient, or an administration beyond the range may be required.

Examples of the oral preparation include oral liquid preparations (for example, elixirs, syrups, pharmaceutically acceptable water, suspensions, emulsions) and oral solid preparations (for example, tablets (including sublingual tablets, orally disintegrating tablets), pills, capsules (including hard capsules, soft capsules, gelatin capsules, microcapsules), powders, granules, lozenges). Examples of the parenteral preparation include liquid preparations (for example, injections (intravitreal injection, subcutaneous injection, intravenous injection, intramuscular injection, intraperitoneal injection, drip infusion, etc.), eye drops (for example, aqueous eye drops (aqueous ophthalmic solution, aqueous suspending ophthalmic solution, viscous ophthalmic solution, solubilized ophthalmic solution, etc.), non-aqueous eye drops (non-aqueous ophthalmic solution, non-aqueous suspending ophthalmic solution, etc.))), external preparations (for example, an ointment (eye ointment or the like)), ear drops, and the like. These formulations may be controlled-release formulations such as rapid release formulations and sustained release formulations. These formulations can be produced by known methods, for example, a method described in the Japanese Pharmacopoeia.

The oral liquid preparation as an oral preparation is produced, for example, by dissolving, suspending or emulsifying an active ingredient in a commonly used diluent (for example, purified water, ethanol, a mixture thereof, or the like). Furthermore, this liquid preparation may contain a wetting agent, a suspending agent, an emulsifying agent, a sweetening agent, a flavoring agent, an aromatic agent, a preservative, a buffering agent, or the like.

The oral solid preparation as an oral preparation is prepared by, for example, mixing an active ingredient with an excipient (for example, lactose, mannitol, glucose, microcrystalline cellulose, starch, or the like), a binder (for example, hydroxypropyl cellulose, polyvinyl pyrrolidone, magnesium aluminate metasilicate, or the like), a disintegrant (for example, calcium fiber glycolate or the like), a lubricant (for example, magnesium stearate or the like), a stabilizer, a solubilizer (glutamic acid, aspartic acid, or the like), or the like, followed by formulation according to a conventional method. If necessary, the preparation may be coated with a coating agent (for example, white sugar, gelatin, hydroxypropyl cellulose, hydroxypropyl methylcellulose phthalate, or the like), or may be coated with two or more layers.

The external preparation as a parenteral preparation is produced by a known method or a commonly used formulation. For example, an ointment is produced by triturating or melting an active ingredient in a base. The ointment base is selected from known or commonly used ones. For example, one or a mixture of two or more selected from a higher fatty acid or a higher fatty acid ester (for example, adipic acid, myristic acid, palmitic acid, stearic acid, oleic acid, adipic acid ester, myristic acid ester, palmitic acid ester, stearic acid ester, oleic acid ester, or the like), a wax (for example, beeswax, spermaceti wax, celesine, or the like), a surfactant (for example, polyoxyethylene alkyl ether phosphoric acid ester or the like), a higher alcohol (for example, cetanol, stearyl alcohol, cetostearyl alcohol, or the like), a silicon oil (for example, dimethylpolysiloxane or the like), a hydrocarbon (for example, hydrophilic petrolatum, white petrolatum, purified lanolin, liquid paraffin, or the like), a glycol (for example, ethylene glycol, diethylene glycol, propylene glycol, polyethylene glycol, macrogol, or the like), a vegetable oil (for example, castor oil, olive oil, sesame oil, turpentine oil, or the like), an animal oil (for example, mink oil, egg yolk oil, squalane, squalene, or the like), water, an absorption promoter, and an antipyretic agent is used. Furthermore, a humectant, a preservative, a stabilizer, an antioxidant, a flavoring agent, or the like may be contained.

The injection as a parenteral agent includes a solution, a suspension, an emulsion, and a solid injection that is dissolved or suspended in a solvent at the time of use. The injection is used, for example, by dissolving, suspending or emulsifying the active ingredient in a solvent. As the solvent, for example, distilled water for injection, physiological saline, vegetable oil, propylene glycol, polyethylene glycol, alcohol such as ethanol, etc., and a combination thereof is used. Furthermore, the injection may contain a stabilizer, a solubilizer (for example, glutamic acid, aspartic acid, polysorbate 80 (registered trademark), or the like), a suspending agent, an emulsifying agent, a soothing agent, a buffering agent, a preservative, an antioxidant, an isotonizing agent, a pH adjusting agent, an excipient, or the like. These preparations are sterilized in the final step or produced by aseptic manipulation. In addition, it is also possible to produce a sterile solid preparation, for example, a freeze-dried product, and dissolve the solid preparation in sterilized or sterile distilled water for injection or another solvent before use.

### EXAMPLES

Hereinafter, a detailed description is made of the present invention with reference to Examples, but the present invention is not limited thereto.

The solvent in parentheses shown at the point of separation by chromatography and in TLC indicate an elution solvent or developing solvent used, and the proportion represents a volume ratio.

Conditions used in LC/MS analysis in the following Examples are shown below. Column: YMC Triart C18, 2.0 mm x 30 mm, 1.9 µm; Flow rate: 1.0 mL/min; Temperature: 30°C; Mobile phase A: 0.1% aqueous TFA (trifluoroacetic acid) solution; Mobile phase B: 0.1% TFA acetonitrile solution: gradient (ratio of mobile phase A : mobile phase B is described): 0 to 0.10 min (95 : 5), 0.10 to 1.20 min (95 : 5 to 5 : 95), 1.20 min to 1.50 min (5 : 95).

The parenthesis shown in the NMR part indicates a solvent used for the measurement.

The compound name used herein is generally named using a computer program ACD/Name (registered trademark) from Advanced Chemistry Development, which performs naming in compliance with the IUPAC nomenclature, or named in compliance with the IUPAC nomenclature.

Reference Example 1: (1R,2R,3S,4R)-3-({[dimethyl(2-methyl-2-propanyl)silyl]oxy}methyl)-2-(1-propen-1-yl)-4-(tetrahydro-2H-pyran-2-yloxy)cyclopentylformate

To a solution of (1S,2R,3S,4R)-3-[[tert-butyl(dimethyl)silyl]oxymethyl]-2-prop-1-enyl-4-tetrahydropyran-2-yloxy-2-cyclopentanol (CAS No.: 1262874-63-4, 7.2 g) in tetrahydrofuran (hereinafter, THF) (5.8 mL) were added triphenylphosphine (10 g) and formic acid (1.5 mL), followed by cooling to -30°C. To the reaction solution was added diethyl azodicarboxylate (18 mL), followed by stirring for 3 hours under ice cooling. The mixture was washed with a saturated aqueous sodium bicarbonate solution, water, and saturated saline, dried over sodium sulfate, and then filtered. The filtrate was concentrated under reduced pressure. The obtained residue was purified by silica gel column chromatography (hexane : ethyl acetate = 97 : 3→90 : 10) to yield the title compound (7.7 g) having the following physical properties.

TLC: Rf 0.85 (hexane : ethyl acetate = 2 : 1).

### Reference Example 2: (1R,2R,3S,4R)-3-({[dimethyl(2-methyl-2-propanyl)silyl]oxy}methyl)-2-(1-propen-1-yl)-4-(tetrahydro-2H-pyran-2-yloxy)cyclopentanol

To a solution of the compound (7.7 g) produced in Reference Example 1 in ethanol (50 mL) was added potassium carbonate (2.67 g), followed by stirring at room temperature for 1 hour. To the reaction mixture was added ethyl acetate, followed by filtering and then washing with ethyl acetate. The obtained filtrate was concentrated under reduced pressure, followed by dilution with ethyl acetate, addition of water, and extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 95 : 5→85 : 15) to yield the title compound (6.0 g) having the following physical properties.

TLC: Rf 0.60 (hexane : ethyl acetate = 2 : 1).

### Reference Example 3: 3-bromo-2-fluoro-6-vinylphenol

To a solution of methyltriphenylphosphonium bromide (2.45 g) in THF (12 mL) was added potassium t-butoxide (666 mg) at 0°C, followed by stirring at room temperature for 1 hour. After cooling to 0°C, a solution of 4-bromo-3-fluoro-2-hydroxy-benzaldehyde (CAS No.: 1427373-29-2, 1.0 g) in THF (15 mL) was added, followed by stirring at 0°C for 1 h. To the reaction mixture was added a 1 N aqueous hydrochloric acid solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 100 : 0→85 : 15) to yield the title compound (1.1 g) having the following physical properties.

TLC: Rf 0.44 (hexane : ethyl acetate = 6 : 4);

¹H-NMR (CDCl₃): δ 7.10, 7.03, 6.91, 5.82, 5.47, 5.38.

### Reference Example 4: {[(1S,2R,3S,5R)-3-(3-bromo-2-fluoro-6-vinylphenoxy)-2-(1-propen-1-yl)-5-(tetrahydro-2H-pyran-2-yloxy)cyclopentyl]methoxy}(dimethyl)(2-methyl-2-propanyl)silane

To a solution of the compound (1.7 g) produced in Reference Example 2 and the compound (1.0 g) produced in Reference Example 3 in THF (17 mL) was added triphenylphosphine (1.7 g), followed by cooling under ice cooling. To the reaction solution was added diethyl azodicarboxylate (2.9 mL), followed by stirring for 3 hours at room temperature. The reaction solution was concentrated under reduced pressure. The obtained residue was purified by silica gel column chromatography (hexane : ethyl acetate = 100 : 0→90 : 10) to yield the title compound (2.39 g) having the following physical properties.

TLC: Rf 0.79 (hexane : ethyl acetate = 9 : 1);

¹H-NMR (CDCl₃): δ 7.12-7.24, 5.62-5.74, 5.46-5.61, 5.27, 4.68-4.78, 4.56, 4.27-4.36, 4.16, 3.83-3.93, 3.69-3.79, 3.54-3.68, 3.38-3.51, 2.57-2.63, 2.45-2.52, 2.09-2.33, 1.91-2.04, 1.76-1.89, 1.41-1.74, 1.23-1.33, 0.84-0.94, 0.01-0.07.

### Reference Example 5: {[(1S,2R,3aS,10aR)-6-bromo-5-fluoro-2-(tetrahydro-2H-pyran-2-yloxy)-2,3,3a, 10a-tetrahydro-1H-benzo[b]cyclopenta[f]oxepin-1-yl]methoxy}(dimethyl)(2-methyl-2-propanyl)silane

A solution of the compound (2.39 g) produced in Reference Example 4 in toluene (83.7 mL) was stirred at 100°C for 30 minutes under a nitrogen stream. After the temperature was lowered to 80°C, tricyclohexylphosphine[1,3-bis(2,4,6-trimethylphenyl)imidazol-2-ylidene][3-phenyl-1H-inden-1-ylidene]ruthenium(II) dichloride (397 mg) was added, and the reaction solution was stirred at 80°C for 100 minutes. The reaction solution was concentrated under reduced pressure. The obtained residue was purified by silica gel column chromatography (hexane : ethyl acetate = 100 : 0→90 : 10) to yield the title compound (2.21 g) having the following physical properties.

TLC: Rf 0.64 (hexane : ethyl acetate = 9 : 1);

¹H-NMR (CDCl₃): δ 7.14, 6.81, 6.30, 6.02, 4.70, 4.21-4.28, 3.96-4.08, 3.81-3.91, 3.48-3.57, 2.72, 2.51-2.66, 2.31-2.47, 2.15-2.28, 1.80-1.90, 1.46-1.78, 1.22-1.35, 0.86-0.93, 0.03-0.11.

### Reference Example 6: ethyl (1S,2R,3aS,10aR)-1-({[dimethyl(2-methyl-2-propanyl)silyl]oxy}methyl)-5-fluoro-2-(tetrahydro-2H-pyran-2-yloxy)-2,3,3a,10a-tetrahydro-1H-benzo[b]cyclopenta[f]oxepine-6-carboxylate

To a solution of the compound (2.06 g) produced in Reference Example 5 in ethanol (12.4 mL)/dimethylformamide (hereinafter, DMF) (6.18 mL) was added diisopropylamine (1.69 mL). Under a nitrogen stream, [1,1-bis(diphenylphosphino)ferrocene]palladium(II) dichloride dichloromethane adduct (319 mg) was added, followed by stirring at 90°C for 19 hours under a carbon monoxide stream. The reaction solution was cooled to room temperature, poured into a cooled saturated aqueous ammonium chloride solution, and then extracted with an ethyl acetate/n-hexane mixed solution. The organic layer was washed with saturated saline, dried over sodium sulfate, and then filtered. The filtrate was concentrated under reduced pressure. The obtained residue was purified by silica gel column chromatography (hexane : ethyl acetate = 95 : 5→75 : 25) to yield the title compound (1.65 g) having the following physical properties.

TLC: Rf 0.43 (hexane : ethyl acetate = 9 : 1);

¹H-NMR (CDCl₃): δ 7.53, 6.95, 6.36, 6.12, 4.68-4.72, 4.35-4.41, 4.19-4.28, 3.96-4.08, 3.82-3.91, 3.47-3.57, 2.76, 2.66, 2.56, 2.32-2.48, 2.15-2.32, 1.47-1.90, 1.39, 1.22-1.33, 0.86-0.93, 0.05-0.11.

### Reference Example 7: ethyl (1S,2R, 3aS,10aR)-1-({[dimethyl(2-methyl-2-propanyl)silyl]oxy}methyl)-5-fluoro-2-(tetrahydro-2H-pyran-2-yloxy)-2,3,3a,9,10,10a-hexahydro-1H-benzo[b]cyclopenta[f]oxepine-6-carboxylate

To a solution of the compound (1.65 g) produced in Reference Example 6 in ethanol (25.1 mL) was added 10% palladium carbon (165 mg), followed by stirring at room temperature for 3 hours under a hydrogen stream. The reaction solution was diluted with ethyl acetate and then filtered. The filtrate was concentrated under reduced pressure. The obtained crude product was subjected to the next reaction without subsequent purification.

TLC: Rf 0.87 (hexane : ethyl acetate = 4 : 6);

¹H-NMR (CDCl₃): δ 7.45, 6.86, 4.70, 4.32-4.41, 4.15-4.22, 3.85-4.00, 3.67-3.81, 3.47-3.56, 3.01-3.12, 2.78, 2.53, 2.25-2.43, 1.97-2.23, 1.81-1.93, 1.47-1.81, 1.38, 1.23-1.29, 0.87-0.93, 0.04-0.09.

### Reference Example 8: ethyl (1S,2R,3aS,10aR)-5-fluoro-1-(hydroxymethyl)-2-(tetrahydro-2H-pyran-2-yloxy)-2,3,3a,9,10,10a-hexahydro-1H-benzo[b]cyclopenta[f]oxepine-6-carboxylate

To a solution of the crude product produced in Reference Example 7 in THF (11.6 mL) was added a 1 M solution of tetrabutylammonium fluoride in THF (4.8 mL) at room temperature, followed by stirring at room temperature overnight. To the reaction solution was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 40 : 60→0 : 100) to yield the title compound (1.20 g) having the following physical properties.

TLC: Rf 0.36 (hexane : ethyl acetate = 4 : 6);

¹H-NMR (CDCl₃): δ 7.45, 6.85, 4.77-4.80, 4.60-4.64, 4.37, 4.07-4.17, 3.83-4.00, 3.62-3.73, 3.46-3.61, 3.01-3.12, 2.92, 2.81, 2.48-2.62, 2.24-2.42, 2.10-2.24, 1.98-2.10, 1.70-1.95, 1.49-1.70, 1.38.

### Reference Example 9: ethyl (1R,2R,3aS,10aR)-5-fluoro-1-formyl-2-(tetrahydro-2H-pyran-2-yloxy)-2,3,3a,9,10,10a-hexahydro-1H-benzo[b]cyclopenta[f]oxepine-6-carboxylate

To a solution of the compound (500 mg) produced in Reference Example 8 in dichloromethane (5 mL) were added dimethyl sulfoxide (hereinafter, DMSO) (1 mL) and triethylamine (854 µL). The reaction solution was cooled to 0°C. To the reaction solution was added a sulfur trioxide-pyridine complex (584 mg), followed by stirring at room temperature for 2 hours. To the reaction solution was added a saturated aqueous ammonium chloride solution, followed by extraction with ethyl acetate. The organic layer was washed with saturated saline, dried over magnesium sulfate, and then filtered. The filtrate was concentrated under reduced pressure. The obtained residue was purified by silica gel column chromatography (hexane : ethyl acetate = 3 : 1) to yield the title compound (446 mg) having the following physical properties.

TLC: Rf 0.40 (hexane : ethyl acetate = 2 : 1);

¹H-NMR (CDCl₃): δ 9.84, 7.48, 6.87, 4.67, 4.32-4.48, 3.78-3.94, 3.43-3.55, 3.06-3.33, 2.72-2.82, 2.51-2.64, 2.28-2.40, 2.16-2.22, 2.00-2.08, 1.68-1.94, 1.47-1.66, 1.38.

### Reference Example 10: methyl (2S)-2-(2,4-difluorophenyl)propanoate

To a solution of (2S)-2-(2,4-difluorophenyl)propanoic acid (CAS No.: 1630485-27-6, 6.50 g) in methanol (100 mL) was added dropwise a solution of 2 M trimethylsilyldiazomethane in n-hexane (82.3 mL) at 0°C. The reaction mixture was stirred at 0°C for 1 h, and then stirred at room temperature for 30 min. To the reaction mixture was added acetic acid, followed by concentration under reduced pressure. The obtained residue was purified by silica gel column chromatography (hexane : ethyl acetate = 100 : 0-83 : 17) to yield the title compound (6.08 g) having the following physical properties.

TLC: Rf 0.50 (hexane : ethyl acetate = 5 : 1);

¹H-NMR (CDCl₃): δ 7.24-7.29, 6.78-6.86, 3.98, 3.69, 1.49.

### Reference Example 11: dimethyl[(3S)-3-(2,4-difluorophenyl)-2-oxobutyl]phosphonate

To a solution of dimethyl methylphosphonate (7.85 g) in THF (50 mL) was added dropwise a solution of 1.55 M n-butyllithium in n-hexane (38.9 mL) at -60°C, followed by stirring at -78°C for 1 hour. To the reaction mixture was added dropwise a solution of the compound (6.05 g) produced in Reference Example 10 in THF (50 mL) at -78°C, followed by stirring for 2 hours. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 50 : 50→0 : 100) to yield the title compound (4.74 g) having the following physical properties.

TLC: Rf 0.41 (ethyl acetate);

¹H-NMR (CDCl₃): δ 7.13-7.17, 6.83-6.89, 4.25, 3.74-3.80, 3.10-3.19, 2.90-2.99, 1.41.

### Reference Example 12: (4S)-4-benzyl-3-[(2R)-2-(2,4-difluorophenyl)propanoyl]-1,3-oxazolidin-2-one

To a solution of 2-(2,4-difluorophenyl)propanoic acid (CAS No.: 1250572-63-4, 1 g) in dichloromethane (17.7 mL) was added (4S)-4-benzyloxazolidin-2-one (CAS No.: 180917-48-0, 1.43 g). Furthermore, 1-ethyl-3-(3-dimethylaminopropyl)carbodiimide hydrochloride (1.54 g), 4-dimethylaminopyridine (131 mg) and triethylamine (1.5 mL) were added at 0°C, followed by stirring at room temperature overnight. To the reaction mixture was added a 1 N aqueous hydrochloric acid solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 90 : 10→65 : 35) to yield the title compound (1.13 g, high polarity material) having the following physical properties.

¹H-NMR (CDCl₃): δ 7.22-7.35, 7.16, 6.79-6.93, 5.20, 4.73, 4.21, 4.12, 3.32, 2.63, 1.51.

### Reference Example 13: (2R)-2-(2,4-difluorophenyl)propanoic acid

To a solution of the compound (1.13 g) produced in Reference Example 12 in THF (15 mL) were added a 30% hydrogen peroxide aqueous solution (2.8 mL) and an aqueous solution of lithium hydroxide (157 mg) (10 mL) at 0°C, followed by stirring at room temperature for 3 hours. To the reaction mixture was added a saturated aqueous sodium thiosulfate solution, followed by addition of a 1 N aqueous hydrochloric acid solution and extraction with ethyl acetate. The organic layer was washed with saturated saline, dried over magnesium sulfate, and then filtered. The filtrate was concentrated under reduced pressure. The obtained residue was purified by silica gel column chromatography (hexane : ethyl acetate = 80 : 20→60 : 40) to yield the title compound (861 mg) having the following physical properties.

¹H-NMR (CDCl₃): δ 7.23-7.34, 6.76-6.92, 4.02, 1.51.

### Reference Example 14: Dimethyl[(3R)-3-(2,4-difluorophenyl)-2-oxobutyl]phosphonate

A procedure for a purpose similar to that for Reference Example 10 → Reference Example 11 was performed using the compound (610 mg) produced in Reference Example 13 in place of (2S)-2-(2,4-difluorophenyl)propanoic acid to yield the title compound (614 mg) having the following physical properties.

¹H-NMR (CDCl₃): δ 1.40, 2.94, 3.15, 3.72-3.83, 4.25, 6.80-6.93, 7.11-7.21.

### Reference Example 15: ethyl (1R,2R,3aS,10aR)-1-[(1E,4S)-4-(2,4-difluorophenyl)-3-oxo-1-penten-1-yl]-5-fluoro-2-(tetrahydro-2H-pyran-2-yloxy)-2,3,3a,9,10,10a-hexahydro-1H-benzo[b]cyclopenta[f]oxepine-6-carboxylate

To a solution of the compound (80 mg) produced in Reference Example 9 in THF (1 mL) were added the compound (115 mg) produced in Reference Example 11, lithium chloride (18 mg) and triethylamine (60 µL), followed by stirring at room temperature overnight. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 10 : 1→75 : 25) to yield the title compound (117 mg) having the following physical properties.

¹H-NMR (CDCl₃): δ 7.46, 7.14, 6.76-6.88, 6.18-6.31, 4.65, 4.20-4.43, 4.04-4.10, 3.80-3.95, 3.48-3.58, 3.37-3.47, 3.28-3.35, 2.82-3.05, 2.53-2.74, 2.16, 1.99-2.06, 1.86-1.94, 1.63-1.83, 1.35-1.53.

### Reference Example 16: ethyl (1R,2R,3aS,10aR)-1-[(1E,4S)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylate

To a solution of the compound (117 mg) produced in Reference Example 15 in methanol (2 mL) was added p-toluenesulfonic acid monohydrate (8 mg) at 0°C, followed by stirring at room temperature for 1 hour. To the reaction mixture was added triethylamine (14 µL), followed by stirring at room temperature for 5 minutes. Then, cerium chloride (151 mg) and sodium tetrahydroboron (19 mg) were added at 0°C, followed by stirring at room temperature for 1 hour. The reaction mixture was concentrated, followed by addition of a 1 N aqueous hydrochloric acid solution and extraction with ethyl acetate. The organic layer was washed with saturated saline, dried over sodium sulfate, and then filtered. The filtrate was concentrated under reduced pressure. The obtained residue was purified by silica gel column chromatography (Gold High Performance Silica manufactured by ISCO, hexane : ethyl acetate = 60 : 40→55 : 45) to yield a mixture containing two diastereomers.

TLC: Rf 0.45, 0.40 (hexane : ethyl acetate = 2 : 3);

¹H-NMR (CDCl₃): δ 7.45-7.51, 7.18-7.31, 6.76-6.89, 5.58-5.69, 5.39-5.56, 4.25-4.40, 3.91, 3.74-3.80, 3.15-3.24, 3.03, 2.63-2.75, 2.42-2.61, 2.00-2.12, 1.71-2.00, 1.61, 1.33-1.40.

### Example 1: (1R,2R,3aS,10aR)-1-[(1E,3R,4S)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

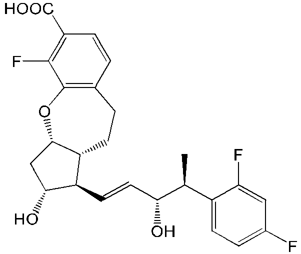

The diastereomer mixture produced in Reference Example 16 was dissolved in dimethoxyethane (0.5 mL)/methanol (1 mL), followed by addition of a 2 N sodium hydroxide aqueous solution (0.5 mL) at room temperature. The reaction mixture was stirred at room temperature for 15 hours. The reaction mixture was concentrated under reduced pressure, followed by addition of 1 N hydrochloric acid and extraction with ethyl acetate. The organic layer was washed with saturated saline, dried over magnesium sulfate, and then filtered. The filtrate was concentrated under reduced pressure. The obtained residue was separated into two diastereomers using an SFC column (CHIRALPAK ID, CO₂ : Methanol = 82 : 18) to yield a high polarity material (17.5 mg). The steric configuration was identified by single crystal X-ray.
HPLC retention time (min): 0.93;
MS (ESI, Pos.): 445 (M+H-H₂O)+.;
¹H-NMR (CD₃OD): δ 7.45, 7.34-7.41, 6.84-6.97, 5.63, 5.49, 4.35, 4.23, 3.87, 3.19, 3.05, 2.80, 2.54-2.67, 1.73-1.96, 1.26.

### Reference Example 17: ethyl (1R,2R,3aS,10aR)-1-[(1E,4R)-4-(2,4-difluorophenyl)-3-oxo-1-penten-1-yl]-5-fluoro-2-(tetrahydro-2H-pyran-2-yloxy)-2,3,3a,9,10,10a-hexahydro-1H-benzo[b]cyclopenta[f]oxepine-6-carboxylate

To a solution of the compound (80 mg) produced in Reference Example 9 in THF (1 mL) were added the compound (115 mg) produced in Reference Example 14, lithium chloride (18 mg) and triethylamine (60 µL), followed by stirring at room temperature overnight. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 10 : 1→75 : 25) to yield the title compound (103 mg) having the following physical properties.

¹H-NMR (CDCl₃): δ 7.46, 7.15, 6.73-6.89, 6.18-6.31, 4.64-4.66, 4.23-4.41, 4.05-4.11, 3.81-3.94, 3.57, 3.40-3.47, 3.33, 2.90-3.02, 2.81-2.90, 2.54-2.71, 2.13-2.21, 2.05, 1.62-1.98, 1.22-1.61.

### Reference Example 18: ethyl (1R,2R,3aS,10aR)-1-[(1E,3ξ,4R)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylate

To a solution of the compound (103 mg) produced in Reference Example 17 in methanol (2 mL) was added p-toluenesulfonic acid monohydrate (7 mg) at 0°C, followed by stirring at room temperature for 1 hour. To the reaction mixture was added triethylamine (13 µL), followed by stirring at room temperature for 5 minutes. Then, cerium chloride (133 mg) and sodium tetrahydroboron (17 mg) were added at 0°C, followed by stirring at room temperature for 1 hour. The reaction mixture was concentrated under reduced pressure, followed by addition of a 1 N aqueous hydrochloric acid solution and extraction with ethyl acetate. The organic layer was washed with saturated saline, dried over sodium sulfate, and then filtered. The filtrate was concentrated under reduced pressure. The obtained residue was purified by silica gel column chromatography (Gold High Performance Silica manufactured by ISCO, hexane : ethyl acetate = 60 : 40→55 : 45) to be separated into two diastereomers, thereby yielding a high polarity material (20.6 mg).

¹H-NMR (CDCl₃): δ 7.48, 7.16-7.23, 6.74-6.88, 5.58, 5.37, 4.37, 4.21-4.30, 3.82-3.92, 3.16, 2.95, 2.39-2.63, 1.95-2.13, 1.80, 1.42-1.76, 1.33-1.41.

### Example 2: (1R,2R,3aS,10aR)-1-[(1E,3ξ,4R)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

To a solution of the compound (high polarity materiam: 20.6 mg) produced in Reference Example 18 in DME (0.5 mL)/methanol (1 mL) was added a 2 N aqueous sodium hydroxide solution (0.5 mL), followed by stirring at room temperature for 15 hours. The reaction mixture was concentrated under reduced pressure, followed by addition of 1 N hydrochloric acid and extraction with ethyl acetate. The organic layer was washed with saturated saline, dried over magnesium sulfate, and then filtered. The filtrate was concentrated under reduced pressure to yield the title compound (14.6 mg) having the following physical properties.
HPLC retention time (min): 0.88;
MS (ESI, Pos.): 445 (M+H-H₂O)+.;
¹H-NMR (CD₃OD): δ 7.39-7.46, 7.29-7.38, 6.88-6.96, 5.51, 5.29, 4.24-4.29, 4.12-4.19, 3.75-3.83, 3.10, 2.89, 2.54-2.65, 2.44, 1.88, 1.64, 1.23-1.46.

### Reference Example 19 (1): (4S)-4-benzyl-3-[(2S)-2-(2-fluorophenyl)propanoyl]-1,3-oxazolidin-2-one

### Reference Example 19 (2): (4S)-4-benzyl-3-[(2R)-2-(2-fluorophenyl)propanoyl]-1,3-oxazolidin-2-one

To a solution of 2-(2-fluorophenyl)propanoic acid (CAS No.: 73041-90-4, 2.5 g) in dichloromethane (49 mL) was added (4S)-4-benzyloxazolidin-2-one (3.95 g). 1-Ethyl-3-(3-dimethylaminopropyl)carbodiimide hydrochloride (4.27 g), 4-dimethylaminopyridine (363 mg) and triethylamine (4.14 mL) were added at 0°C, followed by stirring at room temperature overnight. To the reaction mixture was added a 1 N aqueous hydrochloric acid solution, followed by extraction with dichloromethane and ethyl acetate. The organic layer was washed with saturated saline, dried over magnesium sulfate, and then filtered. The filtrate was concentrated under reduced pressure. The obtained residue was purified by silica gel column chromatography (hexane : ethyl acetate = 90 : 10→65 : 35) to yield the title compounds (low polarity material: 1.76 g, high polarity material: 2.93 g) having the following physical properties.

Low polarity material: ¹H-NMR (CDCl₃): δ 7.20-7.37, 7.12, 7.04, 5.26, 4.63-4.70, 4.12-4.18, 3.35, 2.81, 1.57.

High polarity material: ¹H-NMR (CDCl₃): δ 7.23-7.34, 7.05-7.18, 5.25, 4.74, 4.09-4.22, 3.34, 2.63, 1.53.

### Reference Example 20: dimethyl[(3S)-3-(2-fluorophenyl)-2-oxobutyl]phosphonate

A procedure for a purpose similar to that for Reference Example 13→ Reference Example 10→ Reference Example 11 was performed using the compound (low polarity material: 1.76 g) produced in Reference Example 19(1) in place of the compound produced in Reference Example 12 to yield the title compound (941 mg) having the following physical properties.

¹H-NMR (CDCl₃): δ 7.24-7.30, 7.06-7.21, 4.28, 3.77, 3.11-3.22, 2.87-2.97, 1.39-1.44.

### Reference Example 21: dimethyl[(3R)-3-(2-fluorophenyl)-2-oxobutyl]phosphonate

A procedure for a purpose similar to that for Example 13→ Example 10→ Example 11 was performed using the compound (high polarity material: 2.93 g) produced in Reference Example 19 (2) in place of the compound produced in Reference Example 12 to yield the title compound (970 mg) having the following physical properties.

¹H-NMR (CDCl₃): δ 7.24-7.31, 7.06-7.21, 4.24-4.32, 3.77, 3.12-3.22, 2.87-2.98, 1.41.

### Reference Example 22: ethyl (1R,2R,3aS,10aR)-5-fluoro-1-[(1E,4S)-4-(2-fluorophenyl)-3-oxo-1-penten-1-yl]-2-(tetrahydro-2H-pyran-2-yloxy)-2,3,3a,9,10,10a-hexahydro-1H-benzo[b]cyclopenta[f]oxepine-6-carboxylate

To a solution of the compound (80 mg) produced in Reference Example 9 in THF (1 mL) were added the compound (108 mg) produced in Reference Example 20, lithium chloride (18 mg) and triethylamine (60 µL), followed by stirring at room temperature overnight. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 10 : 1→75 : 25) to yield the title compound (105 mg) having the following physical properties.

TLC: Rf 0.65 (hexane : ethyl acetate = 2 : 1);

¹H-NMR (CDCl₃): δ 7.45, 7.02-7.29, 6.75-6.86, 6.18-6.32, 4.65, 4.24-4.41, 4.04-4.10, 3.91, 3.79-3.86, 3.48-3.56, 3.37-3.44, 3.27-3.32, 2.79-3.03, 2.50-2.74, 2.15, 1.98-2.11, 1.88, 1.72-1.84, 1.22-1.72.

### Reference Example 23: ethyl (1R,2R,3aS,10aR)-5-fluoro-1-[(1E,3R,4S)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylate

To a solution of the compound (105 mg) produced in Reference Example 22 in methanol (2 mL) was added p-toluenesulfonic acid monohydrate (7 mg) at 0°C, followed by stirring at room temperature for 1 hour. To the reaction mixture was added triethylamine (13 µL), followed by stirring at room temperature for 5 minutes. Then, cerium chloride (140 mg) and sodium tetrahydroboron (18 mg) were added at 0°C, followed by stirring at room temperature for 1 hour. The reaction mixture was concentrated under reduced pressure, followed by addition of a 1 N aqueous hydrochloric acid solution and extraction with ethyl acetate. The organic layer was washed with saturated saline, dried over sodium sulfate, and then filtered. The filtrate was concentrated under reduced pressure. The obtained residue was purified by silica gel column chromatography (Gold High Performance Silica manufactured by ISCO, hexane : ethyl acetate = 60 : 40→55 : 45) to be separated into two diastereomers, thereby yielding a high polarity material (18.6 mg).

¹H-NMR (CDCl₃): δ 7.48, 7.00-7.35, 6.87, 5.68, 5.50, 4.29-4.40, 3.85-3.93, 3.25, 3.06, 2.62-2.75, 2.49, 1.99-2.11, 1.72-1.98, 1.56-1.61, 1.38, 1.31.

### Example 3: (1R,2R,3aS,10aR)-5-fluoro-1-[(1E)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

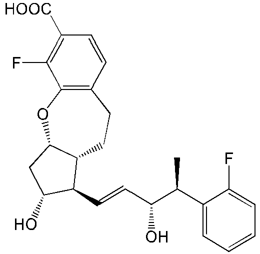

To a solution of the compound (high polarity material: 18.6 mg) produced in Reference Example 23 in dimethoxyethane (0.5 mL)/methanol (1 mL) was added a 2 N aqueous sodium hydroxide solution (0.5 mL). The reaction mixture was stirred at room temperature overnight. The reaction mixture was concentrated under reduced pressure, followed by addition of 1 N hydrochloric acid and extraction with ethyl acetate. The organic layer was washed with saturated saline, dried over magnesium sulfate, and then filtered. The filtrate was concentrated under reduced pressure to yield the title compound (13.1 mg) having the following physical properties. The steric configuration was identified by single crystal X-ray.
HPLC retention time (min): 0.91;
MS (ESI, Pos.): 427 (M+H-H₂O)+.;
¹H-NMR (CD₃OD): δ 7.44, 7.35, 7.16-7.23, 7.11, 7.01, 7.01, 6.93, 5.63, 5.46, 4.33, 4.26, 3.85, 3.19-3.27, 3.04, 2.78, 2.53-2.65, 1.81-1.94, 1.69-1.79, 1.26.

### Reference Example 24: ethyl (1R,2R,3aS,10aR)-5-fluoro-1-[(1E,4R)-4-(2-fluorophenyl)-3-oxo-1-penten-1-yl]-2-(tetrahydro-2H-pyran-2-yloxy)-2,3,3a,9,10,10a-hexahydro-1H-benzo[b]cyclopenta[f]oxepine-6-carboxylate

To a solution of the compound (80 mg) produced in Reference Example 9 in THF (1 mL) were added the compound (108 mg) produced in Reference Example 21, lithium chloride (18 mg) and triethylamine (60 µL), followed by stirring at room temperature overnight. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 10 : 1→75 : 25) to yield the title compound (114 mg) having the following physical properties.
TLC: Rf 0.65 (hexane : ethyl acetate = 2 : 1);
¹H-NMR (CDCl₃): δ 7.45, 7.04-7.28, 6.71-6.85, 6.17-6.32, 4.63-4.66, 4.28-4.40, 4.04-4.10, 3.80-3.92, 3.49-3.59, 3.38-3.46, 3.27, 2.79-3.00, 2.52-2.70, 2.15, 1.99-2.05, 1.33-1.92.

### Reference Example 25: ethyl (1R,2R,3aS,10aR)-5-fluoro-1-[(1E,3ξ,4R)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylate

To a solution of the compound (117 mg) produced in Reference Example 24 in methanol (2 mL) was added p-toluenesulfonic acid monohydrate (8 mg) at 0°C, followed by stirring at room temperature for 1 hour. To the reaction mixture was added triethylamine (14 µL), followed by stirring at room temperature for 5 minutes. Then, cerium chloride (151 mg) and sodium tetrahydroboron (19 mg) were added at 0°C, followed by stirring at room temperature for 1 hour. The reaction mixture was concentrated under reduced pressure, followed by addition of a 1 N aqueous hydrochloric acid solution and extraction with ethyl acetate. The organic layer was washed with saturated saline, dried over sodium sulfate, and then filtered. The filtrate was concentrated under reduced pressure. The obtained residue was purified by silica gel column chromatography (Gold High Performance Silica manufactured by ISCO, hexane : ethyl acetate = 60 : 40→55 : 45) to be separated into two diastereomers, thereby yielding a high polarity material (19 mg) was obtained.

¹H-NMR (CDCl₃): δ 7.47, 7.16-7.25, 7.06-7.13, 6.98-7.04, 6.85, 5.59, 5.37, 4.33-4.40, 4.37, 4.23-4.31, 3.81-3.89, 3.20, 2.93, 2.50-2.60, 2.43, 1.97-2.06, 1.75, 1.62-1.70, 1.34-1.54.

### Example 4: (1R,2R,3aS,10aR)-5-fluoro-1-[(1E,3ξ,4R)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

To a solution of the compound (high polarity material: 19 mg) produced in Reference Example 25 in dimethoxyethane (0.5 mL)/methanol (1 mL) was added a 2 N aqueous sodium hydroxide solution (0.5 mL). The reaction mixture was stirred at room temperature overnight. The reaction mixture was concentrated under reduced pressure, followed by addition of 1 N hydrochloric acid and extraction with ethyl acetate. The organic layer was washed with saturated saline, dried over magnesium sulfate, and then filtered. The filtrate was concentrated under reduced pressure to yield the title compound (14.1 mg) having the following physical properties.
HPLC retention time (min): 0.86;
MS (ESI, Pos.): 427 (M+H-H₂O)+.;
¹H-NMR (CD₃OD): δ 7.35-7.41, 7.26, 7.16-7.23, 7.07-7.13, 6.99-7.05, 6.87, 5.48, 5.23, 4.13-4.23, 3.71-3.78, 3.47, 3.24, 3.08, 2.77-2.86, 2.47-2.58, 2.35-2.43, 1.83, 1.55, 1.38, 1.10-1.34.

### Reference Example 26: Dimethyl {2-[1-(2-fluorophenyl)cyclobutyl]-2-oxoethyl}phosphonate

A procedure for a purpose similar to that for Reference Example 10→ Reference Example 11 was performed using 1-(2-fluorophenyl) cyclobutanecarboxylic acid (CAS No.: 151157-48-1, 10.4 g) in place of (2S)-2-(2,4-difluorophenyl)propanoic acid to yield the title compound (12.64 g) having the following physical properties.

¹H-NMR (CDCl₃): δ 7.25-7.34, 7.17-7.22, 7.01-7.08, 3.69-3.77, 2.99, 2.93, 2.79-2.82, 2.86, 2.41-2.50, 2.03, 1.83-1.94.

### Reference Example 27: dimethyl[2-oxo-2-(1-phenylcyclopropyl)ethyl]phosphonate

A procedure for a purpose similar to that for Reference Example 10→ Reference Example 11 was performed using 1-phenylcyclopropanecarboxylic acid (CAS No.: 6120-95-2, 3.0 g) in place of (2S)-2-(2,4-difluorophenyl)propanoic acid to yield the title compound (2.6 g) having the following physical properties.

¹H-NMR (CDCl₃): δ 7.30-7.42, 3.71-3.75, 3.05, 3.00, 1.71, 1.26-1.30.

### Reference Example 28: ethyl (2,6-difluorophenyl)(difluoro)acetate

To a solution of 1,3-difluoro-2-iodobenzene (CAS No.: 13697-89-7, 4.7 g) in DMSO (24 mL) were added ethyl bromodifluoroacetate (CAS No.: 667-27-6, 2.5 mL) and activated copper (3.2 g), followed by stirring at 65°C for 20 hours. To the reaction mixture was added tert-butyl methyl ether (hereinafter, MTBE) for separation of a DMSO layer. Then, a saturated aqueous ammonium chloride solution was added, followed by extraction with MTBE. The organic layer was washed with saturated saline, dried over sodium sulfate, and then filtered. The filtrate was concentrated under reduced pressure. The obtained residue was purified by silica gel column chromatography (hexane : ethyl acetate = 97 : 3→93 : 7) to yield the title compound (3.31 g) having the following physical properties.
TLC: Rf 0.36 (hexane : ethyl acetate = 9 : 1);
¹H-NMR (CDCl₃): δ 7.42-7.50, 6.95-7.02, 4.39, 1.35.

### Reference Example 29: dimethyl[3-(2,6-difluorophenyl)-3,3-difluoro-2-oxopropyl]phosphonate

A procedure for a purpose similar to that for Reference Example 11 was performed using the compound (3.31 g) produced in Reference Example 28 in place of the compound produced in Reference Example 10 to yield the title compound (4.02 g) having the following physical properties.
TLC: Rf 0.55 (ethyl acetate);
¹H-NMR (CDCl₃): δ 7.41-7.52, 6.98, 3.78-3.86, 3.43-3.54.

### Example 5: (1R,2R,3aS,10aR)-5-fluoro-1-{(1E,3ξ)-3-[1-(2-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

A procedure for a purpose similar to that for Reference Example 17→ Reference Example 18→ Example 2 was performed using the compound produced in Reference Example 26 in place of the compound produced in Reference Example 14 to yield the title compound having the following physical properties. As the intermediate corresponding to the compound produced in Reference Example 18, a high polarity compound separated by a diastereomer separation procedure was used.
HPLC retention time (min): 0.93;
MS (ESI, Pos.): 453 (M-OH)+.;
¹H-NMR (DMSO-d₆): δ 12.95-13.20, 7.33-7.43, 7.16-7.24, 7.09, 5.25-5.43, 4.94, 4.72, 4.27, 4.13-4.20, 3.62-3.72, 2.70-2.98, 2.54-2.62, 2.34-2.44, 2.20-2.31, 1.99, 1.41-1.81.

### Example 5-1 to 5-21:

A procedure for a purpose similar to that for Reference Example 17→ Reference Example 18→ Example 2 was performed using a corresponding phosphonate in place of the compound produced in Reference Example 14 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 10→ Reference Example 11.

### Example 5-1: (1R,2R,3aS,10aR)-5-fluoro-2-hydroxy-1-[(1E,3ξ)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

HPLC retention time (min): 0.88;
MS (ESI, Pos.): 421 (M+H-H₂O)+.;
¹H-NMR (CD₃OD): δ 7.37-7.47, 7.28, 7.18-7.22, 6.96, 5.56, 5.40, 4.32, 3.81-3.92, 3.05, 2.77, 2.51-2.65, 1.78-1.95, 1.61-1.77, 0.89-1.02, 0.71-0.83.

### Example 5-2: (1R,2R,3aS,10aR)-5-fluoro-2-hydroxy-1-{(1E,3ξ)-3-hydroxy-3-[1-(2-methylphenyl)cyclopropyl]-1-propen-1-yl}-2,3,3a,9,10,10a-hexahydro-1H-benzo[b]cyclopenta[f]oxepine-6-carboxylic acid

HPLC retention time (min): 0.94;
MS (ESI, Pos.): 435 (M+H-H₂O)+.;
¹H-NMR (CD₃OD): δ 7.43, 7.25-7.30, 7.07-7.15, 6.95, 5.60, 5.32, 4.30, 4.11-4.15, 3.78-3.86, 3.03, 2.69-2.82, 2.50-2.64, 2.48, 1.91, 1.69-1.82, 1.63, 1.05-1.15, 0.73-0.80, 0.65.

### Example 5-3: (1R,2R,3aS,10aR)-1-{(1E,3ξ)-3-[1-(2-chlorophenyl)cyclobutyl]-3-hydroxy-1-propen-1-yl}-5-fluoro-2-hydroxy-2,3,3a,9,10,10a-hexahydro-1H-benzo[b]cyclopenta[f]oxepine-6-carboxylic acid

HPLC retention time (min): 0.99;
MS (ESI, Pos.): 469, 471 (M+H-H₂O, M+H+2-H₂O)+.;
¹H-NMR (CD₃OD): δ 7.37-7.44, 7.15-7.32, 6.90-6.95, 5.24-5.92, 4.56, 4.29, 3.83, 2.85-3.10, 2.48-2.74, 1.99-2.13, 1.91, 1.79, 1.24-1.39.

### Example 5-4: (1R,2R,3aS,10aR)-5-fluoro-1-[(1E,3ξ)-4-(4-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.88;
MS (ESI, Pos.): 427 (M+H-H₂O)+.;
¹H-NMR (CD₃OD): δ 7.42, 7.28, 6.99-7.05, 6.94, 5.64, 5.46-5.52, 4.37, 4.17, 3.85-3.91, 3.05, 2.76-2.91, 2.56-2.68, 1.85-1.97, 1.79, 1.27.

### Example 5-5: (1R,2R,3aS,10aR)-5-fluoro-1-[(1E,3ξ,4ξ)-4-(4-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.87;
MS (ESI, Pos.): 427 (M+H-H₂O)+.;
¹H-NMR (CD₃OD): δ 7.26-7.49, 7.20-7.25, 7.02, 6.81-6.94, 5.45, 5.26, 4.25, 4.06-4.12, 3.79, 2.73-2.93, 2.59, 2.43, 1.81-1.92, 1.56-1.70, 1.37.

### Example 5-6: (1R,2R,3aS,10aR)-5-fluoro-1-[(1E,3ξ,4ξ)-4-(4-fluoro-2-methylphenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 423 (M+H-2H₂O)+.;
¹H-NMR (CD₃OD): δ 7.41-7.48, 7.24-7.33, 6.86-6.97, 5.69, 5.42-5.59, 4.38, 4.20, 3.85-3.92, 2.97-3.20, 2.82, 2.49-2.73, 2.38, 1.85-2.04, 1.69-1.85, 1.29-1.37, 1.22.

### Example 5-7: (1R,2R,3aS,10aR)-1-[(1E,3ξ,4ξ)-4-(2-chloro-4-fluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.96;
MS (ESI, Pos.): 461, 463 (M-H₂O, M+2-H₂O)+;
¹H-NMR (CD₃OD): δ 7.39-7.50, 7.20, 7.07, 6.94, 5.65, 5.49, 4.29-4.38, 3.80-3.92, 3.35-3.52, 3.00-3.10, 2.75-2.88, 2.54-2.69, 1.81-1.97, 1.70-1.81, 1.29-1.35, 1.26.

### Example 5-8: (1R,2R,3aS,10aR)-1-[(1E,3ξ,4ξ)-4-(2-chloro-4-fluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.93;
MS (ESI, Pos.): 461, 463 (M-H₂O, M+2-H₂O)+;
¹H-NMR (CD₃OD): δ 7.37-7.44, 7.24, 7.06, 6.91, 5.57, 5.27, 4.25, 4.15, 3.79, 3.35-3.45, 2.89, 2.53-2.64, 2.46, 1.88, 1.63, 1.37, 1.24-1.33.

### Example 5-9: (1R,2R,3aS,10aR)-5-fluoro-1-{(1E,3ξ)-3-[1-(2-fluoro-6-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.95;
MS (ESI, Pos.): 453 (M-OH)+.;
¹H-NMR (DMSO-d₆): δ 12.88-13.30, 7.33-7.41, 7.12, 6.95, 5.45-5.58, 5.20-5.33, 5.06-5.18, 4.80, 4.71, 4.21-4.37, 4.09, 3.78-3.95, 3.42, 2.84-2.96, 2.69-2.83, 2.57-2.65, 2.33-2.46, 2.16-2.30, 1.33-1.77, 0.93-1.31, 0.39-0.70.

### Example 5-10: (1R,2R,3aS,10aR)-5-fluoro-1-{(1E,3ξ)-3-[1-(5-fluoro-2-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.96;
MS (ESI, Pos.): 453 (M-OH)+.;
¹H-NMR (DMSO-d₆): δ 12.97-13.22, 7.35-7.42, 7.13, 6.90-7.01, 5.41, 5.13-5.26, 4.81, 4.74, 4.30, 3.94-4.02, 3.64, 2.86-2.98, 2.70-2.83, 2.52-2.60, 2.44, 2.35-2.39, 1.48-1.74, 1.00-1.09, 0.95, 0.64, 0.53.

### Example 5-11: (1R,2R,3aS,10aR)-5-fluoro-1-{(1E,3ξ)-3-[1-(3-fluoro-2-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.95;
¹H-NMR (CD₃OD): δ 7.42, 7.04-7.21, 6.88-7.02, 5.59, 5.34, 4.31, 3.99-4.22, 3.77-3.95, 2.90-3.10, 2.76, 2.46-2.68, 2.39, 1.91, 1.69-1.84, 1.64, 1.04-1.17, 0.72-0.86, 0.66.

### Example 5-12: (1R,2R,3aS,10aR)-5-fluoro-1-{(1E,3ξ)-3-[1-(4-fluoro-2-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 453 (M+H-H₂O)+, 435 (M+H-2H₂O)+.;
¹H-NMR (CD₃OD): δ 7.38-7.50, 7.03-7.27, 6.88-7.03, 5.50-5.74, 5.42, 4.33, 3.94-4.23, 3.83, 3.05, 2.71-2.90, 2.48-2.70, 2.29-2.45, 1.80-1.97, 1.58-1.80, 1.10, 0.63-0.81.

### Example 5-13: (1R,2R,3aS,10aR)-1-[(1E,3ξ,4ξ)-4-(2,3-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.93;
MS (ESI, Pos.): 445 (M-OH)+.;
¹H-NMR (DMSO-d₆): δ 12.91-13.24, 7.37, 7.10-7.26, 6.98, 5.38-5.54, 4.85, 4.77-4.83, 4.35, 4.06-4.12, 3.71, 3.04-3.14, 2.93, 2.82, 2.53-2.63, 2.34-2.46, 1.59-1.84, 1.13-1.28.

### Example 5-14: (1R,2R,3aS,10aR)-1-[(1E,3ξ,4ξ)-4-(2,3-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 445 (M-OH)+.;
¹H-NMR (DMSO-d₆): δ 12.81-13.30, 7.36, 7.14, 6.95, 5.34, 5.16, 5.00, 4.74, 4.25, 4.01, 3.61, 3.04, 2.72-2.83, 2.53-2.65, 2.43, 2.15-2.31, 1.44-1.72, 1.13-1.35.

### Example 5-15: (1R,2R,3aS,10aR)-1-[(1E,3ξ,4ξ)-4-(2,5-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.92;
MS (ESI, Pos.): 445 (M-OH)+.;
¹H-NMR (DMSO-d₆): δ 13.07, 7.37, 7.18, 7.15, 7.02-7.08, 6.98, 5.35-5.51, 4.86, 4.79, 4.35, 4.09, 3.70, 3.06, 2.89-3.00, 2.74-2.86, 2.52-2.56, 2.33-2.45, 1.59-1.82, 1.12-1.25.

### Example 5-16: (1R,2R,3aS,10aR)-1-[(1E,3ξ,4ξ)-4-(2,5-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.89;
MS (ESI, Pos.): 445 (M-OH)+.;
¹H-NMR (DMSO-d₆): δ 13.06, 7.36, 7.17, 7.02-7.09, 6.96, 5.37, 5.15-5.23, 4.97, 4.74, 4.26, 3.98-4.07, 3.62, 2.98, 2.77, 2.61, 2.52-2.56, 2.38-2.46, 2.13-2.31, 1.62-1.72, 1.54, 1.14-1.34.

### Example 5-17: (1R,2R,3aS,10aR)-1-[(1E,3ξ,4ξ)-4-(2,6-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 445 (M+H-H₂O)+.;
¹H-NMR (CD₃OD): δ 7.45, 7.23-7.31, 6.90-6.97, 5.47, 5.30, 4.36, 4.20-4.25, 3.73-3.81, 3.25, 2.80, 2.47-2.60, 2.38, 1.86, 1.50-1.59, 1.46, 0.97-1.23.

### Example 5-18: (1R,2R,3aS,10aR)-1-[(1E,3ξ,4ξ)-4-(2,6-difluorophenyl)-3-hydroxy-1-penten-1-yl]-5-fluoro-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 445 (M+H-H₂O)+.;
¹H-NMR (CD₃OD): δ 7.47, 7.20-7.28, 6.89-6.98, 5.71-5.77, 5.60-5.67, 4.34-4.43, 3.94, 3.24-3.31, 3.07-3.16, 2.87, 2.63-2.72, 1.93-2.03, 1.82-1.91, 1.30.

### Example 5-19: (1R,2R, 3aS,10aR)-5-fluoro-2-hydroxy-1-[(1E,3ξ,4ξ)-3-hydroxy-4-(2,4,6-trifluorophenyl)-1-penten-1-yl]-2,3,3a,9,10,10a-hexahydro-1H-benzo[b]cyclopenta[f]oxepine-6-carboxylic acid

HPLC retention time (min): 0.95;
MS (ESI, Pos.): 463 (M+H-H₂O)+.;
¹H-NMR (CD₃OD): δ 7.47, 6.97, 6.81, 5.60-5.80, 4.40, 4.32, 4.12, 3.93, 3.07-3.31, 2.87, 2.51-2.78, 1.78-2.05, 1.20-1.37.

### Example 5-20: (1R,2R,3aS,10aR)-1-{(1E,3ξ)-3-[1-(2,4-difluorophenyl)cyclobutyl]-3-hydroxy-1-propen-1-yl}-5-fluoro-2-hydroxy-2,3,3a,9,10,10a-hexahydro-1H-benzo[b]cyclopenta[f]oxepine-6-carboxylic acid

HPLC retention time (min): 1.116;
MS (ESI, Pos.): 471 (M+H-H₂O)+, 453 (M+H-2H₂O)+.;
¹H-NMR (CD₃OD): δ 7.42-7.46, 7.10-7.16, 6.94, 6.86, 6.78-6.85, 5.48-5.55, 5.39-5.48, 4.28-4.34, 3.80-3.86, 2.98-3.05, 2.70-2.79, 2.44-2.64, 2.32-2.43, 2.05-2.13, 1.73-1.93, 1.66.

### Example 5-21: (1R,2R,3aS,10aR)-5-fluoro-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.102;
MS (ESI, Pos.): 435 (M+H-2H₂O)+.;
¹H-NMR (CD₃OD): δ 7.43-7.47, 7.12-7.18, 6.94-7.02, 5.39-5.46, 5.31-5.38, 4.29-4.33, 4.24, 3.80-3.86, 2.99-3.07, 2.74-2.82, 2.47-2.64, 2.24-2.42, 2.00-2.08, 1.76-1.92, 1.66-1.75.

### Reference Example 30: ethyl (1R,2R,3aS,10aR)-1-[(1E)-4-(2,6-difluorophenyl)-4,4-difluoro-3-oxo-1-buten-1-yl]-5-fluoro-2-(tetrahydro-2H-pyran-2-yloxy)-2,3,3a,9,10,10a-hexahydro-1H-benzo[b]cyclopenta[f]oxepine-6-carboxylate

To a solution of the compound (309 mg) produced in Reference Example 29 in THF (0.5 mL) was added sodium hydride (17.5 mg, 60% oil suspension), followed by stirring at room temperature for 2 hours. To the reaction mixture were added a solution of the compound (100 mg) produced in Reference Example 9 in THF (0.5 mL) and a solution of zinc chloride in 2-methyltetrahydrofuran (0.37 mL), followed by stirring at 50°C overnight. To the reaction mixture was added a saturated aqueous ammonium chloride solution, followed by extraction with ethyl acetate. The organic layer was washed with saturated saline, dried over sodium sulfate, and then filtered. The filtrate was concentrated under reduced pressure. The obtained residue was purified by silica gel column chromatography (hexane : ethyl acetate = 95 : 5→80 : 20) to yield the title compound (114 mg) having the following physical properties.
TLC: Rf 0.43 (hexane : ethyl acetate = 7 : 3);
¹H-NMR (CDCl₃): δ 7.42-7.50, 7.22, 7.17, 6.98, 6.74-6.88, 4.69-4.72, 4.57, 4.37, 4.01-4.20, 3.85-3.92, 3.71, 3.39-3.51, 3.01-3.19, 2.60-2.82, 2.24, 1.91-2.12, 1.77-1.87, 1.56-1.77, 1.45-1.53, 1.38.

### Example 6: (1R,2R,3aS,10aR)-1-[(1E,3ξ)-4-(2,6-difluorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

A procedure for a purpose similar to that for Reference Example 18→ Example 2 was performed using the compound produced in Reference Example 30 in place of the compound produced in Reference Example 17 to yield the title compound exhibiting the following physical properties. As the intermediate corresponding to the compound produced in Reference Example 18, a high polarity compound separated by a diastereomer separation procedure was used.
HPLC retention time (min): 0.83;
MS (ESI, Pos.): 467 (M-OH)+.;
¹H-NMR (DMSO-d₆): δ 12.95-13.20, 7.54-7.66, 7.35-7.41, 7.20, 6.99, 6.01, 5.56-5.61, 4.86, 4.36-4.50, 4.31, 3.71, 2.84-2.98, 2.69-2.80, 2.53-2.63, 2.35-2.47, 1.73, 1.39-1.62.

### Example 6-1 to 6-17:

A procedure for a purpose similar to that for Reference Example 30→ Reference Example 18→ Example 2 was performed using a corresponding phosphonate in place of the compound produced in Reference Example 29 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 28→ Reference Example 29.

### Example 6-1: (1R,2R,3aS,10aR)-1-[(1E,3ξ)-4,4-difluoro-3-hydroxy-4-phenyl-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.86;
MS (ESI, Pos.): 431 (M+H-H₂O)+.;
¹H-NMR (CD₃OD): δ 7.40-7.55, 6.79-7.03, 5.57-5.65, 4.41-4.56, 4.19-4.40, 3.84-3.91, 3.01, 2.74, 2.55-2.66, 1.89-1.96, 1.81, 1.63.

### Example 6-2: (1R,2R,3aS,10aR)-1-[(1E,3ξ)-4,4-difluoro-4-(4-fluorophenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.87;
MS (ESI, Pos.): 449 (M-OH)+.;
¹H-NMR (DMSO-d₆): δ 12.88-13.32, 7.50-7.57, 7.28-7.40, 6.98, 5.84, 5.54, 5.45, 4.85, 4.37-4.51, 4.32, 3.70, 2.71-3.02, 2.54-2.63, 2.35-2.47, 1.74.

### Example 6-3: (1R,2R,3aS,10aR)-1-[(1E,3ξ)-4,4-difluoro-3-hydroxy-4-(4-methylphenyl)-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 445 (M-OH)+.;
¹H-NMR (DMSO-d₆): δ 12.85-13.34, 7.35, 7.26, 6.99, 5.76, 5.50-5.58, 5.43, 4.83, 4.36-4.46, 4.32, 3.66-3.76, 2.84-2.97, 2.70-2.81, 2.53-2.63, 2.35-2.45, 2.34, 1.44-1.80.

### Example 6-4: (1R,2R,3aS,10aR)-1-[(1E,3ξ)-4-(4-chlorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.92;
MS (ESI, Pos.): 465 (M-OH)+.;
¹H-NMR (DMSO-d₆): δ 13.07, 7.46-7.57, 7.37, 6.99, 5.87, 5.51-5.60, 5.43, 4.85, 4.44, 4.33, 3.70, 2.90, 2.79, 2.53-2.64, 2.45, 1.65-1.81, 1.46-1.65.

### Example 6-5: (1R,2R,3aS,10aR)-1-[(1E,3ξ)-4-(2,4-difluorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.86;
MS (ESI, Pos.): 467 (M+H-H₂O)+.;
¹H-NMR (CD₃OD): δ 7.57-7.64, 7.42, 7.07-7.14, 6.93, 5.63-5.74, 4.61, 4.31-4.35, 3.85-3.92, 2.96-3.04, 2.73, 2.56-2.67, 1.78-1.96, 1.69, 1.57.

### Example 6-6: (1R,2R,3aS,10aR)-1-[(1E,3ξ)-4-(2-chlorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.86;
MS (ESI, Pos.): 465 (M-OH)+.;
¹H-NMR (DMSO-d₆): δ 13.06, 7.44-7.62, 7.37, 6.98, 5.85, 5.46-5.62, 4.86, 4.77, 4.30, 3.66-3.75, 2.82-2.92, 2.68-2.74, 2.52-2.62, 2.45-2.46, 2.34-2.42, 1.63-1.75, 1.32-1.53.

### Example 6-7: (1R,2R,3aS,10aR)-1-[(1E,3ξ)-4,4-difluoro-3-hydroxy-4-(2-methylphenyl)-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.87;
MS (ESI, Pos.): 445 (M+H-H₂O)+.;
¹H-NMR (CD₃OD): δ 7.42-7.49, 7.31-7.38, 7.27, 6.95, 5.68, 5.53-5.61, 4.53-4.62, 4.28-4.35, 3.87, 3.00, 2.67-2.78, 2.54-2.66, 2.48-2.53, 1.92, 1.73-1.85, 1.51-1.71.

### Example 6-8: (1R,2R,3aS,10aR)-1-[(1E,3ξ)-4,4-difluoro-4-(4-fluoro-2-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 463 (M+H-H₂O)+.;
¹H-NMR (CD₃OD): δ 7.36, 6.83-6.93, 5.46-5.60, 4.43, 4.20-4.25, 3.76, 3.23-3.27, 2.91, 2.60-2.73, 2.43-2.55, 2.40, 1.43-1.85.

### Example 6-9: (1R,2R,3aS,10aR)-1-[(1E,3ξ)-4-(2,5-difluorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.84;
MS (ESI, Pos.): 467 (M+H-H₂O)+.;
¹H-NMR (CD₃OD): δ 7.35, 7.11-7.22, 6.83, 5.49-5.64, 4.76, 4.52, 4.19-4.25, 3.77, 2.90, 2.45-2.67, 1.81, 1.65-1.75, 1.51-1.62, 1.40-1.51.

### Example 6-10: (1R,2R,3aS,10aR)-1-[(1E,3ξ)-4,4-difluoro-4-(2-fluoro-6-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.87;
MS (ESI, Pos.): 463 (M-OH)+.;
¹H-NMR (DMSO-d₆): δ 13.06, 7.35-7.42, 7.08-7.15, 6.99, 5.87, 5.50-5.65, 4.85, 4.45, 4.30, 3.71, 2.88, 2.68-2.80, 2.52-2.56, 2.36-2.47, 1.72, 1.49.

### Example 6-11: (1R,2R,3aS,10aR)-1-[(1E,3ξ)-4,4-difluoro-4-(2-fluoro-3-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.89;
MS (ESI, Pos.): 463 (M-OH)+.;
¹H-NMR (DMSO-d₆): δ 12.88-13.22, 7.28-7.46, 7.16-7.22, 6.98, 5.85, 5.51-5.56, 4.85, 4.43-4.59, 4.31, 3.71, 2.82-2.92, 2.69-2.78, 2.52-2.60, 2.45, 2.26, 1.69, 1.37-1.60, 1.23.

### Example 6-12: (1R,2R,3aS,10aR)-1-[(1E,3ξ)-4-(2-chloro-6-fluorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.88;
MS (ESI, Pos.): 483 (M-OH)+.;
¹H-NMR (DMSO-d₆): δ 12.90-13.25, 7.55, 7.33-7.45, 6.99, 5.97, 5.52-5.66, 4.86, 4.49-4.65, 4.30, 3.71, 2.84-2.96, 2.67-2.77, 2.54-2.65, 2.35-2.46, 1.66-1.76, 1.36-1.57, 1.23.

### Example 6-13: (1R,2R,3aS,10aR)-1-[(1E,3ξ)-4,4-difluoro-4-(5-fluoro-2-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 463 (M+H-H₂O)+.;
¹H-NMR (CD₃OD): δ 7.12-7.28, 7.04-7.11, 6.93-7.02, 6.72-6.81, 5.46-5.60, 4.40-4.53, 4.14-4.25, 3.67-3.84, 3.24-3.30, 2.81-2.96, 2.66, 2.50, 2.36, 1.74-1.86, 1.34-1.74.

### Example 6-14: (1R,2R,3aS,10aR)-1-[(1E,3ξ)-4,4-difluoro-4-(2-fluoro-5-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 463 (M+H-H₂O)+.;
¹H-NMR (CD₃OD): δ 7.45, 7.30-7.38, 7.08, 6.94, 5.55-5.71, 4.59-4.68, 4.29-4.34, 3.87, 2.97, 2.53-2.75, 2.38, 1.92, 1.72-1.81, 1.56-1.67, 1.37-1.51.

### Example 6-15: (1R,2R,3aS,10aR)-1-[(1E,3ξ)-4,4-difluoro-4-(3-fluoro-2-methylphenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.91;
MS (ESI, Pos.): 463 (M+H-H₂O)+.;
¹H-NMR (CD₃OD): δ 7.42-7.49, 7.26-7.35, 7.12-7.22, 6.95, 5.65, 4.52-4.61, 4.30-4.36, 3.88, 3.02, 2.75, 2.55-2.67, 2.41, 1.78-1.96, 1.55-1.75.

### Example 6-16: (1R,2R,3aS,10aR)-1-[(1E,3ξ)-4-(5-chloro-2-fluorophenyl)-4,4-difluoro-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.92;
MS (ESI, Pos.): 483 (M-OH)+.;
¹H-NMR (DMSO-d₆): δ 12.90-13.24, 7.63-7.69, 7.48-7.53, 7.39, 6.95-7.00, 5.98, 5.55, 4.86, 4.42-4.58, 4.28-4.38, 3.67, 2.85-2.96, 2.71-2.82, 2.54-2.63, 2.35-2.46, 1.66-1.78, 1.36-1.64.

### Example 6-17: (1R,2R,3aS,10aR)-1-[(1E,3ξ)-4,4-difluoro-4-(2-fluorophenyl)-3-hydroxy-1-buten-1-yl]-5-fluoro-2-hydroxy-2,3,3a,9,10,10a-hexahydro-1H-benzo[b]cyclopenta[f]oxepine-6-carboxylic acid

HPLC retention time (min): 0.86;
MS (ESI, Pos.): 449 (M+H-H₂O)+.;
¹H-NMR (CD₃OD): δ 7.51-7.60, 7.41, 7.30, 7.17-7.25, 6.92, 5.57-5.73, 4.66, 4.30, 3.84-3.91, 2.93-3.01, 2.54-2.73, 1.92, 1.77, 1.60, 1.29-1.52.

### Reference Example 31: 5-bromo-2-vinylphenol

A procedure for a purpose similar to that for Reference Example 3 was performed using 4-bromo-2-hydroxybenzaldehyde (CAS No.: 22532-62-3) in place of 4-bromo-3-fluoro-2-hydroxy-benzaldehyde to yield the title compound having the following physical properties.

¹H-NMR (CDCl₃): δ 7.24, 7.05, 6.98-7.00, 6.80-6.92, 5.77, 5.71, 5.40, 5.09.

### Reference Example 32: ethyl (1R,2R,3aS,10aR)-1-formyl-2-(tetrahydro-2H-pyran-2-yloxy)-2,3,3a,9,10,10a-hexahydro-1H-benzo[b]cyclopenta[floxepine-6-carboxylate

A procedure for a purpose similar to that for Reference Example 4→ Reference Example 5→ Reference Example 6→ Reference Example 7→ Reference Example 8→ Reference Example 9 was performed using the compound produced in Reference Example 31 in place of the compound produced in Reference Example 3 to yield the title compound having the following physical properties.
TLC: Rf 0.50 (hexane : ethyl acetate = 2 : 1);
¹H-NMR (CDCl₃): δ 9.82-9.86, 7.60-7.65, 7.13, 4.64-4.69, 4.32-4.49, 3.79-3.95, 3.44-3.55, 3.23-3.30, 3.14-3.21, 3.02-3.11, 2.77-2.88, 2.52-2.66, 2.18-2.41, 1.98-2.15, 1.70-1.97, 1.59-1.70, 1.48-1.57, 1.39.

### Example 7: (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

A procedure for a purpose similar to that for Reference Example 17→ Reference Example 18→ Example 2 was performed using the compound produced in Reference Example 32 in place of the compound produced in Reference Example 9 and using the compound produced in Reference Example 27 in place of the compound produced in Reference Example 14 to yield the title compound having the following physical properties. The steric configuration was identified by single crystal X-ray.
HPLC retention time (min): 0.91;
MS (ESI, Pos.): 403 (M+H-H₂O)+, 385 (M+H-2H₂O)+.;
¹H-NMR (CD₃OD): δ 7.58, 7.48, 7.34-7.40, 7.22-7.29, 7.15-7.21, 5.53, 5.38, 4.30-4.35, 3.88, 3.77-3.85, 3.00, 2.80, 2.59, 2.42-2.51, 1.65-1.87, 0.87-1.00, 0.68-0.82.

### Example 7-1 to 7-50:

A procedure for a purpose similar to that for Reference Example 17→ Reference Example 18→ Example 2 was performed using the compound produced in Reference Example 32 in place of the compound produced in Reference Example 9 and using a corresponding phosphonate in place of the compound produced in Reference Example 14 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 10→ Reference Example 11 or Reference Example 28→ Reference Example 29.

### Example 7-1: (1R,2R,3aS,10aR)-2-hydroxy-1-[(1E,3S)-3-hydroxy-1-octen-1-yl]-2,3,3a,9,10,10a-hexahydro-1H-benzo[b]cyclopenta[f]oxepine-6-carboxylic acid

MS (ESI, Pos.): 357 (M+H-H₂O)+.;
¹H-NMR (DMSO-d₆): δ 12.80, 7.47, 7.33, 7.18, 5.31-5.53, 4.77, 4.55, 4.35, 3.81-3.94, 3.63-3.76, 2.76-2.98, 2.40-2.58, 2.24-2.38, 1.63-1.85, 1.16-1.49.

### Example 7-2: (1R,2R,3aS,10aR)-2-hydroxy-1-[(1E,3ξ)-3-hydroxy-4-(trifluoromethyl)-1-octen-1-yl]-2,3,3a,9,10,10a-hexahydro-1H-benzo[b]cyclopenta[f]oxepine-6-carboxylic acid,

HPLC retention time (min): 0.97;
MS (ESI, Pos.): 425 (M+H-H₂O)+, 407 (M+H-2H₂O)+.;
¹H-NMR (CD₃OD): δ 7.47, 7.39, 7.06, 5.51-5.63, 4.40-4.45, 4.28, 3.66-3.82, 2.84-2.96, 2.68-2.83, 2.40-2.62, 2.00-2.27, 1.70-1.93, 1.12-1.61, 0.84.

### Example 7-3: (1R,2R,3aS,10aR)-2-hydroxy-1-[(1E,3ξ,4ξ)-3-hydroxy-4-(trifluoromethyl)-1-octen-1-yl]-2,3,3a,9,10,10a-hexahydro-1H-benzo[b]cyclopenta[f]oxepine-6-carboxylic acid,

HPLC retention time (min): 0.99;
MS (ESI, Pos.): 425 (M+H-H₂O)+, 407 (M+H-2H₂O)+.;
¹H-NMR (CD₃OD): δ 7.60, 7.51, 7.19, 5.60-5.75, 4.38-4.46, 3.91, 3.05, 2.86, 2.56-2.70, 2.21-2.32, 1.82-1.97, 1.59-1.74, 1.29-1.53, 0.92-0.98.

### Example 7-4: (1R,2R,3aS,10aR)-2-hydroxy-1-[(1E,3ξ,4ξ)-3-hydroxy-4-(trifluoromethyl)-1-octen-1-yl]-2,3,3a,9,10,10a-hexahydro-1H-benzo[b]cyclopenta[f]oxepine-6-carboxylic acid,

HPLC retention time (min): 0.99;
MS (ESI, Pos.): 425 (M+H-H₂O)+, 407 (M+H-2H₂O)+.;
¹H-NMR (CD₃OD): δ 7.59, 7.51, 7.19, 5.60-5.75, 4.38-4.43, 3.90, 3.04, 2.87, 2.55-2.70, 2.29-2.38, 1.82-1.97, 1.68-1.77, 1.53-1.62, 1.28-1.49, 0.90-0.98.

### Example 7-5: (1R,2R,3aS,10aR)-1-[(1E,3ξ)-4,4-difluoro-3-hydroxy-4-phenyl-1-buten-1-yl]-2-hydroxy-2,3,3a,9,10,10a-hexahydro-1H-benzo[b]cyclopenta[f]oxepine-6-carboxylic acid

HPLC retention time (min): 0.86;
MS (ESI, Pos.): 413 (M+H-H₂O)+.;
¹H-NMR (CD₃OD): δ 7.60, 7.43-7.55, 7.19, 5.57-5.62, 4.94, 4.42-4.57, 4.34, 3.82-3.89, 2.89-3.05, 2.71-2.87, 2.62, 1.85.

### Example 7-6: (1R,2R,3aS,10aR)-2-hydroxy-1-[(1E,3ξ)-3-hydroxy-3-(1-phenylcyclobutyl)-1-propen-1-yl]-2,3,3a,9,10,10a-hexahydro-1H-benzo[b]cyclopenta[f]oxepine-6-carboxylic acid

HPLC retention time (min): 0.97;
MS (ESI, Pos.): 417 (M+H-H₂O)+.;
¹H-NMR (CDCl₃): δ 7.62-7.68, 7.27-7.35, 7.12-7.25, 5.57, 5.39, 4.32-4.38, 3.73-3.82, 2.99-3.08, 2.79, 2.26-2.61, 2.02-2.11, 1.74-2.00.

### Example 7-7: (1R,2R,3aS,10aR)-2-hydroxy-1-[(1E,3ξ,4R)-3-hydroxy-4-phenyl-1-penten-1-yl]-2,3,3a,9,10,10a-hexahydro-1H-benzo[b]cyclopenta[f]oxepine-6-carboxylic acid

HPLC retention time (min): 0.92;
MS (ESI, Pos.): 373 (M+H-2H₂O)+.;
¹H-NMR (CD₃OD): δ 7.46, 7.35, 7.15-7.20, 7.02-7.11, 5.33, 5.13, 4.12-4.16, 3.98-4.03, 3.62-3.68, 2.62-2.75, 2.41-2.54, 2.20-2.28, 1.69, 1.42-1.51, 1.23-1.32.

### Example 7-8: (1R,2R,3aS,10aR)-2-hydroxy-1-[(1E,3ξ,4S)-3-hydroxy-4-phenyl-1-penten-1-yl]-2,3,3a,9,10,10a-hexahydro-1H-benzo[b]cyclopenta[f]oxepine-6-carboxylic acid

HPLC retention time (min): 0.92;
MS (ESI, Pos.): 373 (M+H-2H₂O)+.;
¹H-NMR (CD₃OD): δ 7.47, 7.38, 7.12-7.20, 7.01-7.11, 5.52, 5.35, 4.25, 4.08, 3.74, 2.86-2.95, 2.68-2.78, 2.37-2.56, 1.62-1.80, 1.07-1.24.

### Example 7-9: (1R,2R,3aS,10aR)-1-[(1E,3ξ)-3-(1-butylcyclopropyl)-3-hydroxy-1-propen-1-yl]-2-hydroxy-2,3,3a,9,10,10a-hexahydro-1H-benzo[b]cyclopenta[floxepine-6-carboxylic acid

HPLC retention time (min): 0.99;
MS (ESI, Pos.): 383 (M+H-H₂O)+.;
¹H-NMR (CD₃OD): δ 7.59, 7.51, 7.19, 5.50-5.67, 4.41, 3.86-3.94, 3.03, 2.89, 2.52-2.69, 1.83-1.98, 1.52, 1.24-1.43, 0.89-0.96, 0.50-0.58, 0.26-0.34.

### Example 7-10: (1R,2R,3aS,10aR)-2-hydroxy-1-[(1E,3S)-3-hydroxy-4-(3-thienyl)-1-buten-1-yl]-2,3,3a,9,10,10a-hexahydro-1H-benzo[b]cyclopenta[f]oxepine-6-carboxylic acid

HPLC retention time (min): 0.84;
MS (ESI, Pos.): 383 (M+H-H₂O)+.;
¹H-NMR (CD₃OD): δ 7.59, 7.49, 7.32, 7.18, 7.10, 7.02, 5.63, 5.42, 4.30-4.36, 3.80-3.87, 2.92-3.02, 2.74-2.87, 2.62, 2.49, 1.86, 1.74, 1.58-1.67.

### Example 7-11: (1R,2R,3aS,10aR)-1-{(1E,3ξ,4ξ)-3-[1-(2-fluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 421 (M+H-H₂O);
¹H-NMR (DMSO-d₆): δ 12.78, 7.48, 7.18-7.34, 7.03-7.11, 5.40, 5.19, 4.80, 4.68, 4.22-4.31, 3.96, 3.56-3.66, 2.80-2.92, 2.67-2.79, 2.49, 2.19-2.28, 1.41-1.69, 0.88-1.03, 0.61-0.70, 0.50-0.58.

### Example 7-12: (1R,2R,3aS,10aR)-1-{(1E,3ξ)-3-[1-(3-fluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 421 (M+H-H₂O);
¹H-NMR (DMSO-d₆): δ 12.78, 7.48, 7.32, 7.24-7.31, 7.16-7.21, 7.11-7.15, 7.06-7.11, 6.98, 5.23-5.40, 4.87, 4.69, 4.26-4.32, 3.84, 3.59-3.68, 3.16, 2.81-2.92, 2.70-2.81, 2.41-2.49, 2.21-2.31, 1.51-1.72, 0.80-0.99, 0.58-0.80.

### Example 7-13: (1R,2R,3aS,10aR)-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

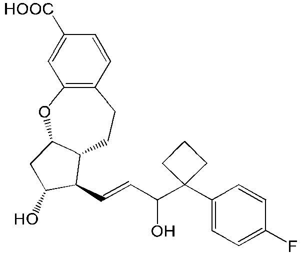

HPLC retention time (min): 0.97;
MS (ESI, Pos.): 435 (M+H-H₂O)+, 417 (M+H-2H₂O)+.;
¹H-NMR (DMSO-d₆): δ 12.78, 7.49, 7.33, 7.21, 7.03-7.10, 5.33, 5.14, 4.83, 4.68, 4.27-4.33, 4.09, 3.60-3.69, 3.16, 2.75-2.93, 2.49, 2.20-2.33, 2.09-2.19, 1.87-2.00, 1.57-1.77.

### Example 7-14: (1R,2R,3aS,10aR)-1-{(1E,3ξ)-3-[1-(3-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.97;
MS (ESI, Pos.): 435 (M+H-H₂O)+.;
¹H-NMR (DMSO-d₆): δ 12.79, 7.48, 7.26-7.34, 7.20, 6.95, 6.88-6.92, 6.80-6.86, 5.34, 5.15, 4.90, 4.68, 4.27-4.32, 4.06-4.12, 3.61-3.69, 3.16, 2.74-2.92, 2.49, 2.11-2.35, 1.89-2.00, 1.58-1.77.

### Example 7-15: (1R,2R,3aS,10aR)-2-hydroxy-1-[(1E,3ξ,4R)-3-hydroxy-4-methyl-1-octen-1-yl]-2,3,3a,9,10,10a-hexahydro-1H-benzo[b]cyclopenta[f]oxepine-6-carboxylic acid,

HPLC retention time (min): 0.96;
MS (ESI, Pos.): 371 (M+H-H₂O)+, 353 (M+H-2H₂O)+.;
¹H-NMR (CD₃OD): δ 7.57, 7.48, 7.16, 5.62, 5.49, 4.38, 3.83-3.92, 3.03, 2.84, 2.49-2.68, 1.79-1.96, 1.45-1.61, 1.21-1.41, 1.02-1.16, 0.86-0.96.

### Example 7-16: (1R,2R,3aS,10aR)-2-hydroxy-1-[(1E,3ξ,4S)-3-hydroxy-4-methyl-1-octen-1-yl]-2,3,3a,9,10,10a-hexahydro-1H-benzo[b]cyclopenta[f]oxepine-6-carboxylic acid

HPLC retention time (min): 0.96;
MS (ESI, Pos.): 371 (M+H-H₂O)+, 353 (M+H-2H₂O)+.;
¹H-NMR (CD₃OD): δ 7.57, 7.49, 7.17, 5.62, 5.46-5.54, 4.38, 3.84-3.91, 3.03, 2.85, 2.47-2.71, 1.76-2.00, 1.46-1.66, 1.21-1.46, 1.05-1.17, 0.86-0.96.

### Example 7-17: (1R,2R,3aS,10aR)-1-{(1E,3ξ)-3-[1-(2-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.1;
MS (ESI, Pos.): 417 (M+H-2H₂O)+.;
¹H-NMR (DMSO-d₆): δ 12.80, 7.49, 7.33, 7.17-7.23, 7.01-7.12, 5.76, 5.28-5.39, 4.92, 4.68, 4.27-4.32, 4.16, 3.60-3.68, 2.83-2.92, 2.71-2.80, 2.52-2.55, 2.39-2.48, 1.91-2.08, 1.50-1.78.

### Example 7-18: (1R,2R,3aS,10aR)-2-hydroxy-1-{(1E,3ξ)-3-hydroxy-3-[1-(3-thienyl)cyclopropyl]-1-propen-1-yl}-2,3,3a,9,10,10a-hexahydro-1H-benzo[b]cyclopenta[f]oxepine-6-carboxylic acid

HPLC retention time (min): 0.88;
MS (ESI, Pos.): 391 (M+H-2H₂O)+.;
¹H-NMR (CD₃OD): δ 7.59, 7.50, 7.29, 7.16-7.20, 7.12, 5.57-5.63, 5.46-5.51, 4.36, 3.94, 3.85, 2.96-3.04, 2.83, 2.62, 2.49, 1.77-1.89, 1.73, 0.76-1.00.

### Example 7-19: (1R,2R,3aS,10aR)-1-[(1E,3ξ,4ξ)-4-(4-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.89;
MS (ESI, Pos.): 409 (M+H-H₂O)+., 391 (M+H-2H₂O)+.;
¹H-NMR (DMSO-d₆): δ 12.80, 7.48, 7.32, 7.16-7.24, 7.04-7.10, 5.26-5.33, 5.14-5.21, 4.80, 4.70, 4.24-4.29, 3.94-4.00, 3.57-3.65, 2.64-2.82, 2.41-2.49, 2.18, 1.51-1.67, 1.34-1.44, 1.23.

### Example 7-20: (1R,2R,3aS,10aR)-1-{(1E,3ξ)-3-[1-(2-chlorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.913;
MS (ESI, Pos.): 437 (M+H-H₂O);
¹H-NMR (CD₃OD): δ 7.58, 7.48, 7.31-7.42, 7.15-7.25, 5.58, 5.31, 4.38, 4.30, 3.75-3.82, 2.97, 2.76, 2.58, 2.41-2.51, 1.61-1.86, 1.51-1.61, 1.29, 1.07-1.21, 0.67-0.83.

### Example 7-21: (1R,2R,3aS,10aR)-1-{(1E,3ξ)-3-[1-(2-chloro-6-fluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.915;
MS (ESI, Pos.): 455 (M+H-H₂O);
¹H-NMR (CD₃OD): δ 7.58, 7.48, 7.16-7.27, 6.99-7.05, 5.65-5.75, 5.38, 5.25, 4.24-4.35, 3.70-3.84, 2.91-3.04, 2.67-2.84, 2.42-2.63, 1.76-1.88, 1.56-1.76, 1.43-1.52, 1.15-1.33, 0.81-0.92, 0.77, 0.62-0.73.

### Example 7-22: (1R,2R,3aS,10aR)-1-{(1E,3ξ)-3-[1-(2,4-difluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.885;
MS (ESI, Pos.): 439 (M+H-H₂O);
¹H-NMR (CD₃OD): δ 7.58, 7.48, 7.38, 7.18, 6.82-6.91, 5.56, 5.38, 4.33, 3.96, 3.80, 2.94-3.04, 2.75-2.85, 2.59, 2.42-2.53, 1.61-1.89, 1.27-1.35, 0.95-1.07, 0.95-0.97, 0.65-0.81.

### Example 7-23: (1R,2R,3aS,10aR)-2-hydroxy-1-{(1E,3ξ)-3-hydroxy-3-[1-(3-thienyl)cyclobutyl]-1-propen-1-yl}-2,3,3a,9,10,10a-hexahydro-1H-benzo[b]cyclopenta[f]oxepine-6-carboxylic acid

HPLC retention time (min): 0.99;
MS (ESI, Pos.): 405 (M+H-2H₂O)+.;
¹H-NMR (CD₃OD): δ 7.60, 7.49, 7.32, 7.19, 7.09, 7.02, 5.36-5.47, 4.32-4.36, 4.23, 3.83, 2.99, 2.81, 2.39-2.65, 2.17-2.32, 1.61-1.99.

### Example 7-24: (1R,2R,3aS,10aR)-1-{(1E,3ξ)-3-[1-(3-chlorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.97;
MS (ESI, Pos.): 437 (M-OH)+.;
¹H-NMR (DMSO-d₆): δ 12.63-13.00, 7.50, 7.19-7.35, 5.32, 4.92, 4.72, 4.31, 3.79-3.84, 3.66, 3.38-3.43, 3.24-3.31, 2.52-2.58, 2.52-2.61, 2.44-2.47, 1.66, 0.90, 0.68.

### Example 7-25: (1R,2R,3aS,10aR)-1-{(1E,3ξ)-3-[1-(2-chlorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.02;
MS (ESI, Pos.): 451 (M-OH)+.;
¹H-NMR (DMSO-d₆): δ 12.62-13.01, 7.48, 7.11-7.34, 5.17-5.60, 4.80-5.00, 4.71, 4.24-4.38, 3.55-3.72, 2.69-2.95, 2.57-2.63, 2.52-2.55, 2.39-2.46, 1.88-1.97, 1.67.

### Example 7-26: (1R,2R,3aS,10aR)-1-{(1E,3ξ)-3-[1-(2-fluoro-6-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.94;
MS (ESI, Pos.): 435 (M-OH)+.;
¹H-NMR (DMSO-d₆): δ 12.48-13.10, 7.49, 7.33, 7.21, 7.08-7.16, 6.86-6.98, 5.49, 5.05-5.33, 4.78, 4.68, 4.25, 4.05-4.12, 3.86-3.92, 2.69-2.92, 2.53-2.63, 2.39-2.46, 2.16-2.31, 1.66, 1.08, 0.38-0.70.

### Example 7-27: (1R,2R,3aS,10aR)-1-{(1E,3ξ)-3-[1-(2,6-difluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.9;
MS (ESI, Pos.): 439 (M-OH)+.;
¹H-NMR (DMSO-d₆): δ 12.68-13.15, 7.50, 7.27-7.39, 7.21, 6.96-7.04, 5.45-5.53, 5.20, 4.89, 4.69, 4.27, 3.93-3.99, 3.51-3.76, 2.69-2.93, 2.52-2.55, 2.44, 2.26, 1.62, 1.48, 1.00-1.11, 0.67, 0.49-0.58.

### Example 7-28: (1R,2R,3aS,10aR)-2-hydroxy-1-[(1E,3ξ)-3-hydroxy-3-{1-[3-(trifluoromethyl)phenyl]cyclopropyl}-1-propen-1-yl]-2,3,3a,9,10,10a-hexahydro-1H-benzo[b]cyclopenta[f]oxepine-6-carboxylic acid

HPLC retention time (min): 0.98;
MS (ESI, Pos.): 471 (M-OH)+.;
¹H-NMR (DMSO-d₆): δ 12.71-12.98, 7.61, 7.50, 7.33, 7.16-7.23, 5.32, 4.97, 4.71, 4.28-4.36, 3.77-3.85, 3.60-3.68, 2.71-2.90, 2.57-2.62, 2.52-2.56, 2.34-2.46, 1.49-1.74, 0.62-1.01.

### Example 7-29: (1R,2R,3aS,10aR)-2-hydroxy-1-[(1E,3ξ)-3-hydroxy-3-{1-[3-(trifluoromethoxy)phenyl]cyclopropyl}-1-propen-1-yl]-2,3,3a,9,10,10a-hexahydro-1H-benzo[b]cyclopenta[f]oxepine-6-carboxylic acid

HPLC retention time (min): 1;
MS (ESI, Pos.): 487 (M-OH)+.;
¹H-NMR (DMSO-d₆): δ 12.68-12.97, 7.49, 7.32-7.41, 7.26, 7.19, 5.31-5.35, 4.94, 4.71, 4.27-4.34, 3.82, 3.58-3.71, 2.71-2.96, 2.55, 2.36-2.46, 2.19-2.31, 1.50-1.75, 0.62-0.99.

### Example 7-30: (1R,2R,3aS,10aR)-1-{(1E,3ξ)-3-[1-(2,5-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

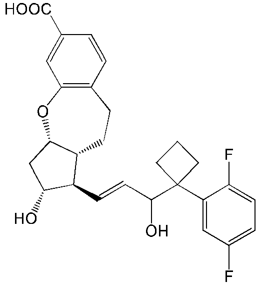

HPLC retention time (min): 1.12;
MS (ESI, Pos.): 435 (M+H-2H₂O)+.;
¹H-NMR (DMSO-d₆): δ 12.70-12.92, 7.48, 7.33, 7.19, 7.00-7.15, 6.79-6.89, 5.32-5.37, 5.01, 4.70, 4.27-4.33, 4.12-4.20, 3.58-3.71, 2.72-2.93, 2.52-2.56, 2.45, 2.18-2.32, 1.99, 1.68.

### Example 7-31: (1R,2R,3aS,10aR)-2-hydroxy-1-{(1E,3ξ)-3-hydroxy-3-[1-(2-methylphenyl)cyclopropyl]-1-propen-1-yl}-2,3,3a,9,10,10a-hexahydro-1H-benzo[b]cyclopenta[f]oxepine-6-carboxylic acid

HPLC retention time (min): 0.96;
MS (ESI, Pos.): 417 (M+H-H₂O)+.;
¹H-NMR (CD₃OD): δ 7.59, 7.49, 7.27, 7.07-7.20, 5.59, 5.32, 4.32, 4.12, 3.76-3.84, 3.35-3.43, 2.96-3.04, 2.80, 2.56-2.65, 2.48, 2.00, 1.69-1.88, 1.64, 1.32-1.36, 1.04-1.14, 0.74-0.79, 0.65.

### Example 7-32: (1R,2R,3aS,10aR)-1-{(1E,3ξ)-3-[1-(2,5-difluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.93;
MS (ESI, Pos.): 439 (M+H-H₂O)+.;
¹H-NMR (CD₃OD): δ 7.59, 7.50, 6.97-7.19, 5.58, 5.39, 4.33, 4.05, 3.79-3.86, 3.36-3.43, 3.01, 2.75-2.87, 2.47-2.65, 1.73-1.89, 1.60-1.72, 1.24-1.38, 1.03-1.10, 0.72-0.86.

### Example 7-33: (1R,2R,3aS,10aR)-1-{(1E,3ξ)-3-[1-(2,4-difluorophenyl)cyclobutyl]-3-hydroxy-1-propen-1-yl1-2-hydroxy-2,3,3a,9,10,10a-hexahydro-1H-benzo[b]cyclopenta[f]oxepine-6-carboxylic acid

HPLC retention time (min): 0.99;
MS (ESI, Pos.): 453 (M+H-H₂O)+.;
¹H-NMR (CD₃OD): δ 7.57, 7.49, 7.13-7.17, 6.84, 6.90, 5.37-5.61, 4.34, 4.33, 3.83, 2.94-3.06, 2.70-2.84, 2.60, 2.49, 2.41, 2.05-2.19, 1.55-1.91.

### Example 7-34: (1R,2R,3aS,10aR)-1-{(1E,3ξ)-3-[1-(2-chloro-6-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.03;
MS (ESI, Pos.): 469, 471 (M-H₂O, M+2-H₂O)+.;
¹H-NMR (CD₃OD): δ 7.47, 7.38, 7.03-7.11, 6.84-6.91, 5.76-5.89, 5.67, 5.33-5.46, 5.20, 4.29-4.40, 4.15-4.25, 3.62-3.78, 2.78-2.94, 2.42-2.69, 2.31-2.39, 1.85-2.06, 1.55-1.79, 1.48, 1.27-1.43, 1.21-1.27.

### Example 7-35: (1R,2R,3aS,10aR)-1-{(1E,3ξ)-3-[1-(2,6-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.99;
MS (ESI, Pos.): 453 (M+H-H₂O)+.;
¹H-NMR (CD₃OD): δ 7.60, 7.50, 7.17-7.26, 6.87, 5.68, 5.48, 4.31-4.40, 3.83, 3.00, 2.79, 2.48-2.67, 2.10-2.22, 1.74-1.91, 1.62-1.72.

### Example 7-36: (1R,2R,3aS,10aR)-2-hydroxy-1-{(1E,3ξ)-3-hydroxy-3-[1-(3-methylphenyl)cyclopropyl]-1-propen-1-yl}-2,3,3a,9,10,10a-hexahydro-1H-benzo[b]cyclopenta[f]oxepine-6-carboxylic acid

HPLC retention time (min): 0.95;
MS (ESI, Pos.): 417 (M+H-H₂O)+.;
¹H-NMR (CD₃OD): δ 7.48, 7.38, 7.01-7.12, 6.90, 5.42, 5.27, 4.23, 3.68-3.79, 2.90, 2.70, 2.49, 2.36, 2.21, 1.53-1.77, 0.74-0.89, 0.56-0.70.

### Example 7-37: (1R,2R,3aS,10aR)-1-{(1E,3ξ)-3-[1-(2,3-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.98;
MS (ESI, Pos.): 453 (M+H-H₂O)+., 435 (M+H-2H₂O)+.;
¹H-NMR (CD₃OD): δ 7.47, 7.38, 7.05, 6.92-7.01, 6.77-6.88, 5.32-5.49, 4.12-4.40, 3.72, 2.87, 2.59-2.77, 2.49, 2.19-2.43, 1.94-2.17, 1.60-1.83, 1.54.

### Example 7-38: (1R,2R,3aS,10aR)-1-{(1E,3ξ)-3-[1-(2-fluoro-3-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.96;
MS (ESI, Pos.): 435 (M+H-H₂O)+.417 (M-2H₂O)+.;
¹H-NMR (CD₃OD): δ 7.58, 7.48, 7.13-7.21, 7.04-7.12, 6.93-6.99, 5.57, 5.35, 4.31, 4.04, 3.75-3.84, 3.33-3.37, 2.98, 2.78, 2.53-2.62, 2.41-2.51, 2.17-2.29, 1.71-1.90, 1.56-1.71, 1.27-1.35, 0.96-1.06, 0.59-0.82.

### Example 7-39: (1R,2R,3aS,10aR)-1-{(1E,3ξ)-3-[1-(5-chloro-2-fluorophenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.96;
MS (ESI, Pos.): 455, 457 (M+H-H₂O)+.437, 439 (M+H-2H₂O)+.;
¹H-NMR (CD₃OD): δ 7.58, 7.46-7.50, 7.33-7.40, 7.14-7.26, 6.99-7.07, 5.55, 5.36, 4.32, 4.03, 3.75-3.85, 3.00, 2.81, 2.40-2.66, 1.70-1.91, 1.55-1.70, 1.27-1.45, 0.98-1.11, 0.65-0.85.

### Example 7-40: (1R,2R,3aS,10aR)-1-{(1E,3ξ)-3-[1-(2-fluoro-5-methylphenyl)cyclopropyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 0.95;
MS (ESI, Pos.): 435 (M+H-H₂O)+.417 (M+H-2H₂O)+.;
¹H-NMR (CD₃OD): δ 7.58, 7.48, 7.13-7.20, 6.99-7.05, 6.84-6.91, 5.56, 5.34, 4.32, 4.03, 3.76-3.83, 2.97, 2.79, 2.58, 2.40-2.51, 1.70-1.90, 1.55-1.67, 1.27-1.37, 0.96-1.06, 0.61-0.83.

### Example 7-41: (1R,2R,3aS,10aR)-2-hydroxy-1-{(1E,3ξ)-3-hydroxy-3-[1-(3-methylphenyl)cyclobutyl]-1-propen-1-yl}-2,3,3a,9,10,10a-hexahydro-1H-benzo[b]cyclopenta[f]oxepine-6-carboxylic acid

HPLC retention time (min): 1;
MS (ESI, Pos.): 413 (M+H-2H₂O)+.;
¹H-NMR (CD₃OD): δ 7.57, 7.47, 7.12-7.19, 6.91-6.99, 5.30-5.45, 4.32, 4.22, 3.81, 2.94-3.03, 2.75-2.85, 2.22-2.62, 1.96-2.08, 1.65-1.87.

### Example 7-42: (1R,2R,3aS,10aR)-2-hydroxy-1-{(1E,3ξ)-3-hydroxy-3-[1-(3-methoxylphenyl)cyclopropyl]-1-propen-1-yl}-2,3,3a,9,10,10a-hexahydro-1H-benzo[b]cyclopenta[f]oxepine-6-carboxylic acid

HPLC retention time (min): 0.89;
MS (ESI, Pos.): 415 (M+H-2H₂O)+.;
¹H-NMR (CD₃OD): δ 7.58, 7.48, 7.13-7.21, 6.95, 6.76, 5.53, 5.38, 4.28-4.35, 3.87-3.92, 3.74-3.85, 2.98, 2.79, 2.54-2.64, 2.41-2.51, 1.62-1.89, 0.86-1.00, 0.64-0.86.

### Example 7-43: (1R,2R,3aS,10aR)-1-{(1E,3ξ)-3-[1-(3-chlorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.02;
MS (ESI, Pos.): 451 (M-OH)+.;
¹H-NMR (DMSO-d₆): δ 12.81, 7.49, 7.34, 7.27-7.32, 7.18-7.24, 7.07, 7.03, 5.36, 5.14, 4.94, 4.70, 4.31, 4.12, 3.67, 2.86, 2.54-2.63, 2.40-2.46, 2.10-2.31, 1.91-2.03, 1.58-1.79.

### Example 7-44: (1R,2R,3aS,10aR)-2-hydroxy-1-{(1E,3ξ)-3-hydroxy-3-[1-(2-methylphenyl)cyclobutyl]-1-propen-1-yl}-2,3,3a,9,10,10a-hexahydro-1H-benzo[b]cyclopenta[f]oxepine-6-carboxylic acid

HPLC retention time (min): 0.99;
MS (ESI, Pos.): 413 (M+H-2H₂O)+.;
¹H-NMR (CD₃OD): δ 7.58, 7.48, 7.17, 6.95-7.11, 5.47-5.61, 5.26-5.47, 4.26-4.43, 3.76-3.85, 2.97, 2.70-2.86, 2.39-2.64, 2.26-2.34, 2.00, 1.58-1.86.

### Example 7-45: (1R,2R,3aS,10aR)-1-[(1E,3ξ)-4,4-difluoro-3-hydroxy-7-methyl-1-octen-1-yl]-2-hydroxy-2,3,3a,9,10,10a-hexahydro-1H-benzo[b]cyclopenta[f]oxepine-6-carboxylic acid

HPLC retention time (min): 0.93;
MS (ESI, Pos.): 407 (M+H-H₂O)+.;
¹H-NMR (CD₃OD): δ 7.57, 7.49, 7.13-7.18, 5.66-5.78, 4.80-4.83, 4.36-4.41, 4.16-4.24, 3.87-3.95, 3.26, 2.99-3.06, 2.79-2.89, 2.55-2.69, 1.79-2.02, 1.52-1.64, 1.35-1.47, 0.91-0.95, 0.93.

### Example 7-46: (1R,2R,3aS,10aR)-1-[(1E,3S)-6-cyclopropyl-3-hydroxy-1-hexen-1-yl]-2-hydroxy-2,3,3a,9,10,10a-hexahydro-1H-benzo[b]cyclopenta[f]oxepine-6-carboxylic acid

HPLC retention time (min): 0.89;
MS (ESI, Pos.): 369 (M+H-H₂O)+, 351 (M+H-2H₂O)+.;
¹H-NMR (CD₃OD): δ 7.55, 7.47, 7.14, 5.55-5.71, 5.32-5.55, 4.28-4.43, 3.96-4.10, 3.86, 3.01, 2.84, 2.62, 2.44-2.56, 1.78-2.00, 1.38-1.64, 1.14-1.31, 0.60-0.74, 0.30-0.51, -0.06-0.08.

### Example 7-47: (1R,2R,3aS,10aR)-1-[(1E,3S)-5-cyclobutyl-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.91;
MS (ESI, Pos.): 369 (M+H-H₂O)+, 351 (M+H-2H₂O)+.;
¹H-NMR (CD₃OD): δ 7.56, 7.48, 7.15, 5.56-5.63, 5.45-5.53, 4.37, 4.00, 3.86, 3.02, 2.84, 2.47-2.67, 2.29, 2.01-2.10, 1.76-1.98, 1.56-1.66, 1.33-1.52.

### Example 7-48: (1R,2R 3aS,10aR)-2-hydroxy-1-[(1E,3S)-6,7,7-trifluoro-3-hydroxy-1, 6-heptadien-1-yl]-2,3,3a,9,10,10a-hexahydro-1H-benzo[b]cyclopenta[f]oxepine-6-carboxylic acid

HPLC retention time (min): 0.86;
MS (ESI, Pos.): 395 (M+H-H₂O)+, 377 (M+H-2H₂O)+.;
¹H-NMR (CD₃OD): δ 7.55, 7.48, 7.13, 5.53-5.68, 4.38, 4.10, 3.87, 2.99, 2.80-2.89, 2.59-2.68, 2.54, 2.37-2.49, 1.73-1.95.

### Example 7-49: (1R,2R 3aS,10aR)-2-hydroxy-1-[(1E,3R)-3-hydroxy-4-propoxy-1-buten-1-yl]-2,3,3a,9,10,10a-hexahydro-1H-benzo[b]cyclopenta[floxepine-6-carboxylic acid

HPLC retention time (min): 0.77;
MS (ESI, Pos.): 399 (M+Na)+.;
¹H-NMR (CD₃OD): δ 7.57, 7.48, 7.15, 5.59-5.69, 4.36-4.40, 4.23-4.28, 3.85-3.91, 3.39-3.51, 2.98-3.05, 2.80-2.88, 2.63, 2.53, 1.80-1.95, 1.61, 0.90-0.98.

### Example 7-50: (1R,2R,3aS,10aR)-1-[(1E,3ξ,4S)-4-ethyl-3-hydroxy-1-octen-1-yl]-2-hydroxy-2,3,3a,9,10,10a-hexahydro-1H-benzo[b]cyclopenta[f]oxepine-6-carboxylic acid

HPLC retention time (min): 1;
MS (ESI, Pos.): 385 (M-OH)+.;
¹H-NMR (DMSO-d₆): δ 12.58-13.08, 7.48, 7.34, 7.18, 5.38-5.52, 4.75, 4.46, 4.34, 3.92, 3.71, 2.76-3.00, 2.54-2.62, 2.35-2.47, 1.66-1.82, 1.06-1.54, 0.80-0.90.

### Reference Example 33: 3-bromo-2-chloro-6-vinylphenol

A procedure for a purpose similar to that for Reference Example 3 was performed using 4-bromo-3-chloro-2-hydroxybenzaldehyde (CAS No.: 1427438-98-9) in place of 4-bromo-3-fluoro-2-hydroxy-benzaldehyde to yield the title compound having the following physical properties.

¹H-NMR (CDCl₃): δ 7.23-7.28, 7.15-7.19, 6.89-6.97, 5.86, 5.80, 5.37.

### Reference Example 34: ethyl (1R,2R,3aS,10aR)-5-chloro-1-formyl-2-(tetrahydro-2H-pyran-2-yloxy)-2,3,3a,9,10,10a-hexahydro-1H-benzo[b]cyclopenta[f]oxepine-6-carboxylate

A procedure for a purpose similar to that for Reference Example 4→ Reference Example 5→ Reference Example 6→ Reference Example 7→ Reference Example 8→ Reference Example 9 was performed using the compound produced in Reference Example 33 in place of the compound produced in Reference Example 3 to yield the title compound having the following physical properties.

¹H-NMR (CDCl₃): δ 9.85, 7.41, 7.02, 4.69, 4.47, 4.38, 4.22-4.27, 3.79-3.86, 3.42-3.54, 3.32-3.42, 3.09-3.24, 2.63-2.72, 2.51-2.60, 2.22-2.37, 1.97-2.05, 1.70-1.88, 1.55-1.67, 1.39, 1.22-1.33.

### Example 8: (1R,2R,3aS,10aR)-5-chloro-1-[(1E,3ξ)-4-(2,6-difluorophenyl)-4,4-difluoro-3-hydroxy-1-buten-1-yl]-2-hydroxy-2,3,3a,9,10,10a-hexahydro-1H-benzo[b]cyclopenta[f]oxepine-6-carboxylic acid

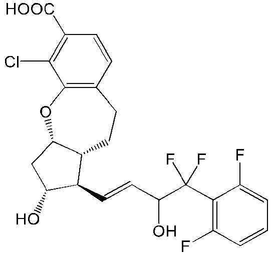

A procedure for a purpose similar to that for Reference Example 30→ Reference Example 18→ Example 2 was performed using the compound produced in Reference Example 34 in place of the compound produced in Reference Example 9 to yield the title compound having the following physical properties. The steric configuration was identified by crystal X-ray.
HPLC retention time (min): 1.045;
MS (ESI, Pos.): 523 (M+Na)+.;
¹H-NMR (CD₃OD): δ 7.50-7.58, 7.37, 7.05-7.14, 5.79, 5.65, 4.52-4.61, 4.20, 3.92, 3.07, 2.57-2.77, 1.98-2.04, 1.80, 1.50-1.70.

### Example 8-1 to 8-8:

A procedure for a purpose similar to that for Reference Example 30→ Reference Example 18→ Example 2 was performed using the compound produced in Reference Example 34 in place of the compound produced in Reference Example 9 and using a corresponding phosphonate in place of the compound produced in Reference Example 29 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 10→ Reference Example 11 or Reference Example 28→ Reference Example 29.

### Example 8-1: (1R,2R,3aS,10aR)-5-chloro-1-[(1E,3ξ)-4,4-difluoro-3-hydroxy-4-phenyl-1-buten-1-yl]-2-hydroxy-2,3,3a,9,10,10a-hexahydro-1H-benzo[b]cyclopenta[f]oxepine-6-carboxylic acid

HPLC retention time (min): 0.85;
MS (ESI, Pos.): 447, 449 (M-H₂O, M+2-H₂O)+;
¹H-NMR (CD₃OD): δ 7.51-7.56, 7.48, 7.39, 7.13, 5.58-5.65, 4.46-4.54, 4.21, 3.90, 3.06, 2.57-2.73, 1.96-2.06, 1.65-1.85, 1.53-1.63.

### Example 8-2: (1R,2R,3aS,10aR)-5-chloro-2-hydroxy-1-[(1E,3ξ)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

HPLC retention time (min): 0.88;
MS (ESI, Pos.): 437, 439 (M-H₂O, M+2-H₂O)+;
¹H-NMR (CD₃OD): δ 7.37-7.42, 7.24-7.32, 7.18-7.22, 7.12, 5.56, 5.36-5.44, 4.21, 3.83-3.92, 3.04-3.16, 2.56-2.71, 1.96-2.04, 1.80, 1.64, 0.89-1.03, 0.78-0.84, 0.71-0.76.

### Example 8-3: (1R,2R,3aS,10aR)-5-chloro-1-{(1E,3ξ)-3-[1-(2-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.117;
MS (ESI, Pos.): 451 (M+H-2H₂O)+.;
¹H-NMR (CD₃OD): δ 7.30, 7.08-7.24, 7.00, 5.54, 5.39-5.46, 4.36, 4.18, 3.83-3.90, 3.02, 2.31-2.68, 2.10, 1.96-2.05, 1.75, 1.58.

### Example 8-4: (1R,2R,3aS,10aR)-5-chloro-1-[(1E,3ξ,4ξ)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.89;
MS (ESI, Pos.): 443, 445 (M-H₂O, M+2-H₂O)+;
¹H-NMR (CD₃OD): δ 7.36, 7.30, 7.18-7.26, 7.02-7.15, 5.52, 5.26, 4.19, 4.08-4.15, 3.81, 3.05-3.16, 2.81-2.93, 2.41-2.59, 1.94, 1.57, 1.41, 1.15-1.26.

### Example 8-5: (1R,2R,3aS,10aR)-5-chloro-1-[(1E,3ξ,4ξ)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 0.92;
MS (ESI, Pos.): 443, 445 (M-H₂O, M+2-H₂O)+;
¹H-NMR (CD₃OD): δ 7.33-7.44, 7.19-7.26, 7.09-7.18, 6.99-7.08, 5.63-5.72, 5.45-5.56, 4.19-4.33, 3.91, 3.22-3.31, 2.98-3.19, 2.52-2.76, 1.97-2.07, 1.78-1.92, 1.56-1.78, 1.24-1.37.

### Example 8-6: (1R,2R,3aS,10aR)-5-chloro-1-[(1E,3ξ,4ξ)-4-(4-fluorophenyl)-3-hydroxy-1-penten-1-yl]-2-hydroxy-2,3,3a,9,10,10a-hexahydro-1H-benzo[b]cyclopenta[f]oxepine-6-carboxylic acid

HPLC retention time (min): 1.081;
MS (ESI, Pos.): 425, 427 (M-2H₂O, M+2-2H₂O)+.;
¹H-NMR (CD₃OD): δ 7.37, 7.29, 7.12, 6.99-7.06, 5.65, 5.49, 4.22-4.27, 4.14-4.21, 3.91, 3.07-3.13, 2.87, 2.58-2.76, 1.96-2.09, 1.81-1.93, 1.73, 1.24-1.30.

### Example 8-7: (1R,2R,3aS,10aR)-5-chloro-1-{(1E,3ξ)-3-[1-(2,4-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.125;
MS (ESI, Pos.): 469 (M+H-2H₂O)+.;
¹H-NMR (CD₃OD): δ 7.39, 7.09-7.17, 6.86-6.92, 6.79-6.86, 5.49-5.56, 5.40-5.47, 4.32, 4.19, 3.83-3.90, 3.02-3.09, 2.51-2.68, 2.33-2.50, 2.05-2.13, 1.94-2.01, 1.71-1.89, 1.56-1.65.

### Example 8-8: (1R,2R,3aS,10aR)-5-chloro-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

HPLC retention time (min): 1.115;
MS (ESI, Pos.): 469 (M+H-H₂O)+.;
¹H-NMR (CD₃OD): δ 7.40, 7.11-7.18, 7.00, 5.40-5.47, 5.32-5.39, 4.24, 4.17-4.21, 3.82-3.90, 3.00-3.11, 2.50-2.72, 2.24-2.43, 2.01, 1.94-2.00, 1.73-1.89, 1.60-1.71.

### Reference Example 35: methyl 3-hydroxy-2-methyl-4-vinylbenzoate

To a solution of methyltriphenylphosphonium bromide (51.5 g) in THF (93.4 mL) was added potassium tert-butoxide (14.0 g) at 0°C, followed by stirring at 0°C for 1 hour. A solution of methyl 4-formyl-3-hydroxy-2-methyl-benzoate (CAS No.: 1427361-42-9, 9.34 g) in THF (46.7 mL) was added, followed by stirring at 0°C for 3 hours. To the reaction mixture were added a saturated ammonium chloride aqueous solution and a 1 N hydrochloric acid aqueous solution at 0°C, followed by extraction with an ethyl acetate/n-hexane mixed solvent. The organic layer was washed with saturated saline, dried over anhydrous sodium sulfate, and then filtered. The filtrate was concentrated under reduced pressure. The obtained residue was purified by silica gel column chromatography (hexane : ethyl acetate = 97 : 3→90 : 10→80 : 20→70 : 30) to yield the title compound (9.22 g) having the following physical properties.

¹H-NMR (CDCl₃): δ 7.10-7.16, 7.00-7.07, 6.79-6.88, 5.69, 5.42, 5.17, 2.35.

### Reference Example 36: methyl 3-{[(1S,2R,3S,4R)-3-({[dimethyl(2-methyl-2-propanyl)silyl]oxy}methyl)-2-(1-propen-1-yl)-4-(tetrahydro-2H-pyran-2-yloxy)cyclopentyl]oxy)-2-methyl-4-vinylbenzoate

To a solution of the compound (7.0 g) produced in Reference Example 35 and the compound (13.5 g) produced in Reference Example 2 in THF (70 mL) was added triphenylphosphine (12.4 g), followed by cooling under ice cooling. To the reaction solution was added a solution of 2.2 M diethyl azodicarboxylate in toluene (22 mL), followed by stirring at room temperature overnight. The reaction solution was concentrated under reduced pressure. To the obtained residue was added MTBE: n-hexane (2 : 1, 150 mL), followed by stirring for 1 hour. The precipitate was filtered, and then the filtrate was concentrated under reduced pressure. The obtained residue was purified by silica gel column chromatography (n-hexane : ethyl acetate = 100 : 0→85 : 15) to yield the title compound (14.3 g) having the following physical properties.

¹H-NMR (CDCl₃): δ 7.58, 7.36-7.41, 7.12-7.23, 5.69-5.86, 5.54-5.64, 5.27-5.34, 4.67-4.70, 4.59, 4.26-4.41, 3.79-3.93, 3.57-3.75, 3.39-3.52, 2.99-3.09, 2.57-2.66, 2.47-2.54, 2.24-2.41, 1.46-1.94, 0.84-0.94, 0.02-0.05.

### Reference Example 37: methyl (1S,2R,3aS,10aR)-1-({[dimethyl(2-methyl-2-propanyl)silyl]oxy}methyl)-5-methyl-2-(tetrahydro-2H-pyran-2-yloxy)-2,3,3a,10a-tetrahydro-1H-benzo[b]cyclopenta[f]oxepine-6-carboxylate

A solution of the compound (14.3 g) produced in Reference Example 36 in toluene (200 mL) was stirred at 60°C for 20 minutes under a nitrogen stream to be degassed. The heating was stopped, and tricyclohexylphosphine [1,3-bis(2,4,6-trimethylphenyl)imidazol-2-ylidene][3-phenyl-1H-inden-1-ylidene]ruthenium(II) dichloride (1.21 g) was added, followed by stirring at 85°C overnight under a nitrogen stream. The reaction solution was concentrated under reduced pressure. The obtained residue was purified by silica gel column chromatography (n-hexane : ethyl acetate = 97 : 3→93 : 7) to yield the title compound (13.2 g) having the following physical properties.

¹H-NMR (CDCl₃): δ 7.52, 7.04, 6.33-6.38, 6.02-6.10, 4.67-4.76, 4.22-4.29, 4.06-4.15, 3.84-4.02, 3.47-3.57, 2.68-2.74, 2.40-2.64, 2.21-2.34, 2.12-2.21, 1.69-1.90, 1.47-1.67, 0.85-0.92, 0.04-0.10.

### Reference Example 38: methyl (1S,2R,3aS,10aR)-1-({[dimethyl(2-methyl-2-propanyl)silyl]oxy}methyl)-5-methyl-2-(tetrahydro-2H-pyran-2-yloxy)-2,3,3a,9,10,10a-hexahydro-1H-benzo[b]cyclopenta[f]oxepine-6-carboxylate

To a solution of the compound (13.2 g) produced in Reference Example 37 in ethyl acetate (132 mL) was added 10% palladium on carbon (1.32 g), followed by stirring at room temperature for 3 hours under a hydrogen stream. The reaction solution was diluted with ethyl acetate and then filtered. The filtrate was concentrated under reduced pressure. Subsequent purification was not performed, but the following reaction was performed.

### Reference Example 39: methyl (1S,2R,3aS,10aR)-1-(hydroxymethyl)-5-methyl-2-(tetrahydro-2H-pyran-2-yloxy)-2,3,3a,9,10,10a-hexahydro-1H-benzo[b]cyclopenta[f]oxepine-6-carboxylate

To a solution of the crude product produced in Reference Example 38 in THF (13.2 mL) was added a 1 mol/L solution of tetrabutylammonium fluoride in THF (52.4 mL) at room temperature, followed by stirring at room temperature overnight. After confirming completion of the reaction by TLC, the reaction solution was poured into ice water and extracted with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate =50 : 50→0 : 100, ethyl acetate : methanol = 85 : 15) to yield the title compound (9.08 g) having the following physical properties.

¹H-NMR (CDCl₃): δ 7.46, 6.94, 4.76-4.80, 4.61-4.67, 4.09-4.20, 3.85-4.01, 3.61-3.72, 3.48-3.59, 3.04-3.14, 2.95-3.00, 2.63-2.73, 2.38-2.55, 2.11-2.21, 1.96-2.06, 1.72-1.89, 1.49-1.67.

### Reference Example 40: methyl (1R,2R,3aS,10aR)-1-formyl-5-methyl-2-(tetrahydro-2H-pyran-2-yloxy)-2,3,3a,9,10,10a-hexahydro-1H-benzo[b]cyclopenta[f]oxepine-6-carboxylate

To a solution of the compound (2.0 g) produced in Reference Example 39 in dichloromethane (20 mL) were added DMSO (10 mL) and triethylamine (3.57 mL), followed by cooling to 0°C. To the reaction mixture was added a sulfur trioxide-pyridine complex (2.45 g), followed by stirring at room temperature for 2 hours. To the reaction mixture were added a saturated ammonium chloride aqueous solution and a 1 N hydrochloric acid aqueous solution, followed by extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 90 : 10→70 : 30→55 : 45) to yield the title compound (2.0 g) having the following physical properties.

¹H-NMR (CDCl₃): δ 9.82-9.86, 7.49, 6.96, 4.64-4.69, 4.44-4.53, 4.14-4.18, 3.80-3.94, 3.43-3.55, 3.34-3.40, 3.25-3.31, 3.09-3.19, 2.51-2.68, 2.46, 2.25-2.35, 2.12-2.18, 1.96-2.04, 1.69-1.88, 1.49-1.67.

### Example 9: (1R,2R,3aS,10aR)-2-hydroxy-1-[(1E,3ξ)-3-hydroxy-3-(1-phenylcyclopropyl)-1-propen-1-yl]-5-methyl-2,3,3a,9,10,10a-hexahydro-1H-benzo[b]cyclopenta[f]oxepine-6-carboxylic acid

A procedure for a purpose similar to that for Reference Example 17→ Reference Example 18→ Example 2 was performed using the compound produced in Reference Example 40 in place of the compound produced in Reference Example 9 and using the compound produced in Reference Example 27 in place of the compound produced in Reference Example 14 to yield the title compound having the following physical properties. As the intermediate corresponding to the compound produced in Reference Example 18, a high polarity compound separated by a diastereomer separation procedure was used.
HPLC retention time (min): 0.9;
MS (ESI, Pos.): 417 (M+H-H₂O)+.;
¹H-NMR (CD₃OD): δ 7.35, 7.28, 7.06-7.19, 6.90, 5.44, 5.28, 3.96-4.01, 3.72-3.80, 2.95, 2.43-2.58, 2.29-2.33, 1.80, 1.60-1.75, 1.46-1.56, 0.77-0.91, 0.59-0.72.

### Example 9-1 to 9-23:

A procedure for a purpose similar to that for Reference Example 30→ Reference Example 18→ Example 2 was performed using the compound produced in Reference Example 40 in place of the compound produced in Reference Example 9 and using a corresponding phosphonate in place of the compound produced in Reference Example 29 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 10→ Reference Example 11 or Reference Example 28→ Reference Example 29.

### Example 9-1: (1R,2R,3aS,10aR)-1-[(1E,3ξ)-4,4-difluoro-3-hydroxy-4-phenyl-1-buten-1-yl]-2-hydroxy-5-methyl-2,3,3a,9,10,10a-hexahydro-1H-benzo[b]cyclopenta[f]oxepine-6-carboxylic acid

HPLC retention time (min): 0.86;
MS (ESI, Pos.): 427 (M+H-H₂O)+.;
¹H-NMR (CD₃OD): δ 7.53-7.55, 7.45-7.50, 7.38-7.44, 6.99, 5.58-5.62, 4.46-4.53, 4.08-4.15, 3.90, 3.02, 2.66-2.74, 2.54-2.63, 2.42, 1.93, 1.66-1.79, 1.50-1.60.

### Example 9-2: (1R,2R,3aS,10aR)-1-{(1E,3ξ)-3-[1-(2-fluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid

HPLC retention time (min): 0.97;
MS (ESI, Pos.): 449 (M+H-H₂O)+.;
¹H-NMR (CD₃OD): δ 7.41, 7.10-7.24, 6.97-7.03, 5.52-5.59, 5.39-5.47, 4.36, 4.07-4.11, 3.84-3.90, 2.99-3.07, 2.48-2.67, 2.38-2.47, 2.07-2.16, 1.82-1.95, 1.68-1.78, 1.56.

### Example 9-3: (1R,2R,3aS,10aR)-1-[(1E,3ξ)-4,4-difluoro-3-hydroxy-1-octen-1-yl]-2-hydroxy-5-methyl-2,3,3a,9,10,10a-hexahydro-1H-benzo[b]cyclopenta[f]oxepine-6-carboxylic acid

HPLC retention time (min): 0.9;
MS (ESI, Pos.): 407 (M+H-H₂O)+.;
¹H-NMR (CD₃OD): δ 7.38, 6.98, 5.69-5.81, 4.15-4.26, 3.95-4.01, 3.08, 2.78, 2.60-2.68, 2.43, 1.85-2.02, 1.73, 1.34-1.56, 1.31.

### Example 9-4: (1R,2R,3aS,10aR)-1-{(1E,3ξ)-3-[1-(2-4-difluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid

HPLC retention time (min): 1.139;
MS (ESI, Pos.): 507 (M+Na)+.;
¹H-NMR (CD₃OD): δ 7.46, 7.10-7.17, 7.00, 6.86-6.92, 6.79-6.86, 5.49-5.55, 5.40-5.48, 4.32, 4.07-4.11, 3.83-3.89, 2.99-3.07, 2.51-2.68, 2.33-2.49, 2.03-2.15, 1.71-1.94, 1.58.

### Example 9-5: (1R,2R,3aS,10aR)-1-{(1E,3ξ)-3-[1-(4-fluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid

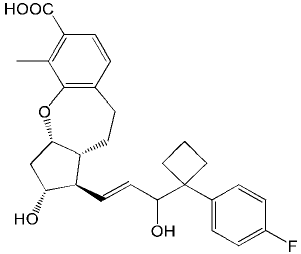

HPLC retention time (min): 1.166;
MS (ESI, Pos.): 489 (M+Na)+.;
¹H-NMR (CD₃OD): δ 7.47, 7.15, 6.97-7.04, 5.40-5.48, 5.31-5.38, 4.25, 4.05-4.13, 3.82-3.90, 3.00-3.09, 2.47-2.67, 2.25-2.43, 1.97-2.13, 1.74-1.93, 1.58-1.69.

### Example 9-6: (1R,2R,3aS,10aR)-1-{(1E,3ξ)-3-[1-(2-6-difluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid

HPLC retention time (min): 1.134;
MS (ESI, Pos.): 507 (M+Na)+.;
¹H-NMR (CD₃OD): δ 7.46, 7.16-7.24, 7.00, 6.82-6.88, 5.63-5.71, 5.43-5.50, 4.37, 4.05-4.09, 3.83-3.89, 3.00-3.08, 2.47-2.71, 2.42, 2.08-2.20, 1.71-1.94, 1.52-1.61.

### Example 9-7: (1R,2R,3aS,10aR)-2-hydroxy-1-[(1E,3ξ)-3-hydroxy-3-(1-phenylcyclobutyl)-1-propen-1-yl]-5-methyl-2,3,3a,9,10,10a-hexahydro-1H-benzo[b]cyclopenta[f]oxepine-6-carboxylic acid

HPLC retention time (min): 1.117;
MS (ESI, Pos.): 471 (M+Na)+.;
¹H-NMR (CD₃OD): δ 7.44-7.47, 7.25-7.30, 7.13-7.18, 6.99-7.02, 5.32-5.45, 4.25, 4.05-4.10, 3.82-3.89, 3.03, 2.51-2.66, 2.30-2.44, 2.01-2.09, 1.72-1.93, 1.60.

### Example 9-8: (1R,2R,3aS,10aR)-2-hydroxy-1-[(1E,3ξ)-3-hydroxy-4-methyl-4-(3-thienyl)-1-penten-1-yl]-5-methyl-2,3,3a,9,10,10a-hexahydro-1H-benzo[b]cyclopenta[f]oxepine-6 carboxylic acid

HPLC retention time (min): 1.083;
MS (ESI, Pos.): 465 (M+Na)+.;
¹H-NMR (CD₃OD): δ 7.45, 7.29-7.32, 7.12-7.16, 7.07-7.10, 6.98, 5.45-5.52, 5.30-5.38, 4.05-4.12, 3.80-3.87, 2.94-3.03, 2.51-2.62, 2.41, 1.86-1.92, 1.63-1.73, 1.48-1.59, 1.26-1.35.

### Example 9-9: (1R,2R,3aS,10aR)-2-hydroxy-1-{(1E,3ξ)-3-hydroxy-3-[1-(3-thienyl)cyclobutyl]-1-propen-1-yl}-5-methyl-2,3,3a,9,10,10a-hexahydro-1H-benzo[b]cyclopenta[f]oxepine-6-carboxylic acid

HPLC retention time (min): 1.099;
MS (ESI, Pos.): 477 (M+Na)+.;
¹H-NMR (CD₃OD): δ 7.46, 7.29-7.32, 7.06-7.08, 6.98-7.02, 5.34-5.45, 4.20-4.23, 4.06-4.10, 3.83-3.89, 2.97-3.05, 2.46-2.64, 2.35-2.44, 2.15-2.30, 1.83-1.99, 1.69-1.78, 1.53-1.62.

### Example 9-10: (1R,2R,3aS,10aR)-2-hydroxy-1-{(1E,3ξ)-3-hydroxy-3-[1-(3-thienyl)cyclopropyl]-1-propen-1-yl}-5-methyl-2,3,3a,9,10,10a-hexahydro-1H-benzo[b]cyclopenta[f]oxepine-6-carboxylic acid

HPLC retention time (min): 1.064;
MS (ESI, Pos.): 463 (M+Na)+.;
¹H-NMR (CD₃OD): δ 7.44-7.47, 7.25-7.29, 7.15-7.17, 7.09-7.12, 6.97-7.00, 5.56-5.63, 5.41-5.49, 4.09, 3.83-3.93, 2.98-3.06, 2.52-2.70, 2.41, 1.87-1.95, 1.72-1.84, 1.60, 0.93-0.98, 0.84-0.92, 0.73-0.83.

### Example 9-11: (1R,2R,3aS,10aR)-1-[(1E,3ξ,4S)-4-ethyl-3-hydroxy-1-octen-1-yl]-2-hydroxy-5-methyl-2,3,3a,9,10,10a-hexahydro-1H-benzo[b]cyclopenta[f]oxepine-6-carboxylic acid

HPLC retention time (min): 1.151;
MS (ESI, Pos.): 439 (M+Na)+.;
¹H-NMR (CD₃OD): δ 7.44, 6.98, 5.62-5.68, 5.46-5.53, 4.12-4.16, 4.03-4.08, 3.89-3.95, 3.04-3.13, 2.57-2.76, 2.42, 1.81-1.97, 1.66-1.77, 1.52-1.63, 1.40-1.50, 1.20-1.39, 0.88-0.97.

### Example 9-12: (1R,2R,3aS,10aR)-1-[(1E,3ξ)-3-(1-butylcyclobutyl)-3-hydroxy-1 propen-1-yl]-2-hydroxy-5-methyl-2,3,3a,9,10,10a-hexahydro-1H-benzo[b]cyclopenta[f]oxepine-6-carboxylic acid

HPLC retention time (min): 1.185;
MS (ESI, Pos.): 451 (M+Na)+.;
¹H-NMR (CD₃OD): δ 7.43-7.47, 6.96-7.00, 5.66-5.73, 5.50-5.57, 4.12-4.16, 3.90-3.98, 3.05-3.12, 2.57-2.78, 2.43, 2.05-2.15, 1.78-1.98, 1.58-1.75, 1.37-1.49, 1.28-1.37, 0.90-0.97, -0.03--0.01.

### Example 9-13: (1R,2R,3aS,10aR)-2-hydroxy-1-[(1E,3ξ)-3-hydroxy-4,4-dimethyl-1-octen-1-yl]-5-methyl-2,3,3a,9,10,10a-hexahydro-1H-benzo[b]cyclopenta[f]oxepine-6-carboxylic acid

HPLC retention time (min): 1.168;
MS (ESI, Pos.): 439 (M+Na)+.;
¹H-NMR (CD₃OD): δ 7.45, 6.98, 5.68-5.75, 5.46-5.52, 4.15, 3.89-3.95, 3.78, 3.05-3.13, 2.58-2.75, 2.43, 1.83-1.99, 1.72, 1.21-1.38, 0.85-0.95.

### Example 9-14: (1R,2R,3aS,10aR)-1-[(1E,3ξ,4R)-4-ethyl-3-hydroxy-1-octen-1-yl]-2-hydroxy-5-methyl-2,3,3a,9,10,10a-hexahydro-1H-benzo[b]cyclopenta[f]oxepine-6-carboxylic acid

HPLC retention time (min): 1.169;
MS (ESI, Pos.): 381 (M+H-2H₂O)+.;
¹H-NMR (CD₃OD): δ 7.44, 6.98, 5.62, 5.51, 4.13-4.16, 4.00-4.04, 3.88-3.96, 3.04-3.13, 2.67-2.76, 2.57-2.66, 2.42, 1.81-1.98, 1.67-1.77, 1.14-1.55, 0.92.

### Example 9-15: (1R,2R,3aS,10aR)-1-[(1E,3ξ)-3-(1-butylcyclopropyl)-3-hydroxy-1 propen-1-yl]-2-hydroxy-5-methyl-2,3,3a,9,10,10a-hexahydro-1H-benzo[b]cyclopenta[f]oxepine-6-carboxylic acid

HPLC retention time (min): 1.118;
MS (ESI, Pos.): 437 (M+Na)+.;
¹H-NMR (CD₃OD): δ 7.46, 6.98, 5.60-5.68, 5.47-5.55, 4.12-4.17, 3.87-3.95, 3.04-3.14, 2.56-2.76, 2.43, 1.82-1.98, 1.67-1.77, 1.45-1.64, 1.25-1.42, 0.87-0.94, 0.48-0.57, 0.23-0.33.

### Example 9-16: (1R,2R 3aS,10aR)-2-hydroxy-1-{(1E,3ξ)-3-hydroxy-3-[1-(3-methylbutyl)cyclopropyl]-1-propen-1-yl}-5-methyl-2,3,3a,9,10,10a-hexahydro-1H-benzo[b]cyclopenta[f]oxepine-6-carboxylic acid

HPLC retention time (min): 1.155;
MS (ESI, Pos.): 451 (M+Na)+.;
¹H-NMR (CD₃OD): δ 7.45, 6.98, 5.59-5.66, 5.48-5.55, 4.14, 3.88-3.96, 3.05-3.13, 2.56-2.77, 2.43, 1.81-1.99, 1.74, 1.22-1.50, 0.84-0.93, 0.49-0.57, 0.23-0.32.

### Example 9-17: (1R,2R,3aS,10aR)-2-hydroxy-1-[(1E,3ξ)-3-hydroxy-4-(trifluoromethyl)-1-octen-1-yl]-5-methyl-2,3,3a,9,10,10a-hexahydro-1H-benzo[b]cyclopenta[f]oxepine-6-carboxylic acid,

HPLC retention time (min): 1.135;
MS (ESI, Pos.): 479 (M+Na)+.;
¹H-NMR (CD₃OD): δ 7.43, 6.97, 5.68-5.75, 5.57-5.65, 4.41-4.45, 4.13, 3.90-3.97, 3.03-3.12, 2.75, 2.57-2.65, 2.42, 2.18-2.31, 1.83-1.98, 1.57-1.75, 1.45, 1.28-1.41, 0.94.

### Example 9-18: (1R,2R,3aS,10aR)-2-hydroxy-1-[(1E,3ξ)-3-hydroxy-4-methyl-4-phenoxy-1-penten-1-yl]-5-methyl-2,3,3a,9,10,10a-hexahydro-1H-benzo[b]cyclopenta[f]oxepine-6 carboxylic acid

HPLC retention time (min): 1.085;
MS (ESI, Pos.): 475 (M+Na)+.;
¹H-NMR (CD₃OD): δ 7.46, 7.24-7.30, 7.02-7.10, 6.98, 5.83-5.90, 5.61-5.69, 4.07-4.17, 3.92-3.99, 3.06-3.14, 2.71-2.81, 2.58-2.69, 2.43, 1.85-2.00, 1.67-1.78, 1.21-1.30.

### Example 9-19: (1R,2R,3aS,10aR)-2-hydroxy-1-[(1E,3ξ)-3-hydroxy-3-(1-phenoxycyclobutyl)-1-propen-1-yl]-5-methyl-2,3,3a,9,10,10a-hexahydro-1H-benzo[b]cyclopenta[f]oxepine-6-carboxylic acid

HPLC retention time (min): 1.104;
MS (ESI, Pos.): 487 (M+Na)+.;
¹H-NMR (CD₃OD): δ 7.45, 7.21-7.27, 6.99, 6.95, 6.88, 5.84-5.90, 5.59-5.66, 4.49, 4.14, 3.90-3.96, 3.13, 2.77, 2.57-2.67, 2.30-2.45, 1.81-1.99, 1.64-1.78.

### Example 9-20: (1R,2R,3aS,10aR)-1-[(1E,3ξ,4ξ)-4-fluoro-3-hydroxy-4-methyl-1-octen-1-yl]-2-hydroxy-5-methyl-2,3,3a,9,10,10a-hexahydro-1H-benzo[b]cyclopenta[f]oxepine-6-carboxylic acid,

HPLC retention time (min): 1.07;
MS (ESI, Pos.): 443 (M+Na)+, 383 (M+H-H₂O-HF)+.;
¹H-NMR (CD₃OD): δ 7.41, 6.97, 5.69-5.76, 5.59-5.66, 4.15, 4.01-4.07, 3.90-3.97, 3.04-3.12, 2.69-2.79, 2.57-2.67, 2.41, 1.68-1.97, 1.46-1.64, 1.26-1.44, 0.94.

### Example 9-21: (1R,2R,3aS,10aR)-1-[(1E,3ξ,4ξ)-4-fluoro-3-hydroxy-4-methyl-1-octen-1-yl]-2-hydroxy-5-methyl-2,3,3a,9,10,10a-hexahydro-1H-benzo[b]cyclopenta[f]oxepine-6-carboxylic acid,

HPLC retention time (min): 1.067;
MS (ESI, Pos.): 443 (M+Na)+, 383 (M+H-H₂O-HF)+.;
¹H-NMR (CD₃OD): δ 7.41, 6.97, 5.61-5.71, 4.13-4.17, 4.05-4.11, 3.90-3.97, 3.04-3.12, 2.58-2.77, 2.41, 1.84-1.97, 1.57-1.76, 1.25-1.45, 0.91-0.97.

### Example 9-22: (1R,2R,3aS,10aR)-2-hydroxy-1-{(1E,3ξ)-3-hydroxy-3-[1-(5-methyl-3-thienyl)cyclobutyl]-1-propen-1-yl}-5-methyl-2,3,3a,9,10,10a-hexahydro-1H-benzo[b]cyclopenta[f]oxepine-6-carboxylic acid

HPLC retention time (min): 1.128;
MS (ESI, Pos.): 491 (M+Na)+.;
¹H-NMR (CD₃OD): δ 7.46, 6.99, 6.76-6.80, 6.66, 5.35-5.45, 4.04-4.22, 3.82-3.90, 2.96-3.07, 2.52-2.67, 2.42, 2.08-2.28, 1.82-1.95, 1.74, 1.52-1.66.

### Example 9-23: (1R,2R,3aS,10aR)-1-[(1E,3ξ)-5-ethoxy-3-hydroxy-4,4-dimethyl-1-penten-1-yl]-2-hydroxy-5-methyl-2,3,3a,9,10,10a-hexahydro-1H-benzo[b]cyclopenta[f]oxepine-6-carboxylic acid

HPLC retention time (min): 1.094;
MS (ESI, Pos.): 441 (M+Na)+.;
¹H-NMR (CD₃OD): δ 7.46, 6.99, 5.69-5.76, 5.47-5.55, 4.13-4.18, 3.89-3.99, 3.42-3.53, 3.32-3.36, 3.28-3.34, 3.23, 3.05-3.14, 2.57-2.76, 2.43, 1.82-1.99, 1.69-1.79, 1.18, 0.87-0.95.

### Example 10: (1R,2R,3aS,10aR)-1-[(1E,3ξ,4ξ)-4-ethyl-3-hydroxy-7-methyl-1-octen-1-yl]-2-hydroxy-5-methyl-2,3,3a,9,10,10a-hexahydro-1H-benzo[b]cyclopenta[f]oxepine-6-carboxylic acid

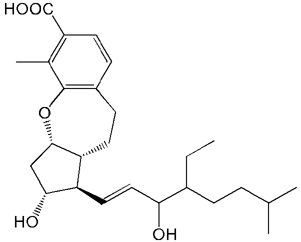

A procedure for a purpose similar to that for Reference Example 17→ Reference Example 18→ Example 2 was performed using the compound produced in Reference Example 40 in place of the compound produced in Reference Example 9 and using a corresponding phosphonate in place of the compound produced in Reference Example 14 to yield the title compound having the following physical properties. As the intermediate corresponding to the compound produced in Reference Example 18, a high polarity compound separated by a diastereomer separation procedure was used. Furthermore, the diastereomer obtained by the procedure in Example 2 was separated using an SFC column (CHIRALPAK IG, CO₂ : 2-propanol = 72 : 28) to yield a high polarity material. A corresponding phosphonate was prepared by performing a similar procedure to that in Reference Example 11 using methyl 2-ethyl-5-methylhexanoate (CAS No.: 1896760-61-4) in place of the compound produced in Reference Example 10.
HPLC retention time (min): 1.196;
MS (ESI, Pos.): 453 (M+Na)+.;
¹H-NMR (CD₃OD): δ 7.44, 6.98, 5.62-5.68, 5.46-5.53, 4.12-4.16, 4.03-4.08, 3.89-3.95, 3.04-3.12, 2.57-2.76, 2.42, 1.81-1.97, 1.67-1.76, 1.40-1.61, 1.18-1.37, 0.88-0.94.

### Reference Example 41: ethyl 6,7,7-trifluoro-3-hydroxy-3-methyl-6-heptenate

To a solution of lithium hexamethyldisilazide (1 mol/L toluene solution, 670 mL) in THF (270 mL) was added dropwise ethyl acetate (65.5 mL) at -78°C, followed by stirring at - 78°C for 15 minutes. To the reaction mixture was added a solution of 5,6,6-trifluoro-5-hexen-2-one (CAS No.: 155630-33-4, 670mmoL) in THF (40 mL), followed by stirring at - 78°C for 30 minutes. To the reaction mixture were added a saturated ammonium chloride aqueous solution and water, followed by extraction with MTBE. The organic layer was washed with saturated saline, dried over anhydrous magnesium sulfate, and then filtered. The filtrate was concentrated under reduced pressure to yield the title compound (130 g) having the following physical properties.

¹H-NMR (CDCl₃): δ 4.14-4.25, 3.68, 2.32-2.57, 1.65-1.85, 1.26-1.34, -0.06-0.20.

### Reference Example 42: 6,7,7-trifluoro-3-hydroxy-3-methyl-6-heptenate

To a solution of the compound (130 g) produced in Reference Example 41 in ethanol (270 mL) was added 5 N aqueous sodium hydroxide solution (162 mL), followed by stirring at room temperature for 1 hour. To the reaction solution, MTBE was added. The separated aqueous layer was acidified with a 5 N aqueous hydrochloric acid solution, and extracted with MTBE. The organic layer was washed with saturated saline, dried over anhydrous magnesium sulfate, and then filtered. The filtrate was concentrated under reduced pressure to yield the title compound (106 g) having the following physical properties.

¹H-NMR (CDCl₃): δ 6.33, 3.24, 2.34-2.64, 1.70-1.93, 1.32.

### Reference Example 43: (1R,2S)-2-hydroxy-1,2-diphenylethanaminium(3S)-6,7,7-trifluoro-3-hydroxy-6-octenate

To a solution of the compound (106 g) produced in Reference Example 42 in 2-butanol (1.06 L) was added (1S,2R)-diphenylethanolamine (106 g) at 80°C, followed by stirring at room temperature for 4 hours. The precipitate was filtered and washed with n-heptane. The washing procedure was further performed twice to yield the title compound (56.94 g) having the following physical properties.

¹H-NMR (CDCl₃): δ 7.18-7.31, 7.05-7.17, 5.04, 4.28, 3.49-3.85, 2.20-2.39, 1.42-1.72, 1.11-1.22, 0.93, 0.00-0.01.

### Reference Example 44: (3S)-6,7,7-trifluoro-3-methyl-6 heptene-1,3-diol

To the compound (56.4 g) produced in Reference Example 43 were added an aqueous hydrochloric acid solution and ethyl acetate for extraction to yield a carboxylic acid form (24.0 g). The carboxylic acid form was dissolved in THF (340 mL). Then, triethylamine (15.8 mL) and isobutyl chloroformate (14.7 mL) were added at -10°C, followed by stirring for 30 minutes. To the filtrate obtained by filtering the precipitate at -10°C were added sodium borohydride (6.4 g) and water (10 mL), followed by stirring at 0°C for 1 hour. To the reaction mixture was added a saturated aqueous ammonium chloride solution, followed by extraction with ethyl acetate. The organic layer was washed with a 1 N aqueous hydrochloric acid solution, water, and saturated saline, dried over anhydrous magnesium sulfate, and then filtered. The filtrate was concentrated under reduced pressure to yield the title compound (17.5 g) having the following physical properties.

¹H-NMR (CDCl₃): δ 3.86-4.08, 2.65-2.82, 2.30-2.52, 1.64-1.86, 1.27.

### Reference Example 45: (3S)-6,7,7-trifluoro-3-methyl-1-[(1-phenyl-1H-tetrazol-5-yl)thio]-6-heptene-3-ol

To a solution of the compound (17.5 g) produced in Reference Example 44 in a toluene (71 mL) were added a 5 N aqueous sodium hydroxide solution (71 mL) and tetrabutylammonium bromide (2.9 g). Furthermore, p-toluenesulfonyl chloride (18.6 g) was added at 0°C, followed by stirring at 0°C for 1 hour. To the mixture was added 1-phenyl-1H-tetrazole-5-thiol (20.5 g), followed by stirring at 60°C for 5 hours. The reaction solution was poured into ice water and extracted with toluene. The organic layer was washed with saturated saline, dried over anhydrous magnesium sulfate, and then filtered. The filtrate was concentrated under reduced pressure. The obtained residue was purified by silica gel chromatography (n-hexane : ethyl acetate = 7 : 1→4 : 1) to yield the title compound (24.8 g) having the following physical properties.

¹H-NMR (CDCl₃): δ 7.47-7.60, 3.37-3.56, 2.36-2.51, 2.29, 2.02-2.10, 1.72-1.81, 1.29.

### Reference Example 46: (3S)-6,7,7-trifluoro-3-methyl-1-[(1-phenyl-1H-tetrazol-5-yl)sulfonyl]-6-heptene-3-ol

To a mixture of sodium tungstate dihydrate (114 mg), benzenephosphonic acid (55 mg), and methyl tri-n-octylammonium chloride (140 mg) was added a hydrogen peroxide (30%) aqueous solution, followed by stirring at room temperature for 15 minutes. To the mixture was added a solution of the compound (24.8 g) produced in Reference Example 45 in toluene (70 mL), followed by stirring at 50°C for 2 hours. The reaction solution was poured into water, followed by extraction with ethyl acetate. The organic layer was washed with saturated saline, dried over anhydrous magnesium sulfate, and then filtered. The filtrate was concentrated under reduced pressure to yield the title compound (26.7 g) exhibiting the following physical properties.

¹H-NMR (CDCl₃): δ 7.55-7.73, 3.81-3.99, 2.32-2.51, 2.04-2.23, 1.71-1.85, 1.30.

### Reference Example 47: 1-phenyl-5-({(3S)-6,7,7-trifluoro-3-methyl-3-[(trimethylsilyl)oxy]-6-hepten-1-yl}sulfonyl)-1H-tetrazole

To a solution of the compound (5 g) produced in Reference Example 46 in DMF (30 mL) were added imidazole (2.6 g) and trimethylchlorosilane (2.4 mL) at room temperature for stirring. The reaction mixture was diluted with ethyl acetate, washed with saturated saline, dried over anhydrous sodium sulfate, and then filtered. The filtrate was concentrated under reduced pressure. The obtained crude product was washed with n-hexane to yield the title compound (5.1 g) having the following physical properties.

¹H-NMR (CDCl₃): δ 7.57-7.73, 3.73-3.88, 2.24-2.44, 1.96-2.19, 1.66-1.82, 1.35, 0.14-0.17.

Reference Example 48: methyl (1R,2R,3aS,10aR)-5-methyl-2-(tetrahydro-2H-pyran-2-yloxy)-1-{(1E,4S)-7,8,8-trifluoro-4-methyl-4-[(trimethylsilyl)oxy]-1,7-octadien-1-yl}-2,3,3a,9,10,10a-hexahydro-1H-benzo[b]cyclopenta[f]oxepine-6-carboxylate

To a solution of the compound (4.29 g) produced in Reference Example 47 in dimethoxyethane (19.2 mL) was added dropwise a 1 mol/L solution of potassium hexamethyldisilazide in THF (9.27 mL) at -78°C, followed by stirring for 20 minutes. To the reaction mixture was added dropwise a solution of the compound (2.4 g) produced in Reference Example 40 in dimethoxyethane (16.8 mL) at -78°C over 40 minutes, followed by stirring for 2 hours. To the reaction mixture were added a saturated aqueous sodium hydrogen carbonate solution and water, followed by extraction with ethyl acetate. The organic layer was washed with saturated saline, dried over sodium sulfate, and then filtered. The filtrate was concentrated under reduced pressure. The obtained residue was purified by silica gel chromatography (n-hexane : ethyl acetate = 90 : 10→80 : 20→70 : 30) to yield a crude product (2.11 g) containing the title compound. The product was not purified here for the next reaction.

### Reference Example 49: methyl (1R,2R,3aS,10aR)-2-hydroxy-5-methyl-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylate

To a solution of the crude product (2.11 g) produced in Reference Example 48 in methanol (21 mL) was added p-toluenesulfonic acid monohydrate (128 mg) at room temperature, followed by stirring at room temperature for 2 hours. To the reaction solution was added triethylamine (0.47 mL), followed by concentration under reduced pressure. The obtained residue was purified by silica gel chromatography (n-hexane : ethyl acetate = 70 : 30→30 : 70→0 : 100) to yield the title compound (1.49 g) exhibiting the following physical properties.

¹H-NMR (CDCl₃): δ 7.50, 6.97, 5.63-5.71, 5.39-5.46, 4.16, 3.97-4.04, 3.87, 3.07-3.15, 2.74-2.81, 2.56-2.63, 2.36-2.47, 2.27, 2.17-2.22, 2.07-2.13, 1.68-1.95, 1.44, 1.23.

### Example 11: (1R,2R,3aS,10aR)-2-hydroxy-5-methyl-1-[(1E,45)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid

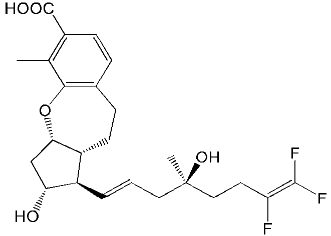

To a solution of the compound (1.49 g) produced in Reference Example 49 in THF (5 mL)/methanol (10 mL) was added a 2 N aqueous sodium hydroxide solution (6.36 mL) at room temperature, followed by stirring at 40°C overnight. The reaction solution was concentrated under reduced pressure, followed by addition of a 2 N aqueous hydrochloric acid solution under ice cooling and extraction with ethyl acetate. The organic layer was washed with saturated saline, dried over sodium sulfate, and then filtered. The filtrate was concentrated under reduced pressure. The obtained residue was crystallized with ethyl acetate (14.5 mL)/n-hexane (5.8 mL) to yield the title compound (1.16 g) exhibiting the following physical properties.
HPLC retention time (min): 1.083;
MS (ESI, Pos.): 477 (M+Na)+, 437 (M+H-H₂O)+, 419 (M+H-2H₂O)+.;
¹H-NMR (CD₃OD): δ 7.44, 6.98, 5.64-5.72, 5.36-5.43, 4.13, 3.87-3.93, 3.04-3.12, 2.56-2.74, 2.35-2.49, 2.27, 1.90-1.97, 1.80-1.87, 1.66-1.77, 1.20.

### Reference Example 50: ethyl 7,7,7-trifluoro-3-hydroxy-3-methylheptanoate

Diethyl ether (52 mL) was added to magnesium (1.53 g), and dibromoethane (0.45 mL) was added at room temperature for activation. Furthermore, bromotrifluorobutane (10 g) was added dropwise, followed by stirring at 50°C to prepare an organometallic reagent. In a separate container, a solution of N-methoxy-N-methylacetamide (5.56 mL) in diethyl ether (45 mL) was cooled to -15°C. To this solution was added dropwise the prepared organometallic reagent, followed by stirring for 90 minutes. To the reaction solution was added a saturated aqueous ammonium chloride solution, followed by extraction with diethyl ether. The organic layer was washed with saturated saline, dried over anhydrous magnesium sulfate, and then filtered to yield a ketone body (6,6,6-trifluorohexan-2-one) as a diethyl ether solution.

To a solution of lithium hexamethyldisilazide (1 mol/L THF solution, 62.8 mL) in THF (52 mL) was added dropwise ethyl acetate (6.14 mL) at -78°C, followed by stirring at - 78°C for 25 minutes. To this was added dropwise a solution of the ketone body in diethyl ether over 20 minutes, followed by stirring at -78°C for 50 minutes. To the reaction mixture were added a saturated aqueous ammonium chloride solution and water, followed by extraction with ethyl acetate. The organic layer was washed with saturated saline, dried over anhydrous magnesium sulfate, and then filtered. The filtrate was concentrated under reduced pressure. The obtained residue was purified by silica gel chromatography (n-hexane : ethyl acetate = 90 : 10→65 : 35) to yield the title compound (16.7 g) having the following physical properties.

¹H-NMR (CDCl₃): δ 4.16-4.23, 3.61, 2.42-2.54, 2.02-2.17, 1.53-1.74, 1.29, 1.25.

### Reference Example 51: 7,7,7-trifluoro-3-hydroxy-3-methylheptanoate

To a solution of the compound (16.7 g) produced in Reference Example 50 in ethanol (130 mL) was added 2 N aqueous sodium hydroxide solution (52 mL), followed by stirring at room temperature for 1 hour. To the reaction solution was added a 2 N aqueous hydrochloric acid solution to make the mixture acidic, followed by extraction with ethyl acetate. The organic layer was washed with saturated saline, dried over anhydrous magnesium sulfate, and then filtered. The filtrate was concentrated under reduced pressure to yield the title compound (8.49 g) having the following physical properties.

¹H-NMR (CDCl₃): δ 2.48-2.64, 2.02-2.21, 1.56-1.76, 1.31.

### Reference Example 52: (1R)-1-phenylethanolaminium(3S)-7,7,7-trifluoro-3-hydroxy-3-methyl heptanoate

To a solution of the compound (8.49 g) produced in Reference Example 51 in ethyl acetate (50 mL) was added (R)-1-phenylethylamine (5 mL), followed by concentration to yield a crude crystal. To the obtained crude crystals were added ethyl methyl ketone (65 mL) and n-heptane (130 mL) at 70°C. The temperature was lowered to room temperature. The precipitate was filtered and washed with n-heptane. The washing procedure was further performed twice to yield the title compound (2.64 g) having the following physical properties.

¹H-NMR (CDCl₃): δ 7.26-7.39, 6.64-7.09, 4.12-4.23, 1.84-2.12, 1.27-1.62, 1.00-1.10.

### Reference Example 53: (3S)-7,7,7-trifluoro-3-methyl-1,3-heptanediol

To the compound (2.64 g) produced in Reference Example 52 were added an aqueous hydrochloric acid solution and ethyl acetate for extraction, followed by concentration under a reduced pressure. The obtained carboxylic acid was dissolved in THF (40 mL). The solution was cooled to 0°C and lithium aluminum hydride (0.3 g) was added, followed by stirring at 60°C. The solution was cooled to 0°C and water was added, followed by stirring. The precipitate was removed by filtration, and then the filtrate was concentrated under reduced pressure to yield the title compound (1.74 g) having the following physical properties.

¹H-NMR (CDCl₃): δ 3.82-4.01, 2.41-2.61, 2.01-2.35, 1.76-1.89, 1.50-1.74.

### Reference Example 54: (3S)-7,7,7-trifluoro-3-methyl-1-[(1-phenyl-1H-tetrazol-5-yl)thio]-3-heptanol

To a solution of the compound (1.74 g) produced in Reference Example 53 in a toluene (20 mL) were added a 5 N aqueous sodium hydroxide solution (6.5 mL) and tetrabutylammonium bromide (254 mg). To the mixture was added p-toluenesulfonyl chloride (1.65 g) at 0°C, followed by stirring at 0°C for 1 hour and 45 minutes.

Furthermore, 1-phenyl-1H-tetrazole-5-thiol (1.69 g) was added, followed by stirring at 60°C overnight. The reaction solution was extracted with MTBE. The organic layer was washed with saturated saline, dried over anhydrous magnesium sulfate, and then filtered. The filtrate was concentrated under reduced pressure. The obtained residue was purified by silica gel chromatography (n-hexane : ethyl acetate = 80 : 20→60 : 40→55 : 45) to yield the title compound (2.65 g) having the following physical properties.

¹H-NMR (CDCl₃): δ 7.54-7.73, 3.85-3.94, 2.02-2.21, 1.53-1.73, 1.42-1.54, 1.28.

### Reference Example 55: (3S)-7,7,7-trifluoro-3-methyl-1-[(1-phenyl-1H-tetrazol-5-yl)sulfonyl]-3-heptanol

To a solution of the compound (2.65 g) produced in Reference Example 54 in dichloromethane (35 mL) was added meta-chloroperbenzoic acid (4.5 g) at 0°C, followed by stirring overnight. The reaction solution was poured into a saturated aqueous sodium bicarbonate solution, followed by extraction with ethyl acetate. The organic layer was washed with saturated saline, dried over anhydrous sodium sulfate, and then filtered. The filtrate was concentrated under reduced pressure. The obtained residue was purified by silica gel chromatography (n-hexane : ethyl acetate = 80 : 20→75 : 25) to yield the title compound (2.76 g) having the following physical properties.

¹H-NMR (CDCl₃): δ 7.54-7.73, 3.85-3.94, 2.02-2.21, 1.53-1.73, 1.42-1.54, 1.28.

### Reference Example 56: 1-phenyl-5-({(3S)-7,7,7-trifluoro-3-methyl-3-[(trimethylsilyl)oxy]heptyl}sulfonyl)-1H-tetrazole

A solution of the compound (2.76 g) produced in Reference Example 55 in DMF (25 mL) was cooled to 0°C, and imidazole (1.44 g) and trimethylchlorosilane (1.34 mL) were added, followed by stirring at room temperature for 3.5 hours. To the reaction solution was added water, followed by extraction with ethyl acetate/n-hexane. The organic layer was washed with saturated saline, dried over anhydrous magnesium sulfate, and then filtered. The filtrate was concentrated under reduced pressure. The obtained residue was purified by silica gel chromatography (n-hexane : ethyl acetate = 95 : 5→85 : 15) to yield the title compound (2.88 g) having the following physical properties.

¹H-NMR (CDCl₃): δ 7.66-7.72, 7.56-7.64, 3.74-3.87, 3.49, 1.95-2.17, 1.50-1.69, 1.32, 0.13-0.15.

### Reference Example 57: methyl (1R,2R,3aS,10aR)-5-methyl-2-(tetrahydro-2H-pyran-2-yloxy)-1-{(1E,4S)-8,8,8-trifluoro-4-methyl-4-[(trimethylsilyl)oxy]-1-octen-1-yl}-2,3,3a,9,10,10a-hexahydro-1H-benzo[b]cyclopenta[f]oxepine-6-carboxylate

To a solution of the compound (3.95 g) produced in Reference Example 56 in dimethoxyethane (17.6 mL) was added dropwise a 1 M solution of potassium hexamethyldisilazide in THF (8.49 mL) at -78°C, followed by stirring for 20 minutes. To the reaction mixture was added dropwise a solution of the compound (2.2 g) produced in Reference Example 40 in dimethoxyethane (15.4 mL) at -78°C over 40 minutes, followed by stirring for 30 minutes. To the reaction mixture were added a saturated aqueous sodium hydrogen carbonate solution and water, followed by extraction with ethyl acetate/n-hexane. The organic layer was washed with saturated saline, dried by addition of anhydrous sodium sulfate, and then filtered. The filtrate was concentrated under reduced pressure. The obtained residue was purified by silica gel chromatography (n-hexane : ethyl acetate = 97 : 3→90 : 10→80 : 20) to yield a crude product (3.53 g) containing the title compound. The product was not purified any more for the next reaction.

### Reference Example 58: methyl (1R,2R,3aS,10aR)-2-hydroxy-5-methyl-1-[(1E,4S)-8,8,8-trifluoro-4-hydroxy-4-methyl-1-octen-1-yl]-2,3,3a,9,10,10a-hexahydro-1H-benzo[b]cyclopenta[f]oxepine-6-carboxylate

To a solution of the crude product (3.53 g) produced in Reference Example 57 in methanol (35.3 mL) was added p-toluenesulfonic acid monohydrate (149 mg) at room temperature, followed by stirring for 3 hours. To the reaction solution was added triethylamine (0.55 mL), followed by concentration under reduced pressure. The obtained residue was purified by silica gel chromatography (n-hexane : ethyl acetate = 50 : 50→30 : 70→0 : 100) to yield the title compound (1.80 g) exhibiting the following physical properties.

¹H-NMR (CDCl₃): δ 7.50, 6.97, 5.62-5.71, 5.38-5.45, 4.16, 3.96-4.03, 3.87, 3.07-3.15, 2.73-2.80, 2.56-2.63, 2.39-2.47, 2.21-2.28, 2.03-2.17, 1.80-1.95, 1.58-1.78, 1.50-1.59, 1.47, 1.21.

### Example 12: (1R,2R,3aS,10aR)-2-hydroxy-5-methyl-1-[(1E,4S)-8,8,8-trifluoro-4-hydroxy-4-methyl-1-octen-1-yl]-2,3,3a,9,10,10a-hexahydro-1H-benzo[b]cyclopenta[f]oxepine-6-carboxylic acid

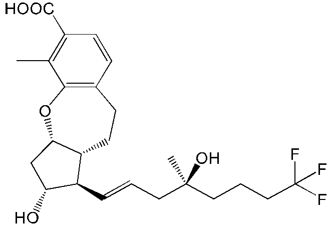

To a solution of the compound (1.80 g) produced in Reference Example 58 in THF (6 mL)-methanol (12 mL) was added a 2 N aqueous sodium hydroxide solution (6 mL) at room temperature, followed by stirring at 40°C overnight. The reaction solution was concentrated under reduced pressure, followed by addition of a 2 N aqueous hydrochloric acid solution under ice cooling and extraction with ethyl acetate. The organic layer was washed with saturated saline, dried over anhydrous sodium sulfate, and then filtered. The filtrate was concentrated under reduced pressure. The obtained crude product (1.8 g) was washed with ethyl acetate/n-hexane to yield the title compound (1.51 g) exhibiting the following physical properties.
HPLC retention time (min): 1.088;
MS (ESI, Pos.): 479 (M+Na)+, 421 (M+H-2H₂O)+.;
¹H-NMR (CD₃OD): δ 7.44, 6.98, 5.63-5.72, 5.33-5.41, 4.13, 3.86-3.94, 3.04-3.14, 2.54-2.74, 2.42, 2.25, 2.07-2.21, 1.86-1.98, 1.82, 1.61-1.75, 1.49-1.60, 1.18.

### Example 12-1 to 12-4:

A procedure for a purpose similar to that for Reference Example 57→ Reference Example 58→ Example 12 was performed using a corresponding tetrazolylsulfonyl compound in place of the compound produced in Reference Example 56 to yield the following Example compound. The corresponding tetrazolylsulfonyl compound is a known compound, or was prepared from a known compound by subjecting the compound to a procedure for a purpose similar to that for Reference Example 50→ Reference Example 51→ Reference Example 52→ Reference Example 53→ Reference Example 54→ Reference Example 55→ Reference Example 56.

### Example 12-1: (1R,2R,3aS,10aR)-2-hydroxy-1-[(1E,4S)-4-hydroxy-4-methyl-1,7-octadien-1-yl]-5-methyl-2,3,3a,9,10,10a-hexahydro-1H-benzo[b]cyclopenta[f]oxepine-6-carboxylic acid

HPLC retention time (min): 1.048;
MS (ESI, Pos.): 423 (M+Na)+.;
¹H-NMR (CD₃OD): δ 7.46, 6.98, 5.80-5.90, 5.64-5.73, 5.32-5.42, 4.99-5.06, 4.90-4.96, 4.10-4.15, 3.86-3.94, 3.04-3.12, 2.55-2.74, 2.43, 2.25, 2.08-2.20, 1.89-2.01, 1.79-1.87, 1.66-1.76, 1.51-1.61, 1.18.

### Example 12-2: (1R,2R,3aS,10aR)-2-hydroxy-1-[(1E,4S)-4-hydroxy-4-methyl-1-nonen-1-yl]-5-methyl-2,3,3a,9,10,10a-hexahydro-1H-benzo[b]cyclopenta[f]oxepine-6-carboxylic acid

HPLC retention time (min): 1.14;
MS (ESI, Pos.): 439 (M+Na)+.;
¹H-NMR (CD₃OD): δ 7.46, 6.98, 5.64-5.73, 5.31-5.39, 4.10-4.16, 3.86-3.94, 3.04-3.13, 2.54-2.74, 2.43, 2.23, 1.89-1.99, 1.78-1.86, 1.67-1.76, 1.26-1.50, 1.16, 0.92.

### Example 12-3: (1R,2R,3aS,10aR)-1-[(1E,4S)-5-cyclopentyl-4-hydroxy-4-methyl-1-penten-yl]-2-hydroxy-5-methyl-2,3,3a,9,10,10a-hexahydro-1H-benzo[b]cyclopenta[f]oxepine-6-carboxylic acid

HPLC retention time (min): 1.171;
MS (ESI, Pos.): 451 (M+Na)+.;
¹H-NMR (CD₃OD): δ 7.44, 6.98, 5.65-5.73, 5.30-5.39, 4.13, 3.87-3.93, 3.04-3.13, 2.56-2.75, 2.42, 2.26, 1.78-2.00, 1.49-1.76, 1.29, 1.13-1.21.

### Example 12-4: (1R,2R,3aS,10aR)-2-hydroxy-1-[(1E,4S)-4-hydroxy-4,7-dimethyl-1,7-octadien-1-yl]-5-methyl-2,3,3a,9,10, 10a-hexahydro-1H-benzo[b]cyclopenta[f]oxepine-6-carboxylic acid

HPLC retention time (min): 1.113;
MS (ESI, Pos.): 437 (M+Na)+.;
¹H-NMR (CD₃OD): δ 7.45, 6.97, 5.65-5.73, 5.34-5.41, 4.69, 4.13, 3.87-3.93, 3.04-3.14, 2.56-2.73, 2.42, 2.21-2.30, 2.06-2.12, 1.90-1.98, 1.79-1.87, 1.74, 1.58-1.65, 1.19.

### Example 13: (1R,2R,3aS,10aR)-2-hydroxy-1-[(1E,4ξ)-4-hydroxy-4,7-dimethyl-1-octen-1-yl]-5-methyl-2,3,3a,9,10,10a-hexahydro-1H-benzo[b]cyclopenta[f]oxepine-6-carboxylic acid

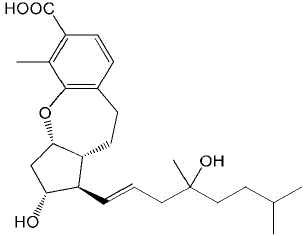

A procedure for a purpose similar to that for Reference Example 57→ Reference Example 58→ Example 12 was performed using a corresponding racemic compound in place of the compound produced in Reference Example 56 for separation into two diastereomers through an SFC column (CHIRALPAK IG, CO₂ : methanol = 69 : 31), thereby yielding the title compound (low-polarity material) exhibiting the following physical properties. The corresponding racemic compound was prepared from a known compound by subjecting the compound to a procedure for a purpose similar to that for Reference Example 50→ Reference Example 51→ Reference Example 52→ Reference Example 53→ Reference Example 54→ Reference Example 55→ Reference Example 56.
HPLC retention time (min): 1.123;
MS (ESI, Pos.): 439 (M+Na)+, 381 (M+H-2H₂O)+.;
¹H-NMR (CD₃OD): δ 7.43, 6.97, 5.72, 5.31-5.39, 4.10-4.15, 3.86-3.94, 3.04-3.12, 2.56-2.74, 2.42, 2.17-2.28, 1.90-1.99, 1.78-1.87, 1.67-1.76, 1.46-1.55, 1.23-1.30, 1.15, 0.90-0.94.

### Example 13-1: (1R,2R,3aS,10aR)-1-[(1E,4ξ)-6-cyclobutyl-4-hydroxy-4-methyl-1-hexen-1-yl]-2-hydroxy-5-methyl-2,3,3a,9,10,10a-hexahydro-1H-benzo[b]cyclopenta[f]oxepine-6-carboxylic acid

A procedure for a purpose similar to that for Reference Example 57→ Reference Example 58→ Example 12 was performed using a corresponding racemic compound in place of the compound produced in Reference Example 56 for separation into two diastereomers through an SFC column (CHIRALPAK IG, CO₂ : 2-propanol = 70 : 30), thereby yielding the title compound (low-polarity material) exhibiting the following physical properties. The corresponding racemic compound was prepared from a known compound by subjecting the compound to a procedure for a purpose similar to that for Reference Example 50→ Reference Example 51→ Reference Example 52→ Reference Example 53→ Reference Example 54→ Reference Example 55→ Reference Example 56.
HPLC retention time (min): 1.149;
MS (ESI, Pos.): 451 (M+Na)+.;
¹H-NMR (CD₃OD): δ 7.45, 6.98, 5.64-5.72, 5.32-5.39, 4.13, 3.87-3.93, 3.05-3.14, 2.56-2.73, 2.42, 2.17-2.28, 2.01-2.10, 1.78-1.98, 1.73, 1.57-1.68, 1.29-1.49, 1.15.

### Reference Example 59: 5-bromo-4-fluoro-2-vinylphenol

A procedure for a purpose similar to that for Reference Example 3 was performed using 4-bromo-5-fuloro-2-hydroxybenzaldehyde (CAS No.: 1427405-76-2) in place of 4-bromo-3-fluoro-2-hydroxy-benzaldehyde to yield the title compound having the following physical properties.

¹H-NMR (CDCl₃): δ 7.15, 7.00, 6.79-6.88, 5.71-5.77, 5.41, 5.20-5.28.

### Reference Example 60: ethyl (1R,2R,3aS,10aR)-7-fluoro-1-formyl-2-(tetrahydro-2H-pyran-2-yloxy)-2,3,3a,9,10, 10a-hexahydro-1H-benzo[b]cyclopenta[f]oxepine-6-carboxylate

A procedure for a purpose similar to that for Reference Example 4→ Reference Example 5→ Reference Example 6→ Reference Example 7→ Reference Example 8→ Reference Example 9 was performed using the compound produced in Reference Example 59 in place of the compound produced in Reference Example 3 to yield the title compound having the following physical properties.

¹H-NMR (CDCl₃): δ 9.81-9.85, 7.48-7.51, 6.84, 4.63-4.67, 4.28-4.48, 3.87-3.93, 3.78-3.85, 3.43-3.54, 3.22-3.29, 3.13-3.19, 2.99-3.08, 2.69-2.78, 2.50-2.63, 2.26-2.37, 2.17-2.23, 1.98-2.11, 1.68-1.93, 1.50-1.67, 1.38.

### Example 14: (1R,2R,3aS,10aR)-7-fluoro-1-{(1E,3ξ)-3-[1-(2-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid

A procedure for a purpose similar to that for Reference Example 17→ Reference Example 18→ Example 2 was performed using the compound produced in Reference Example 60 in place of the compound produced in Reference Example 9 and using the compound produced in Reference Example 26 in place of the compound produced in Reference Example 14 to yield the title compound having the following physical properties. As the intermediate corresponding to the compound produced in Reference Example 18, a high polarity compound separated by a diastereomer separation procedure was used.
HPLC retention time (min): 0.97;
MS (ESI, Pos.): 453 (M-OH)+.;
¹H-NMR (DMSO-d₆): δ 13.04-13.20, 7.17-7.28, 7.01-7.14, 5.32, 4.93, 4.69, 4.22-4.27, 4.16, 3.59-3.72, 2.80-2.93, 2.68-2.77, 2.21-2.32, 1.46-1.81.

### Reference Example 61: 5-bromo-3-fluoro-2-vinylphenol

A procedure for a purpose similar to that for Reference Example 3 was performed using 4-bromo-2-fuloro-6-hydroxybenzaldehyde (CAS No.: 1427438-90-1) in place of 4-bromo-3-fluoro-2-hydroxy-benzaldehyde to yield the title compound having the following physical properties.

¹H-NMR (CDCl₃): δ 6.79-6.90, 6.69, 5.79-5.97, 5.60-5.67.

### Reference Example 62: ethyl (1R,2R,3aS,10aR)-8-fluoro-1-formyl-2-(tetrahydro-2H-pyran-2-yloxy)-2,3,3a,9,10, 10a-hexahydro-1H-benzo[b]cyclopenta[f]oxepine-6-carboxylate

A procedure for a purpose similar to that for Reference Example 4→ Reference Example 5→ Reference Example 6→ Reference Example 7→ Reference Example 8→ Reference Example 9 was performed using the compound produced in Reference Example 61 in place of the compound produced in Reference Example 3 to yield the title compound having the following physical properties.

¹H-NMR (CDCl₃): δ 9.83, 7.35-7.45, 4.65, 4.41-4.54, 4.29-4.42, 4.12, 3.77-3.98, 3.39-3.66, 3.01-3.19, 2.71-2.82, 2.61, 2.28-2.47, 2.16-2.28, 1.93-2.13, 1.67-1.89, 1.48-1.66, 1.38.

### Example 15: (1R,2R,3aS,10aR)-8-fluoro-2-hydroxy-1-[(1E,3ξ)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

A procedure for a purpose similar to that for Reference Example 17→ Reference Example 18→ Example 2 was performed using the compound produced in Reference Example 62 in place of the compound produced in Reference Example 9 and using the compound produced in Reference Example 27 in place of the compound produced in Reference Example 14 to yield the title compound having the following physical properties. As the intermediate corresponding to the compound produced in Reference Example 18, a high polarity compound separated by a diastereomer separation procedure was used.
HPLC retention time (min):;
MS (ESI, Pos.): 403 (M+H-2H₂O)+, 421 (M+H-H₂O)+.;
¹H-NMR (CD₃OD): δ 7.36-7.42, 7.31-7.36, 7.25-7.30, 7.16-7.22, 5.54, 5.40, 4.46, 3.92, 3.79, 3.04, 2.68-2.81, 2.63, 2.27-2.50, 1.70-1.95, 0.87-1.11, 0.70-0.87.

### Reference Example 63: ethyl 6-hydroxy-5-vinyl-2-pyridinecarboxylate

To a solution of ethyl 5-bromo-6-hydroxy-2-pyridinecarboxylate (CAS No.: 1214346-74-3, 482 mg) in DMSO (2.4 mL) were added toluene (7.2 mL), tributylvinyltin (931 mg) and [1,1'-bis(diphenylphosphino)ferrocene]palladium(II) dichloride dichloromethane adduct (160 mg), followed by stirring at 100°C for 3 hours. The reaction mixture was concentrated under reduced pressure. The obtained residue was purified by silica gel column chromatography (hexane : ethyl acetate = 60 : 40→100 : 0) to yield the title compound (170 mg) having the following physical properties.
TLC: Rf 0.52 (hexane : ethyl acetate = 3 : 7);
¹H-NMR (DMSO-d₆): δ 11.50-12.12, 7.73, 7.02, 6.77, 6.24, 5.42, 4.30, 1.27-1.34.

### Reference Example 64: ethyl (6aR,7R,8R,9aS)-7-formyl-8-(tetrahydro-2H-pyran-2-yloxy)-6,6a,7,8,9,9a-hexahydro-5H-cyclopenta[6,7]oxepine[2,3-b]pyridine-2-carboxylate

A procedure for a purpose similar to that for Reference Example 4→ Reference Example 5→ Reference Example 6→ Reference Example 7→ Reference Example 8→ Reference Example 9 was performed using the compound produced in Reference Example 63 in place of the compound produced in Reference Example 3 to yield the title compound having the following physical properties.

¹H-NMR (CDCl₃): δ 9.86, 7.77, 7.56, 4.62-4.70, 4.37-4.49, 3.76-3.83, 3.44-3.50, 3.15-3.24, 2.94, 2.59, 2.31-2.40, 1.93-2.07, 1.77-1.86, 1.66-1.77, 1.55-1.65, 1.48-1.53, 1.41.

### Reference Example 65: ethyl (6aR,7R,8R,9aS)-7-{(1E,3ξ)-3-[1-(2-fluorophenyl)cyclobutyl]-3-hydroxy-1-propen-1-yl }-8-hydroxy-6,6a, 7, 8, 9, 9a-hexahydro-SH-cyclopenta[6,7]oxepine[2,3-b]pyridine-2-carboxylate

A procedure for a purpose similar to that for Reference Example 17 was performed using the compound (45 mg) produced in Reference Example 64 in place of the compound produced in Reference Example 9 and using the compound (49 mg) produced in Reference Example 26 in place of the compound produced in Reference Example 14 to yield an enone form (21 mg). A solution of the enone form (21 mg) in THF (0.2 mL) was cooled to -15°C. To this were added (S)-2-methyl-CBS-oxazaborolidine (0.037 mL, 1 mol/L toluene solution) and N, N-diethylaniline borane (0.013 mL), followed by stirring at -15°C overnight. The completion of the reaction was confirmed by TLC, and the reaction solution was purified by silica gel column chromatography (hexane : ethyl acetate = 50 : 50→100 : 0) to yield a reduced form. A solution of the reduced form in methanol (0.2 mL) was cooled to 0°C, and p-toluenesulfonic acid monohydrate (1.8 mg) was added, followed by stirring at room temperature for 2 hours. To the reaction solution, triethylamine (0.022 mL) was added, and then the reaction mixture was concentrated under reduced pressure. The obtained residue was purified by silica gel column chromatography (hexane : ethyl acetate = 50 : 50→100 : 0) to yield the title compound (7 mg) having the following physical properties.

¹H-NMR (CD₃OD): δ 7.62-7.67, 6.96-7.11, 6.83-6.90, 5.47-5.58, 5.25-5.46, 4.50, 4.24-4.32, 3.71, 3.42-3.61, 3.23-3.31, 2.83, 2.45-2.59, 2.23-2.45, 1.99, 1.80-1.94, 1.53-1.79, 1.29.

### Example 16: (6aR,7R,8R,9aS)-7-{(1E,3ξ)-3-[1-(2-fluorophenyl)cyclobutyl]-3-hydroxy-1-propen-1-yl }-8-hydroxy-6,6a,7,8,9,9a-hexahydro-5H-cyclopenta[6,7]oxepine[2,3-b]pyridine-2-carboxylic acid

A procedure for a purpose similar to that for Example 2 was performed using the compound produced in Reference Example 65 in place of the compound produced in Reference Example 18 to yield the title compound having the following physical properties.
HPLC retention time (min): 0.996;
MS (ESI, Pos.): 454 (M+H)+.;
¹H-NMR (CD₃OD): δ 7.61-7.73, 7.10, 6.92-7.01, 5.39-5.67, 4.52-4.63, 4.39-4.43, 3.74-3.85, 3.36, 3.26, 2.89, 2.31-2.70, 2.03-2.14, 1.59-1.99, 0.90.

### Reference Example 66: ethyl (2R,3R,3aR,11aS)-3-formyl-2-(tetrahydro-2H-pyran-2-yloxy)-1,2,3,3a,4,5,6,11a-octahydrobenzo[b]cyclopenta[g]oxocine-9-carboxylate

A procedure for a purpose similar to that for Reference Example 4→ Reference Example 5→ Reference Example 6→ Reference Example 7→ Reference Example 8→ Reference Example 9 was performed using (1R,2R,3S,4R)-2-allyl-3-([[dimethyl(2-methyl-2-propanyl)silyl]oxy}methyl)-4-(tetrahydro-2H-pyran-2-yloxy)cyclopentanol (CAS No.: 1262874-94-1) in place of the compound produced in Reference Example 2 and using the compound produced in Reference Example 31 in place of the compound produced in Reference Example 3 to yield the title compound having the following physical properties.

¹H-NMR (CDCl₃): δ 9.74, 9.70, 7.66-7.72, 7.16, 4.64-4.68, 4.31-4.43, 3.77-3.97, 3.43-3.56, 3.21, 2.94-3.08, 2.59-2.90, 2.30, 1.47-2.24, 1.40-1.47.

### Example 17: (2R,3R,3aR,11aS)-2-hydroxy-3-[(1E,3S)-3-hydroxy-1-octen-1-yl]-1,2,3,3a,4,5,6,11a-octahydrobenzo[b]cyclopenta[g]oxocine-9-carboxylic acid

A procedure for a purpose similar to that for Reference Example 17→ Reference Example 18→ Example 2 was performed using the compound produced in Reference Example 66 in place of the compound produced in Reference Example 9 and using dimethyl-2-oxoheptyl phosphonate (CAS No.: 36969-89-8) in place of the compound produced in Reference Example 14 to yield the title compound having the following physical properties. As the intermediate corresponding to the compound produced in Reference Example 18, a high polarity compound separated by a diastereomer separation procedure was used.
TLC: Rf 0.37 (CH₂Cl₂/methanol, 9/1);
MS (ESI, Pos.): 777(2M+H)+.;
¹H-NMR (CDCl₃): δ 7.67-7.72, 7.17, 5.61-5.70, 5.49, 4.56-4.64, 4.05-4.16, 3.83-3.93, 2.85-3.03, 2.55-2.68, 2.45, 1.91-2.14, 1.42-1.87, 1.20-1.52, 0.83-0.95, -0.15-0.01.

### Example 17-1 to 17-31:

A procedure for a purpose similar to that for Reference Example 17→ Reference Example 18→ Example 2 was performed using the compound produced in Reference Example 66 in place of the compound produced in Reference Example 9 and using a corresponding phosphonate in place of the compound produced in Reference Example 14 to yield the following Example compound. As the intermediate corresponding to the compound produced in Reference Example 18, a high polarity compound separated by a diastereomer separation procedure was used. The corresponding phosphonate is a known compound, or was prepared from a known compound by subjecting the compound to a procedure for a purpose similar to that for Reference Example 10→ Reference Example 11.

### Example 17-1: (2R,3R,11aS)-2-hydroxy-3-[(1E,3S)-3-hydroxy-7-methyl-1-octen-1-yl]-1,2,3,3a,4,5,6,11a-octahydrobenzo[b]cyclopenta[g]oxocine-9-carboxylic acid

MS (ESI, Neg.): 401 (M-H)-.;
¹H-NMR (CDCl₃): δ 7.66-7.73, 7.16, 5.62, 5.50, 4.59, 4.10, 3.87, 2.93, 2.59, 2.37-2.51, 1.91-2.16, 1.68-1.89, 1.40-1.68, 1.33, 1.04-1.27, 0.86.

### Example 17-2: (2R,3R,11aS)-2-hydroxy-3-[(1E,3S)-3-hydroxy-1-nonen-1-yl]-1,2,3,3a,4,5,6,11a-octahydrobenzo[b]cyclopenta[g]oxocine-9-carboxylic acid

MS (ESI, Neg.): 401 (M-H)-.;
¹H-NMR (CD₃OD): δ 7.57, 7.59, 7.16, 5.40-5.60, 4.38-4.71, 3.99, 3.78, 2.95, 2.51-2.82, 2.33, 1.82-2.10, 1.58-1.81, 1.51, 1.29, 0.75-0.95.

### Example 17-3: (2R,3R,11aS)-3-[(1E,3S)-6-cyclopropyl-3-hydroxy-1-hexen-1-yl]-2-hydroxy-1,2,3,3a,4,5,6,11a-octahydrobenzo[b]cyclopenta[g]oxocine-9-carboxylic acid

MS (ESI, Neg.): 399 (M-H)-.;
¹H-NMR (CD₃OD): δ 7.59, 7.57, 7.16, 5.39-5.63, 4.51-4.67, 4.00, 3.78, 2.94, 2.66, 2.19-2.47, 1.82-2.12, 1.69-1.79, 1.38-1.66, 0.97-1.33, 0.58-0.73, 0.22-0.50, -0.11-0.15.

### Example 17-4: (2R,3R,3aR,11aS)-2-hydroxy-3-[(1E,3ξ,4ξ)-3-hydroxy-7-methyl-4-(trifluoromethyl)-1-octen-1-yl]-1,2,3,3 a,4, 5,6,11 a-octahydrobenzo[b] cyclopenta[g] oxocine-9-carboxylic acid

HPLC retention time (min): 1.01;
MS (ESI, Pos.): 453 (M+H-H₂O)+, 435 (M+H-2H₂O)+.;
¹H-NMR (CD₃OD): δ 7.59-7.62, 7.17, 5.58-5.70, 4.58-4.63, 4.42, 3.82, 2.95, 2.69, 2.36-2.43, 2.22, 1.91-2.04, 1.47-1.86, 1.25-1.42, 0.88-0.95.

### Example 17-5: (2R,3R,3aR,11aS)-2-hydroxy-3-[(1E,3ξ,4R)-3-hydroxy-4-methyl-1-octen-1-yl]-1,2,3,3a,4,5,6,11a-octahydrobenzo[b]cyclopenta[g]oxocine-9-carboxylic acid

MS (ESI, Pos.): 385 (M+H-H₂O)+, 367 (M+H-2H₂O)+.;
¹H-NMR (CDCl₃): δ 7.61-7.78, 7.16, 5.57-5.76, 5.32-5.56, 4.59, 3.78-4.03, 2.77-3.13, 2.52-2.75, 2.47, 1.88-2.26, 1.58-1.87, 1.34-1.56, 1.17-1.33, 1.08, 0.76-0.99.

### Example 17-6: (2R,3R,3aR,11aS)-2-hydroxy-3-[(1E,3S)-3-hydroxy-5-(2-methylcyclopropyl)-1-penten-1-yl]-1,2,3,3a,4,5,6,11a-octahydrobenzo[b]cyclopenta[g]oxocine-9-carboxylic acid

MS (ESI, Pos.): 365 (M+H-2H₂O)+.;
¹H-NMR (CDCl₃): δ 7.64-7.74, 6.98-7.26, 5.58-5.77, 5.49, 4.59, 4.16, 3.74-3.94, 2.78-3.07, 2.60, 2.26-2.50, 1.90-2.25, 1.52-1.88, 1.15-1.51, 1.01, 0.63, -0.17-0.00, -0.34.

### Example 17-7: (2R,3R,3aR,11aS)-2-hydroxy-3-[(1E,3ξ)-3-hydroxy-4-methyl-1-octen-6-yn-1-yl]-1,2,3,3a,4,5,6,11a-octahydrobenzo[b]cyclopenta[g]oxocine-9-carboxylic acid

MS (ESI, Pos.): 381 (M+H-H₂O)+., 363 (M+H-2H₂O)+.;
¹H-NMR (CDCl₃): δ 7.66-7.75, 7.16, 5.46-5.72, 4.59, 4.17, 3.95-4.08, 3.88, 2.77-3.10, 2.60, 2.47, 2.17-2.34, 1.90-2.15, 1.75-1.89, 1.56-1.74, 1.49, 1.38, 1.25, 0.97, 0.76-0.91.

### Example 17-8: (2R,3R,3aR,11aS)-2-hydroxy-3-[(1E,3ξ,4S)-3-hydroxy-4-methyl-1-octen-1-yl]-1,2,3,3a,4,5,6,11a-octahydrobenzo[b]cyclopenta[g]oxocine-9-carboxylic acid

MS (ESI, Pos.): 385 (M+H-H₂O)+, 367 (M+H-2H₂O)+.;
¹H-NMR (DMSO-d₆): δ 12.67-13.00, 7.44-7.50, 7.19, 5.36, 4.73, 4.55, 4.49, 3.71, 3.52-3.67, 2.87-3.01, 2.73-2.85, 2.14, 1.90, 1.61-1.77, 1.49, 1.40, 1.14-1.31, 0.89-1.07, 0.74-0.86.

### Example 17-9: (2R,3R,3aR,11aS)-3-[(1E,3ξ)-4,4-difluoro-3-hydroxy-1-octen-1-yl]-2-hydroxy-1,2,3,3a,4,5,6,11a-octahydrobenzo[b]cyclopenta[g]oxocine-9-carboxylic acid

MS (ESI, Pos.): 407 (M+H-H₂O)+.;
¹H-NMR (DMSO-d₆): δ 12.61-12.98, 7.41-7.54, 7.19, 5.62, 5.37-5.54, 4.80, 4.50-4.64, 4.01-4.18, 3.55-3.74, 2.88-3.06, 2.68-2.88, 2.04-2.29, 1.61-1.94, 1.41-1.62, 1.33, 0.82-0.88.

### Example 17-10: (2R,3R,3aR,11aS)-2-hydroxy-3-[(1E,3ξ)-3-hydroxy-4-methylene-1-octen-1-yl]-1,2,3,3a,4,5,6,11a-octahydrobenzo[b]cyclopenta[g]oxocine-9-carboxylic acid

MS (ESI, Pos.): 383 (M+H-H₂O)+,.365 (M+H-2H₂O)+.;
¹H-NMR (DMSO-d₆): δ 12.73-12.95, 7.39-7.59, 7.20, 5.27-5.54, 5.00, 4.90, 4.66-4.82, 4.58, 4.35, 3.58-3.71, 2.94, 2.81, 2.51, 2.07-2.33, 1.83-2.05, 1.64-1.83, 1.57, 1.45, 1.17-1.42, 0.85.

### Example 17-11: (2R,3R,3aR,11aS)-3-[(1E,3ξ,4ξ)-4-fluoro-3-hydroxy-4-methyl-1-octen-1-yl]-2-hydroxy-1,2,3,3a,4,5,6,11a-octahydrobenzo[b]cyclopenta[g]oxocine-9-carboxylic acid

HPLC retention time (min): 0.95;
MS (ESI, Pos.): 383 (M+H-2H₂O)+.;
¹H-NMR (CD₃OD): δ 7.55-7.65, 7.17, 5.55-5.73, 4.60, 4.02, 3.83, 2.83-3.08, 2.56-2.83, 2.42, 1.86-2.07, 1.79, 1.43-1.63, 1.30-1.44, 1.26-1.28, 0.81-1.06.

### Example 17-12: (2R,3R,3aR,11aS)-3-[(1E,3ξ,4ξ)-4-fluoro-3-hydroxy-4-methyl-1-octen-1-yl]-2-hydroxy-1,2,3,3a,4,5,6,11a-octahydrobenzo[b]cyclopenta[g]oxocine-9-carboxylic acid

HPLC retention time (min): 0.95;
MS (ESI, Pos.): 383 (M+H-2H₂O)+.;
¹H-NMR (CD₃OD): δ 7.58-7.68, 7.17, 5.57-5.72, 4.51-4.66, 3.96-4.15, 3.83, 2.84-3.06, 2.55-2.84, 2.32-2.49, 1.87-2.10, 1.81, 1.53-1.75, 1.24-1.49, 0.85-1.02.

### Example 17-13: (2R,3R,3aR,11aS)-2-hydroxy-3-[(1E,3ξ)-3-hydroxy-4-vinyl-1-octen-1-yl]-1,2,3,3a,4,5,6,11a-octahydrobenzo[b]cyclopenta[g]oxocine-9-carboxylic acid

MS (ESI, Pos.): 379 (M+H-2H₂O)+.;
¹H-NMR (CD₃OD): δ 7.57-7.62, 7.17, 5.52-5.69, 5.40-5.51, 4.96-5.11, 4.56-4.61, 3.93, 3.79, 2.90-2.99, 2.62-2.72, 2.30-2.42, 1.86-2.14, 1.42-1.83, 1.40, 1.13-1.37, 0.83-0.94.

### Example 17-14: (2R,3R,3aR,11aS)-3-[(1E,3ξ)-4-fluoro-3-hydroxy-1-octen-1-yl]-2-hydroxy-1,2,3,3a,4,5,6,11a-octahydrobenzo[b]cyclopenta[g]oxocine-9-carboxylic acid

MS (FAB, Neg.): 405 (M-H)-.;
¹H-NMR (CD₃OD): δ 7.60-7.68, 7.19, 5.57-5.77, 4.51-4.71, 4.33-4.50, 4.20-4.33, 3.96-4.18, 3.83, 2.97, 2.70, 2.33-2.54, 1.89-2.07, 1.78-1.87, 1.46-1.72, 1.31-1.44, 0.93.

### Example 17-15: (2R,3R,3aR,11aS)-3-[(1E,3ξ,4S)-4-ethyl-3-hydroxy-1-octen-1-yl]-2-hydroxy-1,2,3,3a,4,5,6,11a-octahydrobenzo[b]cyclopenta[g]oxocine-9-carboxylic acid

HPLC retention time (min): 1.17;
MS (ESI, Pos.): 381 (M+H-2H₂O)+.;
¹H-NMR (CD₃OD): δ 7.56-7.60, 7.16, 5.54-5.62, 5.42-5.49, 4.55-4.61, 4.02, 3.79, 2.94, 2.62-2.71, 2.31-2.40, 1.86-2.02, 1.73-1.82, 1.12-1.72, 0.84-0.93.

### Example 17-16: (2R,3R,3aR,11aS)-3-[(1E,3ξ,4R)-4-ethyl-3-hydroxy-1-octen-1-yl]-2-hydroxy-1,2,3,3a,4,5,6,11a-octahydrobenzo[b]cyclopenta[g]oxocine-9-carboxylic acid

MS (ESI, Pos.): 399 (M+H-H₂O)+.;
¹H-NMR (CD₃OD): δ 7.57-7.60, 7.16, 5.54-5.62, 5.41-5.50, 4.55-4.61, 3.94-3.99, 3.79, 2.94, 2.62-2.71, 2.31-2.39, 1.86-2.05, 1.09-1.81, 0.83-0.93.

### Example 17-17: (2R,3R,3aR,11aS)-2-hydroxy-3-[(1E,3ξ,4ξ)-3-hydroxy-4-(trifluoromethyl)-1-octen-1-yl]-1,2,3,3a,4,5,6,11a-octahydrobenzo[b]cyclopenta[g]oxocine-9-carboxylic acid

HPLC retention time (min): 1.01;
MS (ESI, Pos.): 439 (M+H-H₂O)+, 421 (M+H-2H₂O)+.;
¹H-NMR (CD₃OD): δ 7.60-7.63, 7.19, 5.58-5.71, 4.59-4.64, 4.42, 3.83, 3.35-3.43, 3.28, 2.97, 2.65-2.74, 2.40, 2.17-2.31, 1.91-2.05, 1.77-1.87, 1.29-1.76, 0.93.

### Example 17-18: (2R,3R,3aR,11aS)-2-hydroxy-3-[(1E,3ξ,4ξ)-3-hydroxy-4-(trifluoromethyl)-1-octen-1-yl]-1,2,3,3a,4,5,6,11a-octahydrobenzo[b]cyclopenta[g]oxocine-9-carboxylic acid

MS (ESI, Pos.): 439 (M+H-H₂O), 421 (M+H-2H₂O)+.;
¹H-NMR (CD₃OD): δ 7.47-7.52, 7.07, 5.45-5.58, 4.46-4.53, 4.24, 3.70, 2.85, 2.58, 2.10-2.32, 1.79-1.92, 1.65-1.75, 1.51-1.66, 1.28-1.51. 1.18-1.28, 0.81.

### Example 17-19: (2R,3R,11aS)-2-hydroxy-3-[(1E,3S)-3-hydroxy-7-methyl-1,7-octadien-1-yl]-1,2,3,3a,4,5,6,11a-octahydrobenzo[b]cyclopenta[g]oxocine-9-carboxylic acid

MS (ESI, Neg.): 399 (M-H)-.;
¹H-NMR (CD₃OD): δ 7.54-7.61, 7.15, 5.41-5.62, 4.51-4.73, 4.02, 3.78, 2.94, 2.54-2.78, 2.33, 1.82-2.14, 1.63-1.78, 1.38-1.63, 1.28.

### Example 17-20: (2R,3R,11aS)-2-hydroxy-3-[(1E,3S,6Z)-3-hydroxy-1,6-octadien-1-yl]-1,2,3,3a,4,5,6,11a-octahydrobenzo[b]cyclopenta[g]oxocine-9-carboxylic acid

MS (ESI, Neg.): 385 (M-H)-.;
¹H-NMR (CD₃OD): δ 7.56-7.60, 7.16, 5.31-5.62, 4.49-4.68, 4.01, 3.78, 2.94, 2.57-2.75, 2.24-2.41, 2.08, 1.96, 1.42-1.79, 1.28.

### Example 17-21: (2R,3R,3aR,11aS)-3-[(1E,3ξ)-3-(1-butylcyclopropyl)-3-hydroxy-1-propen-1-yl]-2-hydroxy-1,2,3,3a,4,5,6,11a-octahydrobenzo[b]cyclopenta[g]oxocine-9-carboxylic acid

MS (ESI, Pos.): 379 (M+H-2H₂O)+.;
¹H-NMR (CD₃OD): δ 7.56-7.70, 7.15, 5.46-5.65, 4.60, 3.86-4.12, 3.81, 2.95, 2.62-2.77, 2.29-2.46, 1.89-2.16, 1.64-1.88, 1.48, 1.16-1.42, 0.83-1.09, 0.42-0.70, 0.17-0.39.

### Example 17-22: (2R,3R,3aR,11aS)-2-hydroxy-3-[(1E,3ξ)-3-hydroxy-3-(1-phenylcyclopropyl)-1-propen-1-yl]-1,2,3,3a,4,5,6,11a-octahydrobenzo[b]cyclopenta[g]oxocine-9-carboxylic acid

MS (ESI, Pos.): 399 (M+H-2H₂O)+.;
¹H-NMR (CD₃OD): δ 7.56-7.62, 7.30-7.37, 7.12-7.24, 5.43-5.51, 5.30-5.38, 4.51-4.56, 3.88, 3.69-3.79, 2.88-2.96, 2.57-2.66, 2.21-2.31, 1.85-1.98, 1.27-1.70, 0.83-0.97, 0.65-0.79.

### Example 17-23: (2R,3R,3aR,11aS)-2-hydroxy-3-[(1E,3ξ)-3-hydroxy-3-(1-phenylcyclobutyl)-1-propen-1-yl]-1,2,3,3a,4,5,6,11a-octahydrobenzo[b]cyclopenta[g]oxocine-9-carboxylic acid

HPLC retention time (min): 1;
MS (ESI, Pos.): 431 (M+H);
¹H-NMR (CDCl₃): δ 7.63-7.67, 7.29, 7.24-7.30, 7.07-7.19, 5.51, 5.25-5.32, 4.48-4.55, 4.30, 3.62, 2.82-2.95, 2.43-2.55, 2.19-2.43, 1.77-2.06, 1.35-1.77.

### Example 17-24: (2R,3R,3aR,11aS)-3-{(1E,3ξ)-3-[1-(2,5-difluorophenyl)cyclopropyl]-3-hydroxy-1-propen-1-yl }-2-hydroxy-1,2,3,3a,4,5,6,11a-octahydrobenzo[b]cyclopenta[g]oxocine-9-carboxylic acid

HPLC retention time (min): 0.91;
MS (ESI, Pos.): 453 (M+H-H₂O)+., 435 (M+H-2H₂O)+.;
¹H-NMR (CD₃OD): δ 7.56-7.61, 7.03-7.17, 6.89-6.99, 5.59, 5.41, 4.50-4.57, 3.89, 3.72, 2.91, 2.57-2.66, 2.22-2.31, 1.85-1.96, 1.55-1.71, 1.49, 1.38, 0.90-1.04, 0.74-0.79.

### Example 17-25: (2R,3R,3aR,11aS)-3-{(1E,3ξ)-3-[1-(2,4-difluorophenyl)cyclobutyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 1;
MS (ESI, Pos.): 467 (M+H-H₂O)+., 449 (M+H-2H₂O)+.;
¹H-NMR (CD₃OD): δ 7.55-7.61, 7.16, 7.10, 6.75-6.86, 5.35-5.49, 4.52-4.58, 4.28, 3.71, 2.84-2.99, 2.43-2.65, 2.19-2.40, 2.00, 1.78-1.95, 1.42-1.69, 1.27-1.38.

### Example 17-26: (2R,3R,3aR,11aS)-3-{(1E,3ξ)-3-[1-(2,5-difluorophenyl)cyclobutyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 0.99;
MS (ESI, Pos.): 467 (M+H-H₂O)+., 449 (M+H-2H₂O)+.;
¹H-NMR (CD₃OD): δ 7.56-7.62, 7.16, 6.91-6.97, 6.80-6.90, 5.43-5.51, 5.33-5.40, 4.50-4.56, 4.29, 3.71, 2.90, 2.44-2.66, 2.19-2.40, 1.98-2.09, 1.79-1.95, 1.43-1.67, 1.28-1.36.

### Example 17-27: (2R,3R,3aR,11aS)-3-{(1E,3ξ)-3-[1-(2,6-difluorophenyl)cyclobutyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 0.79;
MS (ESI, Pos.): 467 (M+H-H₂O)+, 449 (M+H-2H₂O)+.;
¹H-NMR (CD₃OD): δ 7.56-7.62, 7.10-7.19, 6.78, 5.59, 5.40, 4.49-4.55, 4.32, 3.71, 2.85-2.96, 2.44-2.64, 2.04-2.29, 1.77-2.01, 1.52-1.69, 1.42-1.52, 1.31-1.42.

### Example 17-28: (2R,3R,3aR,11aS)-2-hydroxy-3-{(1E,3ξ)-3-hydroxy-3-[1-(3-methylphenyl)cyclopropyl]-1-propen-1-yl}-1,2,3,3a,4,5,6,11a-octahydrobenzo[b]cyclopenta[g]oxocine-9-carboxylic acid

HPLC retention time (min): 0.77;
MS (ESI, Pos.): 431 (M+H-H₂O)+, 413 (M+H-2H₂O)+.;
¹H-NMR (CD₃OD): δ 7.56-7.61, 7.07-7.18, 6.97, 5.47, 5.34, 4.51-4.56, 3.86, 3.72, 3.33-3.37, 3.23-3.29, 2.92, 2.57-2.66, 2.17-2.36, 1.81-2.04, 1.47-1.72, 1.33-1.42, 0.82-0.97, 0.71-0.78, 0.63-0.69.

### Example 17-29: (2R,3R,3aR,11aS)-3-{(1E,3ξ)-3-[1-(3-chlorophenyl)cyclopropyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 0.77;
MS (ESI, Pos.): 452 (M+H-H₂O)+, 434 (M+H-2H₂O)+.;
¹H-NMR (CD₃OD): δ 7.56-7.62, 7.35, 7.14-7.28, 5.34-5.49, 4.51-4.56, 3.86, 3.73, 2.86-2.96, 2.62, 2.23-2.31, 1.85-2.02, 1.49-1.71, 1.32-1.44, 0.85-1.00, 0.68-0.81.

### Example 17-30: (2R,3R,3aR,11aS)-3-{(1E,3ξ)-3-[1-(3-fluorophenyl)cyclobutyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 0.78;
MS (ESI, Pos.): 449 (M+H-H₂O)+, 431 (M+H-2H₂O)+.;
¹H-NMR (CD₃OD): δ 7.60, 7.57, 7.20-7.28, 7.16, 6.93, 6.82-6.88, 5.35-5.43, 5.25-5.32, 4.50-4.55, 4.20, 3.72, 2.91, 2.47-2.66, 2.18-2.43, 1.77-2.07, 1.46-1.72, 1.27-1.40.

### Example 17-31: (2R,3R,3aR,11aS)-3-{(1E,3ξ)-3-[1-(4-fluorophenyl)cyclobutyl]-3-hydroxy-1-propen-1-yl}-2-hydroxy-1,2,3,3a,4,5,6,11a-octahydrobenzo[b]cyclopenta[g]oxocine-9-carboxylic acid

HPLC retention time (min): 0.98;
MS (ESI, Pos.): 450 (M+H-H₂O)+.;
¹H-NMR (CD₃OD): δ 7.62, 7.59, 7.11-7.21, 6.98, 5.28-5.45, 4.54-4.61, 4.22, 3.74, 2.89-3.00, 2.60-2.68, 2.49-2.57, 2.22-2.42, 1.79-2.08, 1.50-1.74, 1.34-1.45.

### Example 18: (2R,3R,3aR,11aS)-10-fluoro-2-hydroxy-3-[(1E,3ξ)-3-hydroxy-3-(1-phenoxycyclobutyl)-1-propen-1-yl]-1,2,3,3a,4,5,6,11a-octahydrobenzo[b]cyclopenta[g]oxocine-9-carboxylic acid

A procedure for a purpose similar to that for Reference Example 17→ Reference Example 18→ Example 2 was performed using a corresponding aldehyde form in place of the compound produced in Reference Example 9 and using a corresponding phosphonate in place of the compound produced in Reference Example 14 to yield the title compound exhibiting the following physical properties. As the intermediate corresponding to the compound produced in Reference Example 18, a high polarity compound separated by a diastereomer separation procedure was used. The corresponding aldehyde form was prepared by performing a procedure for a purpose similar to that for Reference Example 66 using the compound produced in Reference Example 3 in place of the compound produced in Reference Example 31. The corresponding phosphonate was prepared by performing a procedure for a purpose similar to that for Reference Example 10→ Reference Example 11 using 1-phenoxycyclobutanecarboxylic acid (CAS No.: 2287791-08-4) in place of (2S)-2-(2,4-difluorophenyl)propanoic acid.
HPLC retention time (min): 1.107;
MS (ESI, Pos.): 505 (M+Na)+.;
¹H-NMR (CD₃OD): δ 7.51-7.56, 7.22, 6.90-6.98, 6.83-6.88, 5.79-5.86, 5.55-5.62, 4.41-4.48, 3.76-3.84, 2.97-3.07, 2.76-2.86, 2.60-2.70, 2.23-2.51, 2.05-2.17, 1.92-2.03, 1.60-1.82, 1.45-1.57.

### Example 19: (2R,3R,3aR,11aS)-2-hydroxy-3-[(1E,3ξ)-3-hydroxy-3-(1-phenoxycyclobutyl)-1-propen-1-yl]-10-methyl-1,2,3,3a,4,5,6,11a-octahydrobenzo[b]cyclopenta[g]oxocine-9-carboxylic acid

A procedure for a purpose similar to that for Reference Example 17→ Reference Example 18→ Example 2 was performed using a corresponding aldehyde form in place of the compound produced in Reference Example 9 and using a corresponding phosphonate in place of the compound produced in Reference Example 14 to yield the title compound exhibiting the following physical properties. As the intermediate corresponding to the compound produced in Reference Example 18, a high polarity compound separated by a diastereomer separation procedure was used. The corresponding aldehyde form was prepared by performing a procedure for a purpose similar to that for Reference Example 66 using the compound produced in Reference Example 35 in place of the compound produced in Reference Example 31. The corresponding phosphonate was prepared by performing a procedure for a purpose similar to that for Reference Example 10→ Reference Example 11 using 1-phenoxycyclobutanecarboxylic acid in place of (2S)-2-(2,4-difluorophenyl)propanoic acid.
HPLC retention time (min): 1.134;
MS (ESI, Pos.): 501 (M+Na)+.;
¹H-NMR (CD₃OD): δ 7.53, 7.22, 7.00, 6.93, 6.83-6.88, 5.80-5.86, 5.56-5.63, 4.45, 4.22-4.27, 3.78-3.85, 3.01-3.09, 2.61-2.75, 2.48-2.58, 2.46, 2.24-2.42, 2.03-2.10, 1.93-2.02, 1.60-1.77, 1.53, 1.43.

### Example 20: (2R,3R,3aR,11aS)-2-hydroxy-3-[(1E,4S)-7,8,8-trifluoro-4-hydroxy-4-methyl-1,7-octadien-1-yl]-1,2,3,3a,4,5,6,11a-octahydrobenzo[b]cyclopenta[g]oxocine-9-carboxylic acid

A procedure for a purpose similar to that for Reference Example 48→ Reference Example 49→ Example 11 was performed using the compound produced in Reference Example 66 in place of the compound produced in Reference Example 40 to yield the title compound exhibiting the following physical properties.
HPLC retention time (min): 0.94;
MS (ESI, Pos.): 437 (M+H-H₂O)+, 419 (M+H-2H₂O)+.;
¹H-NMR (CD₃OD): δ 7.58-7.64, 7.19, 5.60-5.69, 5.35-5.43, 4.58-4.64, 3.76-3.83, 2.97, 2.62-2.73, 2.32-2.47, 2.20-2.31, 1.88-2.06, 1.65-1.82, 1.47-1.62, 1.20.

### Example 21: (2R,3R,3aR,11aS)-10-fluoro-2-hydroxy-3-[(1E,4S)-7,8,8-trifluoro-4-hydroxy-4-methyl-1,7-octadien-1-yl]-1,2,3,3a,4,5,6,11a-octahydrobenzo[b]cyclopenta[g]oxocine-9-carboxylic acid

A procedure for a purpose similar to that for Reference Example 48→ Reference Example 49→ Example 11 was performed using a corresponding aldehyde form in place of the compound produced in Reference Example 66 to yield the title compound exhibiting the following physical properties. The corresponding aldehyde form was prepared by performing a procedure for a purpose similar to that for Reference Example 66 using the compound produced in Reference Example 3 in place of the compound produced in Reference Example 31.
HPLC retention time (min): 1.079;
MS (ESI, Pos.): 495 (M+Na)+.;
¹H-NMR (CD₃OD): δ 7.50-7.55, 6.96, 5.61-5.69, 5.33-5.42, 4.41-4.46, 3.73-3.82, 2.96-3.06, 2.74-2.86, 2.60-2.69, 2.33-2.47, 2.17-2.30, 2.02-2.14, 1.88-2.02, 1.60-1.82, 1.44-1.57, 1.25-1.40, 1.17.

### Example 22: (2R,3R,3aR,5Z,11aS)-2-hydroxy-3-[(1E,3S)-3-hydroxy-1-octen-1-yl]-1,2,3,3a,4,11a-hexahydrobenzo[b]cyclopenta[g]oxocine-9-carboxylic acid

A procedure for a purpose similar to that for Reference Example 4→ Reference Example 5→ Reference Example 6→ Reference Example 8→ Reference Example 9 was performed using (1R,2R,3S,4R)-2-allyl-3-({[dimethyl(2-methyl-2-propanyl)silyl]oxy}methyl)-4-(tetrahydro-2H-pyran-2-yloxy)cyclopentanol (CAS No.: 1262874-94-1) in place of the compound produced in Reference Example 2 and using the compound produced in Reference Example 31 in place of the compound produced in Reference Example 3 to yield an aldehyde form. A procedure for a purpose similar to that for Reference Example 17→ Reference Example 18→ Example 2 was performed using the aldehyde form in place of the compound produced in Reference Example 9 and using dimethyl-2-oxoheptyl phosphonate (CAS No.: 36969-89-8) in place of the compound produced in Reference Example 14 to yield the title compound having the following physical properties. As the intermediate corresponding to the compound produced in Reference Example 18, a high polarity compound separated by a diastereomer separation procedure was used.
MS (ESI, Pos.): 351 (M+H-2H₂O)+;
¹H-NMR (CDCl₃): δ 7.71, 7.65, 7.16, 6.46, 5.85, 5.47-5.75, 4.68, 4.12, 4.02, 2.27-2.64, 1.91-2.15, 1.45-1.68, 1.18-1.42, 0.75-1.07.

### Reference Example 67: methyl (2R,3R,3aR,11aS)-2-hydroxy-3-[(1E,3ξ,4ξ)-3-hydroxy-4-(trifluoromethyl)-1-octen-1-yl]-1,2,3,3a,4,5,6,11a-octahydrobenzo[b]cyclopenta[g]oxocine-9-carboxylate

To a solution of the compound (125 mg) produced in Example 17-17 in methanol (1 mL) was added dropwise a 2 M solution of trimethylsilyldiazomethane in n-hexane (0.5 mL) at room temperature with stirring until the reaction solution turned yellow. To the reaction mixture was added acetic acid, followed by concentration under a reduced pressure. The obtained residue was purified by silica gel column chromatography (n-hexane : ethyl acetate = 1 : 1) to yield the title compound (126 mg).

### Reference Example 68: methyl (2R,3R,3aR,11aS)-2-{[dimethyl(2-methyl-2-propanyl)silyl]oxy}-3-[(1E,3ξ,4ξ)-3-hydroxy-4-(trifluoromethyl)-1-octen-1-yl]-1,2,3,3a,4,5,6,11a-octahydrobenzo[b]cyclopenta[g]oxocine-9-carboxylate

To a solution of the compound produced in Reference Example 67 in dichloromethane (2 mL) were added triethylamine (0.22 mL) and N,N-dimethyl-4-aminopyridine (13 mg) at room temperature, followed by cooling under ice cooling. Furthermore, tert-butyldiphenylchlorosilane (0.21 mL) was added, followed by stirring at room temperature for 2 days. To the reaction mixture, ethyl acetate was added for dilution. Then, saturated ammonium chloride was added, followed by extraction with ethyl acetate. The organic layer was washed with saturated saline, dried over sodium sulfate, and then filtered. The filtrate was concentrated under reduced pressure. The obtained residue was purified by silica gel column chromatography (n-hexane : ethyl acetate = 10 : 1) to yield the title compound (204 mg).

### Reference Example 69: methyl (2R,3R,3aR,11aS)-3-[(1E,3ξ,4ξ)-3-{[dimethyl(2-methyl-2-propanyl)silyl]oxy}-4-(trifluoromethyl)-1-octen-1-yl]-2-{[(2-methyl-2-propanyl)(diphenyl)silyl]oxy}-1,2,3,3a,4,5,6,11a-octahydrobenzo[b]cyclopenta[g]oxocine-9-carboxylate

To a solution of the compound produced in Reference Example 68 in dichloromethane (1.5 mL) was added 2,6-dimethylpyridine (0.1 mL) at room temperature, followed by cooling under ice cooling. tert-Butyldimethylsilyl trifluoromethanesulfonate (0.1 mL) was added, followed by stirring at room temperature for 2 hours. To the reaction mixture, ethyl acetate was added for dilution. Then, saturated ammonium chloride was added, followed by extraction with ethyl acetate. The organic layer was washed with saturated saline, dried over sodium sulfate, and then filtered. The filtrate was concentrated under reduced pressure. The obtained residue was purified by silica gel column chromatography (n-hexane : ethyl acetate = 10 : 1) to yield the title compound (174 mg).

### Reference Example 70: (2R,3R,3aR,11aS)-3-[(1E,3ξ,4ξ)-3-{[dimethyl(2-methyl-2-propanyl)silyl]oxy}-4-(trifluoromethyl)-1-octen-1-yl]-2-{[(2-methyl-2-propanyl)(diphenyl)silyl]oxy}-1,2,3,3a,4,5,6,11a-octahydrobenzo[b]cyclopenta[g]oxocine-9-carboxylic acid

To a solution of the compound produced in Reference Example 69 in dimethoxyethane (1.5 mL)-methanol (1.5 mL) was added a 2 N aqueous sodium hydroxide solution (0.5 mL) at room temperature, followed by stirring at 35°C for 15 hours. The reaction mixture was concentrated under reduced pressure, followed by addition of 1 N hydrochloric acid and extraction with ethyl acetate. The organic layer was washed with saturated saline, dried over magnesium sulfate, and then filtered. The filtrate was concentrated under reduced pressure to yield the title compound (154 mg) having the following physical properties.

¹H-NMR (CDCl₃): δ 7.63-7.71, 7.35-7.46, 7.14, 5.32-5.40, 4.34-4.40, 3.83-3.97, 2.92, 2.78-2.86, 2.52, 2.17-2.26, 1.95-2.08, 1.80-1.94, 1.38-1.68, 1.26, 1.13-1.21, 1.07, 0.77-0.85, -0.04--0.01.

### Example 23: (2R,3R,3aR,11aS)-2-hydroxy-3-[(1E,3ξ,4ξ)-3-hydroxy-4-(trifluoromethyl)-1-octen-1-yl]-N-(methylsulfonyl)-1,2,3,3a,4,5,6,11a-octahydrobenzo[b]cyclopenta[g]oxocine-9-carboxamide

To a solution of the compound (40 mg) produced in Reference Example 70 in DMF (1 mL) were added methanesulfonamide (9.4 mg), N,N-dimethyl-4-aminopyridine (12 mg) and 1-(3-dimethylaminopropyl)-3-ethylcarbodiimide hydrochloride (19 mg) at room temperature, followed by stirring at room temperature for 1 hour. To the reaction solution, THF (1 mL) and a solution of 1 M tetra-n-butylammonium fluoride in THF (0.25 mL) were added at room temperature, and the reaction mixture was stirred at 45°C for 15 hours. To the reaction mixture was added saturated ammonium chloride, followed by extraction with ethyl acetate. The organic layer was washed with saturated saline, dried over sodium sulfate, and then filtered. The filtrate was concentrated under reduced pressure. The obtained residue was purified by silica gel column chromatography (ethyl acetate : methanol = 5 : 1) to yield the title compound (2.5 mg) having the following physical properties.
HPLC retention time (min): 0.99;
MS (ESI, Pos.): 516 (M+H-H₂O)+.;
¹H-NMR (CDCl₃): δ 7.61-7.67, 7.08, 5.60-5.70, 4.55, 4.40-4.43, 3.81, 3.12, 2.80-3.03, 2.65, 2.34-2.48, 2.15-2.30, 1.90-2.07, 1.27-1.81, 0.93.

### Reference Example 71: (2R,3R,3aR,11aS)-3-[(1E,3ξ,4ξ)-3-{[dimethyl(2-methyl-2-propanyl)silyl]oxy}-4-(trifluoromethyl)-1-octen-1-yl]-2-{[(2-methyl-2-propanyl)(diphenyl)silyl]oxy}-1,2,3,3a,4,5,6,11a-octahydrobenzo[b]cyclopenta[g]oxocine-9-carboxamide

To a solution of the compound (114 mg) produced in Reference Example 70 in DMF (1.1 mL) were added ammonium chloride (15 mg), 7-aza-1-hydroxybenzotriazole (38 mg), diisopropylethylamine (0.1 mL) and 1-(3-dimethylaminopropyl)-3-ethylcarbodiimide hydrochloride (54 mg) at room temperature, followed by stirring at room temperature for 15 hours. To the reaction mixture, ethyl acetate was added for dilution. Then, a saturated ammonium chloride solution was added, followed by extraction with ethyl acetate. The organic layer was washed with saturated saline, dried over sodium sulfate, and then filtered. The filtrate was concentrated under reduced pressure. The obtained residue was purified by silica gel column chromatography (n-hexane : ethyl acetate = 1 : 1) to yield the title compound (105 mg).

### Reference Example 72: (2R,3R,3aR,11aS)-3-[(1E,3ξ,4ξ)-3-{[dimethyl(2-methyl-2-propanyl)silyl]oxy}-4-(trifluoromethyl)-1-octen-1-yl]-2-{[(2-methyl-2-propanyl)(diphenyl)silyl]oxy}-1,2,3,3a,4,5,6,11a-octahydrobenzo[b]cyclopenta[g]oxocine-9-carbonitrile

The compound produced in Reference Example 71 was dissolved in a THF (2 mL) solution, followed by addition of pyridine (0.03 mL) at room temperature. The reaction solution was cooled under ice cooling, followed by addition of trifluoroacetic anhydride (0.03 mL) and stirring at room temperature for 1 hour. To the reaction mixture, ethyl acetate was added for dilution. Then, a saturated ammonium chloride solution was added, followed by extraction with ethyl acetate. The organic layer was washed with saturated saline, dried over sodium sulfate, and then filtered. The filtrate was concentrated under reduced pressure to yield the title compound (99.7 mg) having the following physical properties.

¹H-NMR (CDCl₃): δ 7.64-7.72, 7.34-7.47, 7.18-7.25, 7.10-7.15, 5.36-5.41, 4.33-4.44, 3.89, 2.87, 2.48-2.54, 2.14-2.33, 1.77, 1.44-1.60, 1.13-1.35, 1.07, 0.80-0.89, -0.04-0.03.

### Example 24: (2R,3R,3aR,11aS)-3-[(1E,3ξ,4ξ)-3-hydroxy-4-(trifluoromethyl)-1-octen-1-yl]-9-(1H-tetrazol-5-yl)-1,2,3,3a,4,5,6,11a-octahydrobenzo[b]cyclopenta[g]oxocin-2-ol

To a solution of the compound (99.7 mg) produced in Reference Example 72 in toluene (2 mL) were added dibutyltin oxide (38 mg) and azidotrimethylsilane (58 mg) at room temperature, followed by stirring at 95°C for 15 hours. To the reaction mixture, ethyl acetate was added for dilution, and the resultant solution was purified by silica gel column chromatography (n-hexane : ethyl acetate = 3 : 1) to yield a tetrazole compound (98 mg). To a solution of the obtained tetrazole compound in THF (1 mL) was added 1 M tetra-n-butylammonium fluoride in THF (0.17 mL), followed by stirring at 35°C for 15 hours. To the reaction mixture was added 1 N hydrochloric acid, followed by extraction with ethyl acetate. The organic layer was washed with saturated saline, dried over magnesium sulfate, and then filtered. The filtrate was concentrated under reduced pressure. The obtained residue was purified by silica gel column chromatography (ethyl acetate : methanol = 3 : 1) to yield the title compound (35.2 mg) having the following physical properties.
HPLC retention time (min): 0.99;
MS (ESI, Pos.): 463 (M+H-H₂O)+.;
¹H-NMR (CD₃OD): δ 7.64, 7.60, 7.31, 5.56-5.75, 4.74, 4.43, 3.84, 3.02-3.13, 2.87-3.00, 2.68-2.76, 2.39, 2.16-2.31, 1.79-2.03, 1.52-1.75, 1.30-1.50, 0.93.

### Example 24-1: (1R,2R,3aS,10aR)-1-[(1E,3S)-3-hydroxy-1-octen-1-yl]-6-(1H-tetrazol-5-yl)-2,3,3a,9,10,10a-hexahydro-1H-benzo[b]cyclopenta[f]oxepin-2-ol

A procedure for a purpose similar to that for Reference Example 67→ Reference Example 68→ Reference Example 69→ Reference Example 70→ Reference Example 71→ Reference Example 72→ Example 24 was performed using the compound produced in Example 17 in place of the compound produced in Example 17-17 to yield the title compound having the following physical properties.
TLC: Rf 0.26 (CH₂Cl₂/methanol, 5/1)
MS (APCI, Pos.): 381 (M-H₂O)+.;
¹H-NMR (CD₃OD): δ 7.51-7.61, 7.21, 5.45-5.66, 4.39-4.47, 4.04, 3.87, 2.96-3.08, 2.79-2.92, 2.48-2.76, 1.80-1.97, 1.31-1.64, 0.88-0.98.

### Reference Example 73: ethyl (1S,2R,3aS,10aR)-5-fluoro-2-hydroxy-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

To a solution (50 mL) of the compound (8.3 g) produced in Reference Example 8 in dichloromethane were added triethylamine (9.1 mL), tert-butyldiphenylchlorosilane (8.9 mL) and dimethylaminopyridine (0.31 g) at room temperature, followed by stirring at 35°C for 3 hours. The reaction solution was poured into a 1 N aqueous hydrochloric acid solution/ethyl acetate, followed by extraction with ethyl acetate. The organic layer was washed with saturated saline, dried over anhydrous sodium sulfate, and then filtered. The filtrate was concentrated under reduced pressure. The obtained crude product was dissolved in methanol (50 mL), and p-toluenesulfonic acid monohydrate (378 mg) was added at room temperature, followed by stirring at room temperature for 1 hour. To the reaction solution, triethylamine (3.0 mL) was added, and the solvent was concentrated under reduced pressure. The obtained residue was purified by silica gel column chromatography (n-hexane : ethyl acetate = 90 : 10→30 : 70) to yield the title compound (10.7 g) exhibiting the following physical properties.

¹H-NMR (CDCl₃): δ 7.63-7.70, 7.37-7.52, 6.85, 4.34-4.41, 4.19-4.26, 3.88-3.93, 3.65-3.71, 2.97-3.05, 2.63-2.71, 2.55, 2.20-2.33, 2.12-2.20, 2.00-2.07, 1.84-1.92, 1.71-1.79, 1.38, 1.06.

### Reference Example 74: ethyl (1S,2R,3aS,10aR)-5-fluoro-2-{[(4-methylphenyl)sulfonyl]oxy}-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

To a solution (78 mL) of the compound (15.6 g) produced in Reference Example 73 in acetonitrile were added triethylamine (12.8 mL), trimethylamine hydrochloride (344 mg) and p-toluenesulfonyl chloride (6.34 g) at room temperature, followed by stirring at room temperature for 3 hours. The reaction solution was poured into a 1 N aqueous hydrochloric acid solution/ethyl acetate, followed by extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 90 : 10→70 : 30) to yield the title compound (20.2 g) exhibiting the following physical properties.

¹H-NMR (CDCl₃): δ 7.81, 7.56-7.66, 7.35-7.49, 7.29, 6.82, 5.05-5.11, 4.33-4.40, 4.25, 3.80-3.87, 3.55-3.61, 2.86-2.94, 2.53-2.61, 2.40-2.48, 2.25-2.34, 2.16-2.23, 2.03-2.13, 1.66-1.76, 1.48-1.53, 1.38, 1.03.

### Reference Example 75: ethyl (1S,3aS,10aR)-5-fluoro-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

To a solution of the compound (20.2 g) produced in Reference Example 74 in dimethoxyethane (150 mL)-DMF (10 mL) were added water (15 mL), sodium iodide (16.6 g) and zinc (14.4 g) at room temperature, followed by stirring at 80°C overnight. Furthermore, sodium iodide (4 g) and zinc (3.5 g) were added, followed by stirring at 80°C for 5 hours. The reaction mixture was cooled to room temperature, followed by addition of water and extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 95 : 5→5 : 1) to yield the title compound (13.1 g) exhibiting the following physical properties.

¹H-NMR (CDCl₃): δ 7.64-7.69, 7.36-7.46, 6.84, 4.34-4.41, 3.63-3.74, 2.94-3.02, 2.68-2.76, 2.23-2.33, 1.89-2.12, 1.79-1.87, 1.56-1.62, 1.48-1.54, 1.38, 1.06.

### Reference Example 76: ethyl (1S,3aS,10aR)-5-fluoro-1-formyl-2,3,3a,9,10,10a-hexahydro-1H-benzo[b]cyclopenta[f]oxepine-6-carboxylate

A procedure for a purpose similar to that for Reference Example 8→ Reference Example 9 was performed using the compound produced in Reference Example 75 in place of the compound produced in Reference Example 7 to yield the title compound exhibiting the following physical properties.

¹H-NMR (CDCl₃): δ 9.73, 7.45-7.51, 6.88, 4.43, 4.34-4.41, 2.98-3.12, 2.74-2.85, 2.42-2.50, 2.26-2.38, 2.13-2.23, 1.92-2.07, 1.38.

### Example 25: (1R,3aS,10aR)-5-fluoro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

A procedure for a purpose similar to that for Reference Example 17 was performed using the compound (70 mg) produced in Reference Example 76 in place of the compound produced in Reference Example 9 and using the compound (136 mg) produced in Reference Example 26 in place of the compound produced in Reference Example 14 to yield an enone form (97 mg). A solution of the enone form (97 mg) in THF (1 mL) was cooled to -15°C. To this were added (S)-2-methyl-CBS-oxazaborolidine (1 mol/L toluene solution, 0.204 mL) and N, N-diethylaniline borane (0.073 mL), followed by stirring at -15°C for 8 hours. To the reaction solution were added methanol and water, followed by extraction with ethyl acetate. The organic layer was dried over anhydrous sodium sulfate, and then filtered. The filtrate was concentrated under reduced pressure. The obtained residue was purified by silica gel column chromatography (Gold High Performance Silica manufactured by ISCO, n-hexane : ethyl acetate) to yield a reduced form. The reduced form was dissolved in methanol (1 mL) and dimethoxyethane (0.5 mL), followed by addition of a 2 N aqueous sodium hydroxide solution (0.5 mL) and stirring overnight. After confirming completion of the reaction by TLC, the reaction solution was concentrated, acidified with an aqueous hydrochloric acid solution, and then extracted with ethyl acetate. The organic layer was dried over anhydrous magnesium sulfate, and then filtered. The filtrate was concentrated under reduced pressure. The obtained residue was purified by an SFC column (CHIRALPAK IG, CO₂ : methanol = 65 : 35) to yield the title compound (19 mg) having the following physical properties.
HPLC retention time (min): 1.09;
MS (ESI, Pos.): 437 (M+H-H₂O)+.;
¹H-NMR (CD₃OD): δ 7.28-7.33, 6.97-7.12, 6.81-6.90, 5.26-5.39, 4.77, 4.20-4.26, 2.86-2.93, 2.35-2.64, 2.30, 1.80-2.07, 1.68-1.79, 1.51-1.68, 1.24-1.35.

### Example 25-1 to 25-15:

A procedure for a purpose similar to that for Example 25 was performed using a corresponding phosphonate in place of the compound produced in Reference Example 26 to yield the title compound exhibiting the following physical properties. In Example 25-6, the reduction reaction was performed using (R)-2-methyl-CBS-oxazaborolidine in place of (S)-2-methyl-CBS-oxazaborolidine used in Example 25. The corresponding phosphonate is a known compound, or was prepared from a known compound by subjecting the compound to a procedure for a purpose similar to that for Reference Example 10→ Reference Example 11 or Reference Example 28→ Reference Example 29.

### Example 25-1: (1R,3aS,10aR)-5-fluoro-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

HPLC retention time (min): 1.05;
MS (ESI, Pos.): 423 (M+H-H₂O)+.;
¹H-NMR (DMSO-d₆): δ 12.96-13.32, 7.28-7.47, 7.11-7.20, 7.07, 5.44-5.53, 5.22-5.31, 4.92, 4.41, 3.96-4.01, 3.49-3.49, 2.93-3.04, 2.59-2.63, 2.10-2.21, 1.92-2.04, 1.80-1.92, 1.54-1.71, 1.27-1.41, 0.96-1.08, 0.73, 0.62.

### Example 25-2: (1R,3aS,10aR)-1-[(1E,3ξ)-4,4-difluoro-3-hydroxy-4-phenyl-1-buten-1-yl]-5-fluoro-2,3,3a,9,10,10a-hexahydro-1H-benzo[b]cyclopenta[f]oxepine-6-carboxylic acid

HPLC retention time (min): 0.97;
MS (ESI, Pos.): 433 (M+H)+.;
¹H-NMR (DMSO-d₆): δ 13.12, 7.49-7.57, 7.42-7.48, 7.07, 5.87, 5.46-5.66, 4.40-4.54, 2.89-3.09, 2.74-2.87, 2.58-2.73, 2.13-2.23, 1.97-2.07, 1.70-1.93, 1.66, 1.33-1.45.

### Example 25-3: (1R,3aS,10aR)-1-[(1E,3ξ)-4,4-difluoro-4-(4-fluorophenyl)-3-hydroxy-1-buten-1-yl]-5-fluoro-2,3,3a,9,10,10a-hexahydro-1H-benzo[b]cyclopenta[f]oxepine-6-carboxylic acid

HPLC retention time (min): 1.01;
MS (ESI, Pos.): 451 (M+H)+., 433 (M+H-H₂O)+.;
¹H-NMR (CD₃OD): δ 7.40-7.46, 7.29-7.34, 7.08, 6.83, 5.46-5.54, 5.35-5.43, 4.77, 4.25-4.38, 2.87-2.95, 2.53-2.70, 2.02-2.12, 1.82-2.00, 1.55-1.74, 1.23-1.38.

### Example 25-4: (1R,3aS,10aR)-1-[(1E,3ξ,4ξ)-4-(2,4-difluorophenyl)-3-hydroxy-1-penten-1-yl]-5-fluoro-2,3,3a,9,10,10a-hexahydro-1H-benzo[b]cyclopenta[f]oxepine-6-carboxylic acid

HPLC retention time (min): 1.218;
MS (ESI, Pos.): 429 (M-OH)+.;
¹H-NMR (DMSO-d₆): δ 13.07, 7.33-7.42, 7.07-7.14, 7.00, 5.42-5.49, 5.33-5.41, 4.84, 4.41, 4.02-4.12, 3.17, 2.90-3.09, 2.73-2.83, 2.52-2.61, 2.07-2.18, 1.90-2.02, 1.77-1.89, 1.72, 1.28-1.40, 1.16.

### Example 25-5: (1R,3aS,10aR)-5-fluoro-1-[(1E,3ξ)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

HPLC retention time (min): 1.17;
MS (ESI, Pos.): 405 (M+H-H₂O)+.;
¹H-NMR (CD₃OD): δ 7.41-7.46, 7.32-7.38, 7.22-7.28, 7.14-7.20, 6.95, 5.42-5.48, 5.32-5.40, 4.35, 3.83, 2.98-3.07, 2.69-2.80, 2.58-2.68, 2.09-2.19, 1.90-2.06, 1.68-1.87, 1.34-1.45, 0.93-0.99, 0.84-0.91, 0.75-0.81, 0.68-0.74.

### Example 25-6: (1R,3aS,10aR)-5-fluoro-1-[(1E,3ξ,4S)-3-hydroxy-4-methyl-1-octen-1-yl]-2,3,3a,9,10,10a-hexahydro-1H-benzo[b]cyclopenta[f]oxepine-6-carboxylic acid

HPLC retention time (min): 1.273;
MS (ESI, Pos.): 373 (M-OH)+.;
¹H-NMR (DMSO-d₆): δ 7.30-7.37, 6.97, 5.35-5.50, 4.56, 4.41, 3.73, 2.93-3.02, 2.74-2.82, 2.59, 2.10-2.19, 1.95-2.03, 1.70-1.89, 1.37-1.47, 1.15-1.35, 0.94-1.06, 0.87, 0.79.

### Example 25-7: (1R,3aS,10aR)-5-fluoro-1-[(1E,3ξ)-3-hydroxy-4-methyl-4-phenyl-1-penten-1-yl]-2,3,3a,9,10,10a-hexahydro-1H-benzo[b]cyclopenta[f]oxepine-6-carboxylic acid

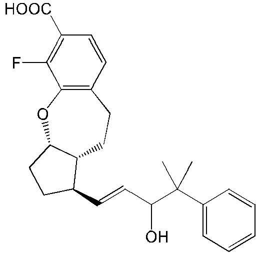

HPLC retention time (min): 1.212;
MS (ESI, Pos.): 447 (M+Na)+.;
¹H-NMR (CD₃OD): δ 7.41-7.46, 7.36-7.41, 7.28, 7.12-7.19, 6.92, 5.25-5.39, 4.28-4.35, 4.15, 2.87-2.98, 2.62-2.72, 2.48-2.59, 2.08-2.17, 1.88-2.04, 1.48-1.68, 1.33.

### Example 25-8: (1R,3aS,10aR)-1-[(1E,3ξ)-3-(1-butylcyclopropyl)-3-hydroxy-1-propen-1-yl]-5-fluoro-2,3,3a,9,10,10a-hexahydro-1H-benzo[b]cyclopenta[f]oxepine-6-carboxylic acid

HPLC retention time (min): 1.27;
MS (ESI, Pos.): 385 (M+H-H₂O)+.;
¹H-NMR (CD₃OD): δ 7.41-7.47, 6.94, 5.47-5.59, 4.41, 3.82-3.88, 3.00-3.11, 2.65-2.85, 2.16-2.25, 2.05-2.14, 1.80-2.03, 1.43-1.62, 1.24-1.40, 0.85-0.96, 0.41-0.56, 0.20-0.34.

### Example 25-9: (1R,3aS,10aR)-5-fluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid

HPLC retention time (min): 1.089;
MS (ESI, Pos.): 421 (M+H-H₂O)+.;
¹H-NMR (CD₃OD): δ 7.44-7.50, 7.36, 7.24-7.29, 7.08, 6.97, 5.49-5.57, 5.31-5.41, 4.97-5.03, 4.92, 4.50, 4.38, 2.98-3.07, 2.73-2.82, 2.60-2.70, 2.13-2.22, 1.93-2.09, 1.68-1.82, 1.38-1.49.

### Example 25-10: (1R,3aS,10aR)-5-fluoro-1-[(1E,3ξ)-3-hydroxy-4-methyl-4-(3-thienyl)-1-penten-1-yl]-2,3,3a,9,10,10a-hexahydro-1H-benzo[b]cyclopenta[f]oxepine-6-carboxylic acid

HPLC retention time (min): 1.18;
MS (ESI, Pos.): 453 (M+Na)+, 413 (M+H-H₂O)+.;
¹H-NMR (CD₃OD): δ 7.39-7.45, 7.28-7.32, 7.11-7.14, 7.06-7.08, 6.92, 5.29-5.41, 4.31-4.36, 4.07, 2.92-3.01, 2.63-2.77, 2.52-2.63, 2.09-2.19, 1.89-2.06, 1.58-1.74, 1.26-1.45.

### Example 25-11: (1R,3aS,10aR)-5-fluoro-1-{(1E,3ξ)-3-[1-(4-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

HPLC retention time (min): 1.23;
MS (ESI, Pos.): 477 (M+Na)+.;
¹H-NMR (CD₃OD): δ 7.43-7.48, 7.14, 6.94-7.02, 5.38-5.47, 5.21-5.30, 4.36, 4.22, 2.97-3.07, 2.71-2.82, 2.56-2.68, 2.47-2.56, 2.24-2.40, 1.91-2.19, 1.71-1.88, 1.35-1.46.

### Example 25-12: (1R,3aS,10aR)-1-[(1E,3ξ)-3-(1-benzylcyclobutyl)-3-hydroxy-1-propen-1-yl]-5-fluoro-2,3,3a,9,10,10a-hexahydro-1H-benzo[b]cyclopenta[f]oxepine-6-carboxylic acid

HPLC retention time (min): 1.276;
MS (ESI, Pos.): 473 (M+Na)+.;
¹H-NMR (CD₃OD): δ 7.33-7.38, 7.08-7.20, 6.85, 5.58-5.65, 5.40-5.47, 4.78, 4.32, 3.86, 2.94-3.02, 2.79, 2.61-2.75, 2.57, 1.72-2.17, 1.53-1.66, 1.34-1.46.

### Example 25-13: (1R,3aS,10aR)-5-fluoro-1-{(1E,3ξ)-3-[3-(2-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid

HPLC retention time (min): 1.095;
MS (ESI, Pos.): 479 (M+Na)+.;
¹H-NMR (CD₃OD): δ 7.42, 7.26-7.33, 7.14-7.21, 7.02-7.10, 6.93, 5.44-5.54, 4.89-5.01, 4.49, 4.32-4.37, 2.94-3.04, 2.58-2.78, 1.91-2.18, 1.61-1.78, 1.35-1.47.

### Example 25-14: (1R,3aS,10aR)-1-[(1E,3ξ)-5-ethoxy-3-hydroxy-4,4-dimethyl-1-penten-1-yl]-5-fluoro-2,3,3a,9,10,10a-hexahydro-1H-benzo[b]cyclopenta[f]oxepine-6-carboxylic acid

HPLC retention time (min): 1.173;
MS (ESI, Pos.): 429 (M+Na)+.;
¹H-NMR (CD₃OD): δ 7.41-7.48, 6.94, 5.59-5.66, 5.47-5.55, 4.39-4.45, 3.94, 3.41-3.52, 3.19-3.23, 3.02-3.11, 2.65-2.86, 2.06-2.25, 1.81-2.03, 1.43-1.55, 1.18, 0.89.

### Example 25-15: (1R,3aS,10aR)-5-fluoro-1-{(1E,3ξ)-3-[3-(4-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid

HPLC retention time (min): 1.071;
MS (ESI, Pos.): 479 (M+Na)+.;
¹H-NMR (CD₃OD): δ 7.42-7.48, 7.07, 6.95, 5.49-5.56, 5.30-5.38, 4.90-4.98, 4.49, 4.37, 2.96-3.05, 2.71-2.83, 2.63, 2.11-2.21, 1.92-2.08, 1.68-1.81, 1.35-1.47.

### Example 26-1 to 26-23:

A procedure for a purpose similar to that for Example 25 was performed using a corresponding aldehyde form in place of the compound produced in Reference Example 76 and using a corresponding phosphonate in place of the compound produced in Reference Example 26 to yield the title compound exhibiting the following physical properties. In Examples 26-1, 26-3 and 26-6, reduction was performed using (R)-2-methyl-CBS-oxazaborolidine in place of (S)-2 methyl-CBS-oxazaborolidine used in the reaction corresponding to Example 25. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 74→ Reference Example 75→ Reference Example 76 using a corresponding phenol form in place of the compound produced in Reference Example 3. The corresponding phosphonate is a known compound, or was prepared from a known compound by subjecting the compound to a procedure for a purpose similar to that for Reference Example 10→ Reference Example 11 or Reference Example 28→ Reference Example 29.

### Example 26-1: (1R,3aS,10aR)-1-[(1E,3S)-3-hydroxy-1-octen-1-yl]-2,3,3a,9,10,10a-hexahydro-1H-benzo[b]cyclopenta[f]oxepine-6-carboxylic acid

MS (FAB, Neg.): 357 (M-H)-.;
¹H-NMR (CDCl₃): δ 7.60-7.67, 7.15, 5.48-5.58, 4.42-4.46, 4.06-4.12, 2.97-3.06, 2.77-2.85, 2.60-2.70, 2.04-2.21, 1.22-2.01, 0.78-0.96.

### Example 26-2: (1R,3aS,10aR)-1-[(1E,3ξ)-3-hydroxy-3-(1-phenylcyclobutyl)-1-propen-1-yl]-2,3,3a,9,10,10a-hexahydro-1H-benzo[b]cyclopenta[f]oxepine-6-carboxylic acid

MS (ESI, Pos.): 401 (M+H-H₂O)+.;
¹H-NMR (CD₃OD): δ 7.45-7.51, 7.36, 7.14-7.20, 7.02-7.10, 5.24-5.36, 5.12-5.22, 4.78, 4.25-4.29, 4.13, 2.85-2.93, 2.65-2.76, 2.38-2.51, 2.16-2.33, 1.86-2.10, 1.58-1.83, 1.15-1.36.

### Example 26-3: (1R,3aS,10aR)-1-[(1E,3ξ)-3-hydroxy-4-phenoxy-1-buten-1-yl]-2,3,3a,9,10,10a-hexahydro-1H-benzo[b]cyclopenta[f]oxepine-6-carboxylic acid

MS (ESI, Pos.): 377 (M+H-H₂O)+.;
¹H-NMR (CD₃OD): δ 7.57-7.61, 7.49, 7.26-7.31, 7.18, 6.92-6.98, 5.66-5.78, 4.43-4.48, 3.91-4.00, 3.27-3.31, 2.98-3.06, 2.78-2.88, 2.65-2.76, 2.07-2.29, 1.81-2.00, 1.47-1.58.

### Example 26-4: (1R,3aS,10aR)-1-[(1E,3ξ)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid

MS (ESI, Pos.): 387 (M+H-H₂O)+.;
¹H-NMR (CD₃OD): δ 7.57-7.63, 7.48, 7.38, 7.25-7.30, 7.17-7.23, 5.36-5.49, 4.37-4.42, 3.85, 2.97-3.05, 2.77-2.85, 2.55-2.65, 2.13-2.23, 1.98-2.07, 1.69-1.95, 1.30-1.46, 0.95-1.00, 0.87-0.93, 0.77-0.83, 0.70-0.76.

### Example 26-5: (1R,3aS,10aR)-1-{(1E,3ξ)-3-[1-(4-fluorophenyl)cyclopropyl]-3-hydroxy-1-propen-1-yl}-2,3,3a,9,10,10a-hexahydro-1H-benzo[b]cyclopenta[f]oxepine-6-carboxylic acid,

MS (ESI, Pos.): 405 (M+H-H₂O)+.;
¹H-NMR (CD₃OD): δ 7.45-7.50, 7.36, 7.26, 7.08, 6.85-6.91, 5.25-5.36, 4.78, 4.27-4.31, 3.68, 2.86-2.94, 2.66-2.75, 2.43-2.53, 2.01-2.11, 1.86-1.95, 1.59-1.83, 1.22-1.33, 0.82-0.89, 0.74-0.81, 0.59-0.69.

### Example 26-6: (1R,3aS,10aR)-1-[(1E,3ξ,4ξ)-3-hydroxy-4-(trifluoromethyl)-1-octen-1-yl]-2,3,3a,9,10,10a-hexahydro-1H-benzo[b]cyclopenta[f]oxepine-6-carboxylic acid

HPLC retention time (min): 1.13;
MS (ESI, Pos.): 409 (M+H-H₂O)+.;
¹H-NMR (CD₃OD): δ 7.57-7.62, 7.50, 7.19, 5.59-5.69, 4.41-4.48, 2.99-3.08, 2.77-2.91, 2.71, 2.17-2.30, 2.06-2.15, 1.81-2.00, 1.27-1.72, 0.89-0.99.

### Example 26-7: (1R,3aS,10aR)-1-{(1E,3ξ)-3-[1-(4-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

HPLC retention time (min): 1.262;
MS (ESI, Pos.): 419 (M+H-H₂O)+;
¹H-NMR (CD₃OD): δ 7.56-7.59, 7.46, 7.11-7.20, 6.96-7.02, 5.39-5.47, 5.22-5.29, 4.36-4.40, 4.22, 2.95-3.03, 2.73-2.86, 2.48-2.61, 2.24-2.40, 1.96-2.20, 1.70-1.93, 1.33-1.44.

### Example 26-8: (1R,3aS,10aR)-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

HPLC retention time (min): 1.07;
MS (ESI, Pos.): 405 (M+H-H₂O)+.;
¹H-NMR (CD₃OD): δ 7.58-7.61, 7.48, 7.35-7.40, 7.22-7.26, 7.20, 7.08-7.12, 7.02, 5.48-5.56, 5.32-5.41, 4.36-4.40, 3.99, 2.95-3.05, 2.81, 2.59, 2.15, 1.97-2.06, 1.86-1.96, 1.65-1.80, 1.30-1.43, 0.99-1.07, 0.76-0.83, 0.72.

### Example 26-9: (1R,3aS,10aR)-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

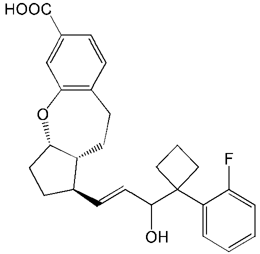

HPLC retention time (min): 1.14;
MS (ESI, Pos.): 419 (M+H-H₂O)+.;
¹H-NMR (CD₃OD): δ 7.58-7.61, 7.48, 7.09-7.23, 6.95-7.02, 5.38-5.50, 4.37, 4.34, 2.94-3.03, 2.80, 2.35-2.64, 1.97-2.22, 1.65-1.94, 1.35-1.48.

### Example 26-10: (1R,3aS,10aR)-1-{(1E)-3-[1-(2,6-difluorophenyl)cyclobutyl]-3-hydroxy-1-propen-1-yl}-2,3,3a,9,10,10a-hexahydro-1H-benzo[b]cyclopenta[f]oxepine-6-carboxylic acid

MS (ESI, Pos.): 437 (M+H)+.;
¹H-NMR (CDCl₃): δ 7.64-7.67, 7.62, 7.11-7.19, 6.81, 5.50-5.60, 4.50-4.54, 4.35-4.42, 2.97-3.06, 2.72-2.83, 2.51-2.71, 2.33-2.45, 1.99-2.17, 1.69-1.97, 1.30-1.41, -0.05--0.01.

### Example 26-11: (1R,3aS,10aR)-1-{(1E,3ξ)-3-[1-(2,4-difluorophenyl)cyclobutyl]-3-hydroxy-1-propen-1-yl}-2,3,3a,9,10,10a-hexahydro-1H-benzo[b]cyclopenta[f]oxepine-6-carboxylic acid

MS (ESI, Pos.): 437 (M+H-H₂O)+.;
¹H-NMR (CDCl₃): δ 7.64-7.67, 7.62, 7.16, 6.98-7.05, 6.80-6.85, 6.70-6.76, 5.43-5.54, 4.40, 2.94-3.05, 2.73-2.83, 2.59-2.70, 2.46-2.56, 2.28-2.46, 2.00-2.18, 1.68-1.97, 1.31-1.42.

### Example 26-12: (1R,3aS,10aR)-1-{(1E,3ξ)-3-[1-(2,4-difluorophenyl)cyclopropyl]-3-hydroxy-1-propen-1-yl}-2,3,3a,9,10,10a-hexahydro-1H-benzo[b]cyclopenta[f]oxepine-6-carboxylic acid

MS (ESI, Pos.): 423 (M+H-H₂O)+.;
¹H-NMR (CDCl₃): δ 7.64-7.67, 7.62, 7.23-7.31, 7.16, 6.73-6.83, 5.46-5.53, 5.37-5.44, 4.38-4.42, 3.82, 2.95-3.05, 2.75-2.83, 2.62, 2.09-2.18, 1.99-2.07, 1.64-1.97, 1.29-1.39, 0.90-1.03, 0.77-0.87.

### Example 26-13: (1R,3aS,10aR)-1-[(1E,3ξ)-4,4-difluoro-3-hydroxy-4-phenyl-1-buten-1-yl]-2,3,3a,9,10,10a-hexahydro-1H-benzo[b]cyclopenta[f]oxepine-6-carboxylic acid

HPLC retention time (min): 1.06;
MS (ESI, Pos.): 397 (M+H-H₂O)+.;
¹H-NMR (CD₃OD): δ 7.40-7.58, 7.11-7.19, 5.42-5.61, 4.32-4.47, 2.84-3.00, 2.70-2.80, 2.59, 2.10-2.19, 1.82-2.08, 1.53-1.77, 1.26-1.47.

### Example 26-14: (1R,3aS,10aR)-1-[(1E,3ξ)-4,4-difluoro-3-hydroxy-1-octen-1-yl]-2,3,3a,9,10,10a-hexahydro-1H-benzo[b]cyclopenta[f]oxepine-6-carboxylic acid

HPLC retention time (min): 1.04;
MS (ESI, Pos.): 377 (M+H-H₂O)+.;
¹H-NMR (CD₃OD): δ 7.47, 7.37, 7.06, 5.60-5.68, 5.47-5.54, 4.76, 4.33, 4.02-4.11, 2.88-2.97, 2.68-2.77, 2.54-2.66, 2.06-2.16, 1.95-2.05, 1.68-1.90, 1.23-1.45, 0.85.

### Example 26-15: (1R,3aS,10aR)-1-[(1E,3ξ)-3-hydroxy-4-methylene-1-octen-1-yl]-2,3,3a,9,10,10a-hexahydro-1H-benzo[b]cyclopenta[f]oxepine-6-carboxylic acid

HPLC retention time (min): 1.06;
MS (ESI, Pos.): 353 (M+H-H₂O)+.;
¹H-NMR (CD₃OD): δ 7.56-7.61, 7.49, 7.18, 5.50-5.64, 5.11, 4.85-4.89, 4.50, 4.43-4.47, 2.99-3.08, 2.78-2.89, 2.63-2.73, 2.18-2.27, 2.05-2.15, 1.82-1.99, 1.31-1.55, 0.96.

### Example 26-16: (1R,3aS,10aR)-5-chloro-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

HPLC retention time (min): 1.2;
MS (ESI, Pos.): 439 (M+H-H₂O)+.;
¹H-NMR (CD₃OD): δ 7.31-7.42, 7.19-7.26, 7.05-7.13, 6.96-7.03, 5.46-5.55, 5.29-5.38, 4.21, 3.97, 2.97-3.10, 2.58-2.79, 1.97-2.17, 1.56-1.81, 1.32-1.47, 0.94-1.09, 0.65-0.83.

### Example 26-17: (1R,3aS,10aR)-5-chloro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

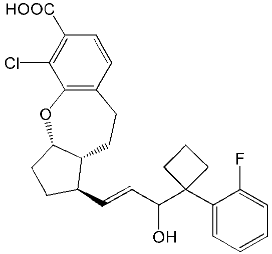

HPLC retention time (min): 1.25;
MS (ESI, Pos.): 453 (M+H-H₂O)+.;
¹H-NMR (CD₃OD): δ 7.36, 7.07-7.21, 6.97, 5.35-5.49, 4.32, 4.21, 2.99-3.07, 2.67-2.77, 2.36-2.64, 1.97-2.16, 1.80-1.88, 1.57-1.78, 1.36-1.47.

### Example 26-18: (1R,3aS,10aR)-5-chloro-1-[(1E,3ξ)-3-hydroxy-4-methylene-1-octen-1-yl]-2,3,3a,9,10,10a-hexahydro-1H-benzo[b]cyclopenta[f]oxepine-6-carboxylic acid

HPLC retention time (min): 1.255;
MS (ESI, Pos.): 387 (M+H-H₂O)+.;
¹H-NMR (CD₃OD): δ 7.30, 7.01, 5.40-5.52, 5.00, 4.72-4.85, 4.39, 4.19, 2.96-3.04, 2.66-2.77, 2.53-2.62, 1.89-2.12, 1.64-1.86, 1.20-1.48, 0.81-0.87.

### Example 26-19: (1R,3aS,10aR)-5-chloro-1-[(1E,3ξ)-3-hydroxy-6-methoxy-4-methylene-1-hexen-1-yl]-2,3,3a,9,10,10a-hexahydro-1H-benzo[b]cyclopenta[f]oxepine-6-carboxylic acid

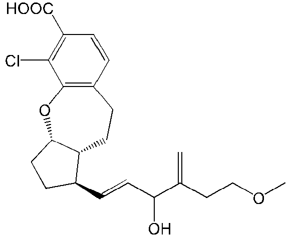

HPLC retention time (min): 1.069;
MS (ESI, Pos.): 389 (M-OH)+;
¹H-NMR (CD₃OD): δ 7.36, 7.09, 5.57-5.64, 5.49-5.56, 5.15, 4.92, 4.52, 4.29, 3.54, 3.06-3.14, 2.75-2.87, 2.63-2.72, 2.25-2.41, 2.02-2.22, 1.74-1.97, 1.47-1.58.

### Example 26-20: (1R,3aS,10aR)-5-chloro-1-[(1E,3ξ,4S)-4-ethyl-3-hydroxy-1-octen-1-yl]-2,3,3a,9,10,10a-hexahydro-1H-benzo[b]cyclopenta[f]oxepine-6-carboxylic acid

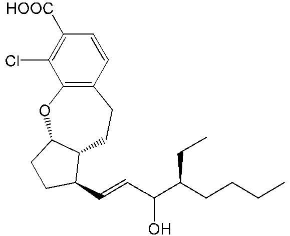

HPLC retention time (min): 1.301;
MS (ESI, Pos.): 443 (M+Na)+, 403 (M+H-H₂O)+.;
¹H-NMR (CD₃OD): δ 7.30, 7.01, 5.36-5.50, 4.78, 4.19, 3.92-3.97, 2.97-3.06, 2.67-2.78, 2.54-2.63, 1.91-2.12, 1.80-1.89, 1.64-1.75, 1.06-1.51, 0.77-0.88.

### Example 26-21: (1R,3aS,10aR)-5-chloro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid

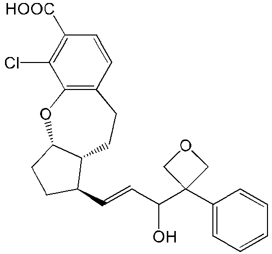

HPLC retention time (min): 1.085;
MS (ESI, Pos.): 477 (M+Na)+.;
¹H-NMR (CD₃OD): δ 7.32-7.40, 7.22-7.27, 7.04-7.13, 5.47-5.54, 5.30-5.36, 4.89-5.01, 4.81-4.84, 4.48, 4.23, 3.02, 2.59-2.77, 1.98-2.16, 1.61-1.78, 1.37-1.49.

### Example 26-22: (1R,3aS,10aR)-1-{(1E,3ξ)-3-[1-(4-fluorophenyl)cyclobutyl]-3-hydroxy-1-propen-1-yl}-5-methyl-2,3,3a,9,10,10a-hexahydro-1H-benzo[b]cyclopenta[f]oxepine-6-carboxylic acid

HPLC retention time (min): 1.278;
MS (ESI, Pos.): 473 (M+Na)+.;
¹H-NMR (CD₃OD): δ 7.38, 7.05, 6.87-6.94, 5.30-5.38, 5.12-5.20, 4.78, 4.14, 4.01-4.06, 2.90-2.98, 2.59-2.69, 2.36-2.53, 2.16-2.33, 1.83-2.08, 1.68-1.80, 1.53-1.64, 1.28-1.39.

### Example 26-23: (1R,3aS,10aR)-8-fluoro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid

MS (ESI, Pos,):437 (M+H-H₂O)+.;
¹H-NMR (CD₃OD): δ 7.31-7.35, 7.31, 7.18-7.24, 7.08-7.17, 6.99, 5.38-5.50, 4.51, 4.34, 2.94-3.03, 2.67-2.81, 2.36-2.60, 2.15-2.26, 1.95-2.13, 1.80-1.92, 1.70-1.78, 1.30-1.43.

### Example 27: (1R,3aS,10aR)-1-[(1E,3ξ)-4,4-difluoro-3-hydroxy-4-phenyl-1-buten-1-yl]-N-(methylsulfonyl)-2,3,3a,9,10,10a-hexahydro-1H-benzo[b]cyclopenta[f]oxepine-6-carboxamide

A procedure for a purpose similar to that for Reference Example 67→ Reference Example 68→ Reference Example 69→ Reference Example 70→ Example 23 was performed using the compound produced in Example 26-13 in place of the compound produced in Example 17-17 to yield the title compound exhibiting the following physical properties.
HPLC retention time (min): 0.99;
MS (ESI, Pos.): 492 (M+H)+.;
¹H-NMR (CD₃OD): δ 7.40-7.53, 7.17-7.25, 5.54-5.61, 5.43-5.50, 4.38-4.52, 2.91-3.01, 2.73-2.84, 2.57-2.65, 2.11-2.22, 1.97-2.07, 1.84-1.95, 1.60-1.79, 1.28-1.47.

### Reference Example 77: ethyl (1S,2S,3aS,10aR)-5-fluoro-2-hydroxy-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

To a solution of the compound (4.87 g) produced in Reference Example 73 in THF (39 mL) was added triphenylphosphine (4.54 g) at room temperature. The mixture was cooled to 0°C, followed by addition of formic acid (0.98 mL) and diethyl azodicarboxylate (2.2 mol/L toluene solution, 7.9 mL), and stirring at room temperature for 3 hours. The reaction solution was concentrated under reduced pressure. The obtained residue was purified by silica gel column chromatography (n-hexane : ethyl acetate = 100 : 0→80 : 20) to yield a formate ester form (4.53 g).

To a solution of the formate ester form (4.53 g) in ethanol (31.7 mL) was added potassium carbonate (2.12 g) at room temperature, followed by stirring at room temperature for 1 hour. The reaction mixture was diluted with MTBE, and then filtered. The filtrate was poured into a saturated aqueous ammonium chloride solution/a 1 N aqueous hydrochloric acid solution, followed by extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 88 : 12→75 : 25) to yield the title compound (4.08 g) exhibiting the following physical properties.

¹H-NMR (CDCl₃): δ 7.64-7.71, 7.39-7.50, 7.31-7.36, 6.81, 4.67-4.72, 4.53-4.58, 4.33-4.39, 4.02-4.08, 3.81-3.87, 2.99, 2.82-2.90, 2.70-2.78, 2.27-2.42, 2.12-2.22, 1.56, 1.37, 1.08.

### Reference Example 78: ethyl (1S,2R,3aS,10aR)-2,5-difluoro-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

A solution of the compound (6.0 g) produced in Reference Example 77 in MTBE (27 mL) was cooled to -78°C, followed by dropwise addition of bis(2-methoxyethyl)aminosulfur trifluoride (9.8 mL) and stirring at -78°C. After confirming the disappearance of the raw material by TLC, the temperature was raised to -20°C. The reaction solution was poured into a saturated aqueous sodium bicarbonate solution, followed by extraction with ethyl acetate. The organic layer was washed with a saturated aqueous sodium bicarbonate solution and saturated saline, dried over anhydrous sodium sulfate, and then filtered. The filtrate was concentrated under reduced pressure. The obtained residue was purified by silica gel column chromatography (n-hexane : ethyl acetate = 93 : 7→86 : 14) to yield the title compound (2.59 g) exhibiting the following physical properties.

¹H-NMR (CDCl₃): δ 7.61-7.68, 7.37-7.50, 6.86, 5.25-5.31, 5.11-5.17, 4.32-4.40, 3.95-3.99, 3.67-3.73, 2.98-3.06, 2.58-2.67, 2.44, 2.21-2.37, 2.12-2.19, 1.85, 1.67-1.76, 1.38, 1.06.

### Reference Example 79: ethyl (1S,2R,3aS,10aR)-2,5-difluoro-1-(hydroxymethyl)-2,3,3a,9,10,10a-hexahydro-1H-benzo[b]cyclopenta[f]oxepine-6-carboxylate

A procedure for a purpose similar to that for Reference Example 8 was performed using the compound produced in Reference Example 78 in place of the compound produced in Reference Example 7 to yield the title compound exhibiting the following physical properties.

¹H-NMR (CDCl₃): δ 7.47-7.51, 6.88, 5.14-5.20, 5.00-5.06, 4.34-4.40, 3.94-4.01, 3.72-3.78, 3.07-3.16, 2.72-2.79, 2.48-2.66, 2.26-2.48, 2.01-2.12, 1.94, 1.44, 1.35-1.41.

### Reference Example 80: ethyl (1S,2R,3aS,10aR)-2,5-difluoro-1-formyl-2,3,3a,9,10,10a-hexahydro-1H-benzo[b]cyclopenta[f]oxepine-6-carboxylate

To a solution of the compound (600 mg) produced in Reference Example 79 in dichloromethane (6 mL) was added 1,1,1-triacetoxy-1,1-dihydro-1,2-benziodoxol-3-(1H)-one (Dess-Martin reagent, 1.17 g) at 0°C, followed by stirring at room temperature. After confirming completion of the reaction by TLC, the reaction mixture was filtered, and the filtrate was concentrated under reduced pressure. The obtained residue was purified by silica gel column chromatography (n-hexane : ethyl acetate = 75 : 25→60 : 40) to yield the title compound (550 mg) exhibiting the following physical properties.

¹H-NMR (CDCl₃): δ 9.98, 7.46-7.54, 6.90, 5.44-5.51, 5.31-5.37, 4.34-4.41, 3.49-3.55, 3.41-3.49, 3.09-3.20, 2.72-2.83, 2.42-2.57, 2.27-2.27, 2.23-2.39, 2.07-2.17, 1.92-2.03, 1.38.

### Example 28: (1R,2R,3aS,10aR)-2,5-difluoro-1-[(1E,3ξ)-3-hydroxy-4-methyl-4-phenyl-1-penten-yl]-2,3,3a,9,10,10a-hexahydro-1H-benzo[b]cyclopenta[f]oxepine-6-carboxylic acid

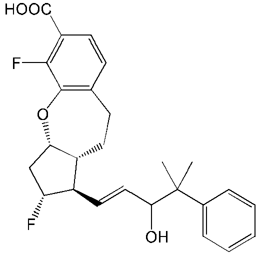

A procedure for a purpose similar to that for Reference Example 24 was performed using the compound (300 mg) produced in Reference Example 80 in place of the compound produced in Reference Example 76 and using dimethyl-3-methyl-2-oxo-3-phenylbutyl phosphonate (CAS No.: 87929-30-4, 375 mg) in place of the compound produced in Reference Example 26 to yield the title compound exhibiting the following physical properties.
HPLC retention time (min): 1.157;
MS (ESI, Pos.): 405 (M+H-H₂O-HF)+.;
¹H-NMR (CD₃OD): δ 7.44-7.49, 7.36-7.42, 7.29, 7.13-7.18, 6.95, 5.44-5.51, 5.31-5.38, 4.78-4.83, 4.64-4.71, 4.19-4.24, 2.92-3.00, 2.75-2.89, 2.44-2.66, 2.05-2.17, 1.68-1.76, 1.48-1.62, 1.35.

### Example 28-1 to 28-3:

A procedure for a purpose similar to that for Example 25 was performed using the compound produced in Reference Example 80 in place of the compound produced in Reference Example 76 and using a corresponding phosphonate in place of the compound produced in Reference Example 26 to yield the title compound exhibiting the following physical properties. The corresponding phosphonate is a known compound, or was prepared from a known compound by subjecting the compound to a procedure for a purpose similar to that for Reference Example 10→ Reference Example 11.

### Example 28-1: (1R,2R,3aS,10aR)-2,5-difluoro-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

HPLC retention time (min): 1.01;
MS (ESI, Pos.): 441 (M-OH)+.;
¹H-NMR (DMSO-d₆): δ 13.10, 7.42, 7.22-7.35, 7.04-7.14, 5.52-5.59, 5.25-5.34, 4.97, 4.73, 4.27, 3.95-4.00, 2.94, 2.69-2.88, 1.92-2.06, 1.71-1.81, 1.53, 0.91-1.04, 0.66-0.73, 0.53-0.61.

### Example 28-2: (1R,2R,3aS,10aR)-2,5-difluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid

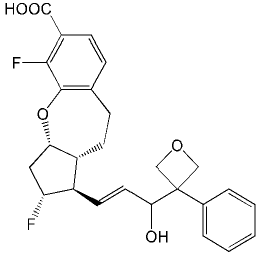

HPLC retention time (min): 1.035;
MS (ESI, Pos.): 439 (M+H-H₂O)+, 419 (M+H-H₂O-HF)+.;
¹H-NMR (CD₃OD): δ 7.36-7.41, 7.25, 7.13-7.18, 6.97, 6.88, 5.34-5.51, 4.75-4.92, 4.59-4.65, 4.42, 4.16, 2.89-2.98, 2.73-2.88, 2.57-2.65, 2.38-2.50, 1.97-2.11, 1.69-1.79, 1.53-1.68.

### Example 28-3: (1R,2R,3aS,10aR)-2,5-difluoro-1-{(1E,3ξ)-3-hydroxy-3-[(2ξ)-2-phenyl-2-oxetanyl]-1-propen-1-yl}-2,3,3a,9,10,10a-hexahydro-1H-benzo[b]cyclopenta[f]oxepine-6-carboxylic acid

HPLC retention time (min): 1.053;
MS (ESI, Pos.): 479 (M+Na)+, 439 (M+H-H₂O)+.;
¹H-NMR (CD₃OD): δ 7.24-7.49, 6.91-6.98, 5.40-5.57, 4.58-4.83, 4.43-4.52, 4.13-4.28, 3.24-3.28, 3.12-3.20, 2.96-3.09, 2.81-2.95, 2.63-2.81, 2.43-2.61, 2.06-2.21, 1.75-1.87, 1.57-1.75.

### Example 29-1 to 29-4:

A procedure for a purpose similar to that for Example 25 was performed using a corresponding aldehyde form in place of the compound produced in Reference Example 76 and using a corresponding phosphonate in place of the compound produced in Reference Example 26 to yield the title compound exhibiting the following physical properties. The corresponding aldehyde form was prepared by performing a procedure for a purpose similar to that for Reference Example 73→ Reference Example 77→ Reference Example 78→ Reference Example 79→ Reference Example 80 using the compound produced in Reference Example 39 in place of the compound produced in Reference Example 8. The corresponding phosphonate is a known compound, or was prepared from a known compound by subjecting the compound to a procedure for a purpose similar to that for Reference Example 10→ Reference Example 11.

### Example 29-1: (1R,2R,3aS,10aR)-2-fluoro-1-[(1E,3ξ)-3-hydroxy-3-(1-phenylcyclobutyl)-1-propen-1-yl]-5-methyl-2,3,3a,9,10,10a-hexahydro-1H-benzo[b]cyclopenta[f]oxepine-6-carboxylic acid

HPLC retention time (min): 1.224;
MS (ESI, Pos.): 473 (M+Na)+.;
¹H-NMR (CD₃OD): δ 7.38-7.42, 7.15-7.21, 7.03-7.09, 6.89-6.96, 5.28-5.41, 4.81-4.90, 4.78, 4.60-4.60, 4.56-4.68, 4.17-4.22, 3.94, 2.81-3.00, 2.20-2.49, 1.91-2.11, 1.63-1.79, 1.52.

### Example 29-2: (1R,2R,3aS,10aR)-1-[(1E,3ξ)-3-(1-butylcyclobutyl)-3-hydroxy-1 propen-1-yl]-2-fluoro-5-methyl-2,3,3a,9,10,10a-hexahydro-1H-benzo[b]cyclopenta[f]oxepine-6-carboxylic acid

HPLC retention time (min): 1.281;
MS (ESI, Pos.): 453 (M+Na)+, 393 (M+H-H₂O-HF)+.;
¹H-NMR (CD₃OD): δ 7.39, 6.92, 5.61-5.68, 5.48-5.56, 4.71-4.91, 4.00, 3.88, 2.94-3.08, 2.40-2.53, 2.35, 1.95-2.16, 1.48-1.88, 1.16-1.37, 0.79-0.89.

### Example 29-3: (1R,2R,3aS,10aR)-2-fluoro-1-{(1E,3ξ)-3-[1-(4-fluorophenyl)cyclobutyl]-3-hydroxy-1-propen-1-yl}-5-methyl-2,3,3a,9,10,10a-hexahydro-1H-benzo[b]cyclopenta[f]oxepine-6-carboxylic acid

HPLC retention time (min): 1.217;
MS (ESI, Pos.): 491 (M+Na)+.;
¹H-NMR (CD₃OD): δ 7.47-7.51, 7.15, 6.97-7.05, 5.38-5.51, 4.70-4.92, 4.28, 4.05, 2.92-3.10, 2.44-2.60, 2.28-2.43, 2.01-2.21, 1.74-1.89, 1.64.

### Example 29-4: (1R,2R,3aS,10aR)-2-fluoro-1-{(1E,3ξ)-3-hydroxy-3-[1-(3-thienyl)cyclobutyl]-1-propen-1-yl}-5-methyl-2,3,3a,9,10,10a-hexahydro-1H-benzo[b]cyclopenta[f]oxepine-6-carboxylic acid

HPLC retention time (min): 1.193;
MS (ESI, Pos.): 479 (M+Na)+.;
¹H-NMR (CD₃OD): δ 7.48, 7.29-7.33, 7.05-7.07, 6.98-7.03, 5.38-5.50, 4.71-4.92, 4.24, 4.03, 2.90-3.07, 2.37-2.56, 2.08-2.30, 1.84-1.99, 1.69-1.83, 1.60.

### Example 30: (1R,2R,3aS,10aR)-1-[(1E,3ξ)-4,4-difluoro-3-hydroxy-4-phenyl-1-buten-1-yl]-2-fluoro-N-(methylsulfonyl)-2,3,3a,9,10,10a-hexahydro-1H-benzo[b]cyclopenta[f]oxepine-6-carboxamide

A procedure for a purpose similar to that for Example 25→ Reference Example 67→ Reference Example 68→ Reference Example 69→ Reference Example 70→ Example 23 was performed using a corresponding aldehyde form in place of the compound produced in Reference Example 9 and using a corresponding phosphonate in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 77→ Reference Example 78→ Reference Example 79→ Reference Example 80 using the compound produced in Reference Example 31 in place of the compound produced in Reference Example 3. The corresponding phosphonate was prepared by performing a procedure for a purpose similar to that for Reference Example 28→ Reference Example 29 using iodobenzene in place of 1,3-difluoro-2-iodobenzene.
HPLC retention time (min): 0.96;
MS (ESI, Pos.): 510 (M+H)+.;
¹H-NMR (DMSO-d₆): δ 12.02, 7.55, 7.45-7.52, 7.25-7.31, 5.88-5.98, 5.53-5.70, 4.73-4.98, 4.40-4.55, 4.26-4.33, 2.57-3.03, 1.74-2.08, 1.59, 1.24.

### Example 31: (2R,3R,3aR,11aS)-2-fluoro-3-[(1E,3ξ)-3-hydroxy-3-(1-phenylcyclobutyl)-1-propen-1-yl]-1,2,3,3a,4,5,6,11a-octahydrobenzo[b]cyclopenta[g]oxocine-9-carboxylic acid

A procedure for a purpose similar to that for Example 25→ Reference Example 67→ Reference Example 68→ Reference Example 69→ Reference Example 70→ Example 23 was performed using a corresponding aldehyde form in place of the compound produced in Reference Example 9 and using a corresponding phosphonate in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 77→ Reference Example 78→ Reference Example 79→ Reference Example 80 using (1R,2R,3S,4R)-2-allyl-3-({[dimethyl(2-methyl-2-propanyl)silyl]oxy}methyl)-4-(tetrahydro-2H-pyran-2-yloxy)cyclopentanol in place of the compound produced in Reference Example 2 and using the compound produced in Reference Example 31 in place of the compound produced in Reference Example 3. The corresponding phosphonate was prepared by performing a procedure for a purpose similar to that for Reference Example 10→ Reference Example 11 using 1-phenylcyclobutanecarboxylic acid (CAS No.: 37828-19-6) in place of (2S)-2-(2,4-difluorophenyl)propanoic acid.
MS (ESI, Pos.): 433 (M+H-H₂O)+, 413 (M+H-H₂O-HF)+.;
¹H-NMR (CD₃OD): δ 7.65-7.69, 7.62, 7.18-7.29, 7.10-7.15, 5.31-5.43, 4.59-4.80, 4.49-4.54, 4.23, 2.94-3.02, 2.82-2.91, 2.48-2.70, 2.24-2.44, 2.10-2.23, 1.96-2.08, 1.77-1.88, 1.52-1.74, 1.24-1.48.

### Reference Example 81: ethyl (1S,2S,3aS,10aR)-5-fluoro-2-{[(4-methylphenyl)sulfonyl]oxy}-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

The compound produced in Reference Example 77 (4.28 g) was dissolved in acetonitrile (34.2 mL), followed by cooling to 0°C. Then, triethylamine (3.71 mL), trimethylamine hydrochloride (145 mg) and p-toluenesulfonyl chloride (2.32 g) were added, followed by stirring at room temperature overnight and further stirring at 50°C for 1 hour. The reaction solution was poured into ice water and extracted with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 100 : 0→75 : 25) to yield the title compound (a mixture with the raw material). Further purification was not performed, but the following reaction was performed.

### Reference Example 82: ethyl (1S,2R,3aS,10aR)-2-chloro-5-fluoro-1-({[(2-methyl-2-propanyl)(diphenyl)silyl]oxy}methyl)-2,3,3a,9,10,10a-hexahydro-1H-benzo[b]cyclopenta[f]oxepine-6-carboxylate

The mixture produced in Reference Example 81 was dissolved in toluene (32.7 mL), followed by addition of tetrabutylammonium chloride (3.17 g) and stirring at 60°C overnight. After confirming completion of the reaction by TLC, the reaction solution was poured into water, followed by extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 100 : 0→83 : 17→70 : 30) to yield the title compound (2.68 g) exhibiting the following physical properties.

¹H-NMR (CDCl₃): δ 7.63-7.69, 7.37-7.48, 6.84, 4.34-4.43, 4.21-4.30, 3.98-4.04, 3.71-3.77, 2.92-3.00, 2.70-2.88, 2.25-2.42, 2.13-2.24, 1.85-1.94, 1.67-1.75, 1.38, 1.07.

### Reference Example 83: ethyl (1S,2R,3aS,10aR)-2-chloro-5-fluoro-1-(hydroxymethyl)-2,3,3a,9,10,10a-hexahydro-1H-benzo[b]cyclopenta[f]oxepine-6-carboxylate

A procedure for a purpose similar to that for Reference Example 8 was performed using the compound (2.68 g) produced in Reference Example 82 in place of the compound produced in Reference Example 7 to yield the title compound (1.10 g) exhibiting the following physical properties.

¹H-NMR (CDCl₃): δ 7.44-7.49, 6.86, 4.34-4.44, 4.06-4.14, 3.96-4.02, 3.79-3.85, 3.01-3.10, 2.79-2.89, 2.32-2.41, 1.93-2.17, 1.43, 1.38.

### Reference Example 84: ethyl (1S,2R,3aS,10aR)-2-chloro-5-fluoro-1-formyl-2,3,3a,9,10,10a-hexahydro-1H-benzo[b]cyclopenta[f]oxepine-6-carboxylate

A procedure for a purpose similar to that for Reference Example 80 was performed using the compound (1.10 g) produced in Reference Example 83 in place of the compound produced in Reference Example 79 to yield the title compound (0.80 g) exhibiting the following physical properties.

¹H-NMR (CDCl₃): δ 9.90, 7.48-7.53, 6.88, 4.34-4.45, 3.35-3.41, 3.08-3.15, 2.79-2.90, 2.34-2.49, 2.09-2.18, 1.93-2.02, 1.39.

### Example 32: (1R,2R,3aS,10aR)-2-chloro-5-fluoro-1-{(1E,3ξ)-3-[3-(2-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid

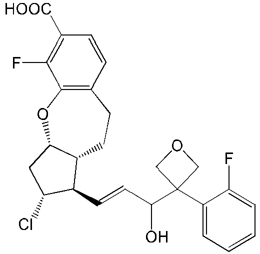

A procedure for a purpose similar to that for Reference Example 24 was performed using the compound (170 mg) produced in Reference Example 84 in place of the compound produced in Reference Example 76 and using a corresponding phosphonate (226 mg) in place of the compound produced in Reference Example 26 to yield the title compound exhibiting the following physical properties. The corresponding phosphonate was prepared by performing a procedure for a purpose similar to that for Reference Example 10→ Reference Example 11 using 3-phenyl-3-oxetanecarboxylic acid (CAS No.: 114012-42-9) in place of (2S)-2-(2,4-difluorophenyl)propanoic acid.
HPLC retention time (min): 1.089;
MS (ESI, Pos.): 513 (M+Na)+, 473 (M+H-H₂O)+.;
¹H-NMR (CD₃OD): δ 7.44-7.48, 7.27-7.33, 7.15-7.20, 7.05-7.11, 6.94, 5.58-5.65, 5.48-5.55, 4.80-4.82, 4.80-5.01, 4.53, 4.34-4.38, 3.84-3.92, 2.84-3.03, 2.68-2.81, 2.15-2.22, 1.84-1.93, 1.63-1.81.

### Example 32-1 to 32-8:

A procedure for a purpose similar to that for Reference Example 24 was performed using the compound (170 mg) produced in Reference Example 84 in place of the compound produced in Reference Example 76 and using a corresponding phosphonate (226 mg) in place of the compound produced in Reference Example 26 to yield the title compound exhibiting the following physical properties. The corresponding phosphonate is a known compound, or was prepared from a known compound by subjecting the compound to a procedure for a purpose similar to that for Reference Example 10→ Reference Example 11.

### Example 32-1: (1R,2R,3aS,10aR)-2-chloro-5-fluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid

HPLC retention time (min): 1.06;
MS (ESI, Pos.): 495 (M+Na)+.;
¹H-NMR (CD₃OD): δ 7.42-7.47, 7.31-7.37, 7.23-7.28, 7.09, 6.94, 5.44-5.54, 4.92-5.01, 4.49-4.52, 4.34-4.38, 3.84-3.92, 2.85-3.03, 2.67-2.82, 2.14-2.22, 1.84-1.93, 1.64-1.82.

### Example 32-2: (1R,2R,3aS,10aR)-2-chloro-5-fluoro-1-[(1E,3ξ)-3-hydroxy-6-methoxy-4-methylene-1-hexen-1-yl]-2,3,3a,9,10,10a-hexahydro-1H-benzo[b]cyclopenta[f]oxepine-6-carboxylic acid

HPLC retention time (min): 1.102;
MS (ESI, Pos.): 407 (M+H-H₂O)+.;
¹H-NMR (CD₃OD): δ 7.43-7.49, 6.94, 5.66-5.73, 5.54-5.62, 5.17, 4.94, 4.58, 4.41-4.45, 3.91-3.99, 3.55, 3.01-3.09, 2.77-2.98, 2.28-2.42, 2.17-2.26, 1.83-2.04.

### Example 32-3: (1R,2R,3aS,10aR)-2-chloro-5-fluoro-1-[(1E,3ξ)-3-hydroxy-4-methyl-4-(3-thienyl)-1-penten-1-yl]-2,3,3a,9,10,10a-hexahydro-1H-benzo[b]cyclopenta[f]oxepine-6-carboxylic acid

HPLC retention time (min): 1.181;
MS (ESI, Pos.): 487 (M+Na)+, 447 (M+H-H₂O)+.;
¹H-NMR (CD₃OD): δ 7.42-7.48, 7.29-7.33, 7.13-7.15, 7.07-7.10, 6.93, 5.50-5.57, 5.28-5.36, 4.33-4.38, 4.15, 3.82-3.90, 2.84-3.01, 2.62-2.79, 2.13-2.21, 1.59-1.85, 1.33.

### Example 32-4: (1R,2R,3aS,10aR)-2-chloro-5-fluoro-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

HPLC retention time (min): 1.163;
MS (ESI, Pos.): 457 (M+H-H₂O)+, 421 (M+H-H₂O-HCl)+.;
¹H-NMR (CD₃OD): δ 7.43-7.49, 7.34-7.40, 7.20-7.26, 7.06-7.10, 6.97-7.03, 6.93-6.97, 5.63-5.71, 5.29-5.36, 4.32-4.37, 4.05, 3.81-3.89, 2.95-3.06, 2.83-2.92, 2.68-2.81, 2.13-2.22, 1.73-1.88, 1.59-1.71, 1.00-1.07, 0.67-0.83.

### Example 32-5: (1R,2R,3aS,10aR)-2-chloro-5-fluoro-1-{(1E,3ξ)-3-[3-(4-fluorophenyl)-3-oxetanyl]-3-hydroxy-1-propen-1-yl }-2,3,3a,9, 1 0, 10a-hexahydro-1H-benzo[b]cyclopenta[f]oxepine-6-carboxylic acid

HPLC retention time (min): 1.078;
MS (ESI, Pos.): 513 (M+Na)+.;
¹H-NMR (CD₃OD): δ 7.43-7.49, 7.04-7.13, 6.95, 5.46-5.56, 4.90-4.99, 4.51-4.55, 4.36-4.40, 3.84-3.92, 2.86-3.04, 2.69-2.84, 2.15-2.22, 1.68-1.94.

### Example 32-6: (1R,2R,3aS,10aR)-2-chloro-5-fluoro-1-{(1E,3ξ)-3-hydroxy-3-[(2ξ)-2-phenyl-2-oxetanyl]-1-propen-1-yl}-2,3,3a,9,10,10a-hexahydro-1H-benzo[b]cyclopenta[f]oxepine-6-carboxylic acid

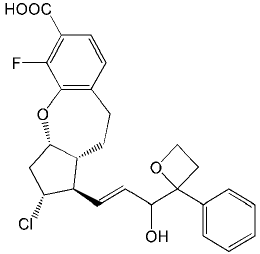

HPLC retention time (min): 1.097;
MS (ESI, Pos.): 455 (M+H-H₂O)+.;
¹H-NMR (CD₃OD): δ 7.41-7.51, 7.31-7.38, 7.23-7.29, 6.94, 5.42-5.51, 4.58-4.64, 4.42-4.49, 4.33-4.38, 4.22-4.25, 3.84-3.91, 3.12-3.20, 2.85-3.01, 2.65-2.79, 2.14-2.21, 1.62-1.90.

### Example 32-7: (1R,2R,3aS,10aR)-2-chloro-1-[(1E,3ξ)-3-(2,3-dihydro-1-benzofuran-3-yl)-3-hydroxy-1-propen-1-yl]-5-fluoro-2,3,3a,9,10,10a-hexahydro-1H-benzo[b]cyclopenta[f]oxepine-6-carboxylic acid

HPLC retention time (min): 1.102;
MS (ESI, Pos.): 481 (M+Na)+.;
¹H-NMR (CD₃OD): δ 7.43-7.50, 7.28-7.39, 7.11, 6.96, 6.93, 6.78-6.85, 6.69-6.74, 5.62-5.71, 5.50-5.58, 5.33-5.41, 4.46-4.61, 4.30-4.41, 4.20, 3.82-3.97, 3.66-3.72, 3.55, 2.62-3.03, 2.13-2.26, 1.88-1.99, 1.70-1.86, 1.56.

### Example 32-8: (1R,2R,3aS,10aR)-2-chloro-5-fluoro-1-{(1E,3ξ)-3-[(2ξ)-2-(4-fluorophenyl)-2-oxetanyl]-3-hydroxy-1-propen-1-yl }-2,3,3a,9, 1 0, 10a-hexahydro-1H-benzo[b]cyclopenta[f]oxepine-6-carboxylic acid

HPLC retention time (min): 1.114;
MS (ESI, Pos.): 473 (M+H-H₂O)+.;
¹H-NMR (CD₃OD): δ 7.40-7.45, 7.32-7.37, 7.05-7.11, 6.93, 5.43-5.53, 4.57-4.63, 4.43-4.49, 4.34-4.38, 4.24-4.26, 3.84-3.91, 3.11-3.20, 2.86-3.02, 2.66-2.80, 2.15-2.22, 1.63-1.92.

### Example 33: (1R,2R,3aS,10aR)-2-chloro-1-{(1E,3ξ)-3-[1-(2-fluorophenyl)cyclopropyl]-3-hydroxy-1-propen-1-yl}-2,3,3a,9,10,10a-hexahydro-1H-benzo[b]cyclopenta[f]oxepine-6-carboxylic acid

A procedure for a purpose similar to that for Example 25 was performed using a corresponding aldehyde form in place of the compound produced in Reference Example 9 and using the compound produced in Reference Example 27 in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 77→ Reference Example 81→ Reference Example 82→ Reference Example 83 Reference Example 84→ using the compound produced in Reference Example 31 in place of the compound produced in Reference Example 3.
HPLC retention time (min): 1.05;
MS (ESI, Pos.): 439 (M-OH)+;
¹H-NMR (DMSO-d₆): δ 12.85, 7.50-7.54, 7.38, 7.29-7.35, 7.21-7.29, 7.05-7.12, 5.53-5.60, 5.19-5.27, 4.97, 4.36, 3.90-4.01, 2.72-2.93, 1.97-2.04, 1.73-1.81, 1.47-1.62, 0.93-1.03, 0.64-0.71, 0.54-0.60.

### Example 34: (1R,2R,3aS,10aR)-2-chloro-1-[(1E,3ξ)-4,4-difluoro-3-hydroxy-4-phenyl-1-buten-1-yl]-2,3,3a,9,10,10a-hexahydro-1H-benzo[b]cyclopenta[f]oxepine-6-carboxylic acid

A procedure for a purpose similar to that for Example 25 was performed using a corresponding aldehyde form in place of the compound produced in Reference Example 9 and using a corresponding phosphonate in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 77→ Reference Example 81→ Reference Example 82→ Reference Example 83 Reference Example 84→ using the compound produced in Reference Example 31 in place of the compound produced in Reference Example 3. The corresponding phosphonate was prepared by performing a procedure for a purpose similar to that for Reference Example 28→ Reference Example 29 using iodobenzene in place of 1,3-difluoro-2-iodobenzene.
HPLC retention time (min): 0.97;
MS (ESI, Pos.): 431 (M-OH)+.;
¹H-NMR (DMSO-d₆): δ 7.50-7.59, 7.44, 7.29, 5.99, 5.58-5.69, 4.50-4.60, 4.42-4.48, 4.04-4.13, 2.90-3.02, 2.69-2.88, 2.59-2.64, 2.04-2.12, 1.87-1.96, 1.58-1.74.

### Example 35: (1R,2R,3aS,10aR)-2-chloro-5 fluoro-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid

A procedure for a purpose similar to that for Reference Example 48→ Reference Example 49→ Example 11 was performed using the compound produced in Reference Example 84 in place of the compound produced in Reference Example 40 to yield the title compound exhibiting the following physical properties.
HPLC retention time (min): 1.194;
MS (ESI, Pos.): 459 (M+H-H₂O)+.;
¹H-NMR (CD₃OD): δ 7.40-7.46, 6.92, 5.69-5.79, 5.30-5.39, 4.42, 3.88, 3.85-3.94, 3.01-3.09, 2.71-2.98, 2.34-2.50, 2.28, 2.16-2.24, 1.79-2.01, 1.71, 1.21.

### Reference Example 85: methyl (1S,2R,3aS,10aR)-2-hydroxy-5-methyl-1-({[(2-methyl-2-propanyl)(diphenyl)silyl]oxylmethyl)-2,3,3a,9,10,10a-hexahydro-1H-benzo[b]cyclopenta[f]oxepine-6-carboxylate

A procedure for a purpose similar to that for Reference Example 73 was performed using the compound produced in Reference Example 39 in place of the compound produced in Reference Example 8 to yield the title compound.

### Reference Example 86: methyl (1S,2R,3aS,10aR)-2-methoxy-5-methyl-1-({[(2-methyl-2-propanyl)(diphenyl)silyl]oxylmethyl)-2,3,3a,9,10,10a-hexahydro-1H-benzo[b]cyclopenta[f]oxepine-6-carboxylate

To the compound (2.01 g) produced in Reference Example 85 were added 1,3-dimethyl-3,4,5,6-tetrahydro-2(1H)-pyrimidinone (15 mL) and THF (4 mL) for dissolution, followed by cooling to -50°C. To this solution was added a solution of sodium tert-butoxide (1.06 g) in THF (1 mL) over 10 minutes, and then was added iodomethane (0.345 mL), followed by stirring at -10°C for 2 hours. To the reaction mixture was added an ice-cooled 1 N hydrochloric acid aqueous solution, followed by extraction with n-hexane : ethyl acetate (3 : 1). The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 10 : 1) to yield the title compound (1.50 g) exhibiting the following physical properties.

¹H-NMR (CDCl₃): δ 7.64-7.73, 7.47, 7.33-7.45, 6.94, 4.06-4.18, 3.85-3.94, 3.74-3.80, 3.36, 3.03, 2.42-2.55, 2.33, 2.19-2.27, 2.12, 1.82-1.93, 1.62-1.72, 1.04-1.08.

### Reference Example 87: methyl (1R,2R,3aS,10aR)-1-formyl-2-methoxy-5-methyl-2,3,3a,9,10,10a-hexahydro-1H-benzo[b]cyclopenta[f]oxepine-6-carboxylate

A procedure for a purpose similar to that for Reference Example 8→ Reference Example 9 was performed using the compound produced in Reference Example 86 in place of the compound produced in Reference Example 7 to yield the title compound exhibiting the following physical properties.

¹H-NMR (CDCl₃): δ 9.94, 7.51, 6.97, 4.09-4.18, 3.87, 3.40, 3.28-3.36, 3.13-3.22, 2.56-2.65, 2.47, 2.31-2.43, 2.20-2.27, 1.94-2.06, 1.78-1.88.

### Example 36: (1R,2R,3aS,10aR)-1-{(1E,3ξ)-3-[1-(4-fluorophenyl)cyclobutyl]-3-hydroxy-1-propen-1-yl}-2-methoxy-5-methyl-2,3,3a,9,10,10a-hexahydro-1H-benzo[b]cyclopenta[f]oxepine-6-carboxylic acid

A procedure for a purpose similar to that for Example 25 was performed using the compound produced in Reference Example 87 in place of the compound produced in Reference Example 76 and using a corresponding phosphonate in place of the compound produced in Reference Example 26 to yield the title compound exhibiting the following physical properties. The corresponding phosphonate was prepared by performing a procedure for a purpose similar to that for Example 11 using methyl 1-(4-fluorophenyl)cyclobutanecarboxylate (CAS No. 1090553-79-9) in place of the compound produced in Reference Example 10.
HPLC retention time (min): 1.206;
MS (ESI, Pos.): 503 (M+Na)+.;
¹H-NMR (CD₃OD): δ 7.45-7.49, 7.13-7.18, 6.97-7.04, 5.48-5.55, 5.32-5.39, 4.26, 4.04-4.07, 3.60-3.67, 3.01-3.08, 2.73-2.82, 2.43-2.59, 2.42, 2.26-2.40, 1.93-2.10, 1.75-1.88, 1.62.

### Example 37: (1R,2R,3aS,10aR)-1-[(1E,3ξ)-3-hydroxy-3-(1-phenoxycyclobutyl)-1-propen-1-yl]-2-methoxy-5-methyl-2,3,3a,9,10,10a-hexahydro-1H-benzo[b]cyclopenta[f]oxepine-6-carboxylic acid

A procedure for a purpose similar to that for Example 25 was performed using the compound produced in Reference Example 87 in place of the compound produced in Reference Example 76 and using a corresponding phosphonate in place of the compound produced in Reference Example 26 to yield the title compound exhibiting the following physical properties. The corresponding phosphonate was prepared by performing a procedure for a purpose similar to that for Reference Example 10→ Reference Example 11 using 1-phenoxycyclobutanecarboxylic acid in place of (2S)-2-(2,4-difluorophenyl)propanoic acid.
HPLC retention time (min): 1.183;
MS (ESI, Pos.): 501 (M+Na)+.;
¹H-NMR (CD₃OD): δ 7.47, 7.24, 7.00, 6.95, 6.86-6.90, 5.83-5.90, 5.66-5.73, 4.50, 4.07-4.12, 3.69-3.75, 3.38, 3.07-3.15, 2.89-2.96, 2.47-2.61, 2.29-2.45, 1.82-2.03, 1.64-1.79.

### Example 38: (1R,2R,3aS,10aR)-2-methoxy-5-methyl-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid

A procedure for a purpose similar to that for Reference Example 48→ Reference Example 49→ Example 11 was performed using the compound produced in Reference Example 87 in place of the compound produced in Reference Example 40 to yield the title compound exhibiting the following physical properties.
HPLC retention time (min): 1.163;
MS (ESI, Pos.): 491 (M+Na)+.;
¹H-NMR (CD₃OD): δ 7.47, 6.99, 5.63-5.72, 5.42-5.49, 4.07-4.12, 3.64-3.71, 3.38, 3.06-3.14, 2.81-2.90, 2.36-2.60, 2.22-2.32, 1.81-2.01, 1.64-1.76, 1.20.

### Example 39: (1R,2R,3aS,10aR)-5-chloro-1-[(1E,3ξ)-4,4-difluoro-3-hydroxy-1-octen-1-yl]-2-methoxy-2,3,3a,9,10,10a-hexahydro-1H-benzo[b]cyclopenta[f]oxepine-6-carboxylic acid

A procedure for a purpose similar to that for Example 25 was performed using a corresponding aldehyde form in place of the compound produced in Reference Example 76 and using a corresponding phosphonate in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 86→ Reference Example 87 using the compound produced in Reference Example 33 in place of the compound produced in Reference Example 3. The corresponding phosphonate was prepared by performing a procedure for a purpose similar to that for Reference Example 11 using methyl 2,2-difluorohexanoate (CAS No. 50889-47-9) in place of the compound produced in Reference Example 10.
HPLC retention time (min): 1.137;
MS (ESI, Pos.): 481 (M+Na)+.;
¹H-NMR (CD₃OD): δ 7.38, 7.10, 5.77-5.84, 5.68-5.75, 4.17-4.24, 3.71-3.76, 3.37-3.39, 3.09-3.16, 2.87-2.94, 2.50-2.68, 2.00-2.10, 1.65-1.99, 1.31-1.54, 0.94.

### Example 40: (1R,2R,3aS,10aR)-5-chloro-2-methoxy-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid

A procedure for a purpose similar to that for Reference Example 48→ Reference Example 49→ Example 11 was performed using a corresponding aldehyde form in place of the compound produced in Reference Example 40 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 8→ Reference Example 73→ Reference Example 86→ Reference Example 87 using the compound produced in Reference Example 33 in place of the compound produced in Reference Example 3.
HPLC retention time (min): 1.142;
MS (ESI, Pos.): 511 (M+Na)+.;
¹H-NMR (CD₃OD): δ 7.41, 7.11, 5.64-5.73, 5.42-5.49, 4.17-4.22, 3.64-3.72, 3.38, 3.08-3.17, 2.80-2.89, 2.72-2.78, 2.60-2.69, 2.35-2.57, 2.22-2.32, 2.02-2.08, 1.63-1.97, 1.20.

### Reference Example 88: [(2S,3R,4R)-2-[(4S)-2,2-dimethyl-1,3-dioxolan-4-yl]-4-{[(2-methyl-2-propanyl)(diphenyl)silyl]oxy}tetrahydro-3-furanyl]acetaldehyde

A procedure for a purpose similar to that for Reference Example 9 was performed using 2-[(2S,3R,4R)-2-((R)-2,2-dimethyl-[1,3]dioxolan-4-yl)-tetrahydrofuran-3-yl]ethanol (CAS No. 385841-97-4) in place of the compound produced in Reference Example 8 to yield the title compound having the following physical properties.

¹H-NMR (CDCl₃): δ 9.67, 7.58-7.64, 7.36-7.46, 4.53-4.56, 4.05-4.11, 3.89-3.95, 3.72-3.78, 3.55-3.63, 2.92-3.00, 2.82, 2.42-2.49, 1.40, 1.31, 1.03-1.08.

### Reference Example 89: ({(3R,4R,5S)-4-allyl-5-[(4S)-2,2-dimethyl-1,3-dioxolan-4-yl]tetrahydro-3-furanyl}oxy)(2-methyl-2-propanyl)diphenylsilane

A procedure for a purpose similar to that for Reference Example 3 was performed using the compound produced in Reference Example 88 in place of 4-bromo-3-fluoro-2-hydroxy-benzaldehyde to yield the title compound exhibiting the following physical properties.

¹H-NMR (CDCl₃): δ 7.63-7.67, 7.35-7.46, 5.74-5.84, 4.99-5.11, 4.37-4.41, 3.96-4.02, 3.79-3.88, 3.50-3.62, 2.56-2.64, 2.28-2.36, 1.92-1.99, 1.52, 1.38, 1.32, 1.07.

### Reference Example 90: ([(3R,4R,5S)-5-[(4S)-2,2-dimethyl-1,3-dioxolan-4-yl]-4-(1-propen-1-yl)tetrahydro-3-furanyl}oxy)(2-methyl-2-propanyl)diphenylsilane

To a solution of the compound (4.59 g) produced in Reference Example 89 in toluene (30 mL) was added carbochlorohydridetris(triphenylphosphine)ruthenium(II) (CAS No.: 16971-33-8, 0.45 g) at room temperature, followed by stirring at 60°C for 4 hours. The reaction solution was concentrated under reduced pressure. The obtained residue was purified by silica gel column chromatography (n-hexane : ethyl acetate = 90 : 10) to yield the title compound (4.45 g).

### Reference Example 91: (3S,4S,5S)-5-[(4S)-2,2-dimethyl-1,3-dioxolan-4-yl]-4-(1-propen-1-yl)tetrahydro-3-furanol

A procedure for a purpose similar to that for Reference Example 8→ Reference Example 1→ Reference Example 2 was performed using the compound (4.45 g) produced in Reference Example 90 in place of the compound produced in Reference Example 7 to yield the title compound (1.51 g) exhibiting the following physical properties.

¹H-NMR (CDCl₃): δ 5.51-5.64, 5.33-5.41, 5.23-5.31, 4.39-4.45, 3.94-4.10, 3.76-3.92, 3.61-3.70, 3.30-3.38, 3.06-3.12, 2.70-2.75, 2.05, 1.68-1.68, 1.65-1.74, 1.53, 1.50, 1.34-1.41.

### Reference Example 92: methyl (1S,3aR,10aS)-1-[(4S)-2,2-dimethyl-1,3-dioxolane-4-yl]-5-methyl-1,3,3a,9,10,10a-hexahydrofuro[3,4-b][1]benzoxepine-6-carboxylate

A procedure for a purpose similar to that for Reference Example 36→ Reference Example 37→ Reference Example 38 was performed using the compound produced in Reference Example 91 in place of the compound produced in Reference Example 2 to yield the title compound exhibiting the following physical properties.

¹H-NMR (CDCl₃): δ 7.48, 6.96, 4.43-4.49, 4.04-4.16, 3.95-4.02, 3.85-3.88, 3.04-3.13, 2.76-2.88, 2.41-2.45, 2.21-2.37, 1.94-2.06, 1.44, 1.37.

### Reference Example 93: methyl (1S,3aR,10aS)-1-formyl-5-methyl-1,3,3a,9,10,10a-hexahydrofuro[3,4-b][1]benzoxepine-6-carboxylate

The compound produced in Reference Example 92 (3.0 g) was dissolved in ethyl acetate (45 mL) and THF (15 mL), and periodic acid (2.64 mL) was added in portions at 0°C, followed by stirring at room temperature for 2 hours. The precipitate was removed by filtration, and the filtrate was poured into an ice-cooled ethyl acetate-saturated sodium thiosulfate aqueous solution, followed by extraction with ethyl acetate. The organic layer was washed with saturated saline, dried over anhydrous sodium sulfate, and then filtered. The filtrate was concentrated under reduced pressure. The obtained residue was purified by silica gel column chromatography (n-hexane : ethyl acetate = 90 : 10→50 : 50→1 : 10) to yield the title compound (1.71 g) exhibiting the following physical properties.

¹H-NMR (CDCl₃): δ 9.77, 7.54, 7.01, 4.32-4.45, 4.22-4.26, 3.88, 3.13-3.24, 2.65-2.74, 2.41-2.47, 2.07-2.18, 2.07-2.09, 1.87-1.97.

### Example 41: (1R,3aR,10aR)-1-[(1E,3ξ,4S)-3-hydroxy-4-methyl-1-octen-1-yl]-5-methyl-1,3,3a,9,10,10a-hexahydrofuro[3,4-b][1]benzoxepine-6-carboxylic acid

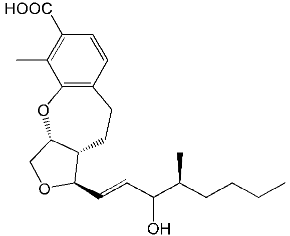

A procedure for a purpose similar to that for Example 25 was performed using the compound (550 mg) produced in Reference Example 93 in place of the compound produced in Reference Example 76, using dimethyl-(2-oxo-3 S-methylheptyl)-phosphonate (CAS No.: 39850-01-6, 582 mg) in place of the compound produced in Reference Example 26 and using (R)-2-methyl-CBS-oxazaborolidine in place of (S)-2-methyl-CBS-oxazaborolidine to yield the title compound exhibiting the following physical properties.
HPLC retention time (min): 1.15;
MS (ESI, Pos.): 411 (M+Na)+, 371 (M+H-H₂O)+.;
¹H-NMR (CD₃OD): δ 7.47, 7.02, 5.77-5.84, 5.59-5.66, 4.47-4.52, 4.42, 4.20-4.25, 4.08-4.12, 3.93, 3.08-3.16, 2.67-2.76, 2.42, 2.07-2.14, 1.91-1.99, 1.77-1.86, 1.49-1.63, 1.22-1.43, 1.06-1.16, 0.86-0.95.

### Example 41-1 to 41-3:

A procedure for a purpose similar to that for Example 25 was performed using the compound produced in Reference Example 93 in place of the compound produced in Reference Example 76 and using a corresponding phosphonate in place of the compound produced in Reference Example 26 to yield the title compound exhibiting the following physical properties. The corresponding phosphonate is a known compound, or was prepared from a known compound by subjecting the compound to a procedure for a purpose similar to that for Reference Example 10→ Reference Example 11.

### Example 41-1: (1R,3aR,10aR)-1-[(1E,3ξ)-3-(1-butylcyclobutyl)-3-hydroxy-1-propen-1-yl]-5-methyl-1,3,3a,9,10,10a-hexahydrofuro[3,4-b][1]benzoxepine-6-carboxylic acid

HPLC retention time (min): 1.25;
MS (ESI, Pos.): 437 (M+Na)+.;
¹H-NMR (CD₃OD): δ 7.50, 7.03, 5.82-5.90, 5.64-5.72, 4.48-4.54, 4.42, 4.21-4.25, 4.07-4.12, 4.00, 3.08-3.16, 2.67-2.75, 2.43, 2.03-2.15, 1.91-1.99, 1.76-1.89, 1.55-1.74, 1.26-1.44, 0.91-0.98.

### Example 41-2: (1R,3aR,10aR)-1-[(1E,3ξ)-3-hydroxy-3-(1-phenoxycyclobutyl)-1-propen-1-yl]-5-methyl-1,3,3a,9,10,10a-hexahydrofuro[3,4-b][1]benzoxepine-6-carboxylic acid

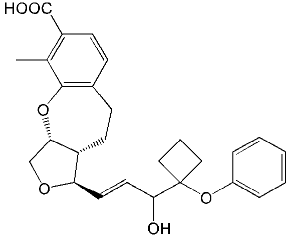

HPLC retention time (min): 1.138;
MS (ESI, Pos.): 473 (M+Na)+.;
¹H-NMR (CD₃OD): δ 7.50, 7.24, 7.03, 6.93-6.98, 6.85-6.91, 5.98-6.05, 5.71-5.79, 4.50-4.56, 4.41, 4.20-4.25, 4.07-4.13, 3.08-3.17, 2.66-2.76, 2.24-2.46, 2.05-2.13, 1.92-2.02, 1.61-1.86.

### Example 41-3: (1R,3aR,10aR)-1-[(1E,3ξ,4ξ)-3-hydroxy-7-methyl-4-(trifluoromethyl)-1-octen-1-yl]-5-methyl-1,3,3a,9,10,10a-hexahydrofuro[3,4-b][1]benzoxepine-6-carboxylic acid

HPLC retention time (min): 1.201;
MS (ESI, Pos.): 439 (M+H-H₂O)+.;
¹H-NMR (CD₃OD): δ 7.46, 7.02, 5.84-5.91, 5.73-5.79, 4.46-4.55, 4.42, 4.20-4.25, 4.10, 3.08-3.15, 2.66-2.74, 2.42, 2.18-2.29, 2.07-2.15, 1.90-1.99, 1.76-1.88, 1.49-1.71, 1.27-1.41, 0.91.

### Example 42: (1R,3aR,10aR)-5-chloro-1-[(1E,3ξ)-6-cyclobutyl-3-hydroxy-4-methylene-1-hexen-1-yl]-1,3,3a,9,10,10a-hexahydrofuro[3,4-b][1]benzoxepine-6-carboxylic acid

A procedure for a purpose similar to that for Example 25 was performed using a corresponding aldehyde form in place of the compound produced in Reference Example 76 and using a corresponding phosphonate in place of the compound produced in Reference Example 26 to yield the title compound exhibiting the following physical properties. The corresponding aldehyde form was prepared by performing a procedure for a purpose similar to that for Reference Example 4→ Reference Example 5→ Reference Example 6→ Reference Example 7→ Reference Example 93 using the compound produced in Reference Example 91 in place of the compound produced in Reference Example 2 and using the compound produced in Reference Example 33 in place of the compound produced in Reference Example 3. The corresponding phosphonate was prepared by performing a procedure for a purpose similar to that for Reference Example 11 using ethyl α-methylenecyclobutanbutanoate (CAS No.: 915203-87-1) in place of the compound produced in Reference Example 10.
HPLC retention time (min): 1.184;
MS (ESI, Pos.): 415 (M+H-H₂O)+.;
¹H-NMR (CD₃OD): δ 7.43, 7.14, 5.76-5.83, 5.65-5.73, 5.09, 4.84-4.91, 4.46-4.56, 4.21-4.26, 4.11-4.16, 3.10-3.19, 2.74-2.82, 2.24-2.36, 2.13-2.17, 2.02-2.10, 1.79-2.00, 1.53-1.67.

### Example 43: (1R,3aR,10aR)-5-fluoro-1-[(1E,4S)-7,8,8-trifluoro-4-hydroxy-4-methyl-1,7-octadien-1-yl]-1,3,3a,9,10,10a-hexahydrofuro[3,4-b][1]benzoxepine-6-carboxylic acid

A procedure for a purpose similar to that for Reference Example 48→ Reference Example 49→ Example 11 was performed using a corresponding aldehyde form in place of the compound produced in Reference Example 40 to yield the title compound exhibiting the following physical properties.

The corresponding aldehyde form was prepared by performing a procedure for a purpose similar to that for Reference Example 4→ Reference Example 5→ Reference Example 6→ Reference Example 7→ Reference Example 93 using the compound produced in Reference Example 91 in place of the compound produced in Reference Example 2.
HPLC retention time (min): 1.121;
MS (ESI, Pos.): 467 (M+Na)+.;
¹H-NMR (CD₃OD): δ 7.47-7.53, 6.98, 5.83-5.92, 5.49-5.57, 4.64, 4.35-4.40, 4.22-4.28, 4.03-4.08, 3.23-3.29, 3.07-3.17, 2.86-2.96, 2.33-2.56, 2.23-2.31, 2.06-2.20, 1.78-2.01, 1.66-1.75, 1.19.

### Pharmacological Example 1: measurement of agonist activity on various human prostanoid receptor

Using CHO cells (EP1-CHO, EP2-CHO, EP3-Chem1, EP4-CHO and IP-CHO) on which various human prostanoid receptors were forcibly expressed, the agonist activity of the test compound on the various prostanoid receptors was examined using the intracellular calcium concentration for EP1 and EP3, and the intracellular cyclic AMP (hereinafter, abbreviated as cAMP) production amount for EP2, EP4 and IP as indices.

### <Compound Treatment>

The test compound and control substances (PGE₂ and iloprost) were dissolved in dimethyl sulfoxide (DMSO) to prepare a 10 mmol/L solution. The prepared 10 mmol/L solution was thawed at the time of use, serially diluted using DMSO, and diluted with a buffer solution 1 for measurement (Hank's balanced salt solution (Thermofischer scientific) containing 1 w/v% human serum albumin (Sigma), 2 µmol/L indomethacin (Sigma), 2.5 mmol/L probenecid, and 10 mmol/L HEPES-NaOH (pH7.4)) or a buffer solution 2 for measurement (D-PBS (Nacalai Tesque) containing 0.1 w/v% human serum albumin, 2 µmol/L diclofenac (Sigma), and 1 mmol/L 3-isobutyl-1-methylxanthine (Sigma)) to perform experiments.

### <Measurement of Agonist Activity>

### (1) Measurement of EP1 and EP3 activities (measurement of intracellular calcium concentration)

The intracellular calcium concentration was measured using Calcium 6 Assay Kit (Molecular Devices). On the day before the measurement, EP1-CHO and EP3-Chem 1 were obtained by thawing the frozen cells, suspended using Ham's F-12 medium (Thermofischer scientific) containing inactivated (56°C, 30 min) 9.8 vol% FBS (Thermofischer scientific) and penicillin-streptomycin (Nacalai Tesque), seeded into a 96 well UV plate to 1.0×10⁵ cells per well, and static cultured at 37°C in the presence of 5%CO₂. On the day of the measurement, the medium was removed, followed by addition of 120 µL of the Calcium indication solution dissolved in the buffer solution 1 for measurement and incubation at room temperature under light-shielding for about 1 hour. After 1 hour incubation, the 96 well UV plates were set in a fluorescence spectrophotometer (FDSS-7000, Hamamatsu Photonics) to measure the intracellular calcium concentration. Thirty microliters of the buffer solution 1 for measurement containing various concentrations of agonists were added, and the mixture was reacted. The intracellular calcium concentration was measured by irradiating the cells with 480 nm excitation light and measuring the fluorescence intensity at 540 nm.

### (2) Measurement of EP2, EP4 and IP agonist activities (measurement of cAMP concentration)

On the day of the measurement, EP2-CHO, EP4-CHO, and IP-CHO were obtained by thawing frozen cells. Subsequently, they were suspended in D-PBS containing 2 µmol/L diclofenac and centrifuged at 500 g for 3 minutes at room temperature, and then the supernatant was removed. The cells were suspended in the buffer solution 2 for measurement, and 10 µL each of the suspension was dispensed into a 384 well plate such that the number of cells per well was 2.0×10⁵ (EP2-CHO), 5.0×10⁴ (EP4-CHO), and 1.0×10⁵ (IP-CHO). Twenty microliters of the buffer solution 2 for measurement containing various concentrations of agonists were added, and the mixture was reacted at room temperature for 1 hour. The cAMP concentration was measured using a cAMP HTRF HiRange kit (CIS bio International). According to the Two step protocol of the kit instruction, 5 µL each of cAMP-D2 and Cryptase diluted with Lysis Buffer was added, followed by incubation at room temperature for 1 hour. After incubation for 1 hour, time-resolved fluorescence at 620 nm and 660 nm when excited at 340 nm was measured using Synergy Neo (Bioteck), and the ratio (TRF ratio) was determined to calculate the cAMP concentration from the calibration curve.

### <Result>

The agonist activity of the present compound on various prostanoid receptors was measured using the above method. The measurement results of the agonist activity (EC₅₀ value) on the EP3 receptor are shown in Table 1, and the measurement results of the agonist activity (EC₅₀ value) on the EP1, EP2, EP4 and IP receptors are shown in Table 2. It has been confirmed that the present compound described in the table has potent agonist activity on the EP3 receptor and has EP3-selective agonist activity as compared with agonist activity on the EP1, EP2 and IP receptors.

**[Table 1]**

| Test substance | EC50 (µmol/L) | Test substance | EC50 (µmol/L) |
|---|---|---|---|
| Example 1 | 0.01 | Example 26-9 | 0.05 |
| Example 3 | 0.02 | Example 26-17 | 0.02 |
| Example 7-13 | 0.02 | Example 26-19 | 0.03 |
| Example 7-30 | 0.04 | Example 26-20 | 0.05 |
| Example 8 | 0.03 | Example 26-21 | 0.02 |
| Example 9-5 | 0.03 | Example 28 | 0.03 |
| Example 10 | 0.003 | Example 28-2 | 0.01 |
| Example 11 | 0.01 | Example 32 | 0.004 |
| Example 12 | 0.01 | Example 32-6 | 0.02 |
| Example 13 | 0.01 | Example 41 | 0.01 |
| Example 25-7 | 0.05 | Example 41-2 | 0.01 |

**[Table 2]**

| Test substance | EC50 (µmol/L) | | | | Test substance | EC50 (µmol/L) | | | |
|---|---|---|---|---|---|---|---|---|---|
| | EP1 | EP2 | EP4 | IP | | EP1 | EP2 | EP4 | IP |
| Example 1 | 3.5 | 6.3 | 0.04 | 6.9 | Example 26-9 | > 10 | > 10 | 0.8 | > 10 |
| Example 3 | 7.3 | 6.2 | 0.02 | 3.1 | Example 26-17 | > 10 | > 10 | 0.6 | > 10 |
| Example 7-13 | 6.3 | 1.4 | 0.9 | > 10 | Example 26-19 | > 10 | > 10 | 0.1 | > 10 |
| Example 7-30 | > 10 | > 10 | 1.5 | > 10 | Example 26-20 | > 10 | > 10 | 8.1 | > 10 |
| Example 8 | 2.7 | 7.2 | 0.02 | 7.4 | Example 26-21 | > 10 | > 10 | 6.6 | > 10 |
| Example 9-5 | > 10 | > 10 | 8.6 | > 10 | Example 28 | > 10 | > 10 | 1.1 | > 10 |
| Example 10 | > 10 | > 10 | > 10 | > 10 | Example 28-2 | > 10 | > 10 | 0.4 | > 10 |
| Example 11 | > 10 | > 10 | > 10 | > 10 | Example 32 | > 10 | > 10 | 7.8 | > 10 |
| Example 12 | 3.5 | > 10 | > 10 | > 10 | Example 32-6 | > 10 | > 10 | 2.5 | > 10 |
| Example 13 | > 10 | > 10 | > 10 | > 10 | Example 41 | > 10 | > 10 | > 10 | > 10 |
| Example 25-7 | > 10 | > 10 | 5.7 | > 10 | Example 41-2 | > 10 | > 10 | > 10 | > 10 |

### Pharmacological Example 2: Evaluation of inhibitory effect on the amount of albumin excreted in urine of the present compound in diabetes model rat

### <Feeding condition>

Male SDT Fatty rats aged 6 weeks (CLEA Japan) were purchased and preliminarily bred for 1 week or more. Experiments were performed at 8 to 16 weeks of age. The animals were fed ad libitum with solid feed CRF-1 (Oriental Yeast) and watered ad libitum.

### <Grouping method>

Animals were housed in metabolic cages 2 days before test substance administration, and urine was collected for 24 hours. On the day before administration of the test substance, urine albumin and urine creatinine were measured, and grouping was performed by a stratification randomization method based on the urine albumin/urine creatinine ratio (ACR) and the average value of body weights for each group.

### <Administration of test substance>

The compounds (test substances) of Examples 9-5, 10, 11, 12, 25-7 and 32, and water for injection (Otsuka Pharmaceutical) as a vehicle were orally administered once at 5 mL/kg. The administration dose is described in Table 3.

**[Table 3]**

| Test substance | Dose (mg/kg) |
|---|---|
| Example 9-5 | 0.1-0.3 |
| Example 10 | 0.01-0.1 |
| Example 11 | 0.01-0.03 |
| Example 12 | 0.01-0.03 |
| Example 25-7 | 0.003-0.01 |
| Example 32 | 0.03-0.1 |

### <Measurement of ACR>

For evaluation of the amount of albumin excreted in urine of each animal, a urine albumin/urine creatinine ratio (ACR) was measured. After administration of the test substance, the animals were housed in metabolic cages, and urine was collected for 24 hours. Each concentration of urine albumin and urine creatinine was measured using an automatic analyzer 7180 (Hitachi High-Tech), and ACR was calculated by dividing the urine albumin concentration by the urine creatinine concentration.

### <Result>

The measurement results are shown in Table 4. It has been confirmed that the present compound described in the table has an effect of suppressing the amount of albumin excreted in urine in a diabetes model rat.

**[Table 4]**

| Test substance | Dose | n | ACR (µg/mg) | |
|---|---|---|---|---|
| | | | mean | S.E. |
| | Control | 7 | 7604.9 | 978.4 |
| Example 9-5 | 0.1 mg/kg | 7 | 4691.4 | 415.8 |
| | 0.3 mg/kg | 7 | 4466.2 | 649 |
| | Control | 6 | 1699.9 | 311.4 |
| Example 10 | 0.01 mg/kg | 6 | 909.3 | 203.7 |
| | 0.1 mg/kg | 6 | 751.6 | 175.3 |
| | Control | 6 | 1822.9 | 811.5 |
| Example 11 | 0.01 mg/kg | 6 | 1010.5 | 183.4 |
| | 0.03 mg/kg | 6 | 575.3 | 114.9 |
| | Control | 6 | 7874.4 | 1459.6 |
| Example 12 | 0.01 mg/kg | 6 | 4602.3 | 517.1 |
| | 0.03 mg/kg | 6 | 3887.3 | 533.3 |
| | Control | 6 | 3419.8 | 504 |
| Example 25-7 | 0.003 mg/kg | 6 | 3217.6 | 382.8 |
| | 0.01 mg/kg | 6 | 2331.6 | 314.3 |
| | Control | 6 | 5709.6 | 359.2 |
| Example 32 | 0.03 mg/kg | 6 | 3391 | 391.2 |
| | 0.1 mg/kg | 6 | 3086.1 | 324.2 |

### Pharmacological Example 3: Evaluation of in vivo drug efficacy of the present compound on renal damage in rat 5/6 kidney-removed model

The in vivo drug efficacy of the present compound on renal damage after 5/6 kidney was removed was evaluated using male rats. This 5/6 kidney-removed model is a model reflecting glomerular hyperfiltration which is a disease state of a patient with chronic renal disease, and is used as a chronic renal disease model (Am J Physiol Renal Physiol, 2022 vol. 322, no. 6, p.639-654).

### <Feeding condition>

Male Jcl: Wistar (Wistar) rats aged 5 weeks were purchased from CLEA Japan, and subjected to experiments. The animals were fed ad libitum and watered ad libitum.

### <5/6 kidney removal>

After acclimation breeding, rats aged 7 weeks having no apparent abnormality were used. After body weight measurement, sodium secobarbital (Ional sodium, Nichi-Iko Pharmaceutical) was intraperitoneally administered at a volume of 1 mL/kg to provide 60 mg/kg for anesthesia. Buprenorphine (0.01 mg/mL solution, Otsuka Pharmaceutical) was subcutaneously administered at a volume of 4 mL/kg for the purpose of analgesia before surgery. After removing hair from the left abdomen of the rat, the left abdomen was disinfected with ethanol and isodine for disinfection and incised to expose the left kidney. The blood flow of the renal artery and vein was stopped using a clamp, and about 2/3 of the left kidney was excised. After hemostatic gauze (SURGICEL) was applied to the resected surface and hemostasis was confirmed, the clamp was removed to return the kidney to the abdomen, and the incision was sutured. The following day, analgesic buprenorphine was administered subcutaneously at a dose of 0.04 mg/kg. One week after the left kidney was removed, the body weight was measured, and then buprenorphine was subcutaneously administered at a dose of 0.04 mg/kg under inhalation anesthesia with isoflurane (Mayran). After removing hair from the right abdomen of the rat, the right abdomen was disinfected with ethanol and isodine for disinfection and incised to expose the right kidney. The renal artery and vein was ligated with a suture to stop the blood flow, the right kidney was totally removed, and the incision was sutured. The following day, analgesic buprenorphine was administered subcutaneously at a dose of 0.04 mg/kg.

### <Grouping method>

Six weeks after the right kidney was removed, the mice were divided into groups so that the average of the above parameters was almost equal between the groups on the basis of body weight, serum urea nitrogen (BUN), serum creatinine (S-CRE), serum total cholesterol (T-CHO), neutral fat (TG), urinary protein (U-TP), urinary albumin (U-ALB), creatinine clearance (CCr), and blood pressure.

### <Administration of test substance>

The test substance and water for injection (Otsuka Pharmaceutical) as a vehicle were orally administered at 5 mL/kg once a day for 8 weeks. The administration dose is described in Table 5.

### <Measurement of biochemical parameter>

Blood was collected by puncturing the tail vein of the rat with an injection needle. Blood was collected in a tube for serum separation (BD Microtainer) and centrifuged to obtain serum. Urine was collected for about 24 hours in a metabolic cage for rat (CLEA Japan) under conditions in which a solid feed and water can be freely taken.

The obtained urine sample was centrifuged, and the supernatant was subjected to measurement. The concentrations of BUN, S-CRE, T-CHO, TG, U-ALB, U-TP and urinary creatinine (U-CRE) were measured by an automatic analyzer Hitachi 7180 (Hitachi High-Tech).

### <Evaluation of renal pathological tissue>

The collected kidney was fixed with 10 vol% neutral buffered formalin solution. Sections were prepared from paraffin-embedded blocks according to a conventional method, and specimens stained with hematoxylin and eosin (H & E), periodic acid-Schiff (PAS), periodic acid-methenamine-silver (PAM), and Masson's trichrome (MT) were prepared. The obtained specimen was observed under an optical microscope, and the proportion of the glomeruli in which mesangial proliferation findings were observed among all the glomeruli on the section was calculated.

### <Result>

The in vivo drug efficacy of the present compound on renal damage in a rat 5/6 kidney-removed model was evaluated using the above method. The measurement results of the amount of albumin excreted in urine per 24 hours after 8 weeks from the start of administration are shown in Table 6, and the proportion of mesangial proliferation in all the glomeruli on the section is shown in Table 7. The present compounds described in the table suppressed an increase in the amount of albumin excreted in urine due to provocation of a disease state, and decreased the proportion of glomeruli in which mesangial proliferation was observed. From the above, it has been confirmed that the present compound has a preventive and/or therapeutic effect on renal damage (for example, chronic renal disease).

**[Table 5]**

| Test substance | Dose (mg/kg) |
|---|---|
| Example 10 | 0.1 |
| Example 11 | 0.01-0.03 |
| Example 12 | 0.01-0.03 |

**[Table 6]**

| Test substance | Dose | Number of examples | Amount of albumin excreted in urine (mg/24 h) | |
|---|---|---|---|---|
| | | | mean | S.E. |
| | Control | 8 | 75.90 | 7.98 |
| Example 10 | 0.1 mg/kg | 9 | 35.47 | 6.52 |
| Example 11 | 0.01 mg/kg | 9 | 46.01 | 5.47 |
| | 0.03 mg/kg | 9 | 37.94 | 3.74 |
| Example 12 | 0.01 mg/kg | 9 | 66.09 | 8.81 |
| | 0.03 mg/kg | 9 | 44.16 | 6.24 |

**[Table 7]**

| Test substance | Dose | Number of examples | Mesangial proliferation (% of total glomeruli) | |
|---|---|---|---|---|
| | | | mean | S.E. |
| | Control | 8 | 38.69 | 1.99 |
| Example 10 | 0.1 mg/kg | 9 | 27.33 | 2.67 |
| Example 11 | 0.01 mg/kg | 9 | 27.65 | 2.14 |
| | 0.03 mg/kg | 9 | 27.07 | 1.07 |
| Example 12 | 0.01 mg/kg | 9 | 27.67 | 2.56 |
| | 0.03 mg/kg | 9 | 26.35 | 1.94 |

### Pharmacological Example 4: Evaluation of in vivo drug efficacy of the present compound in diabetic renal disease model rat

A diabetic renal disease model was created by extracting the unilateral kidneys of male SDT.Cg-Leprfa/JttJcl (SDT-fatty/Jcl, CLEA Japan) rats, a type 2 diabetes model, and then giving saline. The in vivo drug efficacy of the present compound on diabetic renal disease was evaluated in this model. The present model is considered to be a useful model for evaluating the adaptability of the present compound to diabetic renal disease, because findings more similar to those of diabetic renal disease patients than those of normal type 2 diabetes model are observed (Clin Exp Pharmacol Physiol, 2022, vol. 49, no. 4, p. 492-500).

### <Feeding condition>

Male SDT.Cg-Leprfa/JttJcl (SDT-fatty rat) aged 4 weeks was purchased from CLEA Japan, and subjected to experiments. The animals were fed ad libitum with Purina5008 (Japan SLC) from the time of arrival, and watered ad libitum. Water was given from the arrival and until 1 week from single kidney removal, and a 0.2 w/v% NaCl solution was given 1 week after single kidney removal.

### <Single kidney removal treatment>

After acclimation breeding, the body weight was measured, and buprenorphine was subcutaneously administered at a dose of 0.01 mg/kg under inhalation anesthesia with isoflurane (Mayran). After removing hair from the right abdomen of the rat, the right abdomen was disinfected with ethanol and isodine for disinfection and incised to expose the right kidney. The renal artery and vein was ligated with a suture to stop the blood flow, the right kidney was totally removed, and the incision was sutured. The following day, analgesic buprenorphine was administered subcutaneously at a dose of 0.01 mg/kg.

### <Grouping method>

Grouping was performed 3 weeks after single kidney removal. The mice were divided into groups so that the average of the above parameters was almost equal between the groups on the basis of body weight, serum urea nitrogen (BUN), serum creatinine (S-CRE), serum total cholesterol (T-CHO), blood glucose level (GLU), neutral fat (TG), urinary protein (U-TP), urinary albumin (U-ALB), and creatinine clearance (CCr).

### <Administration of test substance>

All of the compounds of Examples 25-7, 10, 11 and 12 were orally administered once a day for 8 weeks in combination with losartan (30 mg/kg), a renin angiotensin system inhibitor, and canagliflozin (10 mg/kg), a glucose co-transporter inhibitor. The compounds of Examples 25-7, 10, 11 and 12, and losartan were each dissolved in water for injection (Otsuka Pharmaceutical), and canagliflozin was suspended in a 0.5% methyl cellulose solution (Fujifilm Wako Pure Chemical Corporation) to prepare an administration solution. In the Control-1 group, water for injection which is a vehicle of losartan or the compound of Examples 25-7, 10, 11 or 12 was orally administered at 5 mL/kg, and a 0.5% methylcellulose solution which is a vehicle of canagliflozin was orally administered at 2.5 mL/kg once a day for 8 weeks. In the Control-2 group, losartan (30 mg/kg) dissolved in water for injection was orally administered at an administration volume of 2.5 mL/kg, canagliflozin (10 mg/kg) suspended in a 0.5% methyl cellulose solution was orally administered at an administration volume of 2.5 mL/kg, and water for injection which is a vehicle of the compounds of Examples 25-7, 10, 11 and 12 was orally administered at an administration volume of 2.5 mL/kg once a day for 8 weeks. The administration dose is described in Table 8.

### <Measurement of biochemical parameter>

Blood was collected by puncturing the tail vein of the rat with an injection needle. Blood was collected in a tube for serum separation (BD Microtainer) and centrifuged to obtain serum. Urine was collected for 24 hours in a metabolic cage for rat under conditions in which a solid feed and water can be freely taken. The obtained urine sample was centrifuged, and the supernatant was subjected to measurement. The concentrations of BUN, S-CRE, T-CHO, TG, U-ALB, U-TP, GLU and U-CRE were measured by an automatic analyzer Hitachi 7180 (Hitachi High-Tech).

### <Result>

The effect of the present compound in a diabetic renal disease model was evaluated using the above method. The measurement results of the serum creatinine (sCre) concentration 8 weeks after the start of administration are shown in Tables 9 and 10. In the group to which the present compound had been administered, an effect of suppressing the increase in serum creatinine (sCre) was observed, as compared with the Control-2 group. From the above, it has been confirmed that the present compound has a preventive and/or therapeutic effect on renal damage (for example, diabetic renal disease).

**[Table 8]**

| Test substance | Dose (mg/kg) |
|---|---|
| Example 25-7 | 0.1 |
| Example 10 | 0.1 |
| Example 11 | 0.01-0.03 |
| Example 12 | 0.01-0.03 |
| Losartan | 30 |
| Canagliflozin | 10 |

**[Table 9]**

| Concomitant drug | Test substance | Dose | Number of examples | Serum creatinine (mg/dL) | |
|---|---|---|---|---|---|
| | | | | mean | S.E. |
| | | Control-1 | 9 | 0.62 | 0.05 |
| Losartan Canagliflozin | | Control-2 | 9 | 0.56 | 0.02 |
| | Example 25-7 | 0.1 mg/kg | 9 | 0.45 | 0.01 |

**[Table 10]**

| Concomitant drug | Test substance | Dose | Number of examples | Serum creatinine (mg/dL) | |
|---|---|---|---|---|---|
| | | | | mean | S.E. |
| | | Control-1 | 9 | 0.58 | 0.03 |
| | | Control-2 | 9 | 0.59 | 0.05 |
| | Example 10 | 0.1 mg/kg | 9 | 0.50 | 0.03 |
| Losartan Canagliflozin | Example 11 | 0.01 mg/kg | 9 | 0.46 | 0.01 |
| | | 0.03 mg/kg | 9 | 0.47 | 0.02 |
| | Example 12 | 0.01 mg/kg | 9 | 0.51 | 0.02 |
| | | 0.03 mg/kg | 9 | 0.45 | 0.02 |

### Pharmacological Example 5: Evaluation of in vivo drug efficacy of the present compound in Alport syndrome model mice

The in vivo drug efficacy of the present compound on Alport syndrome was evaluated using COL4A5 (causative gene of Alport syndrome) knockout mice. Since such mice have findings similar to those of Alport syndrome patients (Biochem Biophys Rep, vol. 12, no. 17, p. 81-86), they are considered to be a useful model for evaluating the adaptability of the present compound to Alport syndrome.

### <Obtainment of animal>

Male wild-type mice and female COL4A5 hetero knockout mice were naturally crossed to obtain offspring. Genotypes of the offspring were confirmed and male COL4A5 homozygote knockout mice were obtained.

### <Feeding condition>

The obtained animals were fed ad libitum with CE-2 (CLEA Japan), and watered ad libitum.

### <Grouping method>

The mice were divided into groups using a statistical analysis system EXSUS10.0 so that the body weight and the urine albumin/urine creatinine value at the time of 6 weeks of age were almost uniform in each group.

### <Administration of test substance>

The test substance and water for injection (Otsuka Pharmaceutical) as a vehicle were orally administered at 10 mL/kg once a day for 17 weeks.

The Control-3 group was orally administered water for injection at 10 mL/kg once a day for 17 weeks. In the Control-4 group, water for injection and an administration solution of irbesartan which is a renin angiotensin system inhibitor were mixed in equal amounts at 5 mL/kg each, and orally administered once a day for 17 weeks.

When the compound of Example 12 and irbesartan, a renin angiotensin system inhibitor, were used in combination, the respective administration solutions were mixed in equal amounts at 5 mL/kg, and the mixture was orally administered once a day for 17 weeks. The administration dose is described in Table 11.

### <Measurement of biochemical parameter>

Blood collection was performed by cutting the tail vein of the mice a little with a razor and using a hematocrit capillary. Blood was centrifuged to obtain plasma. Urine was collected for about 16 hours in a metabolic cage for mouse under conditions in which a solid feed and water can be freely taken. The obtained urine sample was centrifuged, and the supernatant was subjected to measurement. Urine albumin (U-ALB) and urine creatinine (U-CRE) concentrations were measured by an automatic analyzer Hitachi 7180 (Hitachi High-Tech), and the creatinine concentration in plasma was measured using LabAssay Creatinine (Fujifilm Wako Shibayagi).

### <Result>

The in vivo drug efficacy of the present compound in Alport syndrome model mice was evaluated using the above method. The measurement results of the creatinine concentration in plasma 16 weeks after the start of administration are shown in Tables 12 and 13, and the measurement results of the urine albumin/urine creatinine ratio are shown in Tables 14 and 15. The present compound described in the table suppressed the increase in serum creatinine and urine albumin/urine creatinine ratio in the Alport syndrome model mice in both cases of single agent administration and combined administration with irbesartan. From the above, it has been confirmed that the present compound has a preventive and/or therapeutic effect on renal damage (for example, Alport syndrome).

**[Table 11]**

| Test substance | Dose (mg/kg) |
|---|---|
| Example 25-7 | 0.1-0.3 |
| Example 12 | 1-3 |
| Irbesartan | 50 |

**[Table 12]**

| Test substance Dose | | Number of examples | Plasma creatinine (mg/dL) | |
|---|---|---|---|---|
| | | | mean | S.D. |
| | Control-3 | 9 | 0.922 | 0.242 |
| Example 25-7 | 0.1 mg/kg | 9 | 0.758 | 0.055 |
| | 0.3 mg/kg | 9 | 0.628 | 0.055 |

**[Table 13]**

| Concomitant drug | Test substance | Dose | Number of examples | Plasma creatinine (mg/dL) | |
|---|---|---|---|---|---|
| | | | | mean | S.E. |
| | | Control-3 | 9 | 0.885 | 0.054 |
| Irbesartan | | Control-4 | 8 | 0.771 | 0.034 |
| | Example 12 | 1 mg/kg | 9 | 0.709 | 0.082 |
| | | 3 mg/kg | 9 | 0.722 | 0.014 |

**[Table 14]**

| Test substance | Dose | Number of examples | Urine Albumin/Urine Creatinine Ratio (mg/g) | |
|---|---|---|---|---|
| | | | mean | S.D. |
| | Control-3 | 9 | 51455.96 | 11815.49 |
| Example 25-7 | 0.1 mg/kg | 9 | 31781.76 | 6292.97 |
| | 0.3 mg/kg | 9 | 19605.92 | 7198.52 |

**[Table 15]**

| Concomitant drug | Test substance | Dose | Number of examples | Urine Albumin/ Urine Creatinine Ratio (mg/g) | |
|---|---|---|---|---|---|
| | | | | mean | S.E. |
| | | Control-3 | 9 | 49769.31 | 5992.95 |
| Irbesartan | | Control-4 | 8 | 31414.47 | 3969.01 |
| | Example 12 | 1 mg/kg | 9 | 11637.91 | 2042.33 |
| | | 3 mg/kg | 9 | 21945.67 | 3921.74 |

### Pharmacological Example 6: Evaluation of in vivo drug efficacy of the present compound in rat renal ischemia reperfusion (IR) model

The effect of the present compound on renal IR damage was evaluated using male rats. The renal ischemia reperfusion model is a model used to show the effectiveness of a compound (QPI-1002 or the like) for acute renal damage associated with cardiac surgery (J Am Soc Nephrol, 2009, vol. 20, no. 8, p. 1754-1764).

### <Feeding condition>

Male Crl : CD (SD) rats aged 3 or 4 weeks were purchased from Charles River Laboratories Japan, and subjected to experiments. Animals were fed ad libitum with FR-2 (Funabashi Farm) at the time of arrival and with solid feed CE-2 (CLEA Japan) for one week before single kidney removal, and watered ad libitum.

### <Single kidney removal treatment>

After acclimation breeding, the right kidney was removed about 7 days before renal IR treatment. Specifically, the rats were allowed to inhale isoflurane (Mylan) for anesthesia, and administered buprenorphine (Otsuka Pharmaceutical) as an analgesic at 0.01 mg/kg. The right back was incised, the right kidney was removed, and then the incision was sutured.

### <Ischemia reperfusion treatment>

Renal IR treatment was performed about 7 days after single kidney removal. Specifically, rats were anesthetized by intraperitoneal administration of sodium pentobarbital (Tokyo Chemical Industry) at a dose of 50 mg/kg, and administered buprenorphine as an analgesic at 0.01 mg/kg. The left abdomen was incised, and the renal artery and vein was sandwiched with a clamp (Natsume Seisakusho) to cause ischemia for 35 minutes. During ischemia, the rats were allowed to lie on a heat-retaining plate set at 38°C, and after 35 minutes, the clamp was removed for reperfusion. After visually confirming that blood flow to the kidney was recovered, the incision was sutured. The sham group was a sham-operated group in which neither ischemia nor reperfusion treatment was performed.

### <Grouping method>

Animals were distributed on the day before renal IR treatment such that differences in the mean of the body weight in each group were not biased, and assigned animal numbers.

### <Administration of test substance>

The compound of Example 7 (test substance) and physiological saline (Otsuka Pharmaceutical) as a vehicle were subcutaneously administered at 5 mL/kg using an injection needle 5 minutes before renal IR treatment. Saline was administered to the sham group in the same test. The administration dose is described in Table 16.

The respective compounds of Examples 1, 3, 5-2, 7-13, 7-30 and 8, and physiological saline as a vehicle were subcutaneously administered at 1 µL/h for 3 days from the day before renal IR treatment using an osmotic pump (DURECT, 1003D). Specifically, the rats were allowed to inhale isoflurane for anesthesia on the day before renal IR treatment, and administered buprenorphine as an analgesic at 0.01 mg/kg. The back of the rats was incised to insert an osmotic pump filled with the test substance (each compound of Examples 1, 3, 5-2, 7-13, 7-30 and 8). The incision was bonded with Dermabond Advanced (Johnson & Johnson). A pump containing a vehicle was also embedded in the sham group in the same test. The administration dose is described in Table 16.

**[Table 16]**

| Test substance | Administration route | Administration dose | Unit |
|---|---|---|---|
| Example 1 | Subcutaneous osmotic pump continues | 0.3, 1.0 | µg/kg/min |
| Example 3 | Subcutaneous osmotic pump continues | 0.1, 0.3 | µg/kg/min |
| Example 5-2 | Subcutaneous osmotic pump continues | 1.0, 3.0 | µg/kg/min |
| Example 7 | Subcutaneous | 0.1, 1.0 | mg/kg |
| Example 7-13 | Subcutaneous osmotic pump continues | 0.3, 3.0 | µg/kg/min |
| Example 7-30 | Subcutaneous osmotic pump continues | 0.3, 3.0 | µg/kg/min |
| Example 8 | Subcutaneous osmotic pump continues | 0.3, 1.0 | µg/kg/min |

### <Measurement of biochemical parameter>

After renal IR treatment, blood was collected through the tail vein on Day1, Day2, and Day3 to obtain serum. In addition, after renal IR treatment, rats were euthanized by exsanguination through the abdominal aorta under isoflurane anesthesia on Day3. The evaluation of the compound of Example 5-2 was performed as a test until Day2 in the same manner.

The respective concentrations of serum creatinine (sCre) and blood urea nitrogen (BUN) were measured using an automatic analyzer 7180 (Hitachi High-Tech).

### <Result>

The effect of the present compound in a rat renal ischemia reperfusion (IR) model was evaluated using the above method. The measurement results of the serum creatinine (sCre) concentration are shown in Table 17-1 and Table 17-2, and the measurement results of the blood urea nitrogen (BUN) concentration are shown in Table 18-1 and Table 18-2. The present compounds described in the table suppressed an increase in serum creatinine (sCre) and blood urea nitrogen (BUN) due to provocation of a disease state. From the above, it has been confirmed that the present compound has a preventive and/or therapeutic effect on renal damage (for example, acute renal damage).

**[Table 17-1]**

| Example 1 | | day 1 | | day 2 | | day 3 | |
|---|---|---|---|---|---|---|---|
| | n | mean | S.E. | mean | S.E. | mean | S.E. |
| Sham | 4 | 0.28 | 0.01 | 0.27 | 0.01 | 0.25 | 0.00 |
| IR control | 9 | 1.95 | 0.34 ** | 1.66 | 0.41 ** | 1.24 | 0.32 * |
| IR 0.3 µg/kg/min | 9 | 1.30 | 0.27 | 0.84 | 0.17 | 0.56 | 0.09 |
| IR 1.0 µg/kg/min | 6-9 | 1.06 | 0.22 | 0.70 | 0.11 | 0.54 | 0.08 |

| Example 3 | | day 1 | | day 2 | | day 3 | |
|---|---|---|---|---|---|---|---|
| | n | mean | S.E. | mean | S.E. | mean | S.E. |
| Sham | 9 | 0.24 | 0.01 | 0.21 | 0.01 | 0.24 | 0.01 |
| IR control | | 1.53 | 0.26 ** | 1.03 | 0.25 * | 0.83 | 0.21 * |
| IR 0.1 µg/kg/min | 9 9 | 0.75 | 0.10 § | 0.55 | 0.07 | 0.52 | 0.06 |
| IR 0.3 µg/kg/min | 9 | 0.51 | 0.04 § | 0.40 | 0.02 §§ | § 0.39 | 0.02 §§ |

| Example 5-2 | | day 1 | | day 2 | | | |
|---|---|---|---|---|---|---|---|
| | n | mean | S.E. | mean | S.E. | | |
| Sham | 4 | 0.21 | 0.02 | 0.23 | 0.02 | | |
| IR control | 8 | 1.07 | 0.15 ** | 0.72 | 0.09 ** | | |
| IR 1.0 µg/kg/min | 9 | 0.91 | 0.11 | 0.62 | 0.07 | | |
| IR 3.0 µg/kg/min | 8 | 0.79 | 0.16 | 0.50 | 0.05 | | |

| | | | | | | | |
|---|---|---|---|---|---|---|---|
| Compared to the Sham group using an Aspin-Welch test: *p < 0.05, **p < 0.01, ***p < 0.001 Compared to the IR control group using a Steel test: § p < 0.05, §§ p < 0.01 | | | | | | | |

**[Table 17-2]**

| Example 7 | | day 1 | | day 2 | | day 3 | |
|---|---|---|---|---|---|---|---|
| | n | mean | S.E. | mean | S.E. | mean | S.E. |
| Sham | 4 | 0.31 | 0.03 | 0.30 | 0.02 | 0.26 | 0.01 |
| IR control | 9 | 1.75 | 0.23 *** | 1.32 | 0.25 ** | 1.07 | 0.24 ** |
| IR 0.1 mg/kg | 9 | 1.08 | 0.20 | 0.72 | 0.14 § | 0.52 | 0.11 §§ |
| IR 1.0 mg/kg | 8 | 0.99 | 0.18 | 0.62 | 0.09 § | 0.44 | 0.05 §§ |

| Example 7-13 | | day 1 | | day 2 | | day 3 | |
|---|---|---|---|---|---|---|---|
| | n | mean | S.E. | mean | S.E. | mean | S.E. |
| Sham | 4 | 0.25 | 0.00 | 0.25 | 0.00 | 0.26 | 0.01 |
| IR control | 9 | 1.43 | 0.31 ** | 1.08 | 0.27 * | 0.82 | 0.18 * |
| IR 0.3 µg/kg/min | 9 | 1.01 | 0.16 | 0.60 | 0.08 | 0.51 | 0.05 |
| IR 3.0 µg/kg/min | 8-9 | 0.87 | 0.18 | 0.60 | 0.12 | 0.52 | 0.07 |

| Example 7-30 | | day 1 | | day 2 | | day 3 | |
|---|---|---|---|---|---|---|---|
| | n | mean | S.E. | mean | S.E. | mean | S.E. |
| Sham | 9 | 0.26 | 0.03 | 0.26 | 0.00 | 0.27 | 0.01 |
| IR control | 9 | 2.22 | 0.19 *** | 1.56 | 0.22 *** | 1.08 | 0.14 *** |
| IR 0.3 µg/kg/min | 9 | 1.78 | 0.29 | 1.42 | 0.26 | 1.00 | 0.17 |
| IR 3.0 µg/kg/min | 9 | 1.45 | 0.29 | 1.10 | 0.25 | 0.83 | 0.18 |

| Example 8 | | day 1 | | day 2 | | day 3 | |
|---|---|---|---|---|---|---|---|
| | n | mean | S.E. | mean | S.E. | mean | S.E. |
| Sham | 4 | 0.24 | 0.01 | 0.25 | 0.01 | 0.27 | 0.01 |
| IR control | 8-9 | 1.21 | 0.29 ** | 1.00 | 0.39 | 0.49 | 0.08 * |
| IR 0.3 µg/kg/min | 9 | 0.61 | 0.07 | 0.41 | 0.04 | 0.38 | 0.02 |
| IR 1.0 µg/kg/min | 8 | 0.49 | 0.05 | 0.35 | 0.03 | 0.35 | 0.02 |

| | | | | | | | |
|---|---|---|---|---|---|---|---|
| Compared to the Sham group using an Aspin-Welch test: *p < 0.05, **p < 0.01, ***p < 0.001 Compared to the IR control group using a Steel test: § p < 0.05, §§ p < 0.01 | | | | | | | |

**[Table 18-1]**

| Example 1 | | day 1 | | day 2 | | day 3 | |
|---|---|---|---|---|---|---|---|
| | n | mean | S.E. | mean | S.E. | mean | S.E. |
| Sham | 4 | 23.2 | 0.5 | 23.2 | 0.7 | 24.8 | 1.0 |
| IR control | 9 | 119.2 | 16.5 *** | 128.1 | 27.6 ** | 111.3 | 26.2 * |
| IR 0.3 µg/kg/min | 9 | 88.5 | 11.3 | 78.5 | 13.4 | 63.6 | 9.8 |
| IR 1.0 µg/kg/min | 6-9 | 78.0 | 8.9 | 72.0 | 9.4 | 60.8 | 9.3 |

| Example 3 | | day 1 | | day 2 | | day 3 | |
|---|---|---|---|---|---|---|---|
| | n | mean | S.E. | mean | S.E. | mean | S.E. |
| Sham | 9 | 21.5 | 1.1 | 21.5 | 0.5 | 24.1 | 0.8 |
| IR control | 9 | 97.8 | 9.7 *** | 94.4 | 14.9 ** | 79.1 | 16.1 ** |
| IR 0.1 µg/kg/min | 9 | 63.7 | 6.9 § | 59.7 | 8.6 | 56.5 | 8.3 |
| IR 0.3 µg/kg/min | 9 | 45.9 | 3.2 §§ | 44.7 | 2.9 §§ | 41.2 | 3.2 § |

| Example 5-2 | | day 1 | | day 2 | | | |
|---|---|---|---|---|---|---|---|
| | n | mean | S.E. | mean | S.E. | | |
| Sham | 4 | 21.6 | 2.1 | 25.2 | 1.9 | | |
| IR control | 8 | 76.1 | 6.9 *** | 68.3 | 6.2 *** | | |
| IR 1.0 µg/kg/min | 9 | 75.5 | 5.9 | 65.3 | 6.9 | | |
| IR 3.0 µg/kg/min | 8 | 63.4 | 7.1 | 54.9 | 6.4 | | |

| | | | | | | | |
|---|---|---|---|---|---|---|---|
| Compared to the Sham group using an Aspin-Welch test: *p < 0.05, **p < 0.01, ***p < 0.001 Compared to the IR control group using a Dunnett test: # p < 0.05 Compared to the IR control group using a Steel test: § p < 0.05, §§ p < 0.01 | | | | | | | |

**[Table 18-2]**

| Example 7 | | day 1 | | day 2 | | day 3 | |
|---|---|---|---|---|---|---|---|
| | n | mean | S.E. | mean | S.E. | mean | S.E. |
| Sham | 4 | 23.2 | 1.9 | 21.3 | 0.8 | 19.5 | 0.9 |
| IR control | 9 | 104.3 | 10.5 *** | 100.5 | 16.1 ** | 89.0 | 18.4 ** |
| IR 0.1 mg/kg | 9 | 74.6 | 9.9 | 58.7 | 11.1 | 46.3 | 10.3 § |
| IR 1.0 mg/kg | 8 | 73.6 | 8.8 | 53.1 | 9.3 # | 39.7 | 6.1 § |

| Example 7-13 | | day 1 | | day 2 | | day 3 | |
|---|---|---|---|---|---|---|---|
| | n | mean | S.E. | mean | S.E. | mean | S.E. |
| Sham | 4 | 19.0 | 1.0 | 20.2 | 0.7 | 26.1 | 1.3 |
| IR control | 9 | 90.0 | 15.2 ** | 89.1 | 20.8 * | 79.9 | 19.1 * |
| IR 0.3 µg/kg/min | 9 | 81.9 | 9.0 | 62.1 | 9.2 | 56.4 | 6.7 |
| IR 3.0 µg/kg/min | 8-9 | 78.1 | 11.3 | 59.9 | 11.9 | 55.8 | 10.2 |

| Example 7-30 | | day 1 | | day 2 | | day 3 | |
|---|---|---|---|---|---|---|---|
| | n | mean | S.E. | mean | S.E. | mean | S.E. |
| Sham | 9 | 19.0 | 2.2 | 18.8 | 1.2 | 17.8 | 1.3 |
| IR control | 9 | 106.3 | 7.4 *** | 99.3 | 10.7 *** | 81.9 | 9.1 *** |
| IR 0.3 µg/kg/min | 9 | 96.6 | 11.2 | 94.3 | 15.3 | 79.8 | 14.7 |
| IR 3.0 µg/kg/min | 9 | 89.5 | 11.6 | 81.8 | 15.8 | 70.7 | 14.4 |

| Example 8 | | day 1 | | day 2 | | day 3 | |
|---|---|---|---|---|---|---|---|
| | n | mean | S.E. | mean | S.E. | mean | S.E. |
| Sham | 4 | 21.6 | 0.3 | 22.0 | 0.4 | 23.5 | 1.0 |
| IR control | 8-9 | 85.6 | 14.4 ** | 86.6 | 27.6 * | 48.3 | 8.2 * |
| IR 0.3 µg/kg/min | 9 | 49.2 | 5.0 | 40.1 | 3.9 | 39.0 | 3.9 |
| IR 1.0 µg/kg/min | 8 | 41.9 | 3.4 § | 37.9 | 3.2 | 37.6 | 3.8 |

| | | | | | | | |
|---|---|---|---|---|---|---|---|
| Compared to the Sham group using an Aspin-Welch test: *p < 0.05, **p < 0.01, ***p < 0.001 Compared to the IR control group using a Dunnett test: # p < 0.05 Compared to the IR control group using a Steel test: § p < 0.05, §§ p < 0.01 | | | | | | | |

### [Formulation Examples]

### Formulation Example 1

The following ingredients are mixed by a conventional method and then compressed to yield 10,000 tablets containing 10 mg of the active ingredient in one tablet.
- (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid ... 100 g
- Carboxymethyl cellulose calcium ... 20 g
- Magnesium stearate ... 10 g
- Microcrystalline cellulose ... 870 g

### Formulation Example 2

The following ingredients are mixed by a conventional method, then filtered through a dust removing filter, filled in ampoules in 5 ml portions, and heat-sterilized in an autoclave to yield 10,000 ampoules containing 20 mg of the active ingredient in one ampoule.
- (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid ... 200 g
- Mannitol ... 20 g
- Distilled water ... 50 L

### INDUSTRIAL APPLICABILITY

Since the present compound has a potent EP3 agonist activity, it is useful as a preventive and/or therapeutic agent for renal disease.

## Claims

1. A compound represented by general formula (I) or a salt thereof: 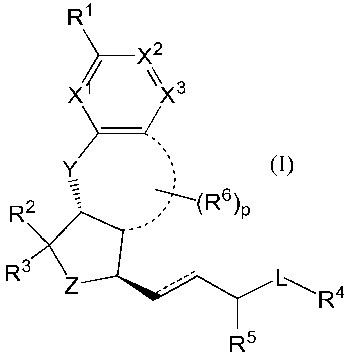 wherein
R¹ represents COOH, COOR¹¹, CONHSO₂R¹² or tetrazolyl,
R¹¹ represents C1-6 alkyl,
R¹² represents C1-6 alkyl or phenyl, which phenyl is optionally substituted with 1 to 5 (1) halogen atoms or (2) C1-4 alkyl groups,
X¹, X² and X³ each independently represent N, CH or CR²¹,
a plurality of R²¹s each independently represent (1) halogen, (2) C1-4 alkyl, (3) C1-4 haloalkyl, (4) -(CH₂)ₜ-(C3-4 saturated carbocycle), or (5) vinyl optionally substituted with 1 to 3 halogen atoms,
t represents an integer of 0 or 1, 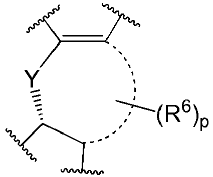
represents 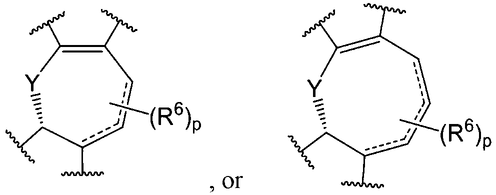
R⁶ represents (1) halogen, (2) C1-4 alkyl, (3) C1-4 haloalkyl, or (4) -(CH₂)_{q}-(C3-4 saturated carbocycle),
q represents an integer of 0 or 1,
p represents an integer of 0 to 3,
a plurality of R⁶s may form a C3-4 saturated carbocycle optionally substituted with 1 to 6 halogen atoms together with a carbon atom to which each R⁶ is bonded,
Y represents an oxygen atom or a sulfur atom,
R² and R³ each independently represent (1) a hydrogen atom, (2) halogen, (3) C1-4 alkyl, or (4) C1-4 haloalkyl,
R² and R³ may form a C3-4 saturated carbocycle optionally substituted with 1 to 6 halogen atoms together with a carbon atom to which R² and R³ are bonded,
Z represents CHR¹⁰ or an oxygen atom,
R¹⁰ represents a hydrogen atom, halogen, a hydroxyl group, or a methoxy group,
L represents -CH₂-, -CHR^{L1}-, -CR^{L2}R^{L3}- or -C(=CH₂)-,
R^{L1} represents (1) halogen, (2) C1-4 alkyl, (3) C1-4 haloalkyl, (4) C2-4 alkenyl, (5) C2-4 haloalkenyl, (6) C2-4 alkynyl or (7) C2-4 haloalkynyl,
R^{L2} and R^{L3} each independently represent (1) halogen, (2) C1-4 alkyl, (3) C1-4 haloalkyl, (4) C2-4 alkenyl, (5) C2-4 haloalkenyl, (6) C2-4 alkynyl, (7) C2-4 haloalkynyl or (8) a hydroxyl group, or
R^{L2} and R^{L3} may form (1) a C3-6 saturated carbocycle optionally substituted with 1 to 10 halogen atoms or (2) a 3-6 membered saturated heterocycle optionally substituted with 1 to 8 halogen atoms, together with a carbon atom to which R^{L2} and R^{L3} are bonded,
R⁴ represents (1) C3-7 alkyl optionally substituted with 1 to 5 R⁴¹s, (2) C3-7 alkenyl optionally substituted with 1 to 5 R⁴²s, (3) C3-7 alkynyl optionally substituted with 1 to 5 R⁴³s, (4) C2-6 alkoxy optionally substituted with 1 to 5 R⁴⁴s or (5) Cyc¹,
R⁴¹, R⁴², R⁴³ and R⁴⁴ each independently represent (1) halogen, (2) a C3-6 carbocycle or (3) a 3-6 membered heterocycle, wherein the C3-6 carbocycle and the 3-6 membered heterocycle is optionally substituted with 1 to 10 (1) halogen atoms or (2) C1-2 alkyl groups,
Cyc¹ represents (1) a C5-10 carbocycle optionally substituted with 1 to 5 R⁵¹s or (2) a 5-10 membered heterocycle optionally substituted with 1 to 5 R⁵²s,
R⁵¹ and R⁵² each independently represent (1) halogen, (2) C1-4 alkyl, (3) C1-4 haloalkyl, (4) C2-4 alkenyl, (5) C2-4 haloalkenyl, (6) C2-4 alkynyl, (7) C2-4 haloalkynyl, (8) C1-4 alkoxy, (9) C1-4 haloalkoxy, (10) a hydroxyl group, (11) a C3-6 saturated carbocycle or (12) a 3-6 membered saturated heterocycle,
R^{L1} and R⁵¹ may form (1) a C3-6 saturated carbocycle or (2) a 3-6 membered saturated heterocycle, together with a carbon atom to which R^{L1} and R⁵¹ are bonded,
R⁵ represents a hydrogen atom or a hydroxyl group,
wherein a plurality of R⁶s, R⁴¹s, R⁴²s, R⁴³s, R⁴⁴s, R⁵¹s and R⁵²s may be the same or different,
-̅ -̅ -̅ -̅ -̅ -̅ represents a single bond or a double bond, provided that adjacent
-̅ -̅ -̅ -̅ -̅ -̅ are not simultaneously a double bond, 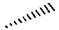
represents a bond beyond the sheet surface (that is, an α configuration), 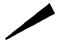
represents a bond in front of the sheet surface (that is, a β configuration), wherein 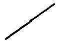
bonded to an asymmetric atom represents the α configuration, the β configuration, or a mixture of the α configuration and the β configuration in any ratio, unless otherwise indicated.

2. The compound or the salt thereof according to claim 1, wherein X¹, X² and X³ are each independently CH or CR²¹.

3. The compound or the salt thereof according to claim 1 or 2, wherein Y is an oxygen atom.

4. The compound or the salt thereof according to any one of claims 1 to 3, wherein R⁴ is C3-7 alkyl optionally substituted with 1 to 3 R⁴¹s, C3-7 alkenyl optionally substituted with 1 to 3 R⁴²s, or Cyc¹.

5. The compound or the salt thereof according to any one of claims 1 to 4, wherein Z is CHR¹⁰, and R¹⁰ is a hydroxyl group.

6. The compound or the salt thereof according to any one of claims 1 to 5, wherein R¹ is COOH.

7. The compound or the salt thereof according to claim 1, wherein general formula (I) is represented by general formula (I-3) 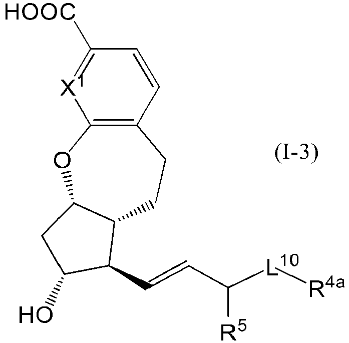 wherein L¹⁰ represents -CHR^{L1}- or -CR^{L2}R^{L3}-, R^{4a} represents C3-7 alkyl optionally substituted with 1 to 3 R⁴¹s, C3-7 alkenyl optionally substituted with 1 to 3 R⁴²s, or Cyc¹, and other symbols represent identical meanings to those in claim 1.

8. The compound or the salt thereof according to claim 1, wherein the compound represented by general formula (I) or the salt thereof is
(1) (1R,2R,3aS,10aR)-1-[(1E,3R,4S)-4-(2,4-difluorophenyl)-3-hydroxy-1-penten-1-yl]-5-fluoro-2-hydroxy-2,3,3a,9,10,10a-hexahydro-1H-benzo[b]cyclopenta[f]loxepine-6-carboxylic acid,
(2) (1R,2R,3aS,10aR)-5-fluoro-1-[(1E)-4-(2-fluorophenyl)-3-hydroxy-1-penten-1-yl]-2-hydroxy-2,3,3a,9,10,10a-hexahydro-1H-benzo[b]cyclopenta[f]oxepine-6-carboxylic acid,
(3) (1R,2R,3aS,10aR)-1-{(1E,3ξ)-3-[1-(4-fluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
(4) (1R,2R,3aS,10aR)-1-{(1E,3ξ)-3-[1-(2,5-difluorophenyl)cyclobutyl]-3-hydroxy-1-propen-1-yl}-2-hydroxy-2,3,3a,9,10,10a-hexahydro-1H-benzo[b]cyclopenta[f]oxepine-6-carboxylic acid,
(5) (1R,2R,3aS,10aR)-5-chloro-1-[(1E,3ξ)-4-(2,6-difluorophenyl)-4,4-difluoro-3-hydroxy-1-buten-1-yl]-2-hydroxy-2,3,3a,9,10,10a-hexahydro-1H-benzo[b]cyclopenta[f]oxepine-6-carboxylic acid,
(6) (1R,2R,3aS,10aR)-1-{(1E,3ξ)-3-[1-(4-fluorophenyl)cyclobutyl]-3-hydroxy-1-propen-1-yl}-2-hydroxy-5-methyl-2,3,3a,9,10,10a-hexahydro-1H-benzo[b]cyclopenta[f]oxepine-6-carboxylic acid,
(7) (1R,2R,3aS,10aR)-1-[(1E,3ξ,4ξ)-4-ethyl-3-hydroxy-7-methyl-1-octen-1-yl]-2-hydroxy-5-methyl-2,3,3a,9,10,10a-hexahydro-1H-benzo[b]cyclopenta[f]oxepine-6-carboxylic acid,
(8) (1R,2R,3aS,10aR)-2-hydroxy-5-methyl-1-[(1E,4S)-7,8,8-trifluoro-4-hydroxy-4-methyl-1,7-octadien-1-yl]-2,3,3a,9,10,10a-hexahydro-1H-benzo[b]cyclopenta[f]oxepine-6-carboxylic acid,
(9) (1R,2R,3aS,10aR)-2-hydroxy-5-methyl-1-[(1E,4S)-8,8,8-trifluoro-4-hydroxy-4-methyl-1-octen-1-yl]-2,3,3a,9,10,10a-hexahydro-1H-benzo[b]cyclopenta[f]oxepine-6-carboxylic acid,
(10) (1R,2R,3aS,10aR)-2-hydroxy-1-[(1E,4ξ)-4-hydroxy-4,7-dimethyl-1-octen-1-yl]-5-methyl-2,3,3a,9,10,10a-hexahydro-1H-benzo[b]cyclopenta[f]oxepine-6-carboxylic acid,
(11) (1R,3aS,10aR)-5-fluoro-1-[(1E,3ξ)-3-hydroxy-4-methyl-4-phenyl-1-penten-1-yl]-2,3,3a,9,10,10a-hexahydro-1H-benzo[b]cyclopenta[f]oxepine-6-carboxylic acid,
(12) (1R,3aS,10aR)-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
(13) (1R,3aS,10aR)-5-chloro-1-{(1E,3ξ)-3-[1-(2-fluorophenyl)cyclobutyl]-3-hydroxy-1-propen-1-yl}-2,3,3a,9,10,10a-hexahydro-1H-benzo[b]cyclopenta[f]oxepine-6-carboxylic acid,
(14) (1R,3aS,10aR)-5-chloro-1-[(1E,3ξ)-3-hydroxy-6-methoxy-4-methylene-1-hexen-1-yl]-2,3,3a,9,10,10a-hexahydro-1H-benzo[b]cyclopenta[f]oxepine-6-carboxylic acid,
(15) (1R,3aS,10aR)-5-chloro-1-[(1E,3ξ,4S)-4-ethyl-3-hydroxy-1-octen-1-yl]-2,3,3a,9,10,10a-hexahydro-1H-benzo[b]cyclopenta[f]oxepine-6-carboxylic acid,
(16) (1R,3aS,10aR)-5-chloro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid,
(17) (1R,2R,3aS,10aR)-2,5-difluoro-1-[(1E,3ξ)-3-hydroxy-4-methyl-4-phenyl-1-penten-yl]-2,3,3a,9,10,10a-hexahydro-1H-benzo[b]cyclopenta[f]oxepine-6-carboxylic acid,
(18) (1R,2R,3aS,10aR)-2,5-difluoro-1-[(1E,3ξ)-3-hydroxy-3-(3-phenyl-3-oxetanyl)-1-propen-1-yl]-2,3,3a,9,10,10a-hexahydro-1H-benzo[b]cyclopenta[f]oxepine-6-carboxylic acid,
(19) (1R,2R,3aS,10aR)-2-chloro-5-fluoro-1-{(1E,3ξ)-3-[3-(2-fluorophenyl)-3-oxetanyl]-3-hydroxy-1-propen-1-yl}-2,3,3a,9,10,10a-hexahydro-1H-benzo[b]cyclopenta[f]oxepine-6-carboxylic acid,
(20) (1R,2R,3aS,10aR)-2-chloro-5-fluoro-1-{(1E,3ξ)-3-hydroxy-3-[(2ξ)-2-phenyl-2-oxetanyl]-1-propen-1-yl }-2,3,3a,9,10,10a-hexahydro-1H-benzo[b]cyclopenta[f]oxepine-6-carboxylic acid,
(21) (1R,3aR,10aR)-1-[(1E,3ξ,4S)-3-hydroxy-4-methyl-1-octen-1-yl]-5-methyl-1,3,3a,9,10,10a-hexahydrofuro[3,4-b][1]benzoxepine-6-carboxylic acid,
(22) (1R,3aR,10aR)-1-[(1E,3ξ)-3-hydroxy-3-(1-phenoxycyclobutyl)-1-propen-1-yl]-5-methyl-1,3,3a,9,10,10a-hexahydrofuro[3,4-b][1]benzoxepine-6-carboxylic acid,
(23) (1R,2R,3aS,10aR)-5-fluoro-2-hydroxy-1-{(1E,3ξ)-3-hydroxy-3-[1-(2-methylphenyl)cyclopropyl]-1-propen-1-yl}-2,3,3a,9,10,10a-hexahydro-1H-benzo[b]cyclopenta[f]oxepine-6-carboxylic acid, or
(24) (1R,2R,3aS,10aR)-2-hydroxy-1-[(1E,3R)-3-hydroxy-3-(1-phenylcyclopropyl)-1-propen-1-yl]-2,3,3a,9,10,10a-hexahydro-1H-benzo[b]cyclopenta[f]oxepine-6-carboxylic acid,
or a salt thereof.

9. A pharmaceutical composition comprising the compound or the salt thereof according to any one of claims 1 to 8.

10. An EP3 agonist comprising the compound or the salt thereof according to any one of claims 1 to 8.
